# EUROPEAN PATENT APPLICATION

(11) **EP 4 786 478 A1**
(43) Date of publication of application: **05.08.2026**
(21) Application number: 24870784.6
(22) Date of filing: 25.09.2024
(51) Int. Cl.: C07H 19/06, C07H 19/067, C07H 19/167, C07H 19/24, C07H 19/16, C07H 21/00

(54) **MODIFIED NUCLEOSIDE COMPOUND, AND OLIGONUCLEOTIDE PREPARED THEREFROM**

(30) Priority: 25.09.2023 CN 202311244208
(71) Applicant: Acurna Ltd., Chengdu, Sichuan 610213 (CN); Chengdu Brilliant Pharmaceutical Co., Ltd., Chengdu, Sichuan 610094 (CN)
(72) Inventor: ZANG, Chao, Chengdu, Sichuan 610094 (CN); KUANG, Shuang, Chengdu, Sichuan 610094 (CN); YU, Shuowen, Chengdu, Sichuan 610094 (CN); HUANG, Haoxi, Chengdu, Sichuan 610094 (CN); SU, Zhonghai, Chengdu, Sichuan 610094 (CN)
(74) Representative: Calysta NV
(86) International application number: PCT/CN2024/121099
(87) International publication number: WO 2025/067240

(57) **Abstract**

A nucleoside phosphoramidite compound, which is a 2'-phosphoramidite group and 4'-modified TNA structure compound. The nucleoside phosphoramidite compound can be incorporated into the end and/or middle of an oligonucleotide, so that the modified oligonucleotide is hybridized with a portion of a target mRNA, resulting in the loss or down-regulation of the normal function of the target mRNA.

## Description

### FIELD OF THE INVENTION

The present invention relates to the field of biopharmaceuticals, specifically to a modified nucleoside compound and oligonucleotide prepared therefrom.

### BACKGROUND OF THE INVENTION

Oligonucleotides are polymers of nucleotides. As nucleic acid inhibitor molecules, oligonucleotides can regulate intracellular mRNA levels and have shown early promise in the treatment of genetic disorders, metabolic diseases, cancers, and viral infections. Nucleic acid inhibitor molecules can modulate mRNA expression through a variety of mechanisms, including RNA interference (RNAi).

RNAi is a conserved pathway found in most eukaryotes, where double-stranded RNA molecules (dsRNA) inhibit the expression of target genes having sequences complementary to the dsRNA. In a typical RNAi pathway, longer dsRNA is cleaved by the endonuclease (Dicer) into shorter RNA duplexes known as small interfering RNA ("siRNA"). It has been confirmed that siRNA associates with endonucleases, trans-activation response RNA-binding protein (TRBP), and Argonaute 2 ("Ago2") to form a complex, sometimes referred to as the RNA-induced silencing complex ("RISC"). Ago2 is an endonuclease that uses the antisense strand (also known as the guide strand) of the siRNA for sequence-specific guidance to cleave the target mRNA.

Over the years, various double-stranded RNAi inhibitor molecular structures have been developed. For example, early work on RNAi inhibitor molecules focused on double-stranded nucleic acid molecules mimicking natural siRNAs, wherein each strand comprises 19-25 nucleotides and includes at least one 3'-overhang of 1 to 5 nucleotides (see, e.g., U.S. Patent No. 8,372,968). Subsequently, longer double-stranded RNAi inhibitor molecules were developed, which are cleaved *in vivo* by endonucleases into active RNAi inhibitor molecules (see, e.g., US Patent No. 8,883,996). Subsequent work developed extended double-stranded nucleic acid inhibitor molecules, wherein at least one end of at least one strand extended beyond the double-stranded targeting region of the molecule, one of the strands comprising a structure of a thermodynamically stable tetraloop (see, e.g., US Patent No. 8,513,207, US Patent No. 8,927,705, WO2010/033225, and WO 2016/100401). Those structures included single-stranded extension structures (on one or both sides of said molecule) and double-stranded extension structures.

In recent years, many modified nucleosides have been used in RNAi, such as common 2'-fluoro modification, 2'-methoxy modification, 2'-methoxyethyl modification, 2',3'-open-ring nucleotide, locked nucleic acid, glycol nucleic acid, 5'-(E)-vinylphosphonate nucleoside modifications, etc. In some cases, chemical modifications are introduced into nucleic acid inhibitor molecules to impart properties that may be desirable under specific conditions, such as those experienced after *in vivo* administration. These modifications include those designed to, for example, counteract nucleases or other enzymes that interfere with the structure or activity of the oligonucleotide, increase cellular uptake of the oligonucleotide, or improve the pharmacokinetic properties of the oligonucleotide.

For example, introducing long-chain lipids into nucleosides (see, e.g., Patent Nos. WO2023283434, and WO2020006050), introducing modified nucleosides into the corresponding oligonucleotide sequences improves the pharmacokinetic properties of oligonucleotide drugs and enhances their bioavailability *in vivo.* Further research on structural modifications of oligonucleotide drugs is needed to obtain oligonucleotide drugs with superior properties in all aspects.

### SUMMARY OF THE INVENTION

In view of the deficiencies of the prior art, the present invention aims to develop a nucleoside-type compound that, when applied in oligonucleotide molecules (including but not limited to siRNA, antisense nucleic acids, saRNA (small activating RNA), miRNA (microRNA), nucleic acid aptamers, etc.), can effectively enhance the activity of the oligonucleotide molecule, while improving the resistance to enzymatic cleavage of the corresponding oligonucleotide against phosphatases, exonucleases, endonucleases, etc. Furthermore, compared to conventional nucleosides, these nucleoside compounds readily accommodate the introduction of functional groups with specific effects, such as various substituted alkyl lipids, without compromising their base complementary pairing ability. This enhances oligonucleotide cell membrane permeability and improves nucleic acid organ targeting. When such modified nucleosides are incorporated into siRNA molecules, they enhance siRNA accumulation in target organs and improve the pharmacokinetic properties of the drug.

The present invention provides an oligonucleotide, which is a single-stranded or double-stranded nucleotide molecule, wherein each nucleotide in the double-stranded nucleotide molecule is a modified or unmodified nucleotide, wherein the double-stranded nucleotide sequence has 16-30 nucleotides; the antisense strand of the double-stranded nucleotide molecule is reverse complementary to at least 14 consecutive nucleotides in the target mRNA; in the direction from the 5' end to the 3' end, at least one nucleotide in the sense strand of the double-stranded nucleotide molecule is replaced by the class of 2',3'-phosphodiester-linked nucleotide (4'-modified TNA).

TNA, i.e., α-L-threose nucleic acid, is a genetic polymer that differs from DNA or RNA, which is 3',5'-phosphate bonded, in that TNA is linked by 2',3'-phosphate bonding and the substituent at 3' position is oriented opposite to that of natural DNA or RNA. Although TNA has a special structure, it can still form Watson-Crick double-stranded structures with its complementary bases. Due to its unique structural characteristics, TNA has good resistance to nuclease cleavage and is used more frequently in nucleic acid aptamers.

The present invention provides a nucleoside compound shown in Formula (1), or a stereoisomer thereof: 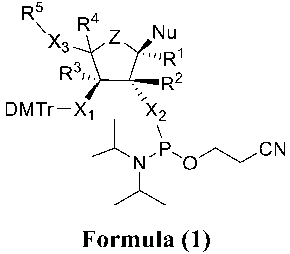 wherein:
R¹, R², R³, and R⁴ are each independently selected from the group consisting of hydrogen, halogen, cycloalkyl, C1-C6 alkyl, substituted C1-C6 alkyl, C1-C6 alkoxy, substituted C1-C6 alkoxy, C2-C6 alkenyl, substituted C2-C6 alkenyl, C2-C6 alkynyl, and substituted C2-C6 alkynyl;
R⁵ is selected from the group consisting of hydrogen, halogen, cyclopropyl, cyclobutyl, C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, and substituted C2-C30 alkynyl;
Nu represents a base or is absent;
Z is -O-, -S-, -NR¹⁰, or -CR¹⁰R¹¹, wherein R¹⁰ and R¹¹ are each independently selected from the group consisting of hydrogen, halogen, acyl, substituted or unsubstituted alkyl, substituted or unsubstituted aryl, substituted or unsubstituted heteroaryl, substituted or unsubstituted heterocyclyl, and substituted or unsubstituted cycloalkyl;
X₁ and X₂ are each independently selected from the group consisting of -(CH₂)ₙO- and -(CH₂)ₙS-, wherein n is any integer from 0 to 10;
X₃ is selected from the group consisting of -(CH₂)ₙO-, -(CH₂)ₙ-, -(CH₂)ₙS-, -(CH₂)ₙNH(CO)-, -(CH₂)ₙ(CO)NH-, and -(CH₂)ₙO(CH₂)ₙ-A-, or, X₃ is selected from the group consisting of -(CH₂)ₙO-, -(CH₂)ₙS-, -(CH₂)ₙNH(CO)-, -(CH₂)ₙ(CO)NH-, and -(CH₂)ₙO(CH₂)ₙ-A-, wherein A is substituted or unsubstituted aryl, substituted or unsubstituted heteroaryl, substituted or unsubstituted heterocyclyl, or substituted or unsubstituted cycloalkyl, wherein n is any integer from 0 to 10.

The nucleoside compound of Formula (1) provided by the present invention is selected from the group consisting of the nucleoside compounds shown in Formula 1-a and 1-b (nucleoside phosphoramidite compounds for modification of oligonucleotides, i.e., 4'-modified TNA backbone structures). 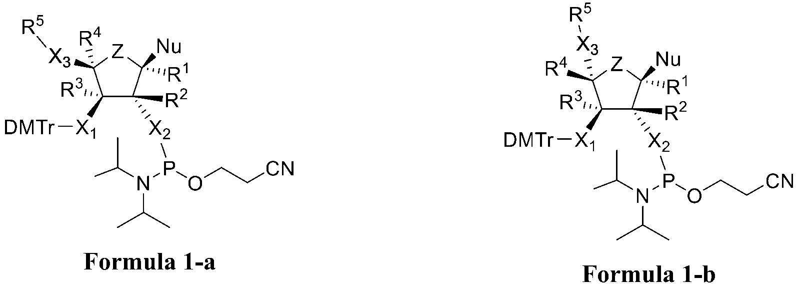 wherein: R¹, R², R³, R⁴, R⁵, Nu, X₁, X₂, and X₃ are as defined above.

The nucleoside compound shown in Formula 1-a or Formula 1-b according to the present invention, wherein,
R¹, R², R³, and R⁴ are each independently selected from the group consisting of hydrogen, halogen, cycloalkyl, C1-C6 alkyl, substituted C1-C6 alkyl, C1-C6 alkoxy, substituted C1-C6 alkoxy, C2-C6 alkenyl, substituted C2-C6 alkenyl, C2-C6 alkynyl, and substituted C2-C6 alkynyl;
R⁵ is selected from the group consisting of hydrogen, cyclopropyl, cyclobutyl, C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, and substituted C2-C30 alkynyl;
Nu represents a base or is absent;
Z is -O-, -S-, or -N(COR')-, wherein R' is C1-C5 alkyl; preferably, R' is selected from the group consisting of methyl, ethyl, n-propyl, isopropyl, n-butyl, isobutyl, and tert-butyl;
X₁ and X₂ are each independently selected from the group consisting of -(CH₂)ₙO- and -(CH₂)ₙS-, wherein n is any integer from 0 to 5; preferably, n is selected from the group consisting of 0, 1, 2, 3, 4, and 5;
X₃ is selected from the group consisting of -(CH₂)ₙO-, -(CH₂)ₙS-, -(CH₂)ₙNH(CO)-, -(CH₂)ₙ(CO)NH-, -(CH₂)ₙ-, or -(CH₂)ₙO(CH₂)ₙ-A-, wherein A is substituted or unsubstituted aryl, substituted or unsubstituted heteroaryl, substituted or unsubstituted heterocyclyl, or substituted or unsubstituted cycloalkyl, wherein n is any integer from 0 to 5; preferably, n is selected from the group consisting of 0, 1, 2, 3, 4, and 5.

The nucleoside compound is shown in Formula 1-a or Formula 1-b according to the present invention, wherein Z is -O-, -S-, or -N(COCH₃)-.

The nucleoside compound is shown in Formula 1-a or Formula 1-b according to the present invention, wherein X₁ is -O-, -S-, or -CH₂O-.

The nucleoside compound is shown in Formula 1-a or Formula 1-b according to the present invention, wherein X₂ is -O- or -S-.

The nucleoside compound is shown in Formula 1-a or Formula 1-b according to the present invention, wherein X₃ is -O-, -CH₂-, -CH₂O-, -CH₂S-, -CH₂NH(CO)-, -(CO)NH-, or -CH₂OCH₂-heteroaryl-; preferably, X₃ is -O-, -CH₂O-, -CH₂S-, -CH₂NH(CO)-, -(CO)NH-, or -CH₂OCH₂-heteroaryl-, for example, X₃ is -CH₂OCH₂-1,2,3-triazolyl-.

The nucleoside compound is shown in Formula 1-a or Formula 1-b according to the present invention, wherein R¹ is hydrogen.

The nucleoside compound is shown in Formula 1-a or Formula 1-b according to the present invention, wherein R² is hydrogen.

The nucleoside compound is shown in Formula 1-a or Formula 1-b according to the present invention, wherein R³ is hydrogen.

The nucleoside compound is shown in Formula 1-a or Formula 1-b according to the present invention, wherein R⁴ is hydrogen or methoxy.

The nucleoside compound is shown in Formula 1-a or Formula 1-b according to the present invention, wherein R⁵ is hydrogen, methyl, halogen, or C10-C18 alkyl; preferably, R⁵ is methyl or C10-C18 alkyl; more preferably, R⁵ is methyl, C10 alkyl, C11 alkyl, C12 alkyl, C13 alkyl, C14 alkyl, C15 alkyl, C16 alkyl, C17 alkyl, or C18 alkyl; even further preferably, R⁵ is methyl, C10 straight-chain alkyl, C11 straight-chain alkyl, C12 straight-chain alkyl, C13 straight-chain alkyl, C14 straight-chain alkyl, C15 straight-chain alkyl, C16 straight-chain alkyl, C17 straight-chain alkyl, or C18 straight-chain alkyl.

The nucleoside compound is shown in Formula 1-a or Formula 1-b according to the present invention, wherein R¹ is H, and Nu is absent.

The nucleoside compound is shown in Formula 1-a or Formula 1-b according to the present invention, wherein Nu is a base selected from the group consisting of:

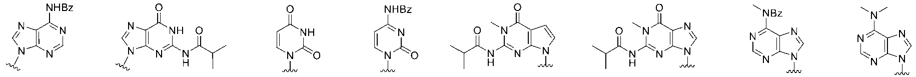

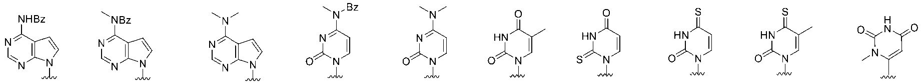

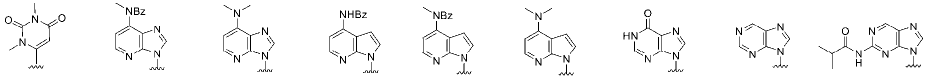

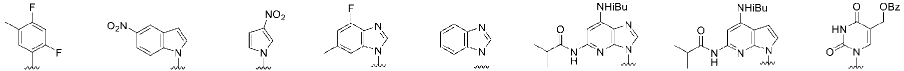

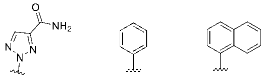

The nucleoside compound is shown in Formula 1-a or Formula 1-b according to the present invention, wherein the nucleoside compound shown in Formula 1-a or 1-b of the present invention is a nucleoside compound shown in Formula 2-a or 2-b, respectively, 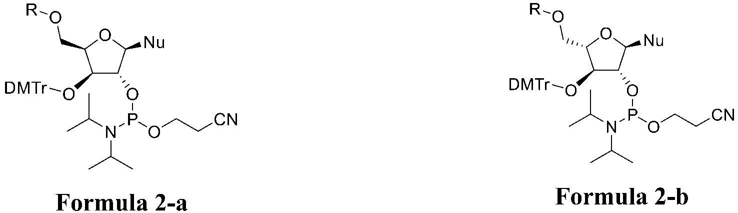
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined above.

The nucleoside compound shown in Formula 1-a according to the present invention, wherein the nucleoside compound shown in Formula 1-a of the present invention is a nucleoside compound shown in Formula 3-a, Formula 5-a, Formula 8-a, or Formula 9-a, respectively, 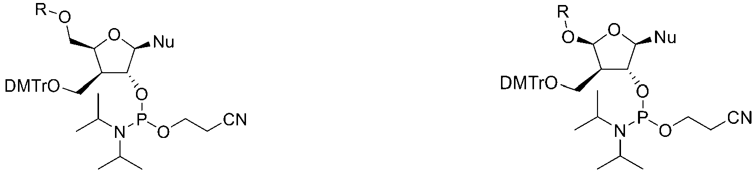 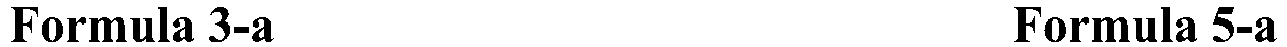 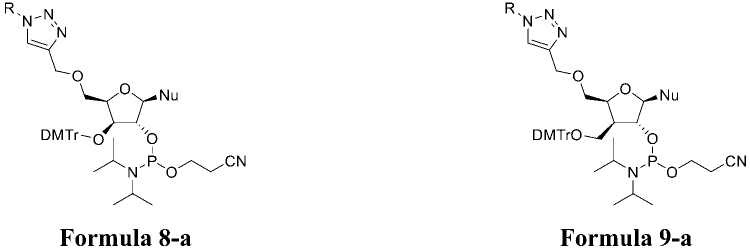
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined above.

The nucleoside compound is shown in Formula 1-a or Formula 1-b according to the present invention, wherein the nucleoside compound shown in Formula 1-a or Formula 1-b of the present invention is a nucleoside compound shown in Formula 4-a or Formula 3-b, respectively, 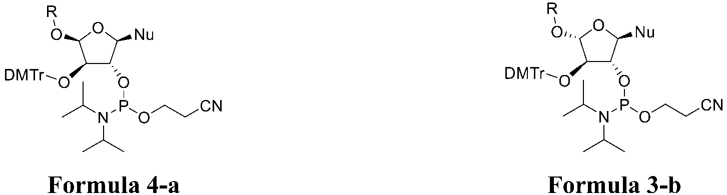
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined above.

The nucleoside compound is shown in Formula 1-a or Formula 1-b according to the present invention, wherein the nucleoside compound shown in formula 1-a or formula 1-b of the present invention is a nucleoside compound shown in formula 6-a or Formula 4-b, respectively, 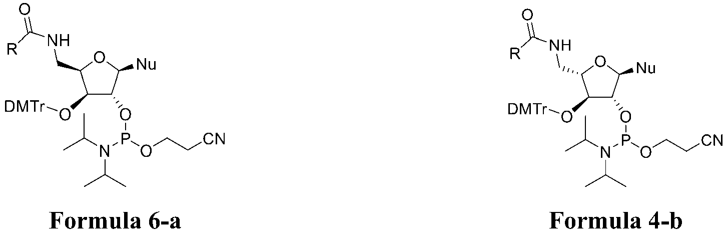
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined above.

The nucleoside compound is shown in Formula 1-a or Formula 1-b according to the present invention, wherein the nucleoside compound shown in Formula 1-a or Formula 1-b of the present invention is a nucleoside compound shown in Formula 7-a or Formula 5-b, respectively, 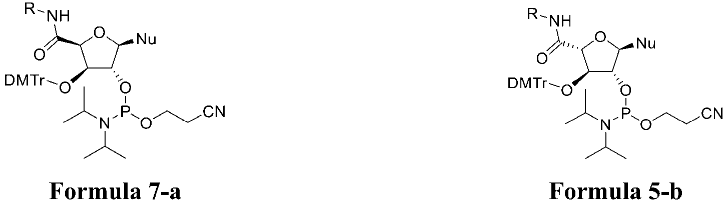
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined above.

The nucleoside compound is shown in Formula 1-a or Formula 1-b according to the present invention, wherein the nucleoside compound shown in Formula 1-a or Formula 1-b of the present invention is a nucleoside compound shown in Formula 10-a or Formula 6-b, respectively, 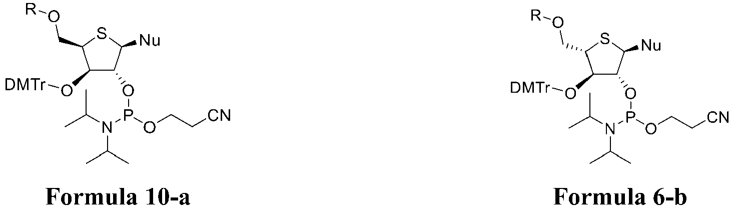
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined above.

The nucleoside compound is shown in Formula 1-a or Formula 1-b according to the present invention, wherein the nucleoside compound shown in Formula 1-a or Formula 1-b of the present invention is a nucleoside compound shown in Formula 11-a or Formula 7-b, respectively, 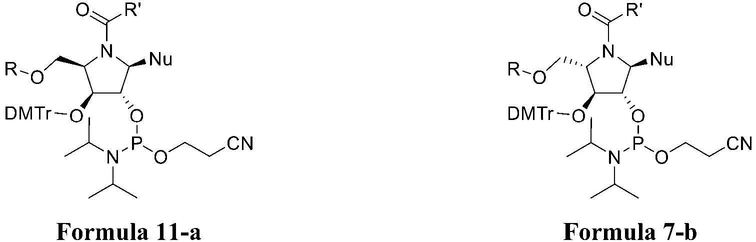
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined above;
R' is a C1-C5 alkyl; preferably, R' is methyl, ethyl, n-propyl, or isopropyl.

The nucleoside compound is shown in Formula 1-a or Formula 1-b according to the present invention, wherein the nucleoside compound shown in Formula 1-a or Formula 1-b of the present invention is a nucleoside compound shown in Formula 12-a or Formula 8-b, respectively, 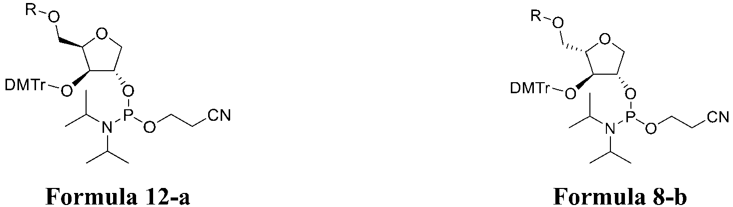 R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl.

The nucleoside compound is shown in Formula 1-a or Formula 1-b according to the present invention, wherein the nucleoside compound shown in Formula 1-a or Formula 1-b of the present invention is a nucleoside compound shown in Formula 13-a or Formula 9-a, respectively, 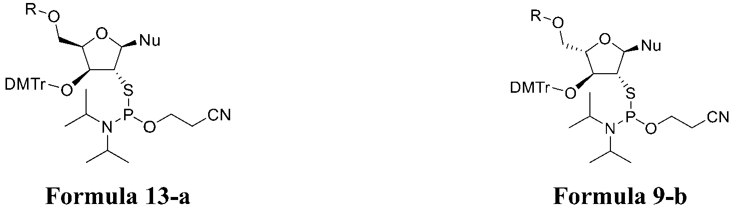
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined above.

The nucleoside compound is shown in Formula 1-a or Formula 1-b according to the present invention, wherein the nucleoside compound shown in Formula 1-a or Formula 1-b of the present invention is a nucleoside compound shown in Formula 14-a or Formula 10-b, respectively, 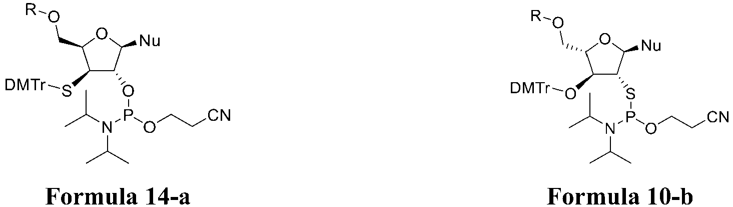
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined above.

The nucleoside compound is shown in Formula 1-a or Formula 1-b according to the present invention, wherein the nucleoside compound shown in Formula 1-a or Formula 1-b of the present invention is a nucleoside compound shown in Formula 15-a or Formula 11-b, respectively, 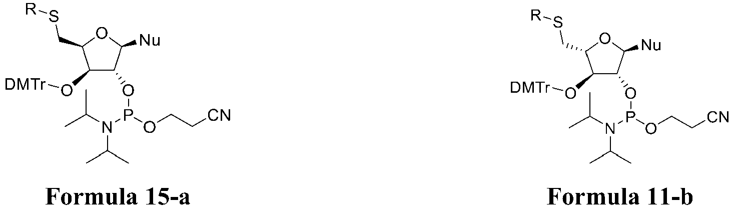
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined above.

The nucleoside compound is shown in Formula 1-a or Formula 1-b according to the present invention, wherein the nucleoside compound shown in Formula 1-a or Formula 1-b of the present invention is a nucleoside compound shown in Formula 16-a or Formula 12-b, respectively, 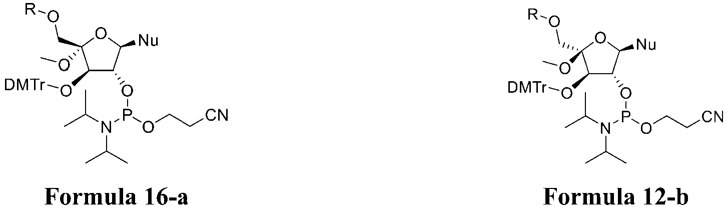
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined above.

The nucleoside compound is shown in Formula 1-a according to the present invention, wherein the nucleoside compound shown in Formula 1-a of the present invention is a nucleoside compound as shown in Table A in the detailed description.

The nucleoside compound is shown in Formula 1-a according to the present invention, wherein the nucleoside compound shown in Formula 1-a of the present invention is a nucleoside compound shown as follows:

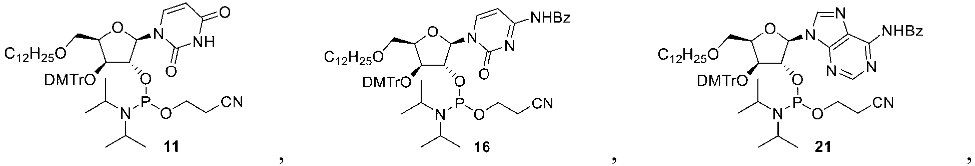

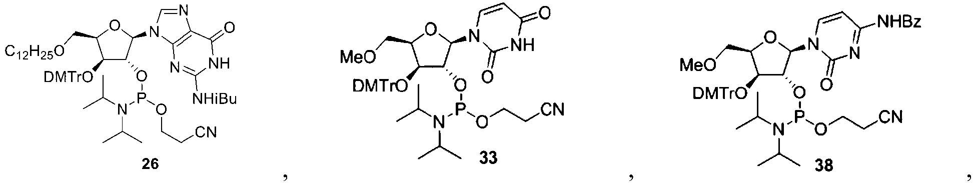

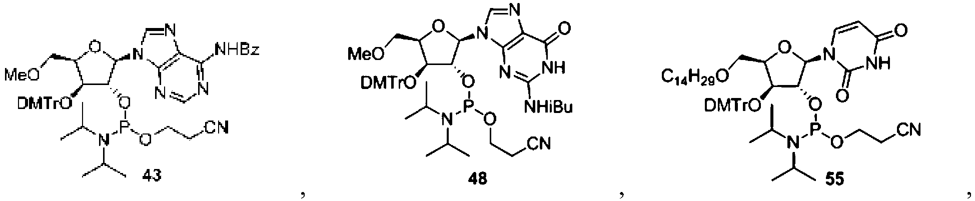

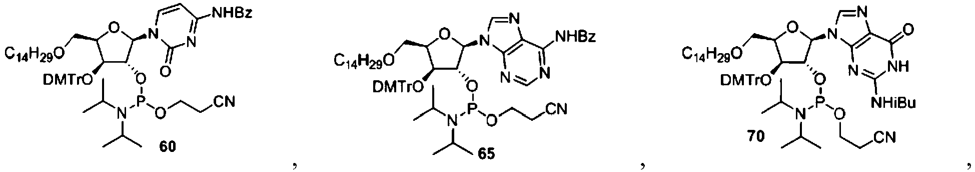

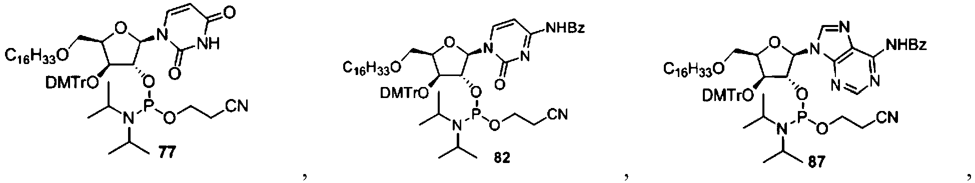

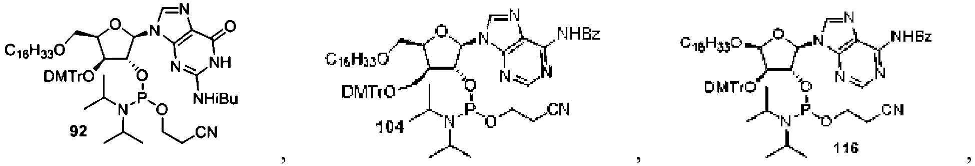

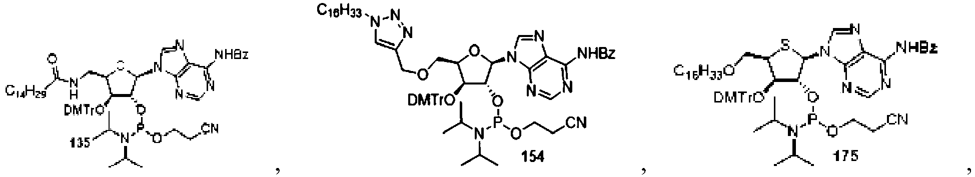

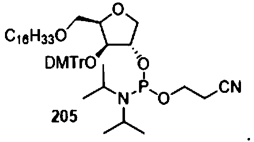

The nucleoside compound is shown in Formula 1-b according to the present invention, wherein the nucleoside compound shown in Formula 1-b of the present invention is a nucleoside compound shown in Table B in the detailed description.

The nucleoside compound is shown in Formula 1-b according to the present invention, wherein the nucleoside compound shown in Formula 1-b of the present invention is a nucleoside compound shown as follows:

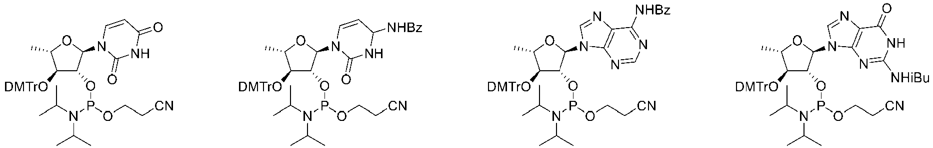

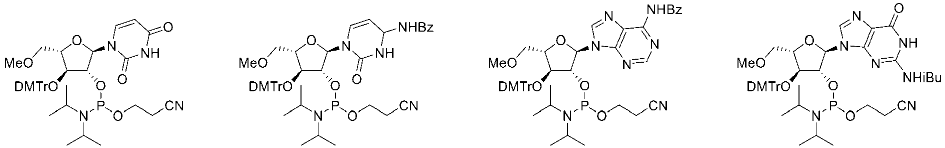

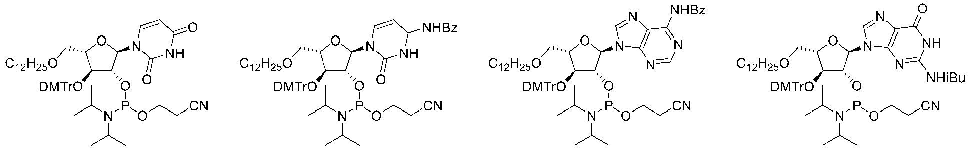

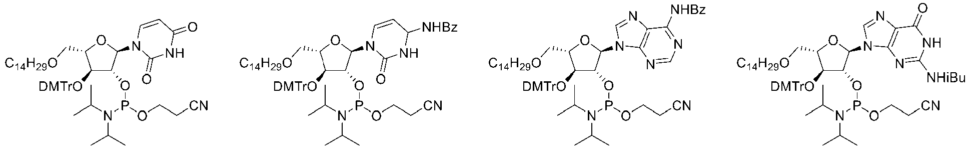

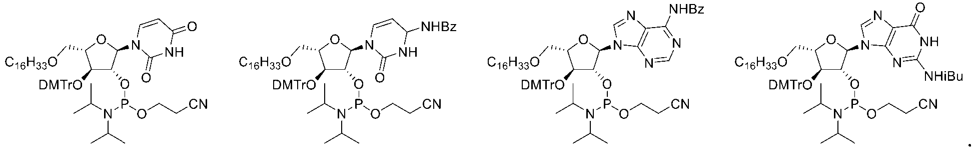

Another object of the present invention is to provide an oligonucleotide, wherein the oligonucleotide is prepared from at least one nucleoside compound shown in Formula 1-a or Formula 1-b as an intermediate; preferably, the oligonucleotide is siRNA, antisense nucleic acid, saRNA, miRNA, or nucleic acid aptamer.

The oligonucleotide according to the present invention, wherein the nucleoside compound in the oligonucleotide has a structure of the following Formula 17-a or Formula 18-a and is linked to the remainder of the oligonucleotide via 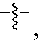 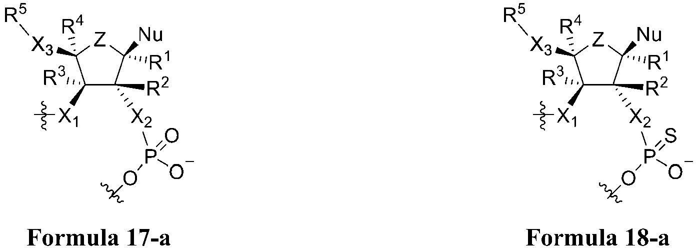
wherein R¹, R², R³, R⁴, R⁵, Z, X₁, X₂, X₃ and Nu are as defined above;
preferably,
the oligonucleotide is siRNA;
the nucleoside compound in the sense strand of siRNA has a structure shown in Formula 17-a or Formula 18-a; more preferably, the structure of Formula 17-a or Formula 18-a is the first, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, or twenty-first nucleotide at the 5' end of the sense strand of the siRNA; further preferably, the structure of Formula 17-a or Formula 18-a is the first, second, third, fourth, fifth, sixth, seventh, eighth, ninth, twelfth, thirteenth, fourteenth, or fifteenth nucleotide at the 5' end of the sense strand of the siRNA; even further preferably, the structure of Formula 17-a or Formula 18-a is the first nucleotide at the 5' end of the sense strand of the siRNA;
or, the nucleoside compound in the antisense strand of siRNA has a structure shown in Formula 17-a or Formula 18-a; more preferably, the structure of Formula 17-a or Formula 18-a is the first, second, third, fourth, fifth, or sixth nucleotide at the 3' end of the antisense strand of the siRNA; further preferably, the structure of Formula 17-a or Formula 18-a is the first or second nucleotide at the 3' end of the antisense strand of the siRNA.

The oligonucleotide according to the present invention, wherein the nucleoside compound in the oligonucleotide has a structure of the following Formula 13-b or Formula 14-b and is linked to the remainder of the oligonucleotide via 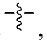 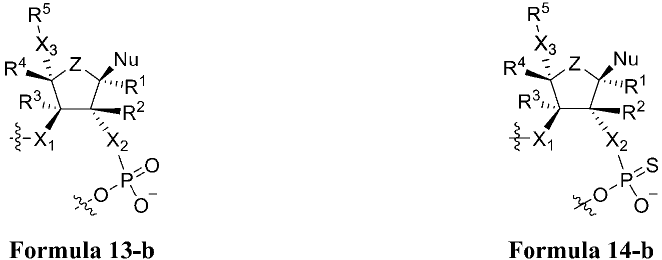
wherein R¹, R², R³, R⁴, R⁵, Z, X₁, X₂, X₃ and Nu are as defined in claim 1;
preferably,
the oligonucleotide is siRNA;
the nucleoside compound in the sense strand of siRNA has a structure shown in Formula 13-b or Formula 14-b; more preferably, the structure of Formula 13-b or Formula 14-b is the first, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, or twenty-first nucleotide at the 5' end of the sense strand of the siRNA; further preferably, the structure of Formula 13-b or Formula 14-b is the first, second, third, fourth, fifth, sixth, seventh, eighth, ninth, twelfth, thirteenth, fourteenth, or fifteenth nucleotide at the 5' end of the sense strand of the siRNA; even further preferably, the structure of Formula 13-b or Formula 14-b is the first nucleotide at the 5' end of the sense strand of the siRNA;
or, the nucleoside compound in the antisense strand of siRNA has a structure shown in Formula 13-b or Formula 14-b; More preferably, the structure of Formula 13-b or Formula 14-b is the first, second, third, fourth, fifth, or sixth nucleotide at the 3' end of the antisense strand of the siRNA; Further preferably, the structure of Formula 13-b or Formula 14-b is the first or second nucleotide at the 3' end of the antisense strand of the siRNA.

A further object of the present invention is to provide use of the nucleoside compound shown in Formula 1-a or 1-b for the preparation of oligonucleotide.

The use according to the present invention, wherein the nucleoside compound in the oligonucleotide has a structure of the following Formula 17-a or Formula 18-a and is linked to the remainder of the oligonucleotide via 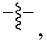 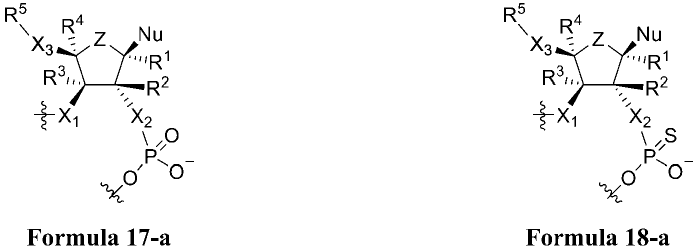
wherein R¹, R², R³, R⁴, R⁵, Z, X₁, X₂, X₃ and Nu are as defined above;
preferably,
the oligonucleotide is siRNA;
the nucleoside compound in the sense strand of siRNA has a structure shown in Formula 17-a or Formula 18-a; more preferably, the structure of Formula 17-a or Formula 18-a is the first, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, or twenty-first nucleotide at the 5' end of the sense strand of the siRNA; further preferably, the structure of Formula 17-a or Formula 18-a is the first, second, third, fourth, fifth, sixth, seventh, eighth, ninth, twelfth, thirteenth, fourteenth, or fifteenth nucleotide at the 5' end of the sense strand of the siRNA; even further preferably, the structure of Formula 17-a or Formula 18-a is the first nucleotide at the 5' end of the sense strand of the siRNA;
or, the nucleoside compound in the antisense strand of siRNA has a structure shown in Formula 17-a or Formula 18-a; more preferably, the structure of Formula 17-a or Formula 18-ais the first, second, third, fourth, fifth, or sixth nucleotide at the 3' end of the antisense strand of the siRNA; further preferably, the structure of Formula 17-a or Formula 18-a is the first or second nucleotide at the 3' end of the antisense strand of the siRNA.

The use according to the present invention, wherein the nucleoside compound in the oligonucleotide has a structure of the following Formula 13-b or Formula 14-b and is linked to the remainder of the oligonucleotide via 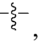 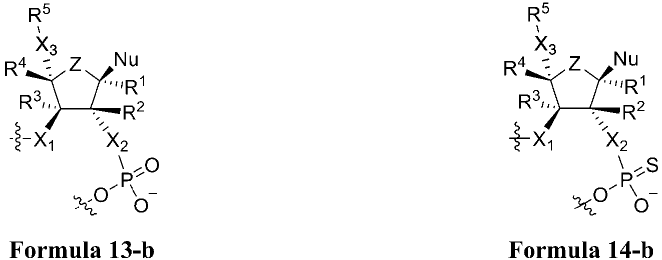
wherein R¹, R², R³, R⁴, R⁵, Z, X₁, X₂, X₃ and Nu are as defined in claim 1;
preferably,
the oligonucleotide is siRNA;
the nucleoside compound in the sense strand of siRNA has a structure shown in Formula 13-b or Formula 14-b; more preferably, the structure of Formula 13-b or Formula 14-b is the first, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, or twenty-first nucleotide at the 5' end of the sense strand of the siRNA; further preferably, the structure of Formula 13-b or Formula 14-b is the first, second, third, fourth, fifth, sixth, seventh, eighth, ninth, twelfth, thirteenth, fourteenth, or fifteenth nucleotide at the 5' end of the sense strand of the siRNA; even further preferably, the structure of Formula 13-b or Formula 14-b is the first nucleotide at the 5' end of the sense strand of the siRNA;
or, the nucleoside compound in the antisense strand of siRNA has a structure shown in Formula 13-b or Formula 14-b; More preferably, the structure of Formula 13-b or Formula 14-b is the first, second, third, fourth, fifth, or sixth nucleotide at the 3' end of the antisense strand of the siRNA; further preferably, the structure of Formula 13-b or Formula 14-b is the first or second nucleotide at the 3' end of the antisense strand of the siRNA.

In the present invention, when the structure of Formula 17-a, Formula 18-a, Formula 13-b, or Formula 14-b is the first nucleotide at the 5' end of the oligonucleotide, X₁ is attached to hydrogen via 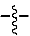 .

In the present invention, when the structure of Formula 17-a, Formula 18-a, Formula 13-b, or Formula 14-b is the first nucleotide at the 3' end of the oligonucleotide, X₂ is not attached to a phosphate group, and is attached to a hydrogen. Alternatively, when the structure of Formula 17-a, Formula 18-a, Formula 13-b, or Formula 14-b is the first nucleotide at the 3' end of the oligonucleotide, X₂ is linked to a targeting ligand via the phosphate group

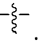

An object of the present invention is to provide a nucleoside compound of Formula 1-a (nucleoside phosphoramidite compounds for modification of oligonucleotides, i.e., 4'-modified TNA backbone structures), 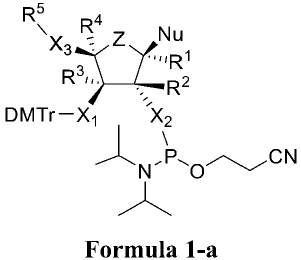 wherein:
R¹, R², R³, and R⁴ are each independently selected from the group consisting of hydrogen, halogen, cycloalkyl, C1-C6 alkyl, substituted C1-C6 alkyl, C1-C6 alkoxy, substituted C1-C6 alkoxy, C2-C6 alkenyl, substituted C2-C6 alkenyl, C2-C6 alkynyl, and substituted C2-C6 alkynyl;
R⁵ is selected from the group consisting of hydrogen, cyclopropyl, cyclobutyl, C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, and substituted C2-C30 alkynyl;
Nu represents a base or is absent;
Z is -O-, -S-, -NR¹⁰, or -CR¹⁰R¹¹, wherein R¹⁰ and R¹¹ are each independently hydrogen, halogen, acyl (acyl is -COR', wherein R' is C1-C5 alkyl), substituted or unsubstituted alkyl, substituted or unsubstituted aryl, substituted or unsubstituted heteroaryl, substituted or unsubstituted heterocyclyl, or substituted or unsubstituted cycloalkyl;
X₁ and X₂ are each independently selected from the group consisting of -(CH₂)ₙO- and -(CH₂)ₙS-, wherein n is any integer from 0 to 10;
X₃ is selected from the group consisting of -(CH₂)ₙO-, -(CH₂)ₙS-, -(CH₂)ₙNH(CO)-, and -(CH₂)ₙO(CH₂)ₙ-A-, wherein A is substituted or unsubstituted aryl, substituted or unsubstituted heteroaryl, substituted or unsubstituted heterocyclyl, or substituted or unsubstituted cycloalkyl, wherein n is any integer from 0 to 10.

The nucleoside compound is shown in Formula 1-a according to the present invention, wherein,
R¹, R², R³, and R⁴ are each independently selected from the group consisting of hydrogen, halogen, cycloalkyl, C1-C6 alkyl, substituted C1-C6 alkyl, C1-C6 alkoxy, substituted C1-C6 alkoxy, C2-C6 alkenyl, substituted C2-C6 alkenyl, C2-C6 alkynyl, and substituted C2-C6 alkynyl;
R⁵ is selected from the group consisting of hydrogen, cyclopropyl, cyclobutyl, C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, and substituted C2-C30 alkynyl;
Nu represents a base or is absent;
Z is -O-, -S-, or -N(COR')-, wherein R' is C1-C5 alkyl;
X₁ and X₂ are each independently selected from the group consisting of -(CH₂)ₙO- and -(CH₂)ₙS-, wherein n is any integer from 0 to 5;
X₃ is selected from the group consisting of -(CH₂)ₙO-, -(CH₂)ₙS-, -(CH₂)ₙNH(CO)-, and -(CH₂)ₙO(CH₂)ₙ-A-, wherein A is substituted or unsubstituted aryl, substituted or unsubstituted heteroaryl, substituted or unsubstituted heterocyclyl, or substituted or unsubstituted cycloalkyl, wherein n is any integer from 0 to 5.

The nucleoside compound is shown in Formula 1-a according to the present invention, wherein Z is -O-, -S-, or -N(COCH₃)-.

The nucleoside compound is shown in Formula 1-a according to the present invention, wherein X₁ is -O-, -S-, or -CH₂O-.

The nucleoside compound is shown in Formula 1-a according to the present invention, wherein X₂ is -O- or -S-.

The nucleoside compound is shown in Formula 1-a according to the present invention, wherein X₃ is -O-, -CH₂O-, -CH₂S-, -CH₂NH(CO)-, -(CO)NH-, or -CH₂OCH₂-heteroaryl, for example, X₃ is -CH₂OCH₂-triazolyl-.

The nucleoside compound is shown in Formula 1-a according to the present invention, wherein R¹ is H.

The nucleoside compound is shown in Formula 1-a according to the present invention, wherein R² is H.

The nucleoside compound is shown in Formula 1-a according to the present invention, wherein R³ is H.

The nucleoside compound is shown in Formula 1-a according to the present invention, wherein R⁴ is H or methoxy.

The nucleoside compound is shown in Formula 1-a according to the present invention, wherein R⁵ is methyl or C10-C18 alkyl.

The nucleoside compound is shown in Formula 1-a according to the present invention, wherein R¹ is H, and Nu is absent.

The nucleoside compound is shown in Formula 1-a according to the present invention, wherein Nu is a base selected from the group consisting of:

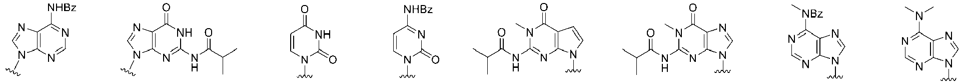

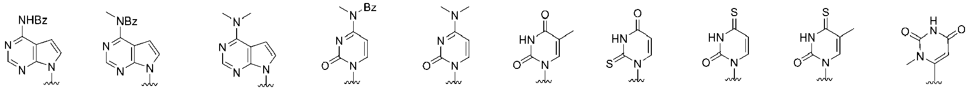

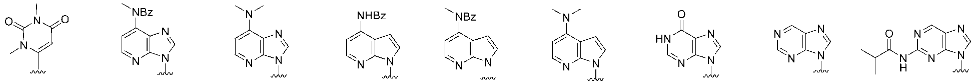

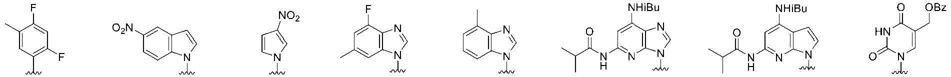

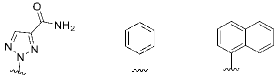

The nucleoside compound is shown in Formula 1-a according to the present invention, wherein the nucleoside compound shown in Formula 1-a of the present invention is a nucleoside compound shown in Formula 2-a: 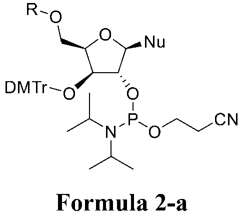
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl;
Nu is as defined above.

The nucleoside compound is shown in Formula 1-a according to the present invention, wherein the nucleoside compound shown in Formula 1-a of the present invention is a nucleoside compound shown in Formula 3-a: 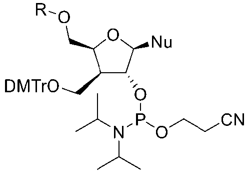 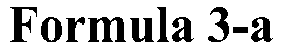
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl;
Nu is as defined above.

The nucleoside compound is shown in Formula 1-a according to the present invention, wherein the nucleoside compound shown in Formula 1-a of the present invention is a nucleoside compound shown in Formula 4-a: 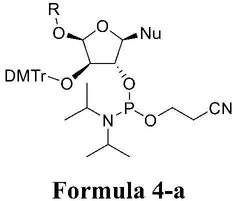
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl;
Nu is as defined above.

The nucleoside compound is shown in Formula 1-a according to the present invention, wherein the nucleoside compound shown in Formula 1-a of the present invention is a nucleoside compound shown in Formula 7-a: 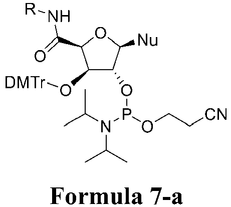
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl;
Nu is as defined above.

The nucleoside compound is shown in Formula 1-a according to the present invention, wherein the nucleoside compound shown in Formula 1-a of the present invention is a nucleoside compound shown in Formula 8-a: 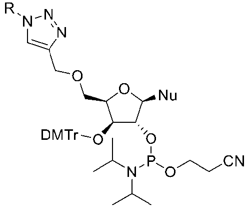 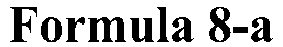
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl;
Nu is as defined above.

The nucleoside compound is shown in Formula 1-a according to the present invention, wherein the nucleoside compound shown in Formula 1-a of the present invention is a nucleoside compound shown in Formula 10-a: 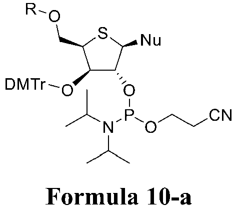
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl;
Nu is as defined above.

The nucleoside compound is shown in Formula 1-a according to the present invention, wherein the nucleoside compound shown in Formula 1-a of the present invention is a nucleoside compound shown in Formula 12-a: 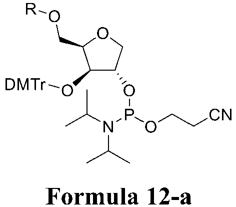 R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl.

Another object of the present invention is to provide an oligonucleotide, wherein the oligonucleotide comprises at least one nucleoside compound shown in Formula 1-a.

The oligonucleotide according to the present invention, wherein the nucleoside compound in the oligonucleotide has a structure of Formula 17-a or Formula 18-a as follows, 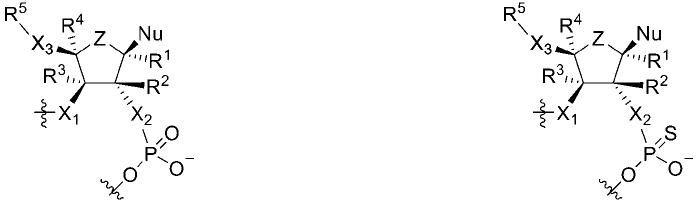 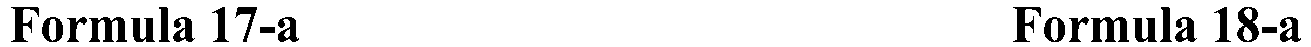 wherein R¹, R², R³, R⁴, R⁵, Z, X₁, X₂, X₃ and Nu are as defined above.

A further object of the present invention is to provide use of the nucleoside compound shown in Formula 1-a for the preparation of oligonucleotide.

The use according to the present invention, wherein the nucleoside compound in the oligonucleotide has a structure of Formula 17-a or Formula 18-a as follows, 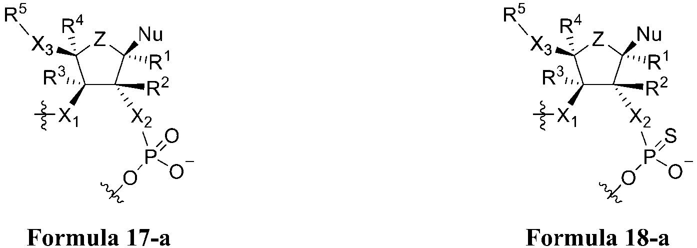 wherein R¹, R², R³, R⁴, R⁵, Z, X₁, X₂, X₃ and Nu are as defined above.

In the present invention, the oligonucleotide may be siRNA, antisense nucleic acid, saRNA, miRNA, or nucleic acid aptamer.

In the present invention, the siRNA has a double-stranded structure comprising a sense strand and an antisense strand; wherein the antisense strand is 17 to 30 nucleotides in length; the sense strand is 17 to 30 nucleotides in length and is at least partially complementary to the antisense strand. Preferably, the sense strand has at least 15, 16, 17, 18, 19, 20, or 21 nucleotides complementary to the antisense strand.

In some embodiments of the present invention, the antisense strand is 21 to 23 nucleotides in length; the sense strand is 19 to 21 nucleotides in length.

In some embodiments of the present invention, the siRNA comprises one or more single-stranded nucleotide overhangs. For example, overhangs of 1, 2, 3 or 4 nucleotides. In some embodiments of the present invention, the overhang may be on the sense chain, the antisense chain, or any combination thereof. In some embodiments of the present invention, the overhang is present on the 5' end, the 3' end, or both ends of the antisense or sense strand of the siRNA. Preferably, the 3' end of the antisense strand of the siRNA has an overhang of 2 nucleotides.

In some embodiments of the present invention, the antisense strand is 23 nucleotides in length, and the sense strand is 21 nucleotides in length; or the antisense strand is 22 nucleotides in length, and the sense strand is 20 nucleotides in length; or the antisense strand is 21 nucleotides in length, and the sense strand is 21 nucleotides in length; or the antisense strand is 21 nucleotides in length, and the sense strand is 19 nucleotides in length; or the antisense strand is 19 nucleotides in length, and the sense strand is 19 nucleotides in length.

In some embodiments of the present invention, the siRNA comprises at least one modified nucleotide.

In some embodiments of the present invention, all of the nucleotides in the sense strand and/or antisense strand of the siRNA are modified nucleotides or nucleotide analogs.

In some embodiments of the present invention, the modified nucleotide is selected from the group consisting of 2'-methoxy nucleotide, 2'-fluoro nucleotide, 2'-deoxy nucleotide, 2',3'-ring-opened nucleotide analogue, 2'-fluoro-arabinonucleotide, 2'-methoxyethyl nucleotide, 2'-amino-modified nucleotide, 2'-alkyl-modified nucleotide, 3'-methoxy nucleotide, 2'-allyl-modified nucleotide, phosphorothioate-containing nucleotide, methylphosphonate-containing nucleotide, 5'-phosphate group-containing nucleotide, 5'-phosphate mimic-containing nucleotide, glycol-modified nucleotide, abasic nucleotide, morpholino nucleotide, locked nucleotide (LNA), unlocked nucleotide (UNA), glycerol nucleotide (GNA), and nucleotide having the structure shown in Formula (1) of the present invention, but the present invention is not limited thereto.

In some embodiments of the present invention, the siRNA-modified nucleotide sequence is selected from the group consisting of:
1) the nucleotides at positions 7, 9, 10, and 11 of the 5' end of the sense strand are 2'-fluoronucleotide; and/or
2) the nucleotides at positions 9, 10, and 11 of the 5' end of the sense strand are 2'-fluoronucleotide; and/or
3) the nucleotides at positions 2, 6, 8, 9, 14, and 16 of the 5' end of the antisense strand are 2'-fluoronucleotide; and/or
4) the nucleotides at positions 2, 6, 14, and 16 of the 5' end of the antisense strand are 2'-fluoronucleotide.

In some embodiments of the present invention, the siRNA-modified nucleotide sequence is selected from the group consisting of:
1) the nucleotides at positions 7, 9, 10, and 11 of the 5' end of the sense strand are 2'-fluoronucleotide, and the nucleotides at remaining positions are 2'-methoxynucleotide or the nucleotide shown in Formula 17-a, Formula 18-a, Formula 13-b, or Formula 14-b of the present invention; and/or
2) the nucleotides at positions 9, 10, and 11 of the 5' end of the sense strand are 2'-fluoronucleotide, and the nucleotides at remaining positions are 2'-methoxynucleotide or the nucleotide shown in Formula 17-a, Formula 18-a, Formula 13-b, or Formula 14-b of the present invention; and/or
3) the nucleotides at positions 2, 6, 8, 9, 14, and 16 of the 5' end of the antisense strand are 2'-fluoronucleotide, and the nucleotides at remaining positions are 2'-methoxynucleotide or the nucleotide shown in Formula 17-a, Formula 18-a, Formula 13-b, or Formula 14-b of the present invention; and/or
4) the nucleotides at positions 2, 6, 14, and 16 of the 5' end of the antisense strand are 2'-fluoronucleotide, and the nucleotides at remaining positions are 2'-methoxynucleotide or the nucleotide shown in Formula 17-a, Formula 18-a, Formula 13-b, or Formula 14-b of the present invention.

In some embodiments of the present invention, the siRNA-modified nucleotide sequence is selected from the group consisting of:
1) the nucleotides at positions 7, 9, 10, and 11 of the 5' end of the sense strand are 2'-fluoronucleotide, the nucleotide at position 1 of the 5' end of the sense strand is the nucleotide shown in Formula 17-a, Formula 18-a, Formula 13-b, or Formula 14-b of the present invention, and the nucleotides at remaining positions are 2'-methoxynucleotide; and/or
2) the nucleotides at positions 9, 10 and 11 of the 5' end of the sense strand are 2'-fluoronucleotide, the nucleotide at position 1 of the 5' end of the sense strand is the nucleotide shown in Formula 17-a, Formula 18-a, Formula 13-b, or Formula 14-b of the present invention, and the nucleotides at remaining positions are 2'-methoxynucleotide; and/or
3) the nucleotides at positions 2, 6, 8, 9, 14, and 16 of the 5' end of the antisense strand are 2'-fluoronucleotide, the nucleotide at position 1 of the 3' end of the antisense strand is the nucleotide shown in Formula 17-a, Formula 18-a, Formula 13-b, or Formula 14-b of the present invention, and the nucleotides at remaining positions are 2'-methoxynucleotide; and/or
4) the nucleotides at positions 2, 6, 8, 9, 14, and 16 of the 5' end of the antisense strand are 2'-fluoronucleotide, the nucleotide at position 2 of the 3' end of the antisense strand is the nucleotide shown in Formula 17-a, Formula 18-a, Formula 13-b, or Formula 14-b of the present invention, and the nucleotides at remaining positions are 2'-methoxynucleotide; and/or
5) the nucleotides at positions 2, 6, 14, and 16 of the 5' end of the antisense strand are 2'-fluoronucleotide, the nucleotide at position 1 of the 3' end of the antisense strand is the nucleotide shown in Formula 17-a, Formula 18-a, Formula 13-b, or Formula 14-b of the present invention, and the nucleotides at remaining positions are 2'-methoxynucleotide; and/or
6) the nucleotides at positions 2, 6, 14, and 16 of the 5' end of the antisense strand are 2'-fluoronucleotide, the nucleotide at position 2 of the 3' end of the antisense strand is the nucleotide shown in Formula 17-a, Formula 18-a, Formula 13-b, or Formula 14-b of the present invention, and the nucleotides at remaining positions are 2'-methoxynucleotide.

In some embodiments of the present invention, the 3' end of the sense strand of the siRNA is further attached to a targeting ligand.

In some embodiments of the present invention, the targeting ligand is L96, having the following structure:

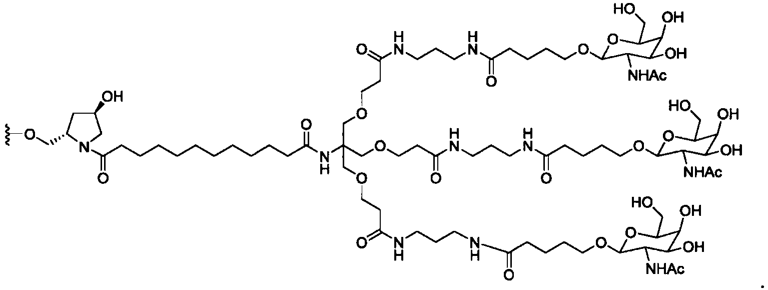

Unless stated to the contrary, terms used in the specification and claims have the following meanings.

In the structure of the compounds of the present application, "DMTr" is 4,4'-dimethoxytrityl.

When Nu represents "absent" in the present application, it means that the nucleotide compound does not have a base, i.e., the base is replaced by hydrogen.

The term "stereoisomer" as used herein refers to a compound having the same chemical construction but with different spatial arrangements of atoms or groups. The compounds of the present invention may comprise asymmetric or chiral centers and thus exist in different stereoisomeric forms. All stereoisomeric forms of the compounds of the present invention, including, but by no means limited to, conformational isomers (rotational isomers), geometric isomers (cis/trans isomers), atropisomers, and the like, and mixtures thereof, e.g., racemic mixtures, constitute a part of the present invention. Many organic compounds exist in optically active forms, i.e., they have the ability to rotate the plane of plane-polarized light. In describing optically active compounds, the prefixes D, L or R, S are used to denote the absolute configuration of the chiral center(s) of the molecule. These stereoisomers have the identical chemical structures but different spatial arrangements. "Stereoisomer" includes enantiomer or diastereomer. A specific stereoisomer may be an enantiomer, and a mixture of isomers is often referred to as an enantiomeric mixture.

The carbon, hydrogen, oxygen, sulfur, nitrogen, or F, Cl, Br, I atoms involved in the groups and compounds described herein comprise their isotopic forms, and the carbon, hydrogen, oxygen, sulfur, or nitrogen atoms involved in the groups and compounds described herein are optionally further replaced by one or more of their corresponding isotopes, wherein the isotopes of carbon comprise ¹²C, ¹³C and ¹⁴C, the isotopes of hydrogen comprise protium (H), deuterium (D, also known as heavy hydrogen), and tritium (T, also known as superheavy hydrogen), the isotopes of oxygen include ¹⁶O, ¹⁷O, and ¹⁸O, the isotopes of sulfur include ³²S, ³³S, ³⁴S and ³⁶S, the isotopes of nitrogen include ¹⁴N and ¹⁵N, the isotopes of fluorine include ¹⁷F and ¹⁹F, the isotopes of chlorine include ³⁵Cl and ³⁷Cl, the isotopes of bromine include ⁷⁹Br and ⁸¹Br, and the isotopes of iodine include ¹²⁷I, ¹²⁹I and ¹³¹I.

"Alkyl" refers to a straight or branched chain saturated aliphatic hydrocarbon group having 1 to 30 carbon atoms, preferably an alkyl group having 1 to 20 (e.g., 1, 2, 3, 4, 5, 6, 7, 8, 9, 10, 11, 12, 13, 14, 15, 16, 17, 18, 19, 20) carbon atoms, and more preferably an alkyl group having 1 to 6 carbon atoms or an alkyl group having 10 to 18 carbon atoms. Non-limiting examples of alkyl include methyl, ethyl, n-propyl, isopropyl, n-butyl, sec-butyl, neo-butyl, tert-butyl, n-pentyl, isopentyl, neopentyl, n-hexyl, and straight or branched chain C₁₀₋₁₈ alkyl. The alkyl group may optionally be further substituted with one or more substituents.

"Alkoxy" refers to a group formed by linking an alkyl group to an oxygen atom. The definition of alkyl is the same as the definition of "alkyl" described above. Non-limiting examples of alkoxy include methoxy, ethoxy, n-propoxy, isopropoxy, n-butoxy, sec-butoxy, tert-butoxy, n-pentoxy, n-hexyloxy, cyclopropoxy, cyclobutoxy, and straight or branched chain C₁₀₋₁₈ alkoxy. The alkoxy group may optionally be further substituted with one or more substituents.

"Cycloalkyl" refers to a saturated cyclic hydrocarbon group in which the ring may be a 3- to 10-membered (e.g., 3, 4, 5, 6, 7, 8, 9, 10-membered) monocyclic ring, a 4- to 12-membered (e.g., 4, 5, 6, 7, 8, 9, 10, 11, 12-membered) bicyclic ring, or a 10- to 20-membered (e.g., 10, 11, 12, 13, 14, 15, 16, 17, 18, 19, 20-membered) polycyclic system, preferably, the number of carbon atoms in the ring are from 3 to 10, further preferably from 3 to 8. Non-limiting examples of cycloalkyl include cyclopropyl, cyclobutyl, cyclopentyl, cyclohexyl, cycloheptyl, cyclooctyl, and the like. The cycloalkyl group may optionally be further substituted with one or more substituents.

"Alkenyl" refers to a straight or branched chain unsaturated aliphatic hydrocarbon group containing 1 to 15 (e.g., 1, 2, 3, 4, 5, 6, 7, 8, 9, 10, 11, 12, 13, 14, 15) carbon-carbon double bonds, consisting of 2 to 30 carbon atoms, preferably an alkenyl group having 2 to 20 (e.g., 2, 3, 4, 5, 6, 7, 8, 9, 10, 11, 12, 13, 14, 15, 16, 17, 18, 19, 20) carbon atoms, and more preferably an alkenyl group having 2 to 8 carbon atoms or an alkenyl group having 10 to 18 carbon atoms. Non-limiting examples of alkenyl include vinyl, propen-2-yl, buten-2-yl, penten-2-yl, penten-4-yl, hexen-2-yl, hexen-3-yl, hepten-2-yl, hepten-3-yl, hepten-4-yl, octen-3-yl, nonen-3-yl, decen-4-yl, undecen-3-yl, and straight or branched chain C₁₂₋₁₈ alkenyl. The alkenyl group may optionally be further substituted with one or more substituents.

"Alkynyl" refers to a straight or branched chain unsaturated aliphatic hydrocarbon group containing 1 to 15 (e.g., 1, 2, 3, 4, 5, 6, 7, 8, 9, 10, 11, 12, 13, 14, 15) carbon-carbon triple bonds, consisting of 2 to 30 carbon atoms, preferably an alkynyl group having 2 to 20 (e.g., 2, 3, 4, 5, 6, 7, 8, 9, 10, 11, 12, 13, 14, 15, 16, 17, 18, 19, 20) carbon atoms, and more preferably an alkynyl group having 2 to 8 carbon atoms or an alkynyl group having 10 to 18 carbon atoms. Non-limiting examples of alkynyl include ethynyl, propynyl-1-yl, propynyl-2-yl, butynyl-1-yl, butynyl-2-yl, butynyl-3-yl, 3,3-dimethylbutynyl-2-yl, pentynyl-1-yl, pentynyl-2-yl, hexynyl-1-yl, 1-heptynyl-1-yl, heptynyl-3-yl, heptynyl-4-yl, octynyl-3-yl, nonynyl-3-yl, decynyl-4-yl, undecyn-3-yl, dodecyn-4-yl, straight or branched chain C₁₃₋₁₈ alkynyl. The alkynyl group may optionally be further substituted with one or more substituents.

Halogens include F, Cl, Br and I.

Acyl refers to a structure formed by a carbonyl-containing functional group bonded to a hydrogen atom or a substituent group, represented as -COR'. In the present invention, R' represents alkyl, alkenyl, or alkynyl. The definitions of alkyl, alkenyl and alkynyl are the same as the definitions of "alkyl", "alkenyl" and "alkynyl" described above. Non-limiting examples of acyl include formyl, acetyl, propionyl, and butyryl.

"Aryl" refers to an aromatic ring group having a conjugated planar ring system, which may be a 5- to 8-membered (e.g., 5, 6, 7, 8-membered) monocyclic ring, a 5- to 12-membered (e.g., 5, 6, 7, 8, 9, 10, 11, 12-membered) bicyclic ring, or a 10- to 15-membered (e.g., 10, 11, 12, 13, 14, 15-membered) tricyclic system, which may be bridged or spiro ring. Non-limiting examples of aryl include phenyl, and naphthyl. The aryl group may optionally be further substituted with one or more substituents.

"Heteroaryl" refers to an aromatic ring group having a conjugated planar ring system and containing a heteroatom, which may be a 3- to 8-membered (e.g., 3, 4, 5, 6, 7, 8-membered) monocyclic ring, a 5- to 12-membered (e.g., 5, 6, 7, 8, 9, 10, 11, 12-membered) bicyclic ring or a 10- to 15-membered (e.g., 10, 11, 12, 13, 14, 15-membered) tricyclic ring system and contains 1 to 6 (e.g., 1, 2, 3, 4, 5, 6) heteroatoms selected from the group consisting of N, O or S. Non-limiting examples of heteroaryl include triazolyl, pyridinyl, furanyl, thienyl, pyranyl, pyrrolyl, pyrimidinyl, pyrazinyl, pyridazinyl, imidazolyl, piperidinyl, benzimidazolyl, benzopyridinyl, pyrrolopyridinyl. The heteroaryl group may optionally be further substituted with one or more substituents.

"Heterocyclyl" or "heterocycle" refers to a saturated or unsaturated aromatic or non-aromatic heterocycle, and when referring to an aromatic heterocycle, it is defined as "heteroaryl" above; when referring to a non-aromatic heterocycle, it may be a 3- to 10-membered (e.g., 3, 4, 5, 6, 7, 8, 9, 10-membered) monocyclic ring, 4- to 12-membered (e.g., 4, 5, 6, 7, 8, 9, 10, 11, 12-membered) bicyclic ring, or 10- to 15-membered (e.g., 10, 11, 12, 13, 14, 15-membered) tricyclic system, and contains 1 to 4 (e.g., 1, 2, 3, 4) heteroatoms selected from the group consisting of N, O or S, preferably a 3- to 8-membered heterocyclyl. Non-limiting examples of "heterocyclyl" or "heterocycle" include oxetanyl, azetanyl, thietanyl, 1,3-dioxolanyl, 1,4-dioxolanyl, 1,3-dioxanyl, azepanyl, oxepanyl, thiepanyl, triazolyl, pyridinyl, piperidinyl, furanyl, thienyl, pyranyl, N-alkylpyrrolyl, pyrimidinyl, pyrazinyl, pyridazinyl, piperazinyl, homopiperazinyl, imidazolyl, piperidinyl, morpholinyl, thiomorpholinyl, thioxanyl, 1,3-dithianyl, dihydrofuranyl, dithiolanyl, tetrahydrofuranyl, tetrahydrothienyl, tetrahydropyranyl, tetrahydrothiopyranyl, tetrahydropyrrolyl, tetrahydroimidazolyl, tetrahydrothiazolyl, tetrahydropyranyl, benzimidazolyl, benzo[d]pyridinyl, pyrrolopyridinyl, benzodihydrofuranyl, 2-pyrrolidinyl, 3-pyrrolidinyl, indolinyl, 2H-pyranyl, 4H-pyranyl, dioxolanyl, 1,3-dioxopentyl, pyrazolinyl, dithianyl, dithiolanyl, dihydrothienyl, pyrrolidinyl, imidazolinyl, imidazolidinyl, and 1,2,3,4-tetrahydroisoquinolinyl. The "heterocyclyl" or "heterocycle" may optionally be further substituted with one or more substituents.

When the aforementioned "alkyl", "alkoxy", "cycloalkyl", "alkenyl", "alkynyl", "aryl", "heteroaryl", "heterocyclyl" groups are substituted, they may optionally be further substituted with 1, 2, 3, 4, 5, 6, 7, 8, 9, or 10 substituents selected from the group consisting of F, Cl, Br, I, hydroxyl, mercapto, nitro, cyano, amino, C1-6 alkylamino, C1-6 alkyl, C1-6 alkoxy, C2-6 alkenyl, C2-6 alkynyl, C3-8 cycloalkyl, C3-8 heterocycloalkyl, C6-10 aryl, and C5-10 heteroaryl.

"Optional" or "optionally" means that the event or condition described thereafter could, but may not, occur, and the description includes situations in which the event or condition occurs and situations in which it does not occur. For example, "heterocyclyl optionally substituted with alkyl" means that the alkyl may be, but is not necessarily present, and the description includes cases where the heterocyclyl is substituted with alkyl and cases where the heterocyclyl is not substituted with alkyl.

### Beneficial effects:

The present invention provides a modified nucleoside compound which can be incorporated into the both ends and/or the middle of an oligonucleotide, so that the modified oligonucleotide is hybridized with a portion of a target RNA, resulting in the loss or down-regulation of the normal function of the target RNA. Such oligonucleotides may also be included in double-stranded compositions. Specifically, the nucleoside compounds of Formulas 1-a and 1-b of the present invention can be applied in oligonucleotides (including but not limited to siRNA, antisense nucleic acids, saRNA, miRNA, aptamers, etc.), effectively enhancing the activity of oligonucleotides and simultaneously improving their resistance to cleavage by enzymes such as phosphatases, exonuclease, and endonuclease. In addition, compared to ordinary nucleosides, the nucleoside compound shown in Formula 1-a of the present invention can readily incorporate various functional groups with specific effects, such as various substituted alkyl lipids, without affecting its base complementary pairing ability. This improves the cell membrane permeability of oligonucleotide sequences and enhances the organ targeting effect of oligonucleotides (for example, when such modified nucleosides are used in siRNA molecules, they can increase the enrichment of siRNA molecules in target organs). Through cell and mouse experiments, the in vitro and in vivo activities, pharmacokinetic properties, and bioavailability of oligonucleotide drugs prepared from the nucleoside compounds represented by Formula 1-a and Formula 1-b of the present invention have been significantly improved.

### BRIEF DESCRIPTION OF THE DRAWINGS

Figure 1 illustrates the residual amount of test sample at each time point in the 3'-exonuclease stability test of single-stranded molecules in Experiment 4.
Figure 2 illustrates the inhibition rate of TTR protein in wild-type mice on day 21 in Experiment 6.
Figure 3 illustrates liver exposure in mice over 14 days in Experiment 7.
Figure 4 illustrates the inhibition rate of TTR protein in wild-type mice on day 21 in Experiment 13.
Figure 5 illustrates the evaluation of TTR protein inhibitory activity in wild-type mice in Experiment 13.

### DETAILED DESCRIPTION OF THE INVENTION

Unless otherwise indicated, the instruments used in the present invention are conventional instruments and the reagents used are conventional reagents.

The structures of the compounds were determined by nuclear magnetic resonance (NMR) and/or mass spectrometry (MS).

NMR shifts (δ) were given in units of 10⁻⁶ (ppm). NMR was determined using an NMR instrument (Broker Avance III 400), the solvents for the determination were deuterated dimethylsulfoxide (DMSO-d6), deuterated chloroform (CDCl₃), deuterated methanol (CD₃OD), and the internal standard was tetramethylsilane (TMS);

MS was determined using Agilent 6120B (ESI) and Agilent 6120B (APCI);

The thin layer chromatography silica gel plates used were Yantai Huanghai HSGF254 or Qingdao GF254 silica gel plates, the specifications for the silica gel plates used in the thin layer chromatography (TLC) were 0.15 mm to 0.20 mm, while those for the silica gel plates used in the separation and purification of products by the thin layer chromatography were 0.4 mm to 0.5 mm;

The carrier used for column chromatography generally was Yantai Huanghai silica gel 200-300 mesh silica gel.

Oligo solid-phase synthesis was carried out, all on an LK- 48E synthesizer (Linkun).

A description of the abbreviated names of the materials used in the present invention:

| Abbreviation of the material or compound used | Names of materials or compounds used |
|---|---|
| TBDPSCl | *tert*-Butyldiphenylchlorosilane |
| Imidazole | Imidazole |
| DCM | Dichloromethane |
| BnBr | Benzyl bromide |
| NaH | Sodium hydride |
| DMF | N,N-Dimethylformamide |
| TBAF | Tetrabutylammonium fluoride |
| Bz | Benzoyl |
| iBu | Isobutyryl |
| THF | Tetrahydrofuran |
| C₁₂H₂₅Br | Bromododecane |
| AcOH | Acetic acid |
| Ac₂O | Acetic anhydride |
| H₂SO₄ | Sulfuric acid |
| Uracil | Uracil |
| BSA | N,O-Bis(trimethylsilyl)acetamide |
| TMSOTf | Trimethylsilyl trifluoromethanesulfonate |
| ACN | Acetonitrile |
| BCl₃ | Boron trichloride |
| DMTrCl | 4,4'-Dimethoxytrityl chloride |
| AgNO₃ | Silver nitrate |
| 2,4,6-collidine | 2,4,6-Trimethylpyridine |
| DCE | 1,2-Dichloroethane |
| CEP-Cl | 2-Cyanoethyl-N,N-diisopropylchlorophosphoramidite |
| DIPEA | N,N-Diisopropylethylamine |
| DMAP | 4-Dimethylaminopyridine |
| PE | Petroleum ether |
| EA | Ethyl acetate |
| TBSCl | *tert*-Butyldimethylsilyl chloride |
| TPSCl | 2,4,6-Triisopropylbenzenesulfonyl chloride |
| TEA | Triethylamine |
| BzCl | Benzoyl chloride |
| pyridine | Pyridine |
| TEA-3HF | Triethylamine trihydrofluoride |
| A(Bz) | N6-Benzoyladenine |
| CH₃ONa/NaOMe | Sodium methoxide |
| G(iBu) | N2-Isobutyrylguanine |
| CH₃I | Iodomethane |
| C₁₄H₂₉Br | Bromotetradecane |
| C₁₆H_{33B}r | Bromohexadecane |
| DMP | Dess-Martin periodinane |
| PPh₃MeBr | Methyltriphenylphosphonium bromide |
| PPh₃MeI | Methyltriphenylphosphonium iodide |
| *t*-BuOK | Potassium tert-butoxide |
| 9-BBN | 9-Borabicyclo[3.3.1]nonane |
| hexane | Hexane |
| NaBO₃-4H₂O | Sodium perborate tetrahydrate |
| KF | Potassium fluoride |
| TEMPO | 2,2,6,6-Tetramethylpiperidinooxy |
| DIAB | (Diacetoxyiodo)benzene |
| EDCl | 1-Ethyl-(3-dimethylaminopropyl)carbodiimide hydrochloride |
| CH₃MgBr | Methylmagnesium bromide |
| m-CPBA | m-Chloroperoxybenzoic acid |
| NaHCO₃ | Sodium bicarbonate |
| PDC | Pyridinium dichromate |
| MsCl | Methanesulfonyl chloride |
| Pd/C | Palladium on carbon |
| HATU | O-(7-Azabenzotriazol-1-yl)-N,N,N',N'-tetramethyluronium hexafluorophosphate |
| TsOH | p-Toluenesulfonic acid |
| H₂O₂ | Hydrogen peroxide |
| LAH | Lithium aluminum hydride |
| K₂OsO₄ | Potassium osmate |
| NMO | N-Methylmorpholine oxide |
| Dioxane | 1,4-Dioxane |
| KNO₂ | Potassium nitrite |
| 18-crown-6 | 18-Crown-6 |
| RuO₂ | Ruthenium dioxide |
| NaIO₄ | Sodium periodate |
| LiBEt₃H | Lithium triethylborohydride |
| NaBH₄ | Sodium borohydride |
| DCA | Dichloroacetic acid |
| PMBCl | 4-Methoxybenzyl chloride |
| DBU | 1,8-Diazabicyclo[5.4.0]undec-7-ene |
| AcSK | Potassium thioacetate |
| DDQ | 2,3-Dichloro-5,6-dicyano-1,4-benzoquinone |
| PPh₃ | Triphenylphosphine |
| Pb₂CO₃ | Lead carbonate |
| I₂ | Iodine |
| PADS | (Phenylacetyl) disulfide |
| ETT | 5-(Ethylthio)-1H-tetrazole |
| TEAA | Triethylammonium acetate |
| M | Mole/liter |
| nM | Nanomole/liter |

The present invention will be described in detail below by way of Examples where specific conditions are not indicated in the Examples, the experimental methods were carried out under conventional conditions. The Examples are given to better illustrate the content of the present invention, but it should not be construed that the content of the present invention is limited to the examples given. Non-essential improvements and adjustments to the Examples by those skilled in the art based on the foregoing invention content still fall within the protection scope of the present invention.

### Example Section

**Examples 1-4 below are the preparation examples of the nucleoside compound shown in Formula 2-a of the present invention, wherein the nucleoside compound shown in Formula 2-a is a TNA structural compound with 2'-phosphoramidite moiety, 3'-ODMTr, and 4'-modification.**

### Example 1: Synthesis of Compound 11

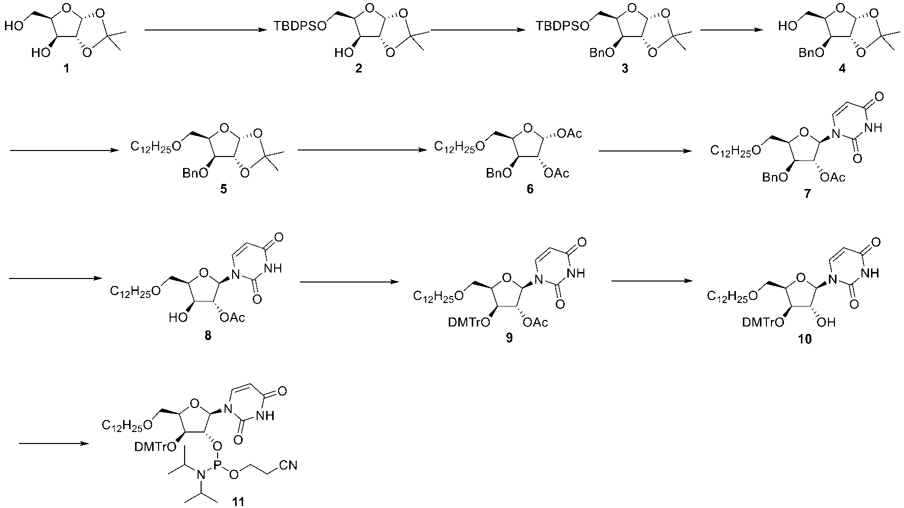

### Synthesis of Compound 2:

Dried compound 1 (20.0 g, 105.15 mmol, 1.0 eq) was dissolved in DCM (200 mL), and imidazole (17.9 g, 262.88 mmol, 2.5 eq) was added to the reaction system. The reaction was cooled to 0°C and stirred for 30 min. Then, tert-butyldiphenylchlorosilane (TBDPSCl, 31.79 g, 115.67 mmol, 1.1 eq) was slowly added dropwise to the reaction system. Once the addition was completed, the ice bath was removed, and the reaction was gradually returned to room temperature and carried out overnight at room temperature. The reaction was monitored by TLC, confirming that compound 1 was fully consumed. Water was added to the reaction system. The resulting mixture was extracted twice with ethyl acetate. The organic phases were combined, and the combined organic phase was washed with water and saturated brine. The organic phase was dried over anhydrous sodium sulfate and concentrated under reduced pressure to give the crude product. The crude product was subjected to column chromatography (PE/EA = 100/15) to afford compound 2 (35.24 g, 82.29 mmol, 78.3% yield).
ESI-MS: m/z 451.2 [M+Na]⁺

### Synthesis of Compound 3:

Compound 2 (38.2 g, 89.13 mmol, 1.0 eq) was dissolved in DMF (300 mL) and stirred until completely dissolved. The reaction system was cooled to around 0°C. NaH (5.35 g, 133.70 mmol, 1.5 eq) was slowly added in batches to the reaction. Once the addition was completed, the reaction system was stirred at 0°C for 30 min. BnBr (22.87 g, 133.70 mmol, 1.5 eq) was slowly added dropwise to the reaction system, and the reaction system was kept at a temperature of around 0°C. Once the addition was completed, the reaction system was returned to room temperature, and the reaction was carried out overnight at room temperature. The completion of the reaction was monitored by TLC, confirming that compound 2 was completely consumed. The reaction system was slowly poured into a cold (0°C) saturated aqueous ammonium chloride solution. The mixed system was extracted twice with ethyl acetate, and the organic phases were combined. The organic phase was washed with water and saturated brine, followed by drying over anhydrous sodium sulfate and concentration under reduced pressure to give crude compound 3 (68.2 g), which was used directly in the next step. ESI-MS: m/z 519.3 [M+H]⁺

### Synthesis of Compound 4:

Crude compound 3 (68.2 g) was dissolved in THF (400 mL) and stirred until completely dissolved. TBAF (1 M in THF, 100 mL, 100 mmol, 1.12 eq) was added to the reaction. The reaction was stirred overnight at room temperature until compound 3 was fully consumed. The reaction solution was concentrated under reduced pressure to remove the solvent, and the resulting crude product was subjected to column chromatography (PE/EA = 10/3) to afford compound 4 (22.07 g, 78.73 mmol, 88.3% yield in two steps).

¹H NMR (400 MHz, DMSO-*d*₆) *δ* 7.43 - 7.23 (m, 5H), 5.84 (d, *J* = 3.6 Hz, 1H), 4.74 (t, *J*= 5.2 Hz, 1H), 4.67 (m, 2H), 4.54 - 4.47 (d, *J* = 12 Hz, 1H), 4.10 (m, 1H), 3.89 (d, *J* = 3.2 Hz, 1H), 3.71 - 3.55 (m, 2H), 1.39 (s, 3H), 1.26 (s, 3H).

### Synthesis of Compound 5:

Compound 4 (22.07 g, 78.73 mmol, 1.0 eq) was dissolved in DMF (200 mL) and stirred well. The reaction was cooled to 0°C and stirred for 30 min. Compound bromododecane (23.55 g, 94.48 mmol, 1.2 eq) was added slowly dropwise to the reaction system. Once the addition was completed, the reaction was returned to room temperature and carried out overnight at room temperature. The complete consumption of compound 4 was monitored by TLC. The reaction system was poured into ice water to quench the reaction completely. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 100/9) to afford compound 5 (32.0 g, 71.37 mmol, 90.6% yield).
¹H NMR (400 MHz, DMSO-*d*₆) *δ* 7.39 - 7.24 (m, 5H), 5.85 (d, *J* = 3.8 Hz, 1H), 4.73 - 4.62 (m, 2H), 4.48 (d, *J* = 12.0 Hz, 1H), 4.17 (td, *J* = 5.9, 3.2 Hz, 1H), 3.88 (d, *J* = 3.2 Hz, 1H), 3.62 (dd, *J* = 10.1, 5.4 Hz, 1H), 3.51 (dd, *J* = 10.1, 6.4 Hz, 1H), 3.46 - 3.30 (m, 2H), 1.47 (m, 2H), 1.39 (s, 3H), 1.24 (d, *J=* 11.6 Hz, 21H), 0.89 - 0.81 (m, 3H). ESI-MS: m/z 471.3 [M+Na]⁺

### Synthesis of Compound 6:

Compound 5 (35.32 g, 78.73 mmol, 1.0 eq) was added to a 500 mL round-bottom three-necked flask, and AcOH (120 mL) and Ac₂O (40.19 g, 393.65 mmol, 5.0 eq) were added to the reaction system and stirred to completely dissolve the compound. The reaction was cooled to 0°C and stirred at this temperature for 30 min. Concentrated sulfuric acid (5 mL) was added slowly dropwise to the reaction system, and the reaction system was kept at a temperature of 0-5°C at all times. Once the addition was completed, the reaction system was returned to room temperature and stirred for additional 8 hours until the compound 5 was completely consumed. The reaction system was cooled to around -5°C and neutralized to a pH of about 7 with aqueous ammonia. Water was added to the reaction system, and the reaction system was extracted twice with ethyl acetate. The organic phases were combined. The organic phase was washed with water and saturated brine, followed by drying over anhydrous sodium sulfate and concentration under reduced pressure to give the crude product 6. The crude product 6 was subjected to column chromatography (PE/EA= 10/2) to afford compound 6 (19.0 g, 38.59 mmol, 49% yield).
¹H NMR (400 MHz, DMSO-*d*₆) *δ* 7.36 - 7.25 (m, 5H), 6.24 (d, *J* = 8.4, 1H), 5.19 - 5.15 (m, 1H), 4.72 - 4.53 (m, 2H), 4.39 - 4.09 (m, 2H), 3.67 - 3.46 (m, 2H), 3.42 - 3.32 (m, 2H), 2.04 (s, 3H), 2.02 (s, 3H), 1.50 - 1.42 (m, 2H), 1.31 - 1.19 (m, 18H), 1.23 (t, *J* = 6.8, 3H). ESI-MS: m/z 493.3 [M+H]⁺

### Synthesis of Compound 7:

Compound 6 (5.0 g, 10.15 mmol, 1.00 eq) and U (2.28 g, 20.30 mmol, 2.0 eq) were added to a 500 mL three-necked round-bottom flask, and ultra-dry ACN (60 mL) was added to the flask with stirring to dissolve the compounds. Subsequently, BSA (6.19 g, 30.45 mmol, 3.0 eq) was added. The reaction system was placed in an oil bath and heated to 80 °C. The reaction system was stirred at this temperature for 1 hour. The entire process was conducted under a nitrogen atmosphere. When the reaction was completed, the reaction system was placed in an ice water bath at 0°C with stirring for 30 min. TMSOTf (2.26 g, 10.15 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound 6 was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product 7. The crude product was subjected to column chromatography (PE/EA= 5/2) to afford compound 7 (4.71 g, 8.65 mmol, 85.2% yield).
¹H NMR (400 MHz, DMSO-*d*₆) *δ* 11.37 (s, 1H), 7.62 (d, *J* = 8.1 Hz, 1H), 7.35 - 7.25 (m, 5H), 5.91 (d, *J* = 2.1 Hz, 1H), 5.58 (dd, *J* = 8.1, 2.2 Hz, 1H), 5.22 (t, *J* = 1.9 Hz, 1H), 4.71 (d, *J* = 12.0 Hz, 1H), 4.54 (d, *J* = 12.0 Hz, 1H), 4.26 (m, 1H), 4.12 (dd, *J=* 4.1, 1.6 Hz, 1H), 3.71 (dd, *J* = 10.6, 4.5 Hz, 1H), 3.64 (dd, *J =* 10.6, 6.1 Hz, 1H), 3.48 - 3.34 (m, 2H), 2.08 (s, 3H), 1.50 - 1.43 (m, 2H), 1.29 - 1.20 (m, 18H), 1.18 (t, *J* = 7.1 Hz, 3H). ESI-MS: m/z 545.3 [M+H]⁺

### Synthesis of Compound 8:

Compound 7 (4.71 g, 8.65 mmol) was added to a 250 ml three-necked flask and ultra-dry DCM (50 mL) was added to the reaction flask. The reaction system was cooled to -20 °C and stirred at this temperature for 30 min. BCl₃ (1 M in toluene, 26 mL, 25.95 mmol, 3.0 eq) was slowly added dropwise to the reaction system and the reaction continued for 5 hours until the compound 7 was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product 8. The crude product was subjected to column chromatography (PE/EA= 2/3) to afford compound 8 (2.0 g, 4.4 mmol, 50.9% yield).
ESI-MS: m/z 455.3 [M+H]⁺

### Synthesis of Compound 9:

Compound 8 (2.0 g, 4.40 mmol, 1.0 eq) was added to a 250 ml round-bottom flask. Ultra-dry DCE (30 mL) was added to the reaction flask with stirring to completely dissolve the compound. DMTrCl (7.45 g, 22.00 mmol, 5.0 eq), AgNO₃ (747 mg, 4.40 mmol, 1.0 eq) and 2,4,6-trimethylpyridine (5.33 g, 44 mmol, 10.0 eq) were added to the reaction at room temperature and stirred well. The reaction was placed in an oil bath and heated to 80°C, then carried out overnight. The complete consumption of compound 8 was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give the crude compound 9. The crude product was subjected to column chromatography (PE/EA= 100/35) to afford compound 9 (3.2 g, 4.23 mmol, 96% yield).
ESI-MS: m/z 757.4 [M+H]⁺

### Synthesis of Compound 10:

Compound 9 (3.2 g, 4.23 mmol, 1.0 eq) was added to a 100 ml round-bottom flask. Then aqueous ammonia (50 mL) was added to the reaction at room temperature. The reaction was stirred at room temperature for 3 hours until the compound 9 was completely consumed. After completion of the reaction, the reaction system was concentrated under reduced pressure at 40°C to give the crude product 10. The crude product was subjected to column chromatography (PE/EA= 1/1) to afford compound 10 (2.9 g, 4.06 mmol, 96% yield).
¹H NMR (400 MHz, DMSO-*d*₆) *δ* 11.28 (s, 1H), 7.90 (d, *J* = 8.1 Hz, 1H), 7.46 - 7.39 (m, 2H), 7.33 - 7.22 (m, 7H), 6.90 - 6.78 (m, 4H), 5.45 (d, *J* = 3.6 Hz, 1H), 4.14 (t, *J* = 4.7 Hz, 1H), 3.74 (s, 6H), 3.68 (dd, *J* = 9.2, 4.5 Hz, 1H), 3.62 - 3.49 (m, 2H), 3.49 - 3.39 (m, 3H), 1.58 (m, 2H), 1.39 - 1.20 (m, 20H), 0.85 (t, *J* = 6.7 Hz, 3H). ESI-MS: m/z 715.4 [M+H]⁺

### Synthesis of Compound 11:

Dried compound 10 (2.9 g, 4.06 mmol, 1.0 eq) was added to a 100 mL round-bottom flask. Ultra-dry DCM (30 mL) was added to the flask with stirring to completely dissolve the compound. DIPEA (1.05 g, 8.12 mmol, 2.0 eq) and DMAP (99 mg, 0.81 mmol, 0.2 eq) were added to the reaction and stirred for 15 min at room temperature. The reaction system was purged with nitrogen and carried out under a nitrogen protection. Compound CEP-Cl (1.44 g, 6.09 mmol, 1.5 eq) was added dropwise to the reaction at room temperature. The reaction was carried out at room temperature for 30-60 min until the compound 10 was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product 11. The crude product was purified by column chromatography (PE/EA= 1/1) to afford compound 11 (3.16 g, 3.46 mmol, 85.2% yield).
¹H NMR (400 MHz, DMSO-*d*₆) *δ* 11.35 (dd, *J* = 14.6, 2.2 Hz, 1H), 7.93 (d, *J* = 8.1 Hz, 1H), 7.47 - 7.36 (m, 2H), 7.33 - 7.23 (m, 7H), 6.89 - 6.83 (m, 4H), 5.71 - 5.51 (m, 2H), 4.34 (dt, *J* = 8.8, 4.7 Hz, 1H), 4.11 - 3.90 (m, 1H), 3.84 - 3.76 (m, 1H), 3.75 (s, 6H), 3.69 - 3.36 (m, 6H), 2.71 (t, *J* = 6.0 Hz, 1H), 2.60 - 2.39 (m, 1H), 1.62 - 1.52 (m, 2H), 1.42 - 1.20 (m, 20H), 1.10 (t, *J= 6.6* Hz, 6H), 1.02 (d, *J* = 6.7 Hz, 6H), 0.89 - 0.81 (m, 3H). ³¹P NMR (162 MHz, DMSO-*d*₆) δ 151.30, 148.65. ESI-MS: m/z 915.6 [M+H]⁺

### Example 2: Synthesis of Compound 16

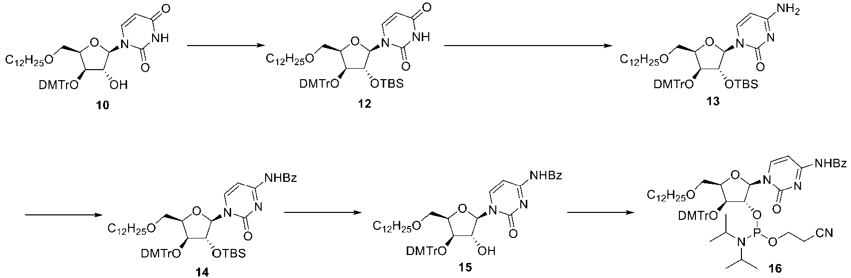

### Synthesis of Compound 12:

Compound 10 (2.6 g, 3.64 mmol, 1.0 eq) was added to a 100 mL round-bottom flask. Ultra-dry DMF (30 mL) was added to the flask and dissolved with stirring. Imidazole (991 mg, 14.56 mmol, 4.0 eq) was added at room temperature and stirred for 10 min. Then, TBSCl (1.10 g, 7.28 mmol, 2.0 eq) was slowly added to the reaction system in batches and the reaction was carried out overnight at room temperature under nitrogen protection. The complete consumption of compound 10 was monitored by TLC and LCMS. The reaction was quenched by adding water. The mixed system was extracted twice with ethyl acetate and the organic phases were combined. The organic phase was washed with water and saturated brine, followed by drying over anhydrous sodium sulfate and removal of the solvent under reduced pressure to give the crude product. The crude product was subjected to column chromatography (PE/EA= 2/1) to afford compound 12 (2.75 g, 3.32 mmol, 91.2% yield).
ESI-MS: m/z 829.5 [M+H]⁺

### Synthesis of Compound 13:

Compound 12 (2.75 g, 3.32 mmol, 1.0 eq) was added to a 250 mL round-bottom flask and ultra-dry ACN (30 mL) was added with stirring to completely dissolve the compound. TEA (672 mg, 6.64 mmol, 2.0 eq) and DMAP (811 mg, 6.64 mmol, 2.0 eq) were added to the reaction system and stirred well. The reaction was cooled to 0-5 °C, and the compound TPSCl (2.01 g, 6.64 mmol, 2.0 eq) was added slowly in batches. Once the addition was completed, the ice bath was removed, and the reaction was returned to room temperature. The reaction was stirred overnight at room temperature. The complete consumption of compound 12 was monitored by TLC. At room temperature, aqueous ammonia (20 mL) was added to the reaction and stirred for about 12 hours until the intermediate was completely consumed. Saturated brine was added to the reaction, and the mixed system was extracted twice with ethyl acetate. The organic phases were combined. The organic phase was washed with water and saturated brine, followed by drying over anhydrous sodium sulfate and concentration under reduced pressure to give the crude product 13 (8.3 g, calculated at 100% yield).
ESI-MS: m/z 828.6 [M+H]⁺

### Synthesis of Compound 14:

Crude compound 13 (8.3 g, 1.0 eq) was added to a 100 mL round-bottom flask, and pyridine (50 mL) was added to the flask with stirring to completely dissolve the crude product 13. The reaction system was cooled to 0 °C, and BzCl (933 mg, 6.64 mmol, 2.0 eq) was slowly added dropwise to the reaction system and stirred at 0 °C for 1 hour until the compound 13 was completely consumed. The reaction was protected by nitrogen throughout. The reaction system was returned to room temperature, and the reaction was quenched by the addition of methanol and water. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA = 1/1) to afford compound 14 (2.35 g, 2.52 mmol, 76% overall yield in 2 steps).
ESI-MS: m/z 931.5 [M+H]⁺

### Synthesis of Compound 15:

Compound 14 (2.35 g, 2.52 mmol, 1.0 eq) was added to a 100 mL round-bottom flask and THF (30 mL) was added with stirring until completely dissolved. Triethylamine trihydrofluoride (5.0 mL) was neutralized to alkaline with triethylamine (17 mL) and added to the above reaction system. The reaction was placed in a 40°C oil bath and stirred overnight under nitrogen protection. The complete consumption of compound 14 was monitored by TLC and LCMS. Water was added to the reaction, and the mixed system was extracted twice with ethyl acetate. The organic phases were combined. The organic phase was washed with water, saturated aqueous sodium bicarbonate solution and saturated brine, followed by drying over anhydrous sodium sulfate and removal of the solvent under reduced pressure to give the crude product. The crude product was subjected to column chromatography (PE/EA= 1/1) to afford compound 15 (1.57 g, 1.92 mmol, 76.2% yield).
ESI-MS: m/z 818.5 [M+H]⁺

### Synthesis of Compound 16:

Dried compound 15 (1.57 g, 1.92 mmol) was added to a 100 mL round-bottom flask, and ultra-dry DCM (20 mL) was added to the flask with stirring to dissolve the compound. DIPEA (496 mg, 3.84 mmol, 2.0 eq) and DMAP (47 mg, 0.38 mmol, 0.2 eq) were added to the reaction, and the reaction was purged with nitrogen. The reaction was cooled to around 0 °C, and CEP-Cl (682 mg, 2.93 mmol, 1.5 eq) was slowly added dropwise. The reaction was carried out at this temperature and under nitrogen protection for 1 hour until the compound 15 was completely consumed. After completion of the reaction, the reaction was quenched by addition of saturated aqueous sodium bicarbonate to the reaction. The mixed system was extracted twice with dichloromethane, and the organic phases were combined. The organic phase was washed with water and saturated brine, followed by drying over anhydrous sodium sulfate and removal of the solvent under reduced pressure to give the crude product. The crude product was purified by column chromatography (PE/EA = 1/1) to afford compound 16 (1.7 g, 1.67 mmol, 87% yield).
¹H NMR (400 MHz, DMSO-*d*₆) *δ* 11.27 (d, *J* = 17.5 Hz, 1H), 8.46 (dd, *J =* 30.3, 7.5 Hz, 1H), 8.01 (d, *J* = 7.6 Hz, 2H), 7.62 (t, *J* = 7.3 Hz, 1H), 7.55 - 7.44 (m, 2H), 7.39 - 7.13 (m, 9H), 6.87 - 6.82 (m, 4H), 5.71 (d, *J* = 25.0 Hz, 1H), 4.23 - 3.90 (m, 3H), 3.78 - 3.38 (m, 13H), 2.74 (dd, *J* = 10.7, 5.6 Hz, 1H), 2.61 - 2.32 (m, 1H), 1.61 - 1.50 (m, 2H), 1.38 - 1.19 (m, 20H), 1.12 - 1.04 (m, 9H), 0.96 (d, *J* = 6.7 Hz, 3H), 0.83 (t, *J* = 6.6 Hz, 3H). ³¹P NMR (162 MHz, DMSO-*d*₆) δ 149.87, 149.34. ESI-MS: m/z 1018.6 [M+H]⁺

### Example 3: Synthesis of Compound 21

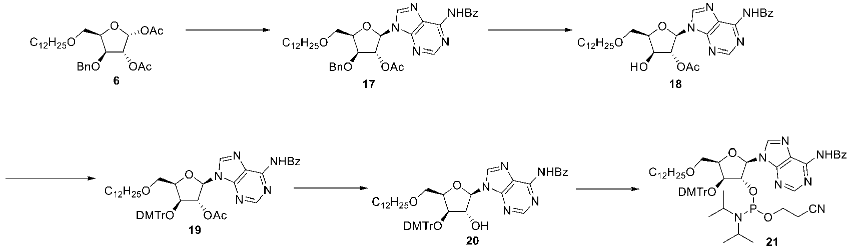

### Synthesis of Compound 17:

Compound 6 (27 g, 54.81 mmol, 1.00 eq) was added to a 500 mL three-necked round-bottom flask, and ultra-dry ACN (250 mL) was added to the flask with stirring to dissolve the compounds. Subsequently, BSA (33.45 g, 164.43 mmol, 3.0 eq) and A(Bz)(26.22 g, 109.62 mmol, 2.0 eq) were added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 30 min. TMSOTf (12.18 g, 54.81 mmol, 1.0 eq) was slowly added dropwise to the reaction system. After completion of addition, the reaction system was placed in an oil bath and slowly warmed to 80°C, and the reaction was carried out at this temperature overnight. The complete consumption of compound 6 was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product 17. The crude product was subjected to column chromatography (PE/EA= 1/1) to afford compound 17 (25 g, 37.23 mmol, 67.9% yield).
¹H NMR (400 MHz, DMSO-*d*₆) *δ* 11.24 (s, 1H), 8.76 (s, 1H), 8.42 (s, 1H), 8.06 (d, *J* = 7.4 Hz, 2H), 7.65 (t, *J* = 7.4 Hz, 1H), 7.55 (t, *J* = 7.6 Hz, 2H), 7.38 - 7.27 (m, 5H), 6.31 (d, J = 1.7 Hz, 1H), 4.78 (d, J = 11.8 Hz, 1H), 4.60 (d, J = 11.8 Hz, 1H), 4.44 (dd, J = 10.3, 4.6 Hz, 1H), 3.83 - 3.67 (m, 2H), 3.46 - 3.39 (m, 2H), 2.13 (s, 3H), 1.53 - 1.43 (m, 2H), 1.32 - 1.16 (m, 20H), 0.84 (t, J = 6.7 Hz, 3H). ESI-MS: m/z 672.4 [M+H]⁺

### Synthesis of Compound 18:

Compound 17 (3.0 g, 4.47 mmol, 1.0 eq) was added to a 250 mL three-necked flask and ultra-dry DCM (30 mL) was added to the reaction flask. The reaction system was cooled to -10 °C and stirred at this temperature for 30 min. 1.0 mol/L of BCl₃ (13.4 mL, 3.0 eq) was slowly added dropwise to the reaction system and the reaction continued for 5 hours until the compound 17 was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product 18. The crude product was subjected to column chromatography (PE/EA= 1/2) to afford compound 18 (1.0 g, 1.72 mmol, 38.5% yield).

### Synthesis of Compound 19:

Compound 18 (1.0 g, 1.72 mmol, 1.0eq) was added to a 100 mL round-bottom flask. Ultra-dry DCE (15 mL) was added to the reaction flask with stirring to completely dissolve the compound. DMTrCl (2.9 g, 8.6 mmol, 5.0 eq), AgNO₃ (0.29 g, 1.72 mmol, 1.0 eq) and 2,4,6-trimethylpyridine (2.08 g, 17.2 mmol, 10.0 eq) were added to the reaction at room temperature and stirred well. The reaction was placed in an oil bath and heated to 80°C, then carried out overnight. The complete consumption of compound 18 was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give a crude compound 19. The crude product was subjected to column chromatography (PE/EA= 2/3) to afford compound 19 (1.2 g, 1.36 mmol, 72% yield).
ESI-MS: m/z 884.5 [M+H]⁺

### Synthesis of Compound 20:

Compound 19 (1.2 g, 1.36 mmol, 1.0 eq) was added to a 100 mL round-bottom flask. THF (10 mL) was added to the reaction, and the reaction was stirred until completely dissolved. To the reaction was added 5.4 mol/L NaOMe solution (0.76 mL, 4.08 mmol, 3.0 eq). The reaction was stirred at room temperature for 3 hours until the compound 19 was completely consumed. After completion of the reaction, the reaction system was concentrated under reduced pressure at 40°C to give the crude product 20. The crude product was subjected to column chromatography to afford compound 20 (0.82 g, 0.97 mmol, 73% yield).
ESI-MS: m/z 842.4 [M+H]⁺

### Synthesis of Compound 21:

Dried compound 20 (0.82 g, 0.97 mmol, 1.0 eq) was added to a 50 mL round-bottom flask. Ultra-dry DCM (10 mL) was added to the flask with stirring to completely dissolve the compound. DIPEA (0.25 g, 1.94 mmol, 2.0 eq) and DMAP (23.8 mg, 0.194 mmol, 0.2 eq) were added to the reaction and stirred for 15 min at room temperature. The reaction system was purged with nitrogen and carried out under a nitrogen protection. Compound CEP-Cl (0.35 g, 1.46 mmol, 1.5 eq) was added dropwise to the reaction at room temperature. The reaction was carried out at room temperature for 30-60 min until the compound 20 was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product 21. The crude product was purified by column chromatography to afford compound 21 (0.74 g, 0.71 mmol, 73.2% yield).
¹H NMR (400 MHz, DMSO-*d*₆) δ 11.21 (d, *J* = 4.0 Hz, 1H), 8.71 (d, *J* = 5.9 Hz, 1H), 8.60 (d, *J* = 29.9 Hz, 1H), 8.04 (d, *J* = 7.6 Hz, 2H), 7.65 (t, *J* = 7.3 Hz, 1H), 7.57 (t, *J* = 7.6 Hz, 2H), 7.35 (t, *J* = 8.6 Hz, 2H), 7.21 - 7.12 (m, 7H), 6.83 - 6.71 (m, 4H), 6.02 (dd, *J* = 36.3, 3.4 Hz, 1H), 4.78 - 4.31 (m, 2H), 3.81 - 3.36 (m, 15H), 2.67 - 2.38 (m, 2H), 1.62 - 1.48 (m, 2H), 1.42 - 1.21 (m, 18H), 1.12 - 0.99 (m, 9H), 0.93 - 0.78 (m, 6H). ESI-MS: m/z 1042.6 [M+H]⁺

### Example 4: Synthesis of Compound 26

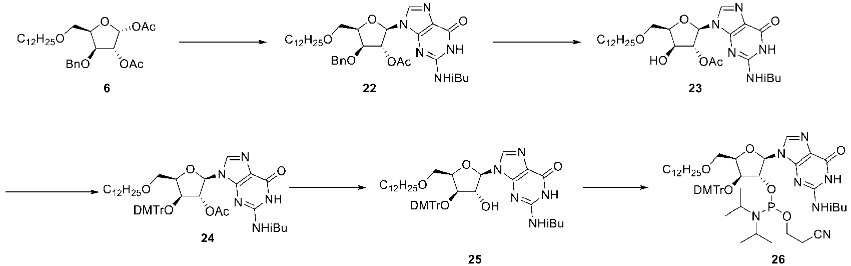

### Synthesis of Compound 22:

Compound 6 (20 g, 40.60 mmol, 1.00 eq) was added to a 500 mL three-necked round-bottom flask, and ultra-dry ACN (200 mL) was added to the flask with stirring to dissolve the compounds. Subsequently, BSA (24.78 g, 121.8 mmol, 3.0 eq) and G(iBu) (17.96 g, 81.2 mmol, 2.0 eq) were added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 30 min. TMSOTf (9.02 g, 40.6 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound 6 was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product 22. The crude product was subjected to column chromatography (PE/EA= 1/1) to afford compound 22 (18 g, 27.53 mmol, 67.8% yield).
ESI-MS: m/z 654.5 [M+H]⁺

### Synthesis of Compound 23:

Compound 22 (5.0 g, 7.64 mmol, 1.0 eq) was added to a 250 mL three-necked flask and ultra-dry DCM (50 mL) was added to the reaction flask. The reaction system was cooled to -10 °C and stirred at this temperature for 30 min. 1.0 mol/L of BCl₃ (22.92 mL, 3.0 eq) was slowly added dropwise to the reaction system and the reaction continued for 5 hours until the compound 22 was completely consumed. The reaction was quenched with triethylamine and methanol at -10°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product 23. The crude product was subjected to column chromatography (PE/EA= 1/2) to afford compound 23 (2.6 g, 4.61 mmol, 60.3% yield).
ESI-MS: m/z 564.3 [M+H]⁺

### Synthesis of Compound 24:

Compound 23 (2.6 g, 4.61 mmol, 1.0eq) was added to a 250 mL round-bottom flask. Ultra-dry DCE (35 mL) was added to the reaction flask with stirring to completely dissolve the compound. DMTrCl (7.8 g, 23.05 mmol, 5.0 eq), AgNO₃ (0.8 g, 4.61 mmol, 1.0 eq), 2,4,6-trimethylpyridine (5.59 g, 46.1 mmol, 10.0 eq) were added to the reaction at room temperature and stirred well. The reaction was placed in an oil bath and heated to 80°C, then carried out overnight. The complete consumption of compound 23 was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give a crude compound 24. The crude product was subjected to column chromatography (PE/EA= 2/3) to afford compound 24 (3.18 g, 3.67 mmol, 79.6% yield).
ESI-MS: m/z 866.6 [M+H]⁺

### Synthesis of Compound 25:

Compound 24 (3.18 g, 3.67 mmol, 1.0 eq) was added to a 100 mL round-bottom flask. THF (30 mL) was added to the reaction and the reaction was stirred until completely dissolved. To the reaction was added 5.4 mol/L NaOMe solution (2.04 mL, 11.01 mmol, 3.0 eq). The reaction was stirred at room temperature for 3 hours until the compound 24 was completely consumed. After completion of the reaction, the reaction system was concentrated under reduced pressure at 40°C to give the crude product 25. The crude product was subjected to column chromatography to afford compound 25 (2.48 g, 3.01 mmol, 82.0% yield).
ESI-MS: m/z 824.5 [M+H]+

### Synthesis of Compound 26:

Dried compound 25 (2.0 g, 2.43 mmol, 1.0 eq) was added to a 50 mL round-bottom flask. Ultra-dry DCM (30 mL) was added to the flask with stirring to completely dissolve the compound. DIPEA (0.63 g, 4.86 mmol, 2.0 eq) and DMAP (59.4 mg, 0.486 mmol, 0.2 eq) were added to the reaction and stirred for 15 min at room temperature. The reaction system was purged with nitrogen and carried out under a nitrogen protection. Compound CEP-Cl (0.86 g, 3.65 mmol, 1.5 eq) was added dropwise to the reaction at room temperature. The reaction was carried out at room temperature for 30-60 min until the compound 25 was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product 26. The crude product was purified by column chromatography to afford compound 26 (2.08 g, 2.06 mmol, 83.5% yield).
¹H NMR (400 MHz, DMSO-*d*₆) *δ* 12.08 (s, 1H), 11.48 (s, 1H), 8.18 (d, *J* = 29.6 Hz, 1H), 7.52 - 7.11 (m, 9H), 6.93 - 6.84 (m, 4H), 5.72 (dd, *J* = 38.6, 4.8 Hz, 1H), 5.21 - 5.04 (m, 1H), 4.53 (t, *J* = 5.5 Hz, 1H), 3.81 - 3.69 (m, 8H), 3.61 - 3.34 (m, 3H), 3.30 - 3.23 (m, 2H), 3.11 (d, *J* = 8.4 Hz, 1H), 2.79 - 2.64 (m, 1H), 2.59 - 2.48 (m, 2H), 2.46 - 2.35 (m, 1H), 1.51 (d, *J=* 6.6 Hz, 2H), 1.38 - 1.18 (m, 18H), 1.13 - 0.98 (m, 13H), 0.88 - 0.74 (m, 8H). ³¹P NMR (162 MHz, DMSO-*d*₆) *δ* 151.94, 148.86. ESI-MS: m/z 1024.6 [M+H]⁺

### Example 5: Synthesis of Compound 33

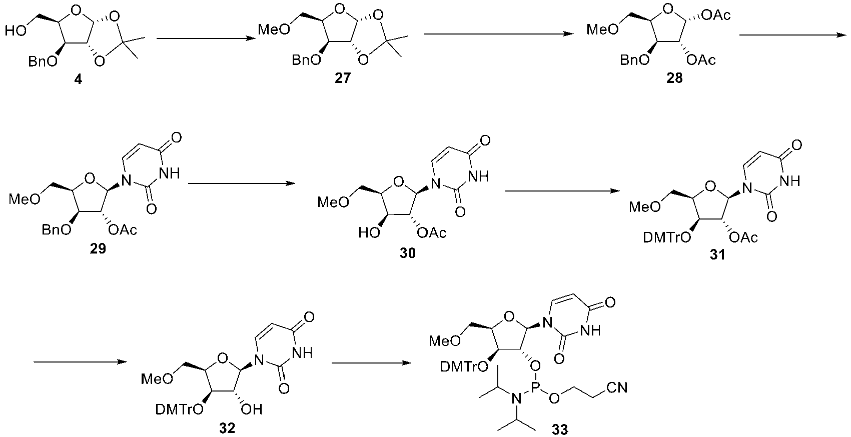

### Synthesis of Compound 27:

Compound 4 (16.6 g, 59.22 mmol, 1.0 eq) was dissolved in DMF (170 mL) and stirred well. The reaction was cooled to 0 °C and stirred for 30 min. Then NaH (2.84 g, 71.06 mmol, 1.2 eq) was added to the reaction and stirred for 30 min. CH₃I (12.61 g, 88.83 mmol, 1.5 eq) was added slowly dropwise to the reaction system. Once the addition was completed, the reaction was returned to room temperature and carried out overnight at room temperature. The complete consumption of compound 4 was monitored by TLC. The reaction system was poured into ice water to quench the reaction completely. The mixed system was extracted twice with ethyl acetate and the organic phases were combined. The organic phase was washed with water and saturated brine, followed by drying over anhydrous sodium sulfate and removal of the solvent under reduced pressure to give the crude product. The crude product was subjected to column chromatography (PE/EA= 100/9) to afford compound 27 (15.0 g, 50.96 mmol, 86.1% yield).
ESI-MS: m/z 295.2 [M+H]⁺

### Synthesis of Compound 28:

Compound 27 (15.0 g, 50.96 mmol, 1.0 eq) was added to a 500 mL round-bottom three-necked flask, and AcOH (136.5 mL) and Ac₂O (26.01 g, 254.95 mmol, 5.0 eq) were added to the reaction system and stirred to completely dissolve the compound. The reaction was cooled to 0°C and stirred at this temperature for 30 min. Concentrated sulfuric acid (1.37 mL) was added slowly dropwise to the reaction system, and the reaction system was kept at a temperature of 0-5°C at all times. Once the addition was completed, the reaction system was returned to room temperature and stirred for additional 8 hours until the compound 27 was completely consumed. The reaction system was cooled to around -5°C and neutralized to a pH of about 7 with aqueous ammonia. Water was added to the reaction system, and the reaction system was extracted twice with ethyl acetate. The organic phases were combined. The organic phase was washed with water and saturated brine, followed by drying over anhydrous sodium sulfate and concentration under reduced pressure to give a crude product 28. The crude product was subjected to column chromatography (PE/EA= 10/2) to afford compound 28 (15.0 g, 44.33 mmol, 87.0% yield).
ESI-MS: m/z 361.1 [M+Na]⁺

### Synthesis of Compound 29:

Compound 28 (15.0 g, 44.33 mmol, 1.00 eq) and uracil (9.94 g, 88.66 mmol, 2.0 eq) were added to a 500 mL three-necked round-bottom flask, and ultra-dry ACN (160 mL) was added to the flask with stirring to dissolve the compounds. Subsequently, BSA (27.05 g, 132.99 mmol, 3.0 eq) was added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 30 min. TMSOTf (9.85 g, 44.36 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound 28 was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product 29. The crude product was subjected to column chromatography (PE/EA= 5/2) to afford compound 29 (15.0 g, 38.42 mmol, 86.7% yield).
ESI-MS: m/z 391.1 [M+H]⁺

### Synthesis of Compound 30:

Compound 29 (15.0 g, 38.42 mmol, 1.0 eq) was added to a 500 mL three-necked flask and ultra-dry DCM (200 mL) was added to the reaction flask. The reaction system was cooled to -15 °C and stirred at this temperature for 30 min. BCl₃ (1M in toluene, 115 mL, 115.26 mmol, 3.0 eq) was slowly added dropwise to the reaction system and the reaction was carried out for additional 5 hours until the compound 29 was completely consumed. The reaction was quenched with triethylamine and methanol at -15°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product 30. The crude product was subjected to column chromatography (PE/EA= 2/3) to afford compound 30 (5.0 g, 16.65 mmol, 43.3% yield).
ESI-MS: m/z 301.2 [M+H]⁺

### Synthesis of Compound 31:

Compound 30 (5.0 g, 16.65 mmol, 1.0 eq) was added to a 250 mL round-bottom flask. Ultra-dry DCE (60 mL) was added to the reaction flask with stirring to completely dissolve the compound. DMTrCl (11.28 g, 33.30 mmol, 2.0 eq), AgNO₃ (2.83 g, 16.65 mmol, 1.0 eq), 2,4,6-trimethylpyridine (20.18 g, 166.5 mmol, 10.0 eq) were added to the reaction at room temperature and stirred well. The reaction was placed in an oil bath and heated to 80°C, then carried out overnight. The complete consumption of compound 30 was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give a crude compound 31. The crude product was subjected to column chromatography (PE/EA= 100/35) to afford compound 31 (8.0 g, 13.28 mmol, 79.7% yield).
ESI-MS: m/z 603.2 [M+H]⁺

### Synthesis of Compound 32:

Compound 31 (8.0 g, 13.28 mmol, 1.0 eq) was added to a 250 round-bottom flask. Then 7M ammonia in methanol (50 mL) was added to the reaction at room temperature. The reaction was stirred at room temperature for 3 hours until the compound 31 was completely consumed. After completion of the reaction, the reaction system was concentrated under reduced pressure at 40°C to give the crude product 32. The crude product was subjected to column chromatography (PE/EA= 1/1) to afford compound 32 (6.0 g, 10.71 mmol, 80.6% yield).
ESI-MS: m/z 561.2 [M+H]⁺

### Synthesis of Compound 33:

Dried compound 32 (2.0 g, 3.57 mmol, 1.0 eq) was added to a 100 mL round-bottom flask. Ultra-dry DCM (20 mL) was added to the flask with stirring to completely dissolve the compound. DIPEA (0.93 g, 7.14 mmol, 2.0 eq) and DMAP (87 mg, 0.714 mmol, 0.2 eq) were added to the reaction and stirred for 15 min at room temperature. The reaction system was purged with nitrogen and carried out under a nitrogen protection. Compound CEP-Cl (1.27 g, 5.36 mmol, 1.5 eq) was added dropwise to the reaction at room temperature. The reaction was carried out at room temperature for 30-60 min until the compound 32 was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product 33. The crude product was purified by column chromatography (PE/EA= 1/1) to afford compound 33 (2.1 g, 2.76 mmol, 77.3% yield).
¹H NMR (400 MHz, DMSO-*d*₆) *δ* 11.34 (s, 1H), 7.86 (dd, *J* = 18.9, 8.2 Hz, 1H), 7.42 - 7.19 (m, 9H), 6.92 - 6.84 (m, 4H), 5.65 - 5.50 (m, 2H), 4.25 - 4.14 (m, 1H), 4.00 - 3.46 (m, 14H), 3.35 - 3.30 (d, *J* = 20 Hz, 3H), 2.73 - 2.68 (m, 1H), 2.65 - 2.41 (m, 1H), 1.09 (t, *J=* 6.1 Hz, 6H), 1.01 (t, *J =* 6.8 Hz, 6H); ³¹P NMR (162 MHz, DMSO-*d*₆) *δ* 151.13, 148.52. ESI-MS: m/z 761.3 [M+H]⁺

### Example 6: Synthesis of Compound 38

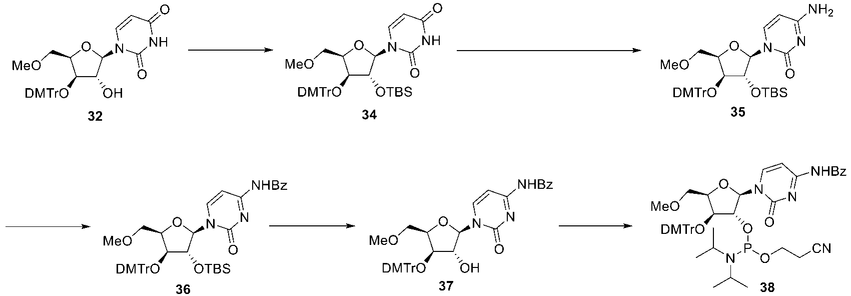

### Synthesis of Compound 34:

Compound 32 (4.0 g, 7.14 mmol, 1.0 eq) was added to a 250 mL round-bottom flask. Ultra-dry DMF (50 mL) was added to the flask with stirring. Imidazole (1.94 g, 28.56 mmol, 4.0 eq) was added at room temperature and stirred for 10 min. Then, TBSCl (2.15 g, 14.28 mmol, 2.0 eq) was slowly added to the reaction system in batches and the reaction was carried out overnight at room temperature under nitrogen protection. The complete consumption of compound 32 was monitored by TLC and LCMS. The reaction was quenched by adding water. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 2/1) to afford compound 34 (3.88 g, 5.75 mmol, 80.5% yield).
ESI-MS: m/z 675.3 [M+H]⁺

### Synthesis of Compound 35:

Compound 34 (3.88 g, 5.76 mmol, 1.0 eq) was added to a 250 mL round-bottom flask. Ultra-dry ACN (30 mL) was added to the flask with stirring to completely dissolve the compound. TEA (1.16 g, 11.52 mmol, 2.0 eq) and DMAP (1.40 g, 11.50 mmol, 2.0 eq) were added to the reaction system and stirred well. The reaction was cooled to 0-5 °C, and the compound TPSCl (3.48 g, 11.50 mmol, 2.0 eq) was added slowly in batches. Once the addition was completed, the ice bath was removed, and the reaction was returned to room temperature. The reaction was stirred overnight at room temperature. The complete consumption of compound 34 was monitored by TLC. At room temperature, aqueous ammonia (40 mL) was added to the reaction and stirred for about 12 hours until the intermediate was completely consumed. Saturated brine was added to the reaction, and the mixed system was extracted twice with ethyl acetate. The organic phases were combined. The organic phase was washed with water and saturated brine, followed by drying over anhydrous sodium sulfate and concentration under reduced pressure to give the crude product 35 (5.1 g, calculated at 100% yield).
ESI-MS: m/z 674.3 [M+H]⁺

### Synthesis of Compound 36:

Crude compound 35 (5.1 g, 1.0 eq) was added to a 100 mL round-bottom flask, and pyridine (50 mL) was added to the flask with stirring to completely dissolve the crude product 35. The reaction system was cooled to 0 °C, and BzCl (1.62 g, 11.50 mmol, 2.0 eq) was slowly added dropwise to the reaction system and stirred at 0 °C for 1 hour until the compound 35 was completely consumed. The reaction was protected by nitrogen throughout. The reaction system was returned to room temperature, and the reaction was quenched by the addition of methanol and water. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA = 1/1) to afford compound 36 (3.7 g, 4.76 mmol, 82.8% overall yield in 2 steps).
ESI-MS: m/z 778.4 [M+H]⁺

### Synthesis of Compound 37:

Compound 36 (3.7 g, 4.76 mmol, 1.0 eq) was added to a 100 mL round-bottom flask and THF (40 mL) was added with stirring until completely dissolved. Triethylamine trihydrofluoride (5.0 mL) was neutralized to alkaline with triethylamine (17 mL) and added to the above reaction system. The reaction was placed in a 40°C oil bath and stirred overnight under nitrogen protection. The complete consumption of compound 36 was monitored by TLC and LCMS. Water was added to the reaction and the mixed system was extracted twice with ethyl acetate. The organic phases were combined. The organic phase was washed with water, saturated aqueous sodium bicarbonate solution and saturated brine, followed by drying over anhydrous sodium sulfate and removal of the solvent under reduced pressure to give the crude product. The crude product was subjected to column chromatography (PE/EA= 1/1) to afford compound 37 (2.6 g, 3.92 mmol, 82.4% yield).
ESI-MS: m/z 664.3 [M+H]⁺

### Synthesis of Compound 38:

Dried compound 37 (2.6 g, 3.92 mmol, 1.0 eq) was added to a 100 mL round-bottom flask, and ultra-dry DCM (30 mL) was added to the flask with stirring to dissolve the compound. DIPEA (1.0 g, 7.84 mmol, 2.0 eq) and DMAP (96 mg, 0.784 mmol, 0.2 eq) were added to the reaction, and the reaction was purged with nitrogen. The reaction was cooled to around 0 °C, and CEP-Cl (1.39 g, 5.88 mmol, 1.5 eq) was slowly added dropwise. The reaction was carried out at this temperature and under nitrogen protection for 1 hour until the compound 37 was completely consumed. After completion of the reaction, the reaction was quenched by addition of saturated aqueous sodium bicarbonate to the reaction. The mixed system was extracted twice with dichloromethane, and the organic phases were combined. The organic phase was washed with water and saturated brine, followed by drying over anhydrous sodium sulfate and removal of the solvent under reduced pressure to give the crude product. The crude product was purified by column chromatography (PE/EA = 1/1) to afford compound 38 (2.3 g, 2.66 mmol, 67.9% yield).
¹H NMR (400 MHz, DMSO-*d*₆) *δ* 11.18 (d, *J =* 24.4 Hz, 1H), 8.49 (dd, *J =* 30.5, 7.3 Hz, 1H), 7.99 (d, *J =* 7.7 Hz, 2H), 7.65 - 7.08 (m, 14H), 6.85 - 6.75 (m, 4H), 5.71 - 5.62 (m, 1H), 4.22 - 3.82 (m, 3H), 3.82 - 3.69 (m, 8H), 3.61 - 3.39 (m, 5H), 3.35 - 3.30 (d, *J =* 20 Hz, 3H), 2.75 - 2.70 (m, 1H), 2.53 - 2.31(m, 1H), 1.08 (t, *J* = 6.1 Hz, 6H), 1.00 (t, *J* = 6.8 Hz, 6H); ³¹P NMR (162 MHz, DMSO-*d*₆) *δ* 149.52, 149.10. ESI-MS: m/z 864.4 [M+H]⁺

### Example 7: Synthesis of Compound 43

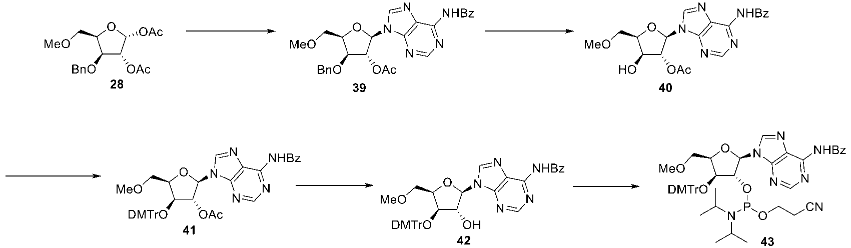

### Synthesis of Compound 39:

Compound 28 (15.0 g, 0.04 mol, 1.00 eq) was added to a 500 mL three-necked round-bottom flask, and ultra-dry ACN (150 mL) was added to the flask with stirring to dissolve the compounds. Subsequently, BSA (24.4 g, 0.12 mol, 3.0 eq) and A(Bz)(19.14 g, 0.08 mol, 2.0 eq) were added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 30 min. TMSOTf (8.9 g, 0.04 mol, 1.0 eq) was slowly added dropwise to the reaction system. Once the addition was completed, the reaction system was placed in an oil bath and slowly warmed to 80°C, and the reaction was carried out at this temperature overnight. The complete consumption of compound 28 was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product 39. The crude product was subjected to column chromatography (PE/EA= 1/1) to afford compound 39 (20.0 g, 38.64 mmol, 87.2% yield).
ESI-MS: m/z 518.2 [M+H]⁺

### Synthesis of Compound 40:

Compound 39 (20.0 g, 38.64 mmol, 1.0 eq) was added to a 500 mL three-necked flask and ultra-dry DCM (200 mL) was added to the reaction flask. The reaction system was cooled to -20 °C and stirred at this temperature for 30 min. 1.0 mol/L of boron trichloride in dichloromethane (116 mL, 115.92 mmol, 3.0 eq) was slowly added dropwise to the reaction system and the reaction continued for 5 hours until the compound 39 was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product 40. The crude product was subjected to column chromatography (PE/EA= 1/2) to afford compound 40 (9.7 g, 22.69 mmol, 58.7% yield).
ESI-MS: m/z 428.2 [M+H]⁺

### Synthesis of Compound 41:

Compound 40 (9.7 g, 22.69 mmol, 1.0eq) was added to a 100 mL round-bottom flask. Ultra-dry DCE (100 mL) was added to the reaction flask with stirring to completely dissolve the compound. DMTrCl (15.4 g, 45.38 mmol, 2.0 eq), AgNO₃ (3.85 g, 22.69 mmol, 1.0 eq), 2,4,6-trimethylpyridine (27.50 g, 226.9 mmol, 10.0 eq) were added to the reaction at room temperature and stirred well. The reaction was placed in an oil bath and heated to 80°C, then carried out overnight. The complete consumption of compound 40 was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give a crude compound 41. The crude product was subjected to column chromatography (PE/EA= 2/3) to afford compound 41 (14.5 g, 19.87 mmol, 87.6% yield).
ESI-MS: m/z 730.3 [M+H]⁺

### Synthesis of Compound 42:

Compound 41 (14.5 g, 19.87 mmol, 1.0 eq) was added to a 100 mL round-bottom flask. THF (150 mL) was added to the reaction, and the reaction was stirred until completely dissolved. To the reaction was added 5.4 mol/L NaOMe solution (5.5 mL, 29.82 mmol, 1.5 eq). The reaction was stirred at room temperature for 3 hours until the compound 41 was completely consumed. After completion of the reaction, the reaction system was concentrated under reduced pressure at 40°C to give the crude product 42. The crude product was subjected to column chromatography to afford compound 42 (12.0 g, 17.45 mmol, 87.8% yield).
ESI-MS: m/z 688.3 [M+H]⁺

### Synthesis of Compound 43:

Dried compound 42 (3.0 g, 4.36 mmol, 1.0 eq) was added to a 50 mL round-bottom flask. Ultra-dry DCM (30 mL) was added to the flask with stirring to completely dissolve the compound. DIPEA (1.13 g, 8.72 mmol, 2.0 eq) and DMAP (106.5 mg, 0.872 mmol, 0.2 eq) were added to the reaction and stirred for 15 min at room temperature. The reaction system was purged with nitrogen and carried out under a nitrogen protection. Compound CEP-Cl (1.55 g, 6.56 mmol, 1.5 eq) was added dropwise to the reaction at room temperature. The reaction was carried out at room temperature for 30-60 min until the compound 42 was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product 43. The crude product was purified by column chromatography to afford compound 43 (3.4 g, 3.83 mmol, 87.8% yield).
¹H NMR (400 MHz, DMSO-*d*₆) *δ* 11.21 (s, 1H), 8.75 - 8.54 (m, 2H), 8.05 (d, *J =* 7.3 Hz, 2H), 7.68 - 7.50 (m, 3H), 7.39 - 7.31 (m, 2H), 7.29 - 7.15 (m, 7H), 6.86 - 6.72 (m, 4H), 6.05 (dd, *J* = 34.0, 3.2 Hz, 1H), 4.74 - 4.30 (m, 2H), 3.87 - 3.78 (m, 1H), 3.79 - 3.46 (m, 12H), 3.30 (d, *J* = 24.0, 3H), 2.70 - 2.57 (m, 2H), 1.11 - 0.87 (m, 12H); ³¹P NMR (162 MHz, DMSO-*d*₆) *δ* 150.68, 149.45; ESI-MS: m/z 888.4 [M+H]⁺

### Example 8: Synthesis of Compound 48

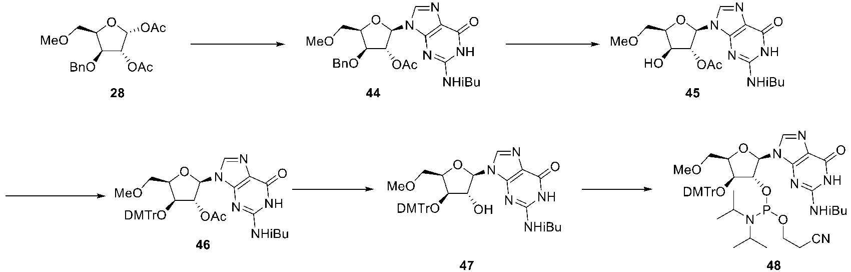

### Synthesis of Compound 44:

Compound 28 (15.0 g, 0.04 mol, 1.00 eq) was added to a 500 mL three-necked round-bottom flask, and ultra-dry ACN (150 mL) was added to the flask with stirring to dissolve the compounds. Subsequently, BSA (24.4 g, 0.12 mol, 3.0 eq) and G(iBu) (17.70 g, 0.08 mol, 2.0 eq) were added. The reaction system was placed in an oil bath and heated to 80 °C. The reaction system was stirred at this temperature for 1 hour. The entire process was conducted under a nitrogen atmosphere. When the reaction was completed, the reaction system was placed in an ice water bath at 0°C with stirring for 30 min. TMSOTf (8.9 g, 0.04 mol, 1.0 eq) was slowly added dropwise to the reaction. Once the addition was completed, the reaction system was placed in an oil bath and slowly warmed to 80°C, and the reaction was carried out at this temperature overnight. The complete consumption of compound 28 was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product 44. The crude product was subjected to column chromatography (PE/EA= 1/1) to afford compound 44 (10.62 g, 21.26 mmol, 48.0% yield).
ESI-MS: m/z 500.2 [M+H]⁺

### Synthesis of Compound 45:

Compound 44 (10.62 g, 21.26 mmol, 1.0 eq) was added to a 500 mL three-necked flask and ultra-dry DCM (100 mL) was added to the reaction flask. The reaction system was cooled to -20 °C and stirred at this temperature for 30 min. 1.0 mol/L of BCl₃ (31.89 mL, 31.92 mmol, 1.5 eq) in dichloromethane was slowly added dropwise to the reaction system and the reaction continued for 5 hours until the compound 44 was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product 45. The crude product was subjected to column chromatography (PE/EA= 1/2) to afford compound (2.03 g, 4.96 mmol, 23.3% yield).
ESI-MS: m/z 410.2 [M+H]⁺

### Synthesis of Compound 46:

Compound 45 (2.0 g, 4.89 mmol, 1.0eq) was added to a 100 mL round-bottom flask. Ultra-dry DCE (25 mL) was added to the reaction flask with stirring to completely dissolve the compound. DMTrCl (3.32 g, 9.78 mmol, 2.0 eq), AgNO₃ (0.83 g, 4.89 mmol, 1.0 eq), 2,4,6-trimethylpyridine (5.93 g, 48.9 mmol, 10.0 eq) were added to the reaction at room temperature and stirred well. The reaction was placed in an oil bath and heated to 80°C, then carried out overnight. The complete consumption of compound 45 was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give a crude compound 46. The crude product was subjected to column chromatography (PE/EA= 2/3) to afford compound 46 (2.96 g, 4.16 mmol, 85.1% yield).
ESI-MS: m/z 712.4 [M+H]⁺

### Synthesis of Compound 47:

Compound 46 (2.96 g, 4.16 mmol, 1.0 eq) was added to a 100 mL round-bottom flask. THF (30 mL) was added to the reaction, and the reaction was stirred until completely dissolved. To the reaction was added 5.4 mol/L NaOMe solution (2.31 mL, 12.48 mmol, 3.0 eq). The reaction was stirred at room temperature for 3 hours until the compound 46 was completely consumed. After completion of the reaction, the reaction system was concentrated under reduced pressure at 40°C to give the crude product 47. The crude product was subjected to column chromatography to afford compound 47 (2.54 g, 3.79 mmol, 91.1% yield).
ESI-MS: m/z 670.4 [M+H]⁺

### Synthesis of Compound 48:

Dried compound 47 (2.54 g, 3.79 mmol, 1.0 eq) was added to a 50 mL round-bottom flask. Ultra-dry DCM (30 mL) was added to the flask with stirring to completely dissolve the compound. DIPEA (0.98 g, 7.58 mmol, 2.0 eq) and DMAP (93 mg, 0.758 mmol, 0.2 eq) were added to the reaction and stirred for 15 min at room temperature. The reaction system was purged with nitrogen and carried out under a nitrogen protection. Compound CEP-Cl (1.35 g, 5.69 mmol, 1.5 eq) was added dropwise to the reaction at room temperature. The reaction was carried out at room temperature for 30-60 min until the compound 47 was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product 48. The crude product was purified by column chromatography to afford compound 48 (2.57 g, 2.96 mmol, 77.8% yield).
¹H NMR (400 MHz, DMSO-*d*₆) *δ* 12.708 (s, 1H), 11.59 (s, 1H), 8.21 (d, *J =* 29.6 Hz, 1H), 7.55 - 7.18 (m, 9H), 6.88 - 6.79 (m, 4H), 5.72 (dd, *J* = 38.6, 4.8 Hz, 1H), 5.25 - 5.12 (m, 1H), 4.50 (t, *J* = 5.5 Hz, 1H), 3.80 - 3.68 (m, 8H), 3.62 - 3.35 (m, 3H), 3.30 (d, *J =* 24.0, 3H), 3.25 - 3.20 (m, 1H), 3.12 (d, *J =* 8.4 Hz, 1H), 2.78 - 2.65 (m, 1H), 2.57 (m, 1H), 2.44 - 2.35 (m, 1H), 1.11 - 0.87 (m, 18H); ³¹P NMR (162 MHz, DMSO-*d*₆) *δ* 151.65, 148.95; ESI-MS: m/z 870.5 [M+H]⁺

### Example 9: Synthesis of Compound 55

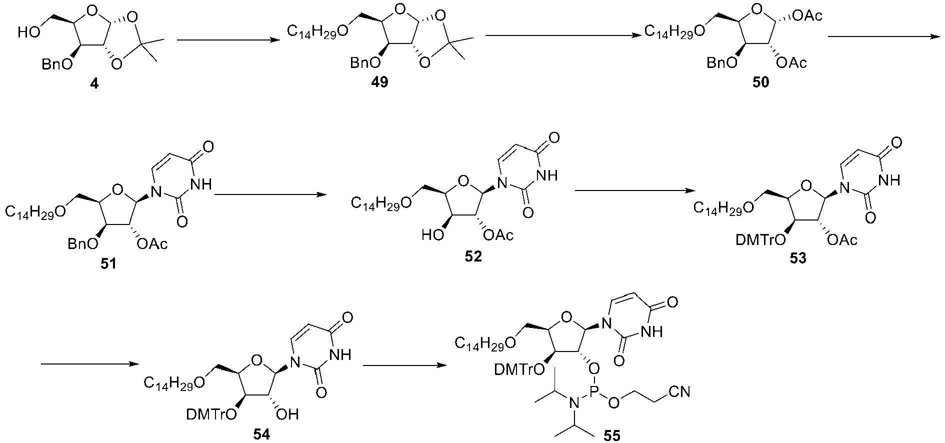

### Synthesis of Compound 49:

Compound 4 (36.0 g, 128.42 mmol, 1.0 eq) was dissolved in DMF (400 mL) and stirred well. The reaction was cooled to 0 °C and stirred for 30 min, and NaH (7.7 g, 192.63 mmol, 1.5 eq) was added and stirred for 30 min. Compound bromotetradecane (42.7 g, 154.10 mmol, 1.2 eq) was added slowly dropwise to the reaction system. Once the addition was completed, the reaction was returned to room temperature and carried out overnight at room temperature. The complete consumption of compound 4 was monitored by TLC. The reaction system was poured into ice water to quench the reaction completely. The mixed system was extracted twice with ethyl acetate and the organic phases were combined. The organic phase was washed with water and saturated brine, followed by drying over anhydrous sodium sulfate and removal of the solvent under reduced pressure to give the crude product. The crude product was subjected to column chromatography (PE/EA= 100/9) to afford compound 49 (55.8 g, 117.10 mmol, 91% yield).
ESI-MS: m/z 477.5 [M+H]⁺

### Synthesis of Compound 50:

Compound 49 (55.8 g, 117.10 mmol, 1.0 eq) was added to a 1000 mL round-bottom three-necked flask, and AcOH (500 mL) and AC₂O (60.0 g, 0.585 mol, 5.0 eq) were added to the reaction system and stirred to completely dissolve the compound. The reaction was cooled to 0°C and stirred at this temperature for 30 min. Concentrated sulfuric acid (10 mL) was added slowly dropwise to the reaction system, and the reaction system was kept at a temperature of 0-5°C at all times. Once the addition was completed, the reaction system was returned to room temperature and stirred for additional 8 hours until the compound 49 was completely consumed. The reaction system was cooled to around -5°C and neutralized to a pH of about 7 with aqueous ammonia. Water was added to the reaction system, and the reaction system was extracted twice with ethyl acetate. The organic phases were combined. The organic phase was washed with water and saturated brine, followed by drying over anhydrous sodium sulfate and concentration under reduced pressure to give a crude product 50. The crude product was subjected to column chromatography (PE/EA= 10/2) to afford compound 50 (31.0 g, 59.58 mmol, 50.8% yield).
ESI-MS: m/z 521.3 [M+H]⁺

### Synthesis of Compound 51:

Compound 50 (13.0 g, 0.025 mol, 1.00 eq) and uracil (5.6 g, 0.05 mmol, 2.0 eq) were added to a 500 mL three-necked round-bottom flask, and ultra-dry ACN (130 mL) was added to the flask with stirring to dissolve the compounds. Subsequently, BSA (15.3 g, 0.075 mol, 3.0 eq) was added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 30 min. TMSOTf (5.56 g, 0.025 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound 50 was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product 51. The crude product was subjected to column chromatography (PE/EA= 5/2) to afford compound 51 (11.0 g, 19.21 mmol, 76.9% yield).
ESI-MS: m/z 573.5 [M+H]⁺

### Synthesis of Compound 52:

Compound 51 (11.0 g, 19.21 mmol, 1.0 eq) was added to a 500 mL three-necked flask and ultra-dry DCM (120 mL) was added to the reaction flask. The reaction system was cooled to -20 °C and stirred at this temperature for 30 min. Boron trichloride (1M in toluene, 77 mL, 76.84 mmol, 4.0 eq) was slowly added dropwise to the reaction system and the reaction continued for 5 hours until the compound 51 was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product 52. The crude product was subjected to column chromatography (PE/EA= 2/3) to afford compound 52 (7.7 g, 15.96 mmol, 83.0% yield).
ESI-MS: m/z 483.3 [M+H]⁺

### Synthesis of Compound 53:

Compound 52 (7.7 g, 15.96 mmol, 1.0 eq) was added to a 250 mL round-bottom flask. Ultra-dry DCE (80 mL) was added to the reaction flask with stirring to completely dissolve the compound. DMTrCl (27.04 g, 79.8 mmol, 5.0 eq), AgNO₃ (2.72 g, 15.96 mmol, 1.0 eq), 2,4,6-trimethylpyridine (19.4 g, 159.6 mmol, 10.0 eq) were added to the reaction at room temperature and stirred well. The reaction was placed in an oil bath and heated to 80°C, then carried out overnight. The complete consumption of compound 52 was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give a crude compound 53. The crude product was subjected to column chromatography (PE/EA= 100/35) to afford compound 53 (11.37 g, 14.48 mmol, 90.7% yield).
ESI-MS: m/z 785.4 [M+H]⁺

### Synthesis of Compound 54:

Compound 53 (11.37 g, 14.48 mmol, 1.0 eq) was added to a 250 ml round-bottom flask. Then aqueous ammonia (50 mL) was added to the reaction at room temperature. The reaction was stirred at room temperature for 3 hours until the compound 53 was completely consumed. After completion of the reaction, the reaction system was concentrated under reduced pressure at 40°C to give the crude product 54. The crude product was subjected to column chromatography (PE/EA= 1/1) to afford compound 54 (10.0 g, 13.46 mmol, 93.0% yield).
ESI-MS: m/z 743.4 [M+H]⁺

### Synthesis of Compound 55:

Dried compound 54 (5.0 g, 6.73 mmol, 1.0 eq) was added to a 100 mL round-bottom flask. Ultra-dry DCM (50 mL) was added to the flask with stirring to completely dissolve the compound. DIPEA (1.74 g, 13.46 mmol, 2.0 eq) and DMAP (164.4 mg, 1.346 mmol, 0.2 eq) were added to the reaction and stirred for 15 min at room temperature. The reaction system was purged with nitrogen and carried out under a nitrogen protection. Compound CEP-Cl (2.40 g, 10.1 mmol, 1.5 eq) was added dropwise to the reaction at room temperature. The reaction was carried out at room temperature for 30-60 min until the compound 54 was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product 55. The crude product was purified by column chromatography (PE/EA= 1/1) to afford compound 55 (4.54 g, 4.82 mmol, 71.6% yield).
¹H NMR (400 MHz, DMSO-*d*₆) *δ* 11.35 (d, *J =* 14.5 Hz, 1H), 7.93 (d, *J =* 8.1 Hz, 1H), 7.41 (dd, *J =* 16.0, 7.5 Hz, 2H), 7.34 - 7.20 (m, 7H), 6.86 (t, *J =* 8.5 Hz, 4H), 5.73 - 5.49 (m, 2H), 4.41 - 4.30 (m, 1H), 4.10 - 3.90 (m, 1H), 3.83 - 3.33 (m, 15H), 2.71 (t, *J =* 6.0 Hz, 1H), 2.60 - 2.39 (m, 1H), 1.60 - 1.52 (m, 2H), 1.42 - 1.21 (m, 22H), 1.10 (t, *J* = 6.5 Hz, 6H), 1.02 (d, *J* = 6.7 Hz, 6H), 0.84 (t, *J =* 6.5 Hz, 3H); ³¹P NMR (162 MHz, DMSO-*d*₆) *δ* 151.28, 148.64; ESI-MS: m/z 943.5 [M+H]⁺

### Example 10: Synthesis of Compound 60

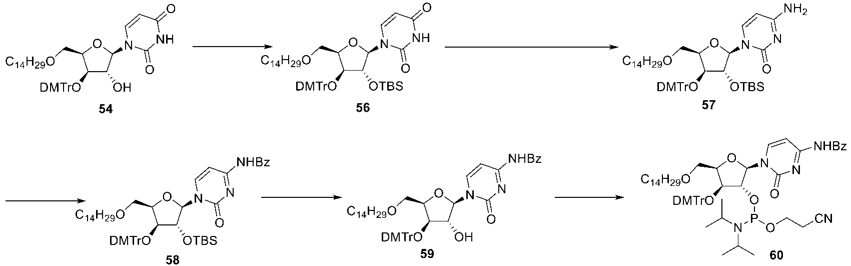

### Synthesis of Compound 56:

Compound 54 (6.0 g, 8.08 mmol, 1.0 eq) was added to a 100 mL round-bottom flask. Ultra-dry DMF (60 mL) was added to the flask with stirring. Imidazole (1.65 g, 24.24 mmol, 3.0 eq) was added at room temperature and stirred for 10 min. Then, TBSCl (1.83 g, 12.12 mmol, 1.5 eq) was slowly added to the reaction system in batches and the reaction was carried out overnight at room temperature under nitrogen protection. The complete consumption of compound 54 was monitored by TLC and LCMS. The reaction was quenched by adding water. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 2/1) to afford compound 56 (5.6 g, 6.54 mmol, 81.0% yield).
ESI-MS: m/z 857.5 [M+H]⁺

### Synthesis of Compound 57:

Compound 56 (5.6 g, 6.54 mmol, 1.0 eq) was added to a 250 mL round-bottom flask. Ultra-dry ACN (60 mL) was added to the flask with stirring to completely dissolve the compound. Triethylamine (1.32 g, 13.08 mmol, 2.0 eq) and DMAP (1.60 g, 13.08 mmol, 2.0 eq) were added to the reaction system and stirred well. The reaction was cooled to 0-5 °C, and the compound TPSCl (3.96 g, 13.08 mmol, 2.0 eq) was added slowly in batches. Once the addition was completed, the ice bath was removed, and the reaction was returned to room temperature. The reaction was stirred overnight at room temperature. The complete consumption of compound 56 was monitored by TLC. At room temperature, aqueous ammonia (40 mL) was added to the reaction and stirred for about 12 hours until the intermediate was completely consumed. Saturated brine was added to the reaction, and the mixed system was extracted twice with ethyl acetate. The organic phases were combined. The organic phase was washed with water and saturated brine, followed by drying over anhydrous sodium sulfate and concentration under reduced pressure to give the crude product 57 (9.0 g, calculated at 100% yield).
ESI-MS: m/z 856.5 [M+H]⁺

### Synthesis of Compound 58:

Crude compound 57 (9.0 g, 1.0 eq) was added to a 100 mL round-bottom flask, and pyridine (50 mL) was added to the flask with stirring to completely dissolve the crude product 57. The reaction system was cooled to 0 °C, and BzCl (1.38 g, 9.81 mmol, 1.5 eq) was slowly added dropwise to the reaction system and stirred at 0 °C for 1 hour until the compound 57 was completely consumed. The reaction was protected by nitrogen throughout. The reaction system was returned to room temperature, and the reaction was quenched by the addition of methanol and water. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA = 1/1) to afford compound 58 (5.5 g, 5.73 mmol, 87.6% overall yield in 2 steps).
ESI-MS: m/z 960.6 [M+H]⁺

### Synthesis of Compound 59:

Compound 58 (5.5 g, 5.73 mmol, 1.0 eq) was added to a 250 mL round-bottom flask and THF (60 mL) was added with stirring until completely dissolved. Triethylamine trihydrofluoride (6.0 mL) was neutralized to alkaline with triethylamine (20 mL) and added to the above reaction system. The reaction was placed in a 40°C oil bath and stirred overnight under nitrogen protection. The complete consumption of compound 58 was monitored by TLC and LCMS. Water was added to the reaction and the mixed system was extracted twice with ethyl acetate. The organic phases were combined. The organic phase was washed with water, saturated aqueous sodium bicarbonate solution and saturated brine, followed by drying over anhydrous sodium sulfate and removal of the solvent under reduced pressure to give the crude product. The crude product was subjected to column chromatography (PE/EA= 1/1) to afford compound 59 (4.6 g, 5.44 mmol, 94.9% yield).
ESI-MS: m/z 846.5 [M+H]⁺

### Synthesis of Compound 60:

Dried compound 59 (1.0 g, 1.18 mmol, 1.0 eq) was added to a 100 mL round-bottom flask, and ultra-dry DCM (15 mL) was added to the flask with stirring to dissolve the compound. DIPEA (305.1 mg, 2.36 mmol, 2.0 eq) and DMAP (28.8 mg, 0.236 mmol, 0.2 eq) were added to the reaction, and the reaction was purged with nitrogen. The reaction was cooled to around 0 °C, and CEP-Cl (418.9 mg, 1.77 mmol, 1.5 eq) was slowly added dropwise. The reaction was carried out at this temperature and under nitrogen protection for 1 hour until the compound 59 was completely consumed. After completion of the reaction, the reaction was quenched by addition of saturated aqueous sodium bicarbonate to the reaction. The mixed system was extracted twice with dichloromethane, and the organic phases were combined. The organic phase was washed with water and saturated brine, followed by drying over anhydrous sodium sulfate and removal of the solvent under reduced pressure to give the crude product. The crude product was purified by column chromatography (PE/EA = 1/1) to afford compound 60 (1.1 g, 1.05 mmol, 89.0% yield).
¹H NMR (400 MHz, DMSO-*d*₆) *δ* 11.25 (d, *J =* 24.4 Hz, 1H), 8.46 (dd, *J =* 30.5, 7.3 Hz, 1H), 8.01 (d, *J =* 7.7 Hz, 2H), 7.66 - 7.10 (m, 14H), 6.89 - 6.79 (m, 4H), 5.75 - 5.66 (m, 1H), 4.27 - 3.87 (m, 3H), 3.81 - 3.67 (m, 8H), 3.66 - 3.35 (m, 7H), 2.77 - 2.70 (m, 1H), 2.57 - 2.32 (m, 1H), 1.62 - 1.49 (m, 2H), 1.36 - 1.19 (m, 22H), 1.13 - 1.02 (m, 9H), 0.96 (d, *J =* 6.7 Hz, 3H), 0.83 (t, *J =* 6.8 Hz, 3H); ³¹P NMR (162 MHz, DMSO-*d*₆) *δ* 149.86, 149.33; ESI-MS: m/z 1046.6 [M+H]⁺

### Example 11: Synthesis of Compound 65

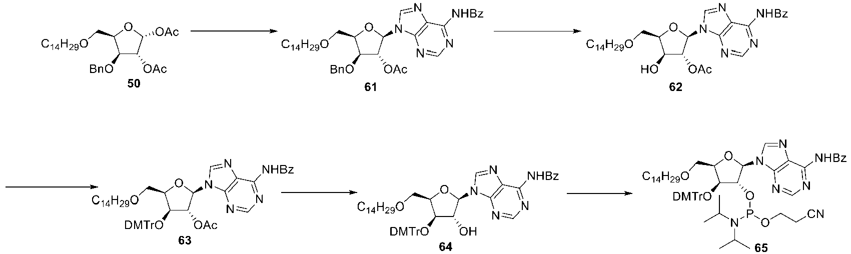

### Synthesis of Compound 61:

Compound 50 (8.0 g, 15.37 mmol, 1.00 eq) was added to a 250 mL three-necked round-bottom flask, and ultra-dry ACN (80 mL) was added to the flask with stirring to dissolve the compounds. Subsequently, BSA (9.38 g, 46.11 mmol, 3.0 eq) and A(Bz)(7.35 g, 30.74 mmol, 2.0 eq) were added. The reaction system was placed in an oil bath and heated to 80 °C. The reaction system was stirred at this temperature for 1 hour. The entire process was conducted under a nitrogen atmosphere. When the reaction was completed, the reaction system was placed in an ice water bath at 0°C with stirring for 30 min. TMSOTf (3.4 g, 15.37 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound 50 was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product 61. The crude product was subjected to column chromatography (PE/EA= 1/1) to afford compound 61 (7.8 g, 11.15 mmol, 72.6% yield).
ESI-MS: m/z 700.4 [M+H]⁺

### Synthesis of Compound 62:

Compound 61 (7.8 g, 11.15 mmol, 1.0 eq) was added to a 250 mL three-necked flask and ultra-dry DCM (80 mL) was added to the reaction flask. The reaction system was cooled to -20 °C and stirred at this temperature for 30 min. 1.0 mol/L of boron trichloride in dichloromethane (40 mL, 39.03 mmol, 3.5 eq) was slowly added dropwise to the reaction system and the reaction continued for 5 hours until the compound 61 was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product 62. The crude product was subjected to column chromatography (PE/EA= 1/2) to afford compound 62 (5.5 g, 9.03 mmol, 80.9% yield).
ESI-MS: m/z 610.4 [M+H]⁺

### Synthesis of Compound 63:

Compound 62 (5.5 g, 9.03 mmol, 1.0eq) was added to a 250 mL round-bottom flask. Ultra-dry DCE (60 mL) was added to the reaction flask with stirring to completely dissolve the compound. DMTrCl (7.65 g, 22.58 mmol, 2.5 eq), AgNO₃ (1.53 g, 9.03 mmol, 1.0 eq), 2,4,6-trimethylpyridine (10.94 g, 90.3 mmol, 10.0 eq) were added to the reaction at room temperature and stirred well. The reaction was placed in an oil bath and heated to 80°C, then carried out overnight. The complete consumption of compound 62 was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give a crude compound 63. The crude product was subjected to column chromatography (PE/EA= 2/3) to afford compound 63 (6.6 g, 7.24 mmol, 80.2% yield).
ESI-MS: m/z 912.5 [M+H]⁺

### Synthesis of Compound 64:

Compound 63 (6.6 g, 7.24 mmol, 1.0 eq) was added to a 250 mL round-bottom flask. THF (70 mL) was added to the reaction, and the reaction was stirred until completely dissolved. To the reaction was added 5.4 mol/L NaOMe solution (2.01 mL, 10.86 mmol, 1.5 eq). The reaction was stirred at room temperature for 3 hours until the compound 63 was completely consumed. After completion of the reaction, the reaction system was concentrated under reduced pressure at 40°C to give the crude product 64. The crude product was subjected to column chromatography to afford compound 64 (5.8 g, 6.67 mmol, 92.1% yield).
ESI-MS: m/z 870.5 [M+H]⁺

### Synthesis of Compound 65:

Dried compound 64 (1.0 g, 1.15 mmol, 1.0 eq) was added to a 50 mL round-bottom flask. Ultra-dry DCM (10 mL) was added to the flask with stirring to completely dissolve the compound. DIPEA (297.3 g, 2.3 mmol, 2.0 eq) and DMAP (28.1 mg, 0.23 mmol, 0.2 eq) were added to the reaction and stirred for 15 min at room temperature. The reaction system was purged with nitrogen and carried out under a nitrogen protection. Compound CEP-Cl (408.3 mg, 1.73 mmol, 1.5 eq) was added dropwise to the reaction at room temperature. The reaction was carried out at room temperature for 30-60 min until the compound 64 was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product 65. The crude product was purified by column chromatography to afford compound 65 (1.0 g, 0.93 mmol, 80.9% yield).
¹H NMR (400 MHz, DMSO-*d*₆) *δ* 11.20 (d, *J =* 4.0 Hz, 1H), 8.70 (d, *J =* 5.9 Hz, 1H), 8.61 (d, *J* = 29.9 Hz, 1H), 8.05 (d, *J =* 7.6 Hz, 2H), 7.64 (t, *J =* 7.3 Hz, 1H), 7.54 (t, *J =* 7.6 Hz, 2H), 7.37 (t, *J* = 8.6 Hz, 2H), 7.26 - 7.18 (m, 7H), 6.84 - 6.72 (m, 4H), 6.05 (dd, *J =* 36.3, 3.4 Hz, 1H), 4.79 - 4.32 (m, 2H), 3.80 - 3.35 (m, 15H), 2.69 - 2.39 (m, 2H), 1.62 - 1.47 (m, 2H), 1.40 - 1.20 (m, 22H), 1.11 - 0.97 (m, 9H), 0.92 - 0.79 (m, 6H); ³¹P NMR (162 MHz, DMSO-*d*₆) *δ* 151.29, 149.24; ESI-MS: m/z 1070.6 [M+H]⁺

### Example 12: Synthesis of Compound 70

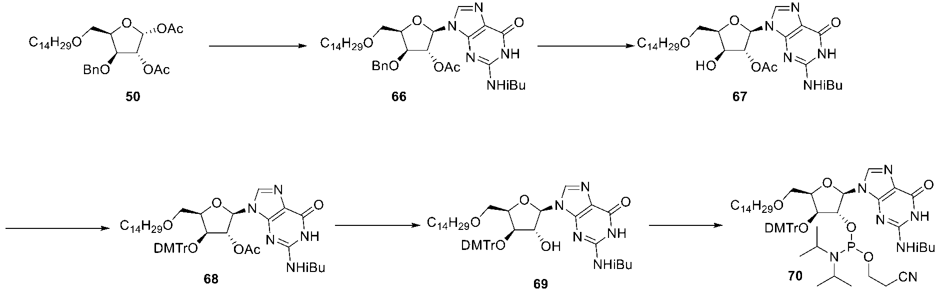

### Synthesis of Compound 66:

Compound 50 (10.0 g, 19.22 mmol, 1.00 eq) was added to a 250 mL three-necked round-bottom flask, and ultra-dry ACN (80 mL) was added to the flask with stirring to dissolve the compounds. Subsequently, BSA (11.73 g, 57.66 mmol, 3.0 eq) and G(iBu) (8.5 g, 38.44 mmol, 2.0 eq) were added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 50 min. TMSOTf (4.27 g, 19.22 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound 50 was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product 66. The crude product was subjected to column chromatography (PE/EA= 1/1) to afford compound 66 (7.8 g, 11.45 mmol, 59.6% yield).
ESI-MS: m/z 682.4 [M+H]⁺

### Synthesis of Compound 67:

Compound 66 (7.8 g, 11.45 mmol, 1.0 eq) was added to a 250 mL three-necked flask and ultra-dry DCM (80 mL) was added to the reaction flask. The reaction system was cooled to -20 °C and stirred at this temperature for 30 min. 1.0 mol/L of BCl₃ in dichloromethane (40.1 mL, 40.1 mmol, 3.5 eq) was slowly added dropwise to the reaction system and the reaction continued for 5 hours until the compound 66 was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product 67. The crude product was subjected to column chromatography (PE/EA= 1/2) to afford compound 67 (3.8 g, 6.43 mmol, 56.2% yield).
ESI-MS: m/z 592.4 [M+H]⁺

### Synthesis of Compound 68:

Compound 67 (3.8 g, 6.43 mmol, 1.0eq) was added to a 250 mL round-bottom flask. Ultra-dry DCE (50 mL) was added to the reaction flask with stirring to completely dissolve the compound. DMTrCl (5.45 g, 16.1 mmol, 2.5 eq), AgNO₃ (1.1 g, 6.43 mmol, 1.0 eq), 2,4,6-trimethylpyridine (7.8 g, 64.3 mmol, 10.0 eq) were added to the reaction at room temperature and stirred well. The reaction was placed in an oil bath and heated to 80°C, then carried out overnight. The complete consumption of compound 67 was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give a crude compound 68. The crude product was subjected to column chromatography (PE/EA= 2/3) to afford compound 68 (1.5 g, 1.68 mmol, 26.1% yield).
ESI-MS: m/z 894.5 [M+H]⁺

### Synthesis of Compound 69:

Compound 68 (1.5 g, 1.68 mmol, 1.0 eq) was added to a 100 mL round-bottom flask. THF (30 mL) was added to the reaction, and the reaction was stirred until completely dissolved. To the reaction was added 5.4 mol/L NaOMe solution (0.46 mL, 2.52 mmol, 1.5 eq). The reaction was stirred at room temperature for 3 hours until the compound 68 was completely consumed. After completion of the reaction, the reaction system was concentrated under reduced pressure at 40°C to give the crude product. The crude product was subjected to column chromatography to afford compound 69 (0.7 g, 0.82 mmol, 48.8% yield).
ESI-MS: m/z 852.5 [M+H]⁺

### Synthesis of Compound 70:

Dried compound 69 (0.7 g, 0.82 mmol, 1.0 eq) was added to a 25 mL round-bottom flask. Ultra-dry DCM (10 mL) was added to the flask with stirring to completely dissolve the compound. DIPEA (212.0 g, 1.64 mmol, 2.0 eq) and DMAP (20.04 mg, 0.164 mmol, 0.2 eq) were added to the reaction and stirred for 15 min at room temperature. The reaction system was purged with nitrogen and carried out under a nitrogen protection. Compound CEP-Cl (291.1 mg, 1.23 mmol, 1.5 eq) was added dropwise to the reaction at room temperature. The reaction was carried out at room temperature for 30-60 min until the compound 69 was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product 70. The crude product was purified by column chromatography to afford compound 70 (0.56 g, 0.53 mmol, 64.6% yield).
¹H NMR (400 MHz, DMSO-*d*₆) *δ* 12.09 (s, 1H), 11.53 (s, 1H), 8.19 (d, *J =* 29.6 Hz, 1H), 7.53 - 7.12 (m, 9H), 6.92 - 6.82 (m, 4H), 5.73 (dd, *J =* 38.6, 4.8 Hz, 1H), 5.22 - 5.05 (m, 1H), 4.54 (t, *J=* 5.5 Hz, 1H), 3.80 - 3.68 (m, 8H), 3.62 - 3.35 (m, 3H), 3.31 - 3.25 (m, 2H), 3.12 (d, *J* = 8.4 Hz, 1H), 2.78 - 2.68 (m, 1H), 2.57 (m, 2H), 2.47 - 2.38 (m, 1H), 1.50 (d, *J =* 6.6 Hz, 2H), 1.37 - 1.19 (m, 22H), 1.15 - 0.99 (m, 13H), 0.89 - 0.75 (m, 8H); ³¹P NMR (162 MHz, DMSO-*d*₆) *δ* 151.78, 148.87; ESI-MS: m/z 1052.6 [M+H]⁺

### Example 13: Synthesis of Compound 77

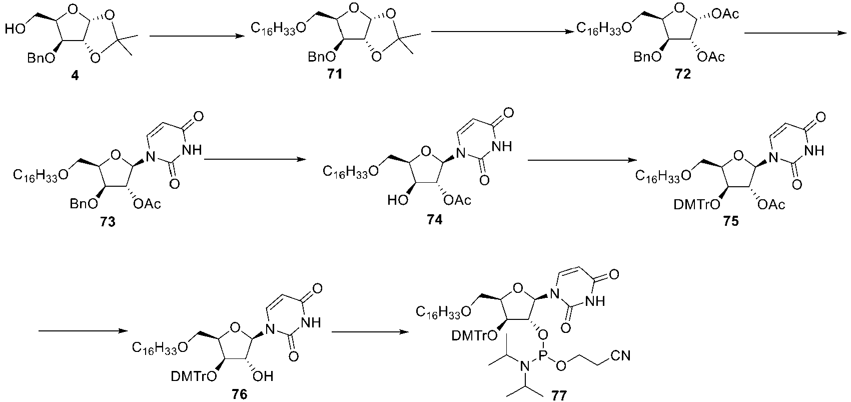

### Synthesis of Compound 71:

Compound 4 (35 g, 124.85 mmol, 1.0 eq) was dissolved in DMF (350 mL) and stirred well. The reaction was cooled to 0 °C and stirred for 30 min, and then NaH (7.5 g, 187.28 mmol, 1.5 eq) was added. Compound bromohexadecane (45.75 g, 149.82 mmol, 1.2 eq) was added slowly dropwise to the reaction system. Once the addition was completed, the reaction was returned to room temperature and carried out overnight at room temperature. The complete consumption of compound 4 was monitored by TLC. The reaction system was poured into ice water to quench the reaction completely. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 100/9) to afford compound 71 (57.5 g, 114.0 mmol, 91.3% yield).
ESI-MS: m/z 505.4 [M+H]⁺

### Synthesis of Compound 72:

Compound 71 (57.5 g, 114.0 mmol, 1.0 eq) was added to a 1000 mL round-bottom three-necked flask, and AcOH (535 mL) and Ac₂O (58.19 g, 569.98 mmol, 5.0 eq) were added to the reaction system and stirred to completely dissolve the compound. The reaction was cooled to 0°C and stirred at this temperature for 30 min. Concentrated sulfuric acid (5.35 mL) was added slowly dropwise to the reaction system, and the reaction system was kept at a temperature of 0-5°C at all times. Once the addition was completed, the reaction system was returned to room temperature and stirred for additional 8 hours until the compound 71 was completely consumed. The reaction system was cooled to around -5°C and neutralized to a pH of about 7 with aqueous ammonia. Water was added to the reaction system, and the reaction system was extracted twice with ethyl acetate. The organic phases were combined. The organic phase was washed with water and saturated brine, followed by drying over anhydrous sodium sulfate and concentration under reduced pressure to give a crude product 72. The crude product was subjected to column chromatography (PE/EA= 10/2) to afford compound 72 (47.5 g, 86.6 mmol, 76% yield).
ESI-MS: m/z 549.4 [M+H]⁺

### Synthesis of Compound 73:

Compound 72 (20.3 g, 37.02 mmol, 1.00 eq) and uracil (8.3 g, 74.04 mmol, 2.0 eq) were added to a 500 mL three-necked round-bottom flask, and ultra-dry ACN (200 mL) was added to the flask with stirring to dissolve the compounds. Subsequently, BSA (22.59 g, 111.06 mmol, 3.0 eq) was added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 30 min. TMSOTf (8.23 g, 37.02 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound 72 was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product 73. The crude product was subjected to column chromatography (PE/EA= 5/2) to afford compound 73 (20.0 g, 33.3 mmol, 90% yield).
ESI-MS: m/z 601.4 [M+H]⁺

### Synthesis of Compound 74:

Compound 73 (10.0 g, 16.66 mmol, 1.0eq) was added to a 250 mL three-necked flask and ultra-dry DCM (100 mL) was added to the reaction flask. The reaction system was cooled to -20 °C and stirred at this temperature for 30 min. Boron trichloride (1M in toluene, 66.7 mL, 66.64 mmol, 4.0 eq) was slowly added dropwise to the reaction system and the reaction continued for 5 hours until the compound 73 was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product 74. The crude product was subjected to column chromatography (PE/EA= 2/3) to afford compound 74 (5.66 g, 11.09 mmol, 66.6% yield).
ESI-MS: m/z 511.3 [M+H]⁺

### Synthesis of Compound 75:

Compound 74 (5.6 g, 11.09 mmol, 1.0 eq) was added to a 250 mL round-bottom flask. Ultra-dry DCE (70 mL) was added to the reaction flask with stirring to completely dissolve the compound. DMTrCl (7.52 g, 22.18 mmol, 2.0 eq), AgNO₃ (1.87 g, 11.09 mmol, 1.0 eq), 2,4,6-trimethylpyridine (13.4 g, 110.9 mmol, 10.0 eq) were added to the reaction at room temperature and stirred well. The reaction was placed in an oil bath and heated to 80°C, then carried out overnight. The complete consumption of compound 74 was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give a crude compound 75. The crude product was subjected to column chromatography (PE/EA= 100/35) to afford compound 75 (8.9 g, 10.95 mmol, 98.8% yield).
ESI-MS: m/z 813.5 [M+H]⁺

### Synthesis of Compound 76:

Compound 75 (8.9 g, 10.95 mmol, 1.0 eq) was added to a 250 round-bottom flask. Then aqueous ammonia (70 mL) was added to the reaction at room temperature. The reaction was stirred at room temperature for 3 hours until the compound 75 was completely consumed. After completion of the reaction, the reaction system was concentrated under reduced pressure at 40°C to give the crude product 76. The crude product was subjected to column chromatography (PE/EA= 1/1) to afford compound 76 (7.3 g, 9.47 mmol, 86.5% yield).
ESI-MS: m/z 771.5 [M+H]⁺

### Synthesis of Compound 77:

Dried compound 76 (3.3 g, 4.28 mmol, 1.0 eq) was added to a 100 mL round-bottom flask. Ultra-dry DCM (35 mL) was added to the flask with stirring to completely dissolve the compound. DIPEA (1.11 g, 8.56 mmol, 2.0 eq) and DMAP (105 mg, 0.856 mmol, 0.2 eq) were added to the reaction and stirred for 15 min at room temperature. The reaction system was purged with nitrogen and carried out under a nitrogen protection. Compound CEP-Cl (1.52 g, 6.42 mmol, 1.5 eq) was added dropwise to the reaction at room temperature. The reaction was carried out at room temperature for 30-60 min until the compound 76 was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product 77. The crude product was purified by column chromatography (PE/EA= 1/1) to afford compound 77 (3.9 g, 4.02 mmol, 93.9% yield).
¹H NMR (400 MHz, DMSO-*d*₆) *δ* 11.28 (d, *J* = 14.5 Hz, 1H), 7.90 (d, *J =* 8.1 Hz, 1H), 7.35 (dd, *J* = 16.0, 7.5 Hz, 2H), 7.30 - 7.16 (m, 7H), 6.88 (t, *J =* 8.5 Hz, 4H), 5.77 - 5.55 (m, 2H), 4.42 - 4.32 (m, 1H), 4.11 - 3.95 (m, 1H), 3.84 - 3.68 (m, 8H), 3.62 - 3.35 (m, 7H), 2.68 (t, *J* = 6.0 Hz, 1H), 2.55 - 2.38 (m, 1H), 1.65 - 1.55 (m, 2H), 1.43 - 1.23 (m, 26H), 1.09 (t, *J =* 6.5 Hz, 6H), 1.01 (d, *J* = 6.7 Hz, 6H), 0.83 (t, *J =* 6.5 Hz, 3H); ³¹P NMR (162 MHz, DMSO-*d*₆) *δ* 150.13, 148.46; ESI-MS: m/z 971.6 [M+H]⁺

### Example 14: Synthesis of Compound 82

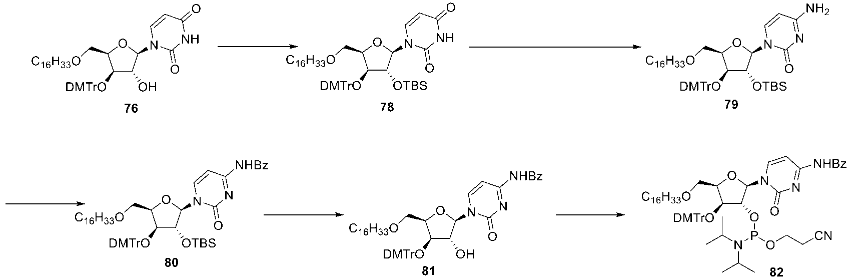

### Synthesis of Compound 78:

Compound 76 (4.0 g, 5.19 mmol, 1.0 eq) was added to a 100 mL round-bottom flask. Ultra-dry DMF (40 mL) was added to the flask with stirring. Imidazole (1.06 g, 15.57 mmol, 3.0 eq) was added at room temperature and stirred for 10 min. Then, TBSCl (1.17 g, 7.79 mmol, 1.5 eq) was slowly added to the reaction system in batches and the reaction was carried out overnight at room temperature under nitrogen protection. The complete consumption of compound 76 was monitored by TLC and LCMS. The reaction was quenched by adding water. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 2/1) to afford compound 78 (2.9 g, 3.28 mmol, 63% yield).
ESI-MS: m/z 885.5 [M+H]⁺

### Synthesis of Compound 79:

Compound 78 (2.9 g, 3.28 mmol, 1.0 eq) was added to a 100 mL round-bottom flask. Ultra-dry ACN (30 mL) was added to the flask with stirring to completely dissolve the compound. TEA (663 mg, 6.56 mmol, 2.0 eq) and DMAP (801 mg, 6.56 mmol, 2.0 eq) were added to the reaction system and stirred well. The reaction was cooled to 0-5 °C, and the compound TPSCl (1.98 g, 6.56 mmol, 2.0 eq) was added slowly in batches. Once the addition was completed, the ice bath was removed, and the reaction was returned to room temperature. The reaction was stirred overnight at room temperature. The complete consumption of compound 78 was monitored by TLC. At room temperature, aqueous ammonia (20 mL) was added to the reaction and stirred for about 12 hours until the intermediate was completely consumed. Saturated brine was added to the reaction, and the mixed system was extracted twice with ethyl acetate. The organic phases were combined. The organic phase was washed with water and saturated brine, followed by drying over anhydrous sodium sulfate and concentration under reduced pressure to give the crude product 79 (4.3 g, calculated at 100% yield).
ESI-MS: m/z 884.6 [M+H]⁺

### Synthesis of Compound 80:

Crude compound 79 (4.3 g, 1.0 eq) was added to a 100 mL round-bottom flask, and pyridine (50 mL) was added to the flask with stirring to completely dissolve the crude product 79. The reaction system was cooled to 0 °C, and BzCl (922 mg, 6.56 mmol, 2.0 eq) was slowly added dropwise to the reaction system and stirred at 0 °C for 1 hour until the compound 79 was completely consumed. The reaction was protected by nitrogen throughout. The reaction system was returned to room temperature, and the reaction was quenched by the addition of methanol and water. The mixed system was extracted twice with ethyl acetate and the organic phases were combined. The organic phase was washed with water and saturated brine, followed by drying over anhydrous sodium sulfate and removal of the solvent under reduced pressure to give the crude product. The crude product was subjected to column chromatography (PE/EA = 1/1) to afford compound 80 (2.0 g, 2.03 mmol, 61.8% overall yield in 2 steps).
ESI-MS: m/z 988.6 [M+H]⁺

### Synthesis of Compound 81:

Compound 80 (2.0 g, 2.03 mmol, 1.0 eq) was added to a 100 mL round-bottom flask and THF (20 mL) was added with stirring until completely dissolved. Triethylamine trihydrofluoride (5.0 mL) was neutralized to alkaline with triethylamine (17 mL) and added to the above reaction system. The reaction was placed in a 40°C oil bath and stirred overnight under nitrogen protection. The complete consumption of compound 80 was monitored by TLC and LCMS. Water was added to the reaction, and the mixed system was extracted twice with ethyl acetate. The organic phases were combined. The organic phase was washed with water, saturated aqueous sodium bicarbonate solution and saturated brine, followed by drying over anhydrous sodium sulfate and removal of the solvent under reduced pressure to give the crude product. The crude product was subjected to column chromatography (PE/EA= 1/1) to afford compound 81 (1.37 g, 1.57 mmol, 77.3% yield).
ESI-MS: m/z 874.5 [M+H]⁺

### Synthesis of Compound 82:

Dried compound 81 (1.37 g, 1.57 mmol, 1.0 eq) was added to a 100 mL round-bottom flask, and ultra-dry DCM (15 mL) was added to the flask with stirring to dissolve the compound. DIPEA (405 mg, 3.14 mmol, 2.0 eq) and DMAP (38.4 mg, 0.314 mmol, 0.2 eq) were added to the reaction, and the reaction was purged with nitrogen. The reaction was cooled to around 0 °C, and CEP-Cl (557.4 mg, 2.35 mmol, 1.5 eq) was slowly added dropwise. The reaction was carried out at this temperature and under nitrogen protection for 1 hour until the compound 81 was completely consumed. After completion of the reaction, the reaction was quenched by addition of saturated aqueous sodium bicarbonate to the reaction. The mixed system was extracted twice with dichloromethane, and the organic phases were combined. The organic phase was washed with water and saturated brine, followed by drying over anhydrous sodium sulfate and removal of the solvent under reduced pressure to give the crude product. The crude product was purified by column chromatography (PE/EA = 1/1) to afford compound 82 (1.0 g, 0.93 mmol, 59.2% yield).
¹H NMR (400 MHz, DMSO-*d*₆) *δ* 11.24 (d, *J* = 24.2 Hz, 1H), 8.55 - 8.39 (m, 1H), 8.01 (d, *J* = 7.7 Hz, 2H), 7.66 - 7.10 (m, 14H), 6.92 - 6.73 (m, 4H), 5.70 (d, *J =* 26.4 Hz, 1H), 4.25 - 3.88 (m, 3H), 3.81 - 3.35 (m, 15H), 2.73 (dd, *J =* 10.4, 5.6 Hz, 1H), 2.61 - 2.31 (m, 1H), 1.55 (dt, *J* = 14.1, 7.1 Hz, 2H), 1.37 - 1.18 (m, 26H), 1.13 - 0.94 (m, 12H), 0.84 (t, *J =* 6.5 Hz, 3H); ³¹P NMR (162 MHz, DMSO-*d*₆) *δ* 149.86, 149.32; ESI-MS: m/z 1074.6 [M+H]⁺

### Example 15: Synthesis of Compound 87

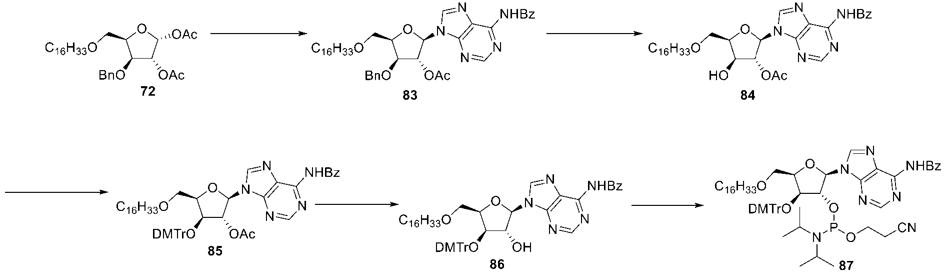

### Synthesis of Compound 83:

Compound 72 (17 g, 31.0 mmol, 1.00 eq) was added to a 500 mL three-necked round-bottom flask, and ultra-dry ACN (200 mL) was added to the flask with stirring to dissolve the compounds. Subsequently, BSA (18.91 g, 93 mmol, 3.0 eq) and A(Bz)(14.83 g, 62.0 mmol, 2.0 eq) were added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 30 min. TMSOTf (6.89 g, 31.0 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound 72 was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product 83. The crude product was subjected to column chromatography (PE/EA= 1/1) to afford compound 83 (18.0 g, 24.74 mmol, 79.8% yield).
ESI-MS: m/z 728.4 [M+H]⁺

### Synthesis of Compound 84:

Compound 83 (18.0 g, 24.74 mmol, 1.0 eq) was added to a 500 mL three-necked flask and ultra-dry DCM (300 mL) was added to the reaction flask. The reaction system was cooled to -20 °C and stirred at this temperature for 30 min. 1.0 mol/L of boron trichloride in dichloromethane (74.2 mL, 74.22 mmol, 3.0 eq) was slowly added dropwise to the reaction system and the reaction continued for 5 hours until the compound 83 was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product 84. The crude product was subjected to column chromatography (PE/EA= 1/2) to afford compound 84 (10.0 g, 15.68 mmol, 63.4% yield).
ESI-MS: m/z 638.4 [M+H]⁺

### Synthesis of Compound 85:

Compound 84 (10.0 g, 15.68 mmol, 1.0eq) was added to a 500 mL round-bottom flask. Ultra-dry DCE (150 mL) was added to the reaction flask with stirring to completely dissolve the compound. DMTrCl (10.63 g, 31.36 mmol, 2.0 eq), AgNO₃ (2.66 g, 15.68 mmol, 1.0 eq), 2,4,6-trimethylpyridine (19 g, 156.8 mmol, 10.0 eq) were added to the reaction at room temperature and stirred well. The reaction was placed in an oil bath and heated to 80°C, then carried out overnight. The complete consumption of compound 84 was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give a crude compound 85. The crude product was subjected to column chromatography (PE/EA= 2/3) to afford compound 85 (13.0 g, 13.84 mmol, 88.3% yield).
ESI-MS: m/z 940.5 [M+H]⁺

### Synthesis of Compound 86:

Compound 85 (13.0 g, 13.84 mmol, 1.0 eq) was added to a 250 mL round-bottom flask. THF (130 mL) was added to the reaction, and the reaction was stirred until completely dissolved. To the reaction was added 5.4 mol/L NaOMe solution (3.84 mL, 20.76 mmol, 1.5 eq). The reaction was stirred at room temperature for 3 hours until the compound 85 was completely consumed. After completion of the reaction, the reaction system was concentrated under reduced pressure at 40°C to give the crude product 86. The crude product was subjected to column chromatography to afford compound 86 (10.5 g, 11.7 mmol, 84.5% yield).
ESI-MS: m/z 898.5 [M+H]⁺

### Synthesis of Compound 87:

Dried compound 86 (1.0 g, 1.11 mmol, 1.0 eq) was added to a 50 mL round-bottom flask. Ultra-dry DCM (10 mL) was added to the flask with stirring to completely dissolve the compound. DIPEA (286.9 mg, 2.22 mmol, 2.0 eq) and DMAP (26.9 mg, 0.22 mmol, 0.2 eq) were added to the reaction and stirred for 15 min at room temperature. The reaction system was purged with nitrogen and carried out under a nitrogen protection. Compound CEP-Cl (395.3 g, 1.67 mmol, 1.5 eq) was added dropwise to the reaction at room temperature. The reaction was carried out at room temperature for 30-60 min until the compound 86 was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product 87. The crude product was purified by column chromatography to afford compound 87 (1.0 g, 0.91 mmol, 82.1% yield).
¹H NMR (400 MHz, DMSO-*d*₆) *δ* 11.24 - 11.16 (m, 1H), 8.70 (d, *J* = 6.5 Hz, 1H), 8.61 (d, *J* = 30.2 Hz, 1H), 8.05 (d, *J =* 7.6 Hz, 2H), 7.64 (t, *J* = 7.3 Hz, 1H), 7.54 (t, *J =* 7.5 Hz, 2H), 7.41 - 7.34 (m, 2H), 7.27 - 7.17 (m, 7H), 6.84 - 6.73 (m, 4H), 6.05 (dd, *J =* 35.6, 3.3 Hz, 1H), 4.78 - 4.30 (m, 2H), 3.81 - 3.76 (m, 1H), 3.74 - 3.37 (m, 14H), 2.66 (t, *J* = 6.1 Hz, 1H), 2.62 - 2.39 (m, 1H), 1.63 - 1.48 (m, 2H), 1.36 - 1.18 (m, 26H), 1.13 - 0.96 (m, 9H), 0.92 - 0.79 (m, 6H); ³¹P NMR (162 MHz, DMSO-*d*₆) *δ* 151.26, 149.26; ESI-MS: m/z 1098.6 [M+H]⁺

### Example 16: Synthesis of Compound 92

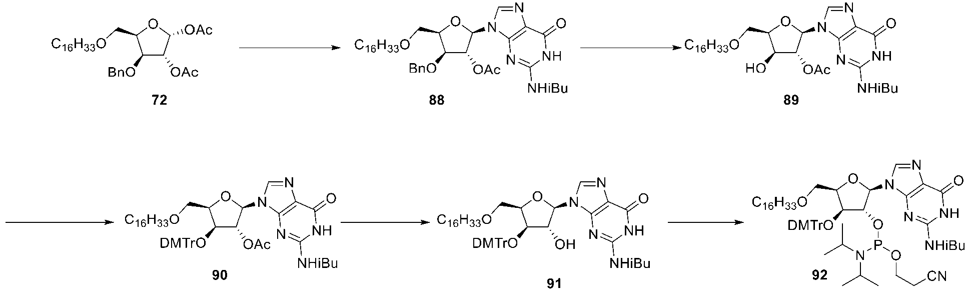

### Synthesis of Compound 88:

Compound 72 (12.0 g, 21.88 mmol, 1.00 eq) was added to a 500 mL three-necked round-bottom flask, and ultra-dry ACN (130 mL) was added to the flask with stirring to dissolve the compounds. Subsequently, BSA (13.35 g, 65.65 mmol, 3.0 eq) and G(iBu) (9.67 g, 43.76 mmol, 2.0 eq) were added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 50 min. TMSOTf (4.86 g, 21.88 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound 72 was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product 88. The crude product was subjected to column chromatography (PE/EA= 1/1) to afford compound 88 (11.2 g, 15.79 mmol, 72.2% yield).
ESI-MS: m/z 710.4 [M+H]⁺

### Synthesis of Compound 89:

Compound 88 (11.2 g, 15.79 mmol, 1.0 eq) was added to a 250 mL three-necked flask and ultra-dry DCM (100 mL) was added to the reaction flask. The reaction system was cooled to -20 °C and stirred at this temperature for 30 min. 1.0 mol/L of BCl₃ in dichloromethane (47.4 mL, 47.37mmol, 3.0 eq) was slowly added dropwise to the reaction system and the reaction continued for 5 hours until the compound 88 was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product 89. The crude product was subjected to column chromatography (PE/EA= 1/2) to afford compound 89 (6.3 g, 10.17 mmol, 64.4% yield).
ESI-MS: m/z 620.5 [M+H]⁺

### Synthesis of Compound 90:

Compound 89 (6.3 g, 10.17 mmol, 1.0 eq) was added to a 250 mL round-bottom flask. Ultra-dry DCE (70 mL) was added to the reaction flask with stirring to completely dissolve the compound. DMTrCl (6.89 g, 20.34 mmol, 2.0 eq), AgNO₃ (1.73 g, 10.17 mmol, 1.0 eq), 2,4,6-trimethylpyridine (12.3 g, 101.7 mmol, 10.0 eq) were added to the reaction at room temperature and stirred well. The reaction was placed in an oil bath and heated to 80°C, then carried out overnight. The complete consumption of compound 89 was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give a crude compound 90. The crude product was subjected to column chromatography (PE/EA= 2/3) to afford compound 90 (7.8 g, 8.46 mmol, 83.2% yield).
ESI-MS: m/z 922.5 [M+H]⁺

### Synthesis of Compound 91:

Compound 90 (7.8 g, 8.46 mmol, 1.0 eq) was added to a 250 mL round-bottom flask. THF (80 mL) was added to the reaction, and the reaction was stirred until completely dissolved. To the reaction was added 5.4 mol/L NaOMe solution (2.4 mL, 12.69 mmol, 1.5 eq). The reaction was stirred at room temperature for 3 hours until the compound 90 was completely consumed. After completion of the reaction, the reaction system was concentrated under reduced pressure at 40°C to give the crude product. The crude product was subjected to column chromatography to afford compound 91 (6.43 g, 7.31 mmol, 86.4% yield).
ESI-MS: m/z 880.5 [M+H]⁺

### Synthesis of Compound 92:

Dried compound 91 (3.0 g, 3.41 mmol, 1.0 eq) was added to a 100 mL round-bottom flask. Ultra-dry DCM (30 mL) was added to the flask with stirring to completely dissolve the compound. DIPEA (881 mg, 6.82 mmol, 2.0 eq) and DMAP (83.3 mg, 0.682 mmol, 0.2 eq) were added to the reaction and stirred for 15 min at room temperature. The reaction system was purged with nitrogen and carried out under a nitrogen protection. Compound CEP-Cl (1.21 g, 5.12 mmol, 1.5 eq) was added dropwise to the reaction at room temperature. The reaction was carried out at room temperature for 30-60 min until the compound 91 was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product 92. The crude product was purified by column chromatography to afford compound 92 (3.1 g, 2.87 mmol, 84.2% yield).
¹H NMR (400 MHz, DMSO-*d*₆) *δ* 12.05 (s, 1H), 11.45 (s, 1H), 8.23 (d, *J =* 29.6 Hz, 1H), 7.58 - 7.15 (m, 9H), 6.95 - 6.89 (m, 4H), 5.66 (dd, *J =* 38.6, 4.8 Hz, 1H), 5.25 - 5.09 (m, 1H), 4.55 (t, *J* = 5.5 Hz, 1H), 3.81 - 3.65 (m, 8H), 3.63 - 3.38(m, 3H), 3.31 - 3.21 (m, 2H), 3.10 (d, *J* = 8.4 Hz, 1H), 2.74 - 2.65 (m, 1H), 2.58 (m, 2H), 2.48 - 2.31 (m, 1H), 1.51 (d, *J =* 6.6 Hz, 2H), 1.38- 1.18 (m, 26H), 1.13 - 0.98 (m, 13H), 0.88 - 0.73 (m, 8H); ³¹P NMR (162 MHz, DMSO-*d*₆) *δ* 151.58, 148.93; ESI-MS: m/z 1080.6 [M+H]⁺

**Example 17 below is the preparation example of the nucleoside compound shown in Formula 3-a of the present invention (Synthetic route), wherein the nucleoside compound shown in Formula 3-a is a TNA structural compound with 2'-phosphoramidite moiety, 3'-CH₂ODMTr, and 4'-modification.**

### Example 17: Synthesis of Compound 104

Compound 104A was prepared by the following route:

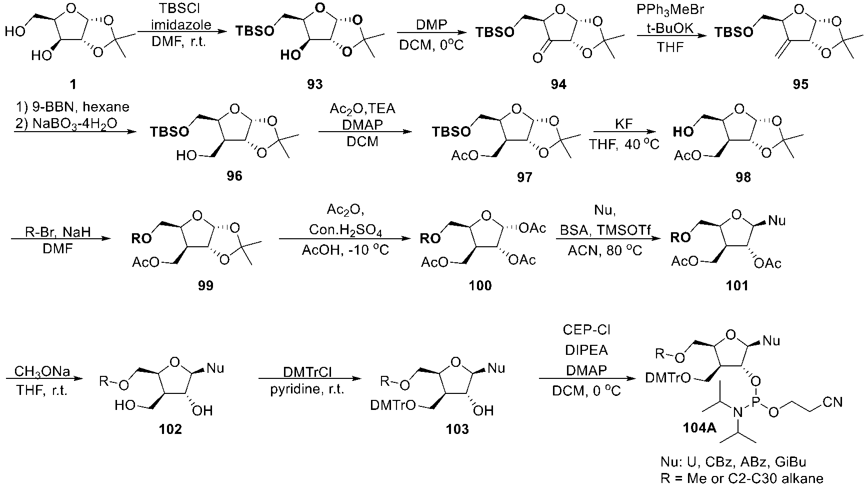

Nucleoside compound 104 is a TNA structural compound with 2'-phosphoramidite moiety, 3'-ODMTr, Nu=ABz, and R=C₁₆H₃₃.

In the above preparation route, when compound 98 was used to prepare compound 99, R-Br was selected as C₁₆H₃₃-Br; when compound 100 was used to prepare compound 101, Nu was selected as adenine A. Additionally, referring to the synthesis method of compound 87 in Example 15, the structure and nuclear magnetic resonance data of the obtained nucleoside compound 104 were presented as follows:

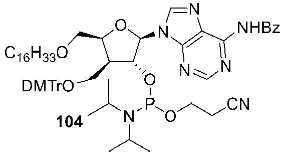

¹H NMR (400 MHz, DMSO-*d*₆) *δ* 11.24 - 11.16 (m, 1H), 8.70 (d, *J =* 6.5 Hz, 1H), 8.61 (d, *J* = 30.2 Hz, 1H), 8.05 (d, *J* = 7.6 Hz, 2H), 7.64 (t, *J* = 7.3 Hz, 1H), 7.54 (t, *J =* 7.5 Hz, 2H), 7.41 - 7.34 (m, 2H), 7.27 - 7.17 (m, 7H), 6.84 - 6.73 (m, 4H), 6.05 (dd, *J* = 35.6, 3.3 Hz, 1H), 4.78 - 4.30 (m, 2H), 4.13 - 3.88 (m, 2H), 3.81 - 3.76 (m, 1H), 3.74 - 3.37 (m, 14H), 2.66 (t, *J* = 6.1 Hz, 1H), 2.62 - 2.39 (m, 1H), 1.63 - 1.48 (m, 2H), 1.36 - 1.18 (m, 26H), 1.13 - 0.96 (m, 9H), 0.92 - 0.79 (m, 6H); ³¹P NMR (162 MHz, DMSO-*d*₆) *δ* 150.26, 148.26; ESI-MS: m/z 1112.6 [M+H]⁺

**Example 18 below is the preparation example of the nucleoside compound shown in Formula 4-a of the present invention (Synthetic route), wherein the nucleoside compound shown in Formula 4-a is a TNA structural compound with 2'-phosphoramidite moiety, 3'-ODMTr, and 4'-modification.**

### Example 18: Synthesis of Compound 116

Compound 116A was prepared by the following route:

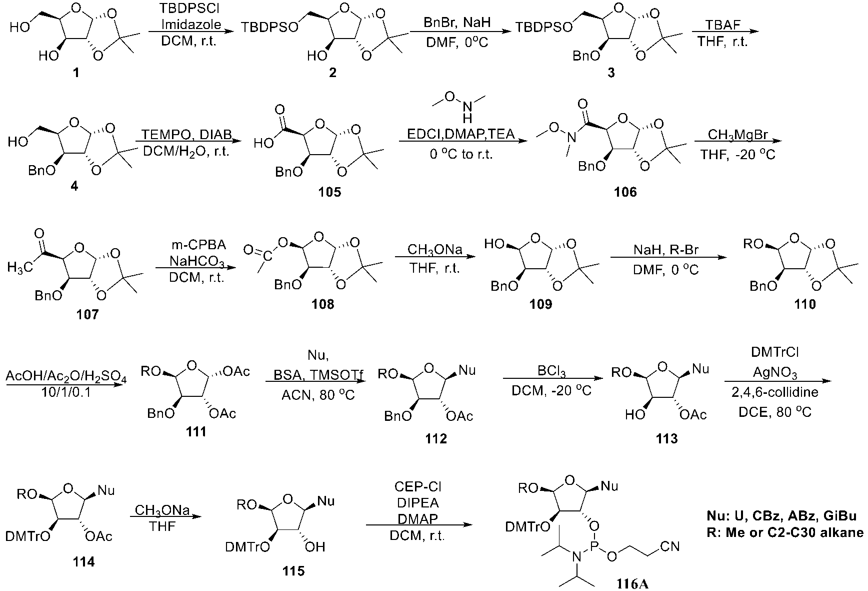

Nucleoside compound 116 is a TNA structural compound with 2'-phosphoramidite moiety, 3'-ODMTr, Nu=ABz, and R=C₁₆H₃₃.

In the above preparation route, when compound 109 was used to prepare compound 110, R-Br was selected as C₁₆H₃₃-Br; when compound 111 was used to prepare compound 112, Nu was selected as adenine A. Additionally, referring to the synthesis method of compound 87 in Example 15, the structure and nuclear magnetic resonance data of the obtained nucleoside compound 116 were presented as follows:

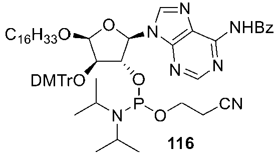

¹H NMR (400 MHz, DMSO-*d*₆) *δ* 11.22 (d, *J =* 4.0 Hz, 1H), 8.72 (d, *J =* 5.9 Hz, 1H), 8.61 (d, *J* = 29.9 Hz, 1H), 8.03 (d, *J =* 7.6 Hz, 2H), 7.64 (t, *J =* 7.3 Hz, 1H), 7.57 (t, *J =* 7.6 Hz, 2H), 7.35 (t, *J* = 8.6 Hz, 2H), 7.21 - 7.12 (m, 7H), 6.82 - 6.72 (m, 4H), 6.01 (dd, *J* = 36.3, 3.4 Hz, 1H), 4.79 - 4.32 (m, 2H), 3.83 - 3.37 (m, 17H), 2.68 - 2.37 (m, 2H), 1.63 - 1.47 (m, 2H), 1.41 - 1.21 (m, 22H), 1.13 - 0.99 (m, 9H), 0.92 - 0.77 (m, 6H). ³¹P NMR (162 MHz, DMSO-*d*₆) *δ* 151.83, 149.06. ESI-MS: m/z 1028.5 [M+H]⁺

**Example 19 below is the preparation example of the nucleoside compound shown in Formula 5-a of the present invention (Synthetic route), wherein the nucleoside compound shown in Formula 5-a is a TNA structural compound with 2'-phosphoramidite moiety, 3'-CH₂ODMTr, and 4'-modification.**

### Example 19:

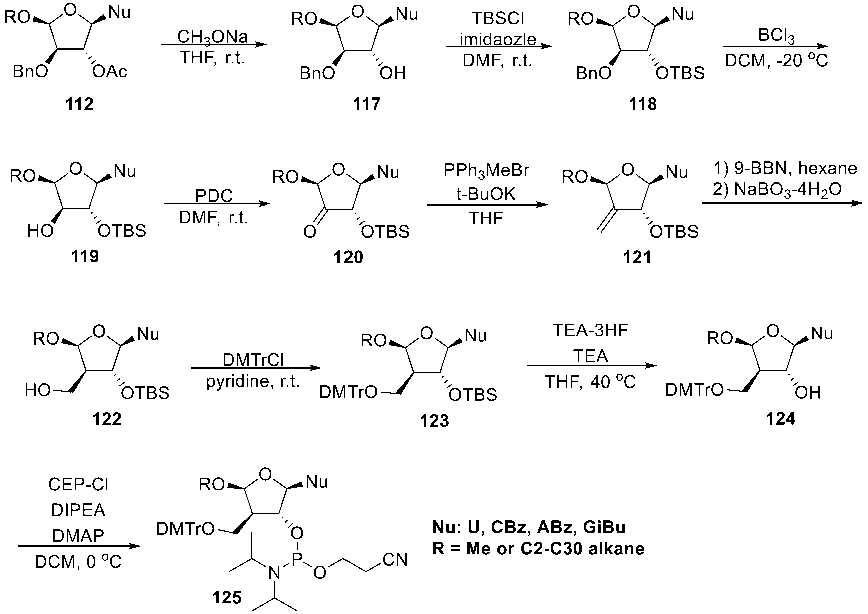

**Example 20 below is the preparation example of the nucleoside compound shown in Formula 6-a of the present invention (Synthetic route), wherein the nucleoside compound shown in Formula 6-a is a TNA structural compound with 2'-phosphoramidite moiety, 3'-ODMTr, and 4'-modification.**

### Example 20: Synthesis of Compound 135

Compound 135A was prepared by the following route:

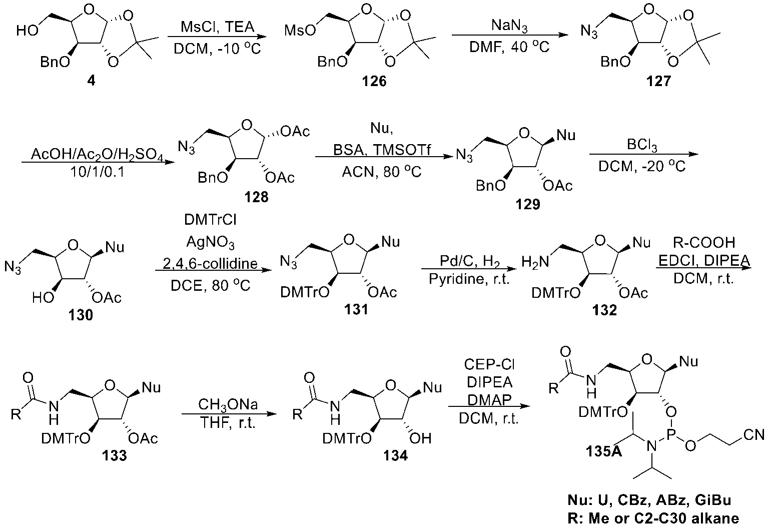

Nucleoside compound 135 is a TNA structural compound with 2'-phosphoramidite moiety, 3'-ODMTr, Nu=ABz, and R=C₁₄H₂₉.

By the above preparation route of Example 20, when compound 128 was used to prepare compound 129, Nu was selected as ABz, where A is adenine; when compound 132 was used to prepare compound 133, R-COOH was selected as C₁₄H₂₉-COOH. Additionally, referring to the synthesis of compound 87 in Example 15, the structure and nuclear magnetic resonance data of the obtained nucleoside compound 135 were presented as follows:

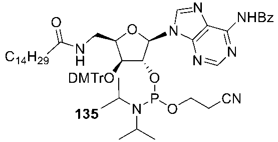

¹H NMR (400 MHz, DMSO-*d*₆) *δ* 11.21 (d, *J =* 4.0 Hz, 1H), 8.71 (d, *J =* 5.9 Hz, 1H), 8.62 (d, *J* = 29.9 Hz, 1H), 8.05 - 7.99 (m, 3H), 7.64 (t, *J =* 7.3 Hz, 1H), 7.57 (t, *J =* 7.6 Hz, 2H), 7.35 (t, *J =* 8.6 Hz, 2H), 7.21 - 7.12 (m, 7H), 6.82 - 6.72 (m, 4H), 6.01 (dd, *J =* 36.3, 3.4 Hz, 1H), 4.79 - 4.32 (m, 2H), 3.83 - 3.37 (m, 15H), 2.68 - 2.37 (m, 2H), 1.63 - 1.47 (m, 2H), 1.41 - 1.21 (m, 20H), 1.13 - 0.99 (m, 9H), 0.92 - 0.77 (m, 6H). ³¹P NMR (162 MHz, DMSO-*d*₆) *δ* 151.92, 148.91. ESI-MS: m/z 1055.6 [M+H]⁺

### Example 21: Synthesis of Compound 143

Compound 143A was prepared by the following route:

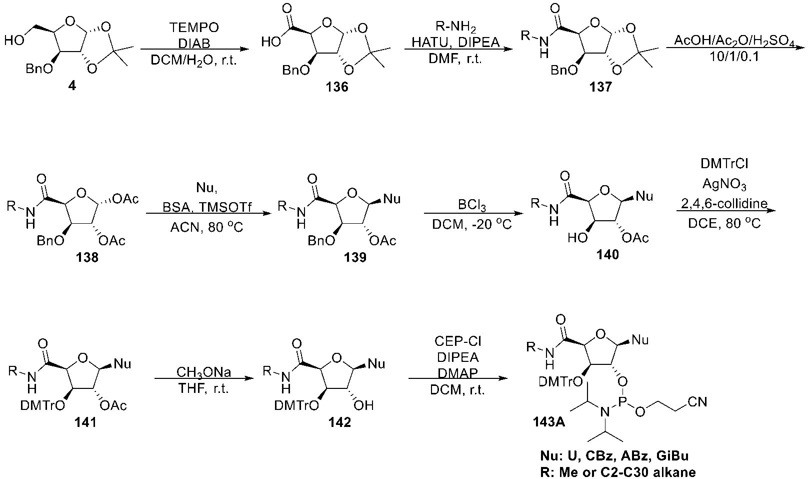

**Example 22 below is the preparation example of the nucleoside compound shown in Formula 8-a of the present invention (Synthetic route), wherein the nucleoside compound shown in Formula 8-a is a TNA structural compound with 2'-phosphoramidite moiety, 3'-ODMTr, and 4'-modification.**

### Example 22: Synthesis of Compound 154

Compound 154A was prepared by the following route:

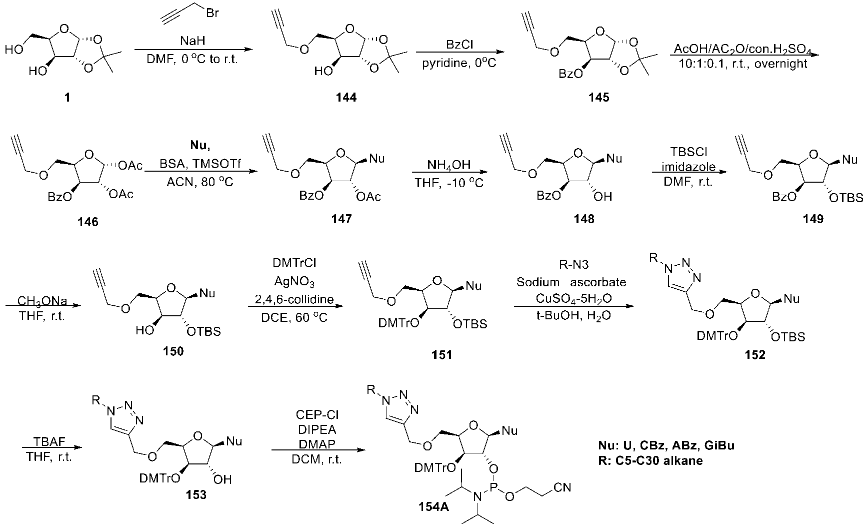

Nucleoside compound 154 is a TNA structural compound with 2'-phosphoramidite moiety, 3'-ODMTr, Nu=ABz, and R=C₁₆H₃₃.

By the above preparation route of Example 22, when compound 151 was used to prepare compound 152, R was selected as C₁₆H₃₃; when compound 146 was used to prepare compound 147, Nu was selected as ABz, where A is adenine. The structure and nuclear magnetic resonance data of the obtained nucleoside compound 154 were presented as follows:

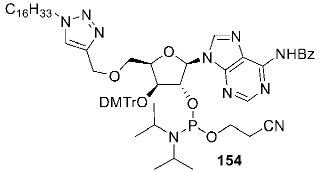

¹H NMR (400 MHz, DMSO-*d*₆) *δ* 11.21 (d, *J =* 4.0 Hz, 1H), 8.71 (d, *J =* 5.9 Hz, 1H), 8.60 (d, *J* = 29.9 Hz, 1H), 8.04 (d, *J=* 7.6 Hz, 2H), 7.65 (t, *J =* 7.3 Hz, 1H), 7.61 - 7.51 (m, 3H), 7.35 (t, *J* = 8.6 Hz, 2H), 7.21 - 7.12 (m, 7H), 6.83 - 6.71 (m, 4H), 6.02 (dd, *J* = 36.3, 3.4 Hz, 1H), 4.78 - 4.31 (m, 4H), 3.81 - 3.36 (m, 17H), 2.67 - 2.38 (m, 2H), 1.62 - 1.48 (m, 2H), 1.42 - 1.21 (m, 22H), 1.12 - 0.99 (m, 9H), 0.93 - 0.78 (m, 6H). ³¹P NMR (162 MHz, DMSO-*d*₆) *δ* 151.63, 149.23. ESI-MS: m/z 1123.6 [M+H]⁺

**Example 23 below is the preparation example of the nucleoside compound shown in Formula 9-a of the present invention (Synthetic route), wherein the nucleoside compound shown in Formula 9-a is a TNA structural compound with 2'-phosphoramidite moiety, 3'-CH₂ODMTr, and 4'-modification.**

### Example 23:

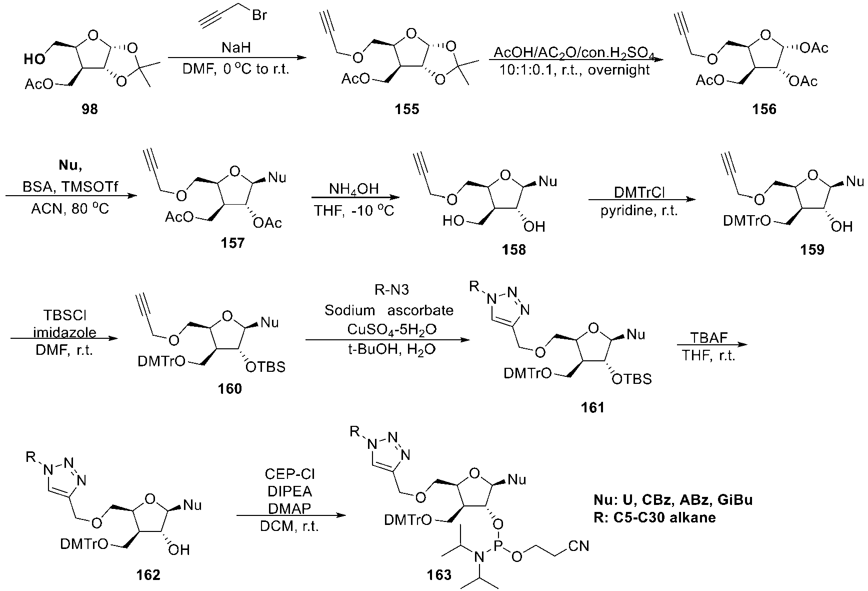

Example 24 below is the preparation example of the nucleoside compound shown in **Formula 10-a of the present invention (Synthetic route), wherein the nucleoside compound shown in Formula 10-a is a TNA structural compound with 2'-phosphoramidite moiety, 3'-ODMTr, and 4'-modification.**

### Example 24:

**Compound 175A** was prepared by the following route:

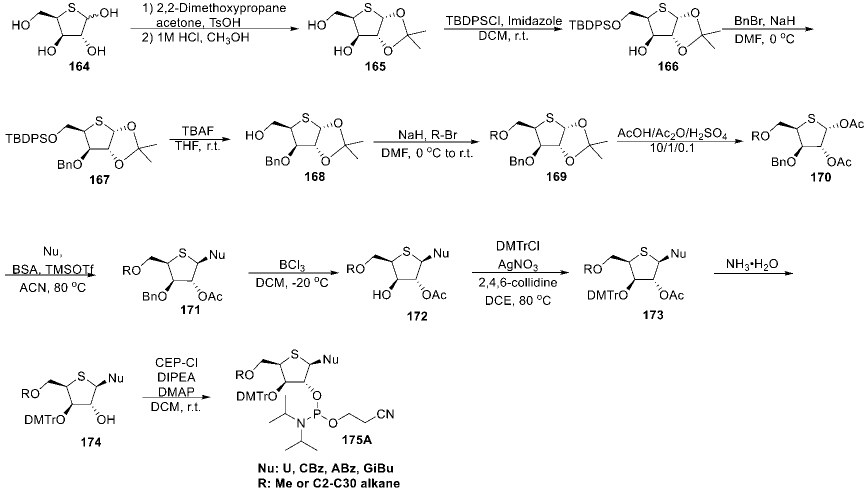

Nucleoside compound 175 is a TNA structural compound with 2'-phosphoramidite moiety, 3'-ODMTr, Nu=ABz, and R=C₁₆H₃₃.

By the above preparation route of Example 24, when compound 168 was used to prepare compound 169, R-Br was selected as C₁₆H₃₃-Br; when compound 170 was used to prepare compound 171, Nu was selected as ABz, where A is adenine. Additionally, referring to the synthesis method of compound 87 in Example 15, the structure and nuclear magnetic resonance data of the obtained nucleoside compound 175 were presented as follows:

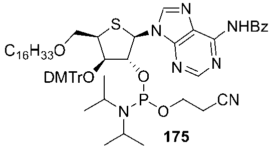

¹H NMR (400 MHz, DMSO-*d*₆) δ 11.22 (d, *J* = 17.5 Hz, 1H), 8.47 (dd, *J =* 30.3, 7.5 Hz, 1H), 8.02 (d, *J =* 7.6 Hz, 2H), 7.63 (t, *J* = 7.3 Hz, 1H), 7.56 - 7.43 (m, 2H), 7.38 - 7.12 (m, 9H), 6.88 - 6.81 (m, 4H), 5.72 (d, *J =* 25.0 Hz, 1H), 4.24 - 3.91 (m, 3H), 3.77 - 3.39 (m, 17H), 2.75 (dd, *J* = 10.7, 5.6 Hz, 1H), 2.62 - 2.31 (m, 1H), 1.62 - 1.51 (m, 2H), 1.37 - 1.18 (m, 24H), 1.11 - 1.03 (m, 9H), 0.96 (d, *J* = 6.7 Hz, 3H), 0.82 (t, *J* = 6.6 Hz, 3H). ³¹P NMR (162 MHz, DMSO-*d*₆) δ 149.78, 149.21. ESI-MS: m/z 1058.6 [M+H]⁺

**Example 25 below is the preparation example of the nucleoside compound shown in Formula 11-a of the present invention (Synthetic route), wherein the nucleoside compound shown in Formula 11-a is a TNA structural compound with 2'-phosphoramidite moiety, 3'-ODMTr, and 4'-modification.**

### Example 25:

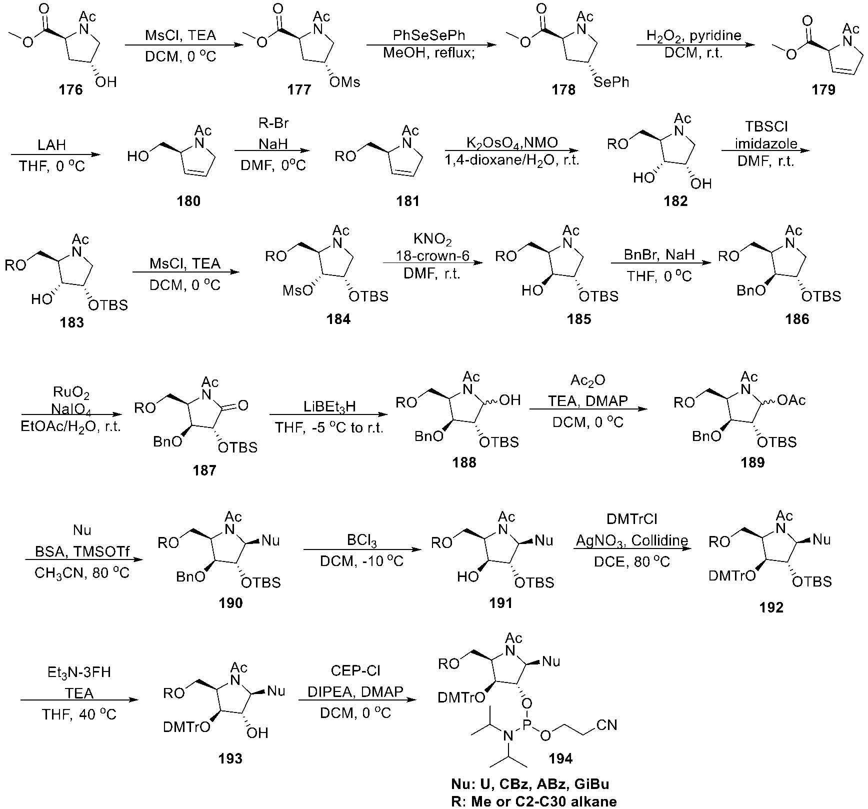

**Example 26 below is the preparation example of the nucleoside compound shown in Formula 12-a of the present invention (Synthetic route), wherein the nucleoside compound shown in Formula 12-a is a TNA structural compound with 1'-H, 2'-phosphoramidite moiety, 3'-ODMTr, and 4'-modification.**

### Example 26:

**Compound 205A** was prepared by the following route:

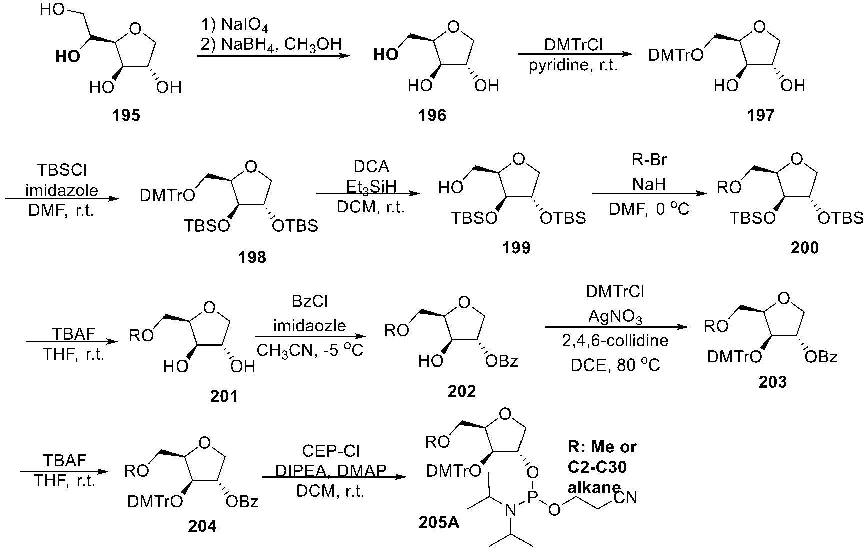

Nucleoside compound 205 is a TNA structural compound with 2'-phosphoramidite moiety, 3'-ODMTr, and R=C₁₆H₃₃.

By the above preparation route of Example 26, when compound 199 was used to prepare compound 200, R-Br was selected as C₁₆H₃₃-Br. The structure and nuclear magnetic resonance data of the obtained nucleoside compound 205 were presented as follows:

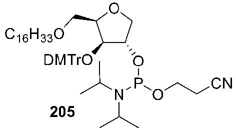

¹H NMR (400 MHz, DMSO-*d*₆) *δ* 7.35 - 7.21 (m, 9=H), 6.92 - 6.81 (m, 4H), 4.24 - 3.91 (m, 4H), 3.77 - 3.39 (m, 17H), 2.75 (dd, *J =* 10.7, 5.6 Hz, 1H), 2.62 - 2.31 (m, 1H), 1.62 - 1.51 (m, 2H), 1.37 - 1.18 (m, 24H), 1.11 - 1.03 (m, 9H), 0.96 (d, *J* = 6.7 Hz, 3H), 0.82 (t, *J* = 6.6 Hz, 3H). ³¹P NMR (162 MHz, DMSO-*d*₆) δ 150.13, 149.31. ESI-MS: m/z 805.5 [M+H]⁺

**Example 27 below is the preparation example of the nucleoside compound shown in Formula 13-a of the present invention (Synthetic route), wherein the nucleoside compound shown in Formula 13-a is a TNA structural compound with 2'-S-phosphoramidite moiety, 3'-ODMTr, and 4'-modification.**

### Example 27:

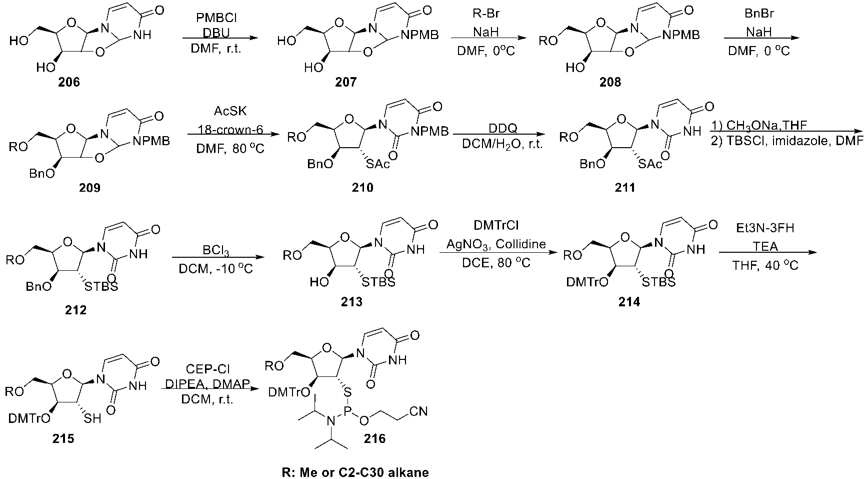

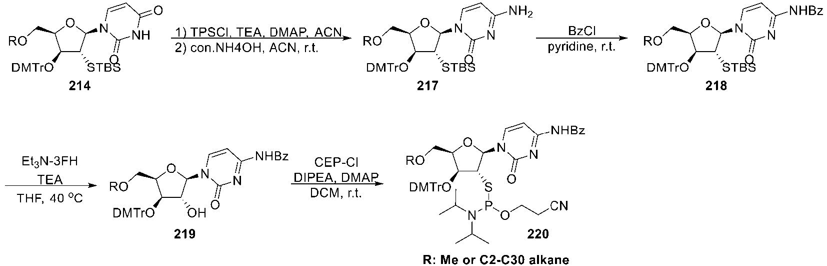

**Example 28 below is the preparation example of the nucleoside compound shown in Formula 14-a of the present invention (Synthetic route), wherein the nucleoside compound shown in Formula 14-a is a TNA structural compound with 2'-phosphoramidite moiety, 3'-SDMTr, and 4'-modification.**

### Example 28:

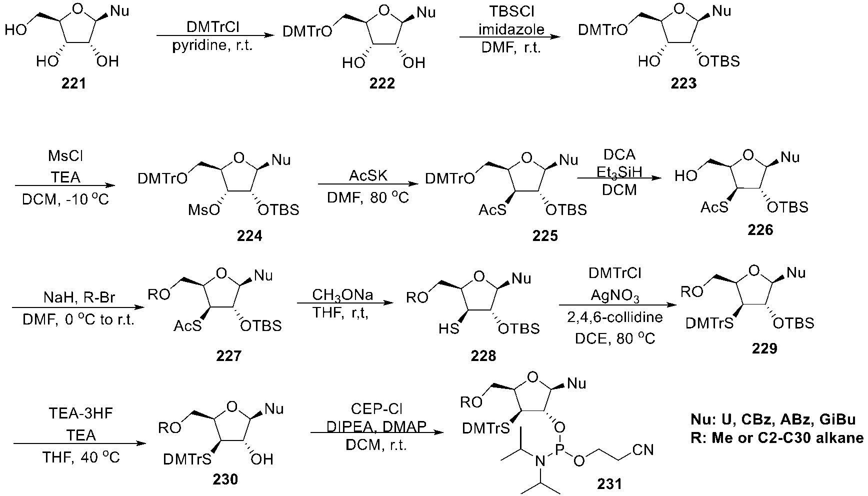

**Example 29 below is the preparation example of the nucleoside compound shown in Formula 15-a of the present invention (Synthetic route), wherein the nucleoside compound shown in Formula 15-a is a TNA structural compound with 2'-phosphoramidite moiety, 3'-ODMTr, and 4'-modification.**

### Example 29:

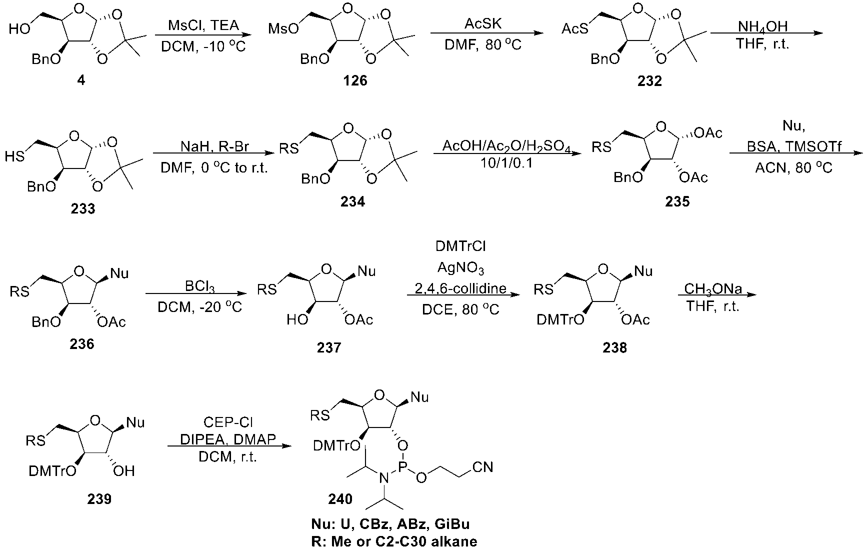

**Example 30 below is the preparation example of the nucleoside compound shown in Formula 16-a of the present invention (Synthetic route), wherein the nucleoside compound shown in Formula 16-a is a TNA structural compound with 2'-phosphoramidite moiety, 3'-ODMTr, and 4'-modification.**

### Example 30:

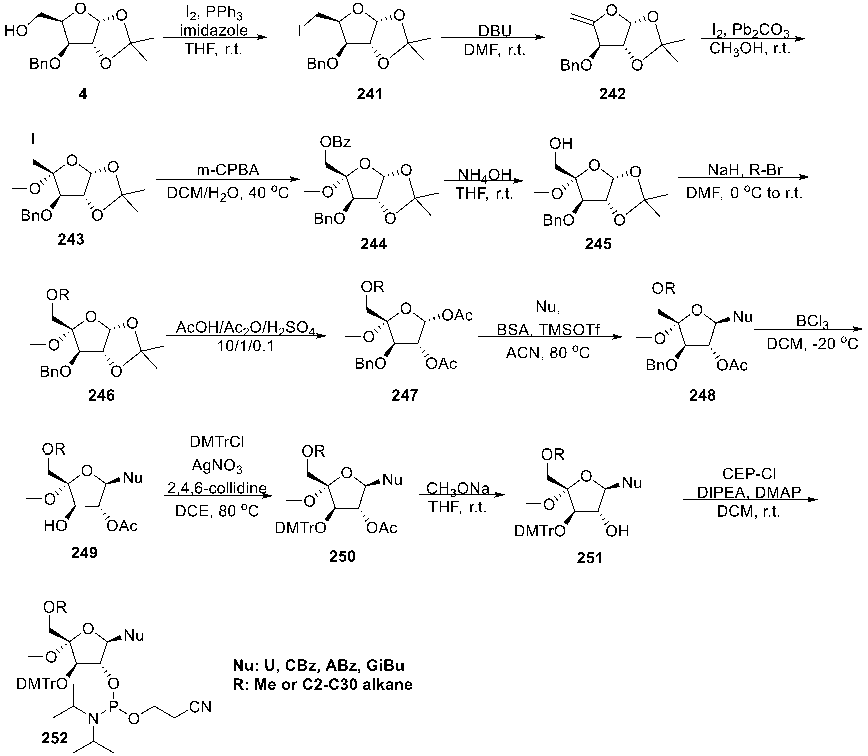

The structures of some compounds prepared by the above preparation methods are shown in Table A below (nucleoside compounds shown in Formula 1-a).

| Compound No. and Structure | | Compound No. and Structure | |
|---|---|---|---|
| LT0- Nu | 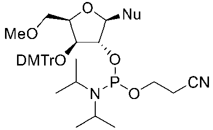 | LT54- Nu | 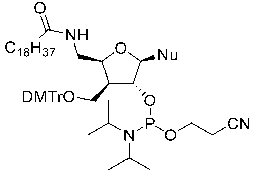 |
| LT1- Nu | 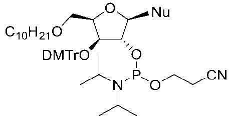 | LT55- Nu | 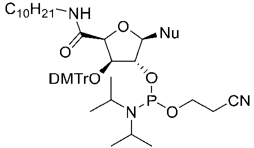 |
| LT2- Nu | 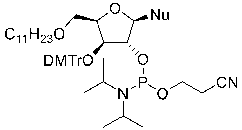 | LT56- Nu | 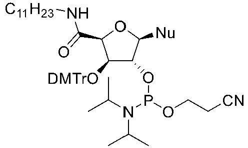 |
| LT3- Nu | 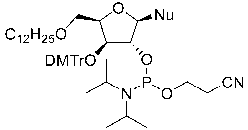 | LT57- Nu | 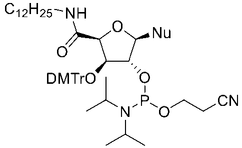 |
| LT4- Nu | 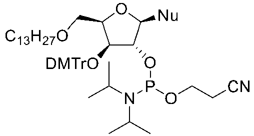 | LT58- Nu | 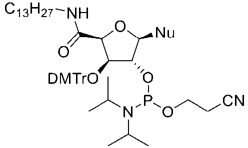 |
| LT5- Nu | 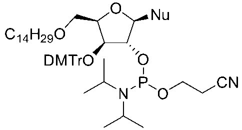 | LT59- Nu | 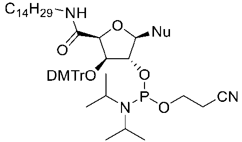 |
| LT6- Nu | 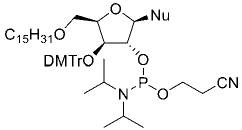 | LT60- Nu | 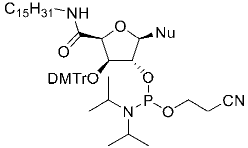 |
| LT7- Nu | 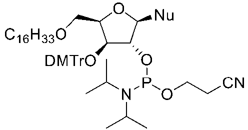 | LT61- Nu | 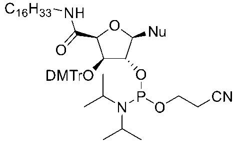 |
| LT8- Nu | 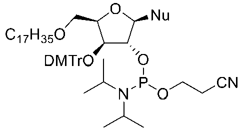 | LT62- Nu | 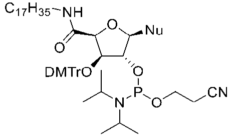 |
| LT9- Nu | 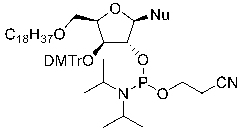 | LT63- Nu | 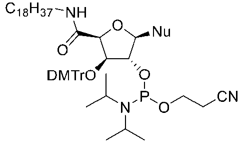 |
| LT10- Nu | 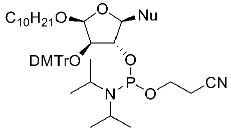 | LT64- Nu | 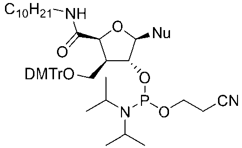 |
| LT11- Nu | 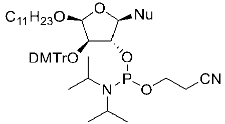 | LT65- Nu | 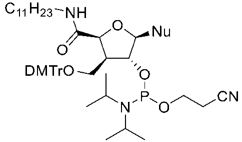 |
| LT12- Nu | 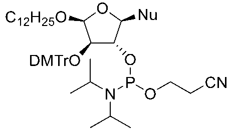 | LT66- Nu | 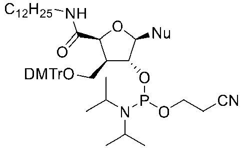 |
| LT13- Nu | 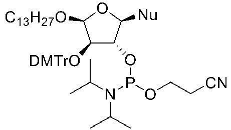 | LT67- Nu | 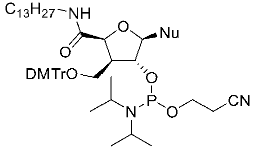 |
| LT14- Nu | 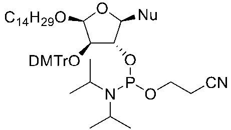 | LT68- Nu | 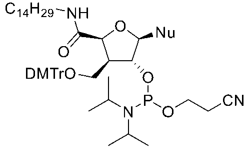 |
| LT15- Nu | 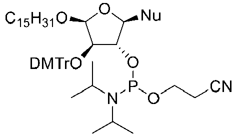 | LT69- Nu | 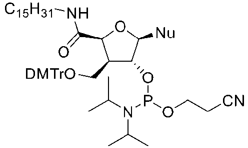 |
| LT16- Nu | 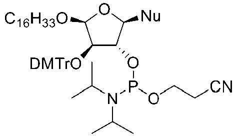 | LT70- Nu | 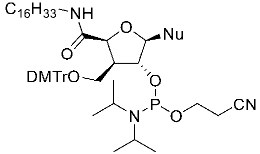 |
| LT17- Nu | 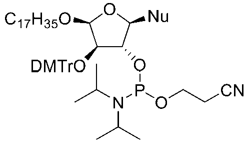 | LT71- Nu | 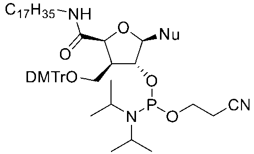 |
| LT18- Nu | 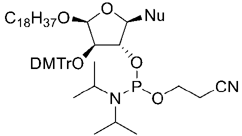 | LT72- Nu | 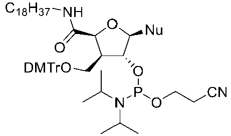 |
| LT19- Nu | 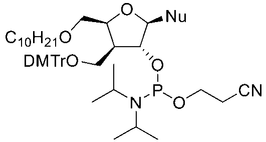 | LT73- Nu | 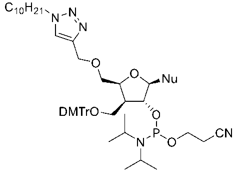 |
| LT20- Nu | 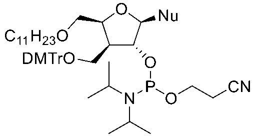 | LT74- Nu | 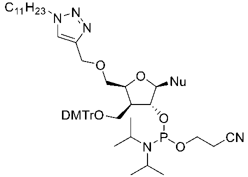 |
| LT21- Nu | 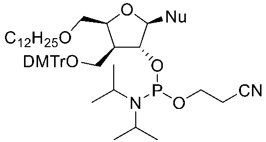 | LT75- Nu | 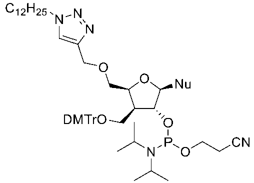 |
| LT22- Nu | 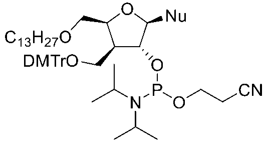 | LT76- Nu | 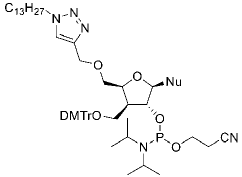 |
| LT23- Nu | 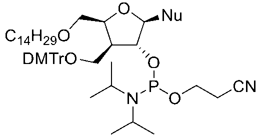 | LT77- Nu | 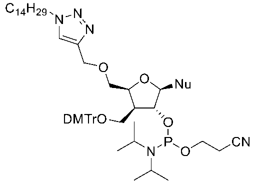 |
| LT24- Nu | 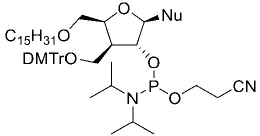 | LT78- Nu | 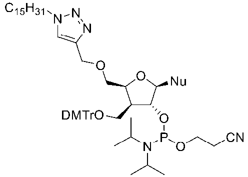 |
| LT25- Nu | 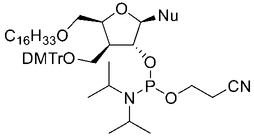 | LT79- Nu | 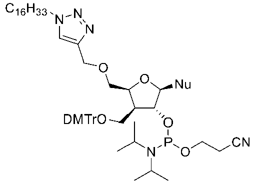 |
| LT26- Nu | 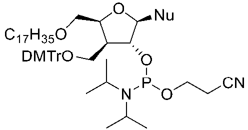 | LT80- Nu | 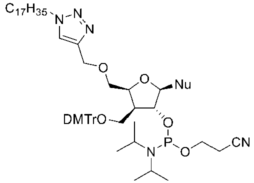 |
| LT27- Nu | 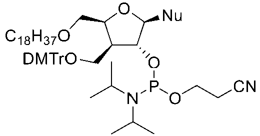 | LT81- Nu | 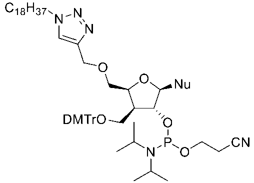 |
| LT28- Nu | 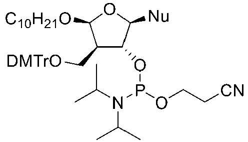 | LT82- Nu | 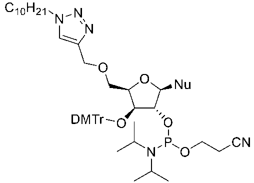 |
| LT29- Nu | 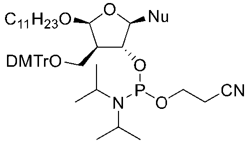 | LT83- Nu | 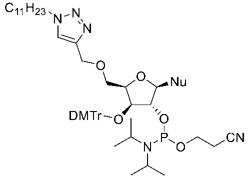 |
| LT30- Nu | 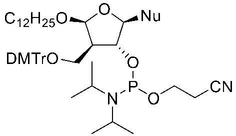 | LT84- Nu | 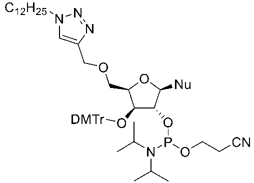 |
| LT31- Nu | 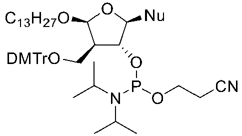 | LT85- Nu | 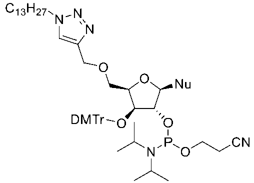 |
| LT32- Nu | 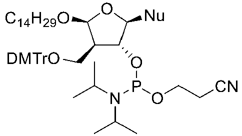 | LT86- Nu | 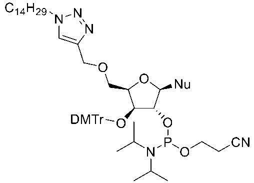 |
| LT33- Nu | 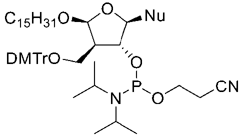 | LT87- Nu | 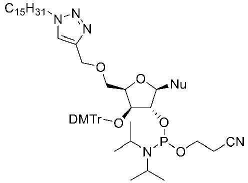 |
| LT34- Nu | 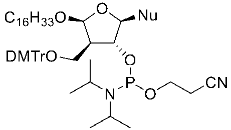 | LT88- Nu | 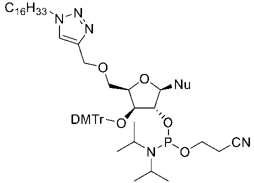 |
| LT35- Nu | 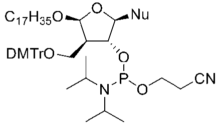 | LT89- Nu | 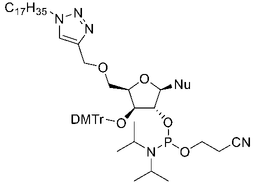 |
| LT36- Nu | 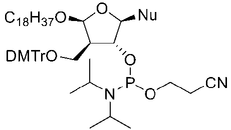 | LT90- Nu | 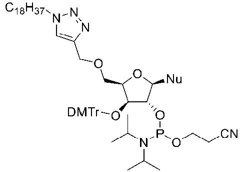 |
| LT37- Nu | 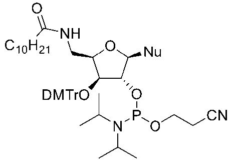 | LT91- Nu | 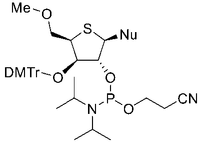 |
| LT38- Nu | 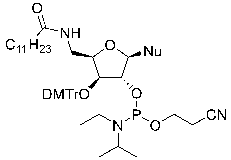 | LT92- Nu | 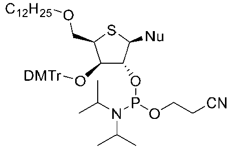 |
| LT39- Nu | 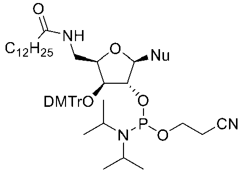 | LT93- Nu | 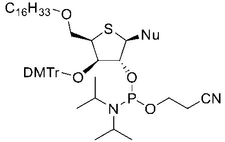 |
| LT40- Nu | 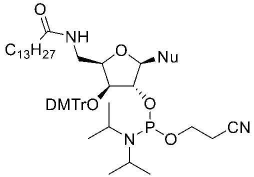 | LT94- Nu | 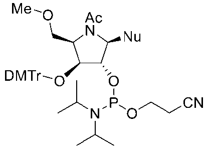 |
| LT41- Nu | 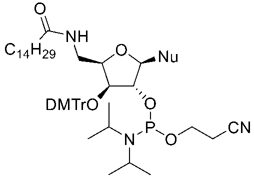 | LT95-without Nu | 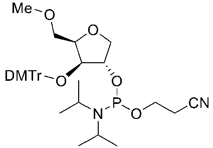 |
| LT42- Nu | 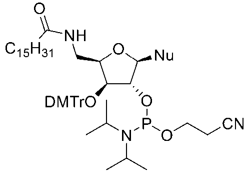 | LT96-without Nu | 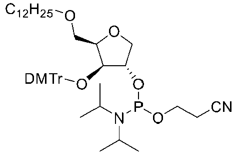 |
| LT43- Nu | 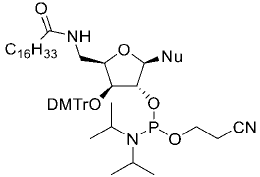 | LT97-without Nu | 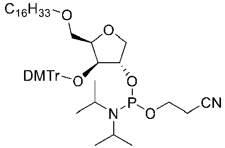 |
| LT44- Nu | 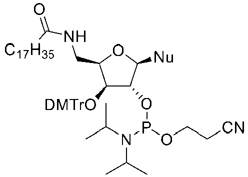 | LT98- Nu | 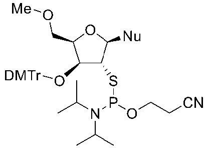 |
| LT45- Nu | 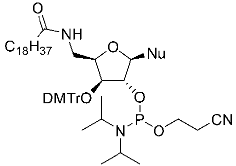 | LT99- Nu | 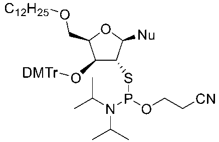 |
| LT46- Nu | 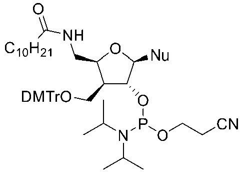 | LT100- Nu | 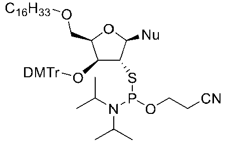 |
| LT47- Nu | 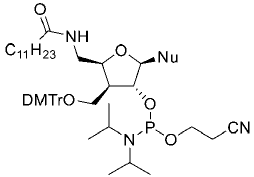 | LT101- Nu | 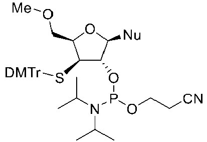 |
| LT48- Nu | 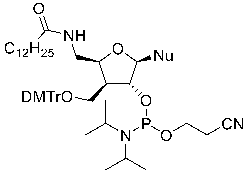 | LT102- Nu | 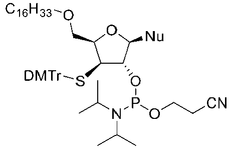 |
| LT49- Nu | 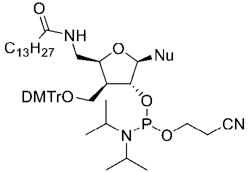 | LT103- Nu | 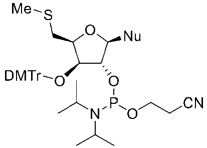 |
| LT50- Nu | 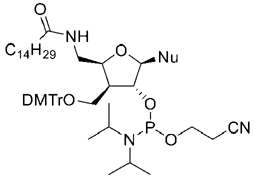 | LT104- Nu | 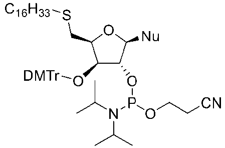 |
| LT51- Nu | 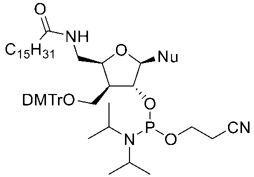 | LT105- Nu | 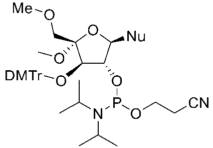 |
| LT52- Nu | 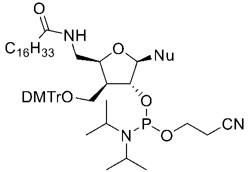 | LT106- Nu | 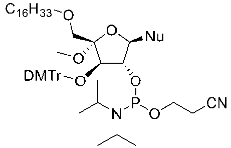 |
| LT53- Nu | 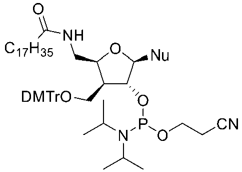 | | |

wherein Nu represents a base or is absent.

**Examples 31-34 below are the preparation examples of the nucleoside compound shown in Formula 1-b of the present invention, wherein the nucleoside compound shown in Formula 1-b is a TNA structural compound with 2'-phosphoramidite moiety, 3'-ODMTr, and 4'-modification.**

### Example 31: Synthesis of Compound 11'

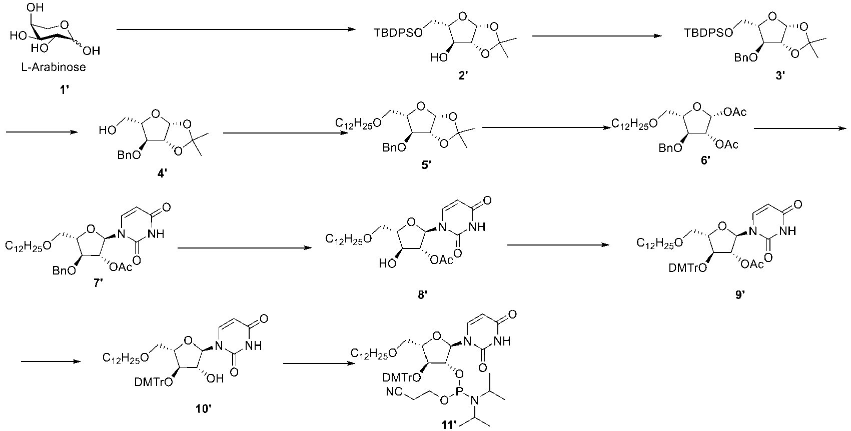

### Synthesis of Compound 2':

Dried compound 1' (20.0 g, 133.22 mmol, 1.0 eq) was dissolved in DMF (200 mL), and imidazole (22.67 g, 333.06 mmol, 2.5 eq) was added to the reaction system. The reaction was cooled to 0°C and stirred for 30 min. Then, tert-butyldiphenylchlorosilane (40.28 g, 146.54 mmol, 1.1 eq) was slowly added dropwise to the reaction system. Once the addition was completed, the ice bath was removed, and the reaction was warmed to 60 °C and carried out overnight. The reaction was monitored by TLC, confirming that compound **1'** was fully consumed. Water was added to the reaction system. The resulting mixture was extracted twice with ethyl acetate. The organic phases were combined, and the combined organic phase was washed with water and saturated brine. The organic phase was dried over anhydrous sodium sulfate and concentrated under reduced pressure to obtain 35.38 g of crude intermediate. The crude intermediate was dissolved in acetone (300 mL), followed by addition of p-toluenesulfonic acid (1.15 g, 6.66 mmol, 0.05 eq), then 2,2-dimethoxypropane (69.37 g, 666.1 mmol, 5.0 eq). Once the addition was completed, the reaction was stirred at room temperature. The reaction was monitored by TLC, confirming that the intermediate was fully consumed. P-toluenesulfonic acid was quenched by addition of sodium bicarbonate solid. The solid was removed by filtration. The filtrate was concentrated to give 38.24 g of crude product. The crude product was subjected to column chromatography (PE/EA= 20/3) to give compound 2' (19.27 g, 44.96 mmol, 33.75% yield).
ESI-MS: m/z 451.2 [M+Na]⁺

### Synthesis of Compound 3':

Compound **2'** (19.27 g, 44.96 mmol, 1.0 eq) was dissolved in DMF (200 mL) and stirred until completely dissolved. The reaction system was cooled to around 0°C. 60% NaH (2.70 g, 67.44 mmol, 1.5 eq) was slowly added in batches to the reaction.

Once the addition was completed, the reaction system was stirred at 0°C for 30 min. BnBr (11.53 g, 67.44 mmol, 1.5 eq) was slowly added dropwise to the reaction system, and the reaction system was kept at a temperature of around 0°C. Once the addition was completed, the reaction system was returned to room temperature, and the reaction was carried out overnight at room temperature. The completion of the reaction was monitored by TLC, confirming that compound 2' was completely consumed. The reaction system was slowly poured into a cold (0°C) saturated aqueous ammonium chloride solution. The mixed system was extracted twice with ethyl acetate, and the organic phases were combined. The organic phase was washed with water and saturated brine, followed by drying over anhydrous sodium sulfate and concentration under reduced pressure to give crude compound **3'** (38.2 g), which was used directly in the next step.
ESI-MS: m/z 519.3 [M+H]⁺

### Synthesis of Compound 4':

Crude compound **3'** (68.2 g) was dissolved in THF (200 mL) and stirred until completely dissolved. TBAF (1 M in THF, 50 mL, 50 mmol, 1.11 eq) was added to the reaction. The reaction was stirred overnight at room temperature until compound **3'** was fully consumed. The reaction solution was concentrated under reduced pressure to remove the solvent and the resulting crude product was subjected to column chromatography (PE/EA = 10/3) to afford compound **4'** (10.77 g, 38.41 mmol, 85.43% yield in two steps).

¹H NMR (400 MHz, DMSO-*d*₆) *δ* 7.42 - 7.26 (m, 5H), 5.85 (d, *J* = 3.6 Hz, 1H), 4.73 (t, *J* = 5.2 Hz, 1H), 4.69 (m, 2H), 4.52 - 4.41 (d, *J =* 12 Hz, 1H), 4.03 (m, 1H), 3.81 (d, *J* = 3.2 Hz, 1H), 3.70 - 3.51 (m, 2H), 1.32 (s, 3H), 1.25 (s, 3H).

### Synthesis of Compound 5':

Compound **4'** (10.77 g, 38.41 mmol, 1.0 eq) was dissolved in DMF (200 mL) and stirred well. The reaction system was cooled to 0°C and stirred for 30 min. 60% NaH (1.84 g, 46.09 mmol, 1.2 eq) was slowly added in batches to the reaction. Compound bromododecane (11.49 g, 46.09 mmol, 1.2 eq) was added slowly dropwise to the reaction system. Once the addition was completed, the reaction was returned to room temperature and carried out overnight at room temperature. The complete consumption of compound **4** was monitored by TLC. The reaction system was poured into ice water to quench the reaction completely. The mixed system was extracted twice with ethyl acetate and the organic phases were combined. The organic phase was washed with water and saturated brine, followed by drying over anhydrous sodium sulfate and removal of the solvent under reduced pressure to give the crude product. The crude product was subjected to column chromatography (PE/EA= 100/9) to afford compound **5'** (15.74 g, 35.08 mmol, 91.33% yield).
¹H NMR (400 MHz, DMSO-*d*₆) δ 7.40 - 7.21 (m, 5H), 5.78 (d, *J =* 3.8 Hz, 1H), 4.74 - 4.61 (m, 2H), 4.44 (d, *J =* 12.0 Hz, 1H), 4.22 (td, *J =* 5.9, 3.2 Hz, 1H), 3.89 (d, *J =* 3.2 Hz, 1H), 3.63 (dd, *J =* 10.1, 5.4 Hz, 1H), 3.52 (dd, *J =* 10.1, 6.4 Hz, 1H), 3.47 - 3.28 (m, 2H), 1.48(m, 2H), 1.38 (s, 3H), 1.21 (d, *J* = 11.6 Hz, 21H), 0.88 - 0.80 (m, 3H). ESI-MS: m/z 471.3 [M+Na]⁺

### Synthesis of Compound 6':

Compound **5'** (15.74 g, 35.08 mmol, 1.0 eq) was added to a 500 mL round-bottom three-necked flask, and AcOH (180 mL) and Ac₂O (17.91 g, 175.4 mmol, 5.0 eq) were added to the reaction system and stirred to completely dissolve the compound. The reaction was cooled to 0°C and stirred at this temperature for 30 min. Concentrated sulfuric acid (2 mL) was added slowly dropwise to the reaction system, and the reaction system was kept at a temperature of 0-5°C at all times. Once the addition was completed, the reaction system was returned to room temperature and stirred for additional 8 hours until the compound 5' was completely consumed. The reaction system was cooled to around -5°C and neutralized to a pH of about 7 with aqueous ammonia. Water was added to the reaction system, and the reaction system was extracted twice with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 5/1) to afford compound **6'** (10.93 g, 22.19 mmol, 63.26% yield).
¹H NMR (400 MHz, DMSO-*d*₆) *δ* 7.32 - 7.22 (m, 5H), 6.22 (d, *J* = 8.4, 1H), 5.29 - 5.12 (m, 1H), 4.71 - 4.52 (m, 2H), 4.35 - 4.11 (m, 2H), 3.68 - 3.41 (m, 2H), 3.41 - 3.34 (m, 2H), 2.05 (s, 3H), 2.01 (s, 3H), 1.50 - 1.41 (m, 2H), 1.32 - 1.18 (m, 18H), 1.22 (t, *J* = 6.8, 3H). ESI-MS: m/z 493.3 [M+H]⁺

### Synthesis of Compound 7':

Compound **6'** (5.0 g, 10.15 mmol, 1.00 eq) and U (2.28 g, 20.30 mmol, 2.0 eq) were added to a 500 mL three-necked round-bottom flask, and ultra-dry ACN (60 mL) was added to the flask with stirring to dissolve the compounds. Subsequently, BSA (6.19 g, 30.45 mmol, 3.0 eq) was added. The reaction system was placed in an oil bath and heated to 80 °C. The reaction system was stirred at this temperature for 1 hour. The entire process was conducted under a nitrogen atmosphere. When the reaction was completed, the reaction system was placed in an ice water bath at 0°C with stirring for 30 min. TMSOTf (2.26 g, 10.15 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound **6'** was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 5/2) to afford compound 7' (4.44 g, 8.15 mmol, 80.30% yield).
¹H NMR (400 MHz, DMSO-*d*₆) *δ* 11.31 (s, 1H), 7.63 (d, *J =* 8.1 Hz, 1H), 7.31 - 7.22 (m, 5H), 5.93 (d, *J* = 2.1 Hz, 1H), 5.60 (dd, *J* = 8.1, 2.2 Hz, 1H), 5.21 (t, *J* = 1.9 Hz, 1H), 4.69 (d, *J=* 12.0 Hz, 1H), 4.51 (d, *J =* 12.0 Hz, 1H), 4.31 - 4.21 (m, 1H), 4.11 (dd, *J* = 4.1*,* 1.6 Hz, 1H), 3.72 (dd, *J* = 10.6, 4.5 Hz, 1H), 3.65 (dd, *J* = 10.6, 6.1 Hz, 1H), 3.48 - 3.37 (m, 2H), 2.05 (s, 3H), 1.51 - 1.41 (m, 2H), 1.27 - 1.21 (m, 18H), 1.18 (t, *J =* 7.1 Hz, 3H). ESI-MS: m/z 545.3 [M+H]⁺

### Synthesis of Compound 8':

Compound **7'** (4.44 g, 8.15 mmol) was added to a 250 mL three-necked flask and ultra-dry DCM (50 mL) was added to the reaction flask. The reaction system was cooled to -30 °C and stirred at this temperature for 30 min. Boron trichloride (1M in toluene, 25 mL, 24.45 mmol, 3.0 eq) was slowly added dropwise to the reaction system and the reaction was carried out until the compound 7' was completely consumed. The reaction was quenched with triethylamine and methanol at -30°C. The reaction was returned to room temperature, and water was added. The mixed system was extracted with DCM, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 2/3) to afford compound **8'** (2.31 g, 5.08 mmol, 62.33% yield).
ESI-MS: m/z 455.3 [M+H]⁺

### Synthesis of Compound 9':

Compound **8'** (2.31 g, 5.08 mmol, 1.0 eq) was added to a 250 mL round-bottom flask. Ultra-dry DCE (30 mL) was added to the reaction flask with stirring to completely dissolve the compound. DMTrCl (3.44 g, 10.16 mmol, 2.0 eq), silver nitrate (863 mg, 5.08 mmol, 1.0 eq) and 2,4,6-trimethylpyridine (3.08 g, 25.4 mmol, 5.0 eq) were added to the reaction at room temperature and stirred well. The reaction was placed in an oil bath and heated to 80°C, then carried out overnight. The complete consumption of compound **8'** was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give the crude product. The crude product was subjected to column chromatography (PE/EA= 20/7) to afford compound **9'** (3.31 g, 4.37 mmol, 86.02% yield).
ESI-MS: m/z 757.4 [M+H]⁺

### Synthesis of Compound 10':

Compound **9'** (3.31 g, 4.37 mmol, 1.0 eq) was added to a 100 mL round-bottom flask, and 30 mL of THF was added to dissolve the compound. Then 0.97 mL of NaOMe solution (5.4 M in Methanol) (5.24 mmol, 1.2 eq) was added to the reaction at room temperature. The reaction was stirred at room temperature for 15 min until the compound **9'** was completely consumed. After the reaction is complete, the reaction system was poured into water, and the resulting mixture was extracted with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 1/1) to afford compound **10'** (3.01 g, 4.21 mmol, 96.34% yield).
¹H NMR (400 MHz, DMSO-*d*₆) δ 11.27 (s, 1H), 7.91 (d, *J =* 8.1 Hz, 1H), 7.47 - 7.38 (m, 2H), 7.32 - 7.21 (m, 7H), 6.91 - 6.75 (m, 4H), 5.46 (d, *J =* 3.6 Hz, 1H), 4.16 (t, *J =* 4.7 Hz, 1H), 3.76 (s, 6H), 3.66 (dd, *J* = 9.2, 4.5 Hz, 1H), 3.66 - 3.46 (m, 2H), 3.46 - 3.38 (m, 3H), 1.56 (m, 2H), 1.36 - 1.21 (m, 20H), 0.85 (t, *J* = 6.7 Hz, 3H). ESI-MS: m/z 715.4 [M+H]⁺

### Synthesis of Compound 11':

Dried compound **10'** (3.01 g, 4.21 mmol, 1.0 eq) was added to a 100 mL round-bottom flask. Ultra-dry DCM (30 mL) was added to the flask with stirring to completely dissolve the compound. DIPEA (1.09 g, 8.42 mmol, 2.0 eq) and DMAP (103 mg, 0.84 mmol, 0.2 eq) were added to the reaction and stirred for 15 min at room temperature. The reaction system was purged with nitrogen and carried out under a nitrogen protection. Compound CEP-Cl (1.50 g, 6.32 mmol, 1.5 eq) was added dropwise to the reaction at room temperature. The reaction was carried out at room temperature for 30-60 min until the compound **10'** was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product. The crude product was purified by column chromatography (PE/EA= 1/1) to afford compound **11'** (3.22 g, 3.52 mmol, 83.61% yield).
¹H NMR (400 MHz, DMSO-*d*₆) δ 11.32 (dd, *J =* 14.6, 2.2 Hz, 1H), 7.92 (d, *J =* 8.1 Hz, 1H), 7.46 - 7.37 (m, 2H), 7.35 - 7.25 (m, 7H), 6.88 - 6.84 (m, 4H), 5.73 - 5.52 (m, 2H), 4.33 (dt, *J =* 8.8, 4.7 Hz, 1H), 4.12 - 3.92 (m, 1H), 3.84 - 3.75 (m, 1H), 3.76(s, 6H), 3.66 - 3.39 (m, 6H), 2.72 (t, *J =* 6.0 Hz, 1H), 2.61 - 2.37 (m, 1H), 1.61 - 1.53 (m, 2H), 1.41 - 1.21 (m, 20H), 1.11 (t, *J =* 6.6 Hz, 6H), 1.03 (d, *J =* 6.7 Hz, 6H), 0.88 - 0.80 (m, 3H). ³¹P NMR (162 MHz, DMSO-*d*₆) *δ* 151.12, 148.78. ESI-MS: m/z 913.5 [M+H]⁺

### Example 32: Synthesis of Compound 16'

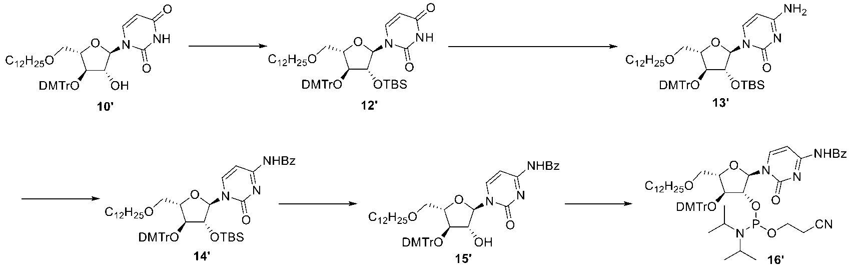

### Synthesis of Compound 12':

Compound **10'** (5.0 g, 6.99 mmol, 1.0 eq) was added to a 250 mL round-bottom flask. Ultra-dry DMF (50 mL) was added to the flask with stirring. Imidazole (1.90 g, 27.96 mmol, 4.0 eq) was added at room temperature and stirred for 10 min. Then, TBSCl (952 mg, 13.98 mmol, 2.0 eq) was slowly added to the reaction system in batches and the reaction was carried out overnight at room temperature under nitrogen protection. The complete consumption of compound **10'** was monitored by TLC and LCMS. The reaction was quenched by adding water. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 2/1) to afford compound **12'** (4.84 g, 5.84 mmol, 83.58% yield).
ESI-MS: m/z 829.5 [M+H]⁺

### Synthesis of Compound 13':

Compound **12'** (4.84 g, 5.84 mmol, 1.0 eq) was added to a 250 mL round-bottom flask. Ultra-dry ACN (50 mL) was added to the flask with stirring to completely dissolve the compound. TEA (1.18 g, 11.68 mmol, 2.0 eq) and DMAP (1.43 g, 11.68 mmol, 2.0 eq) were added to the reaction system and stirred well. The reaction was cooled to 0-5 °C, and the compound TPSCl (3.54 g, 11.68 mmol, 2.0 eq) was added slowly in batches. Once the addition was completed, the ice bath was removed, and the reaction was returned to room temperature. The reaction was stirred overnight at room temperature. The complete consumption of compound **12'** was monitored by TLC. At room temperature, aqueous ammonia (40 mL) was added to the reaction and stirred for about 12 hours until the intermediate was completely consumed. Saturated brine was added to the reaction, and the mixed system was extracted twice with ethyl acetate. The organic phases were combined. The organic phase was washed with water and saturated brine, followed by drying over anhydrous sodium sulfate and concentration under reduced pressure to give the crude product **13'** (10.31 g, calculated at 100% yield).
ESI-MS: m/z 826.5 [M-H]⁻

### Synthesis of Compound 14':

Crude compound **13'** (10.31 g, 1.0 eq) was added to a 100 mL round-bottom flask, and pyridine (100 mL) was added to the flask with stirring to completely dissolve the crude product **13'.** The reaction system was cooled to 0 °C, and BzCl (1.64 g, 11.68 mmol, 2.0 eq) was slowly added dropwise to the reaction system and stirred at 0 °C for 1 hour until the compound 13' was completely consumed. The reaction was protected by nitrogen throughout. The reaction system was returned to room temperature, and the reaction was quenched by the addition of methanol and water. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA = 1/1) to afford compound **14'** (3.90 g, 4.18 mmol, 71.58% overall yield in 2 steps).
ESI-MS: m/z 930.5 [M-H]⁻

### Synthesis of Compound 15':

Compound **14'** (3.90 g, 4.18 mmol, 1.0 eq) was added to a 100 mL round-bottom flask and THF (30 mL) was added with stirring until completely dissolved. Triethylamine trihydrofluoride (5.0 mL) was neutralized to alkaline with triethylamine (17 mL) and added to the above reaction system. The reaction was placed in a 40°C oil bath and stirred overnight under nitrogen protection. The complete consumption of compound **14'** was monitored by TLC and LCMS. Water was added to the reaction, and the mixed system was extracted twice with ethyl acetate. The organic phases were combined. The organic phase was washed with water, saturated aqueous sodium bicarbonate solution and saturated brine, followed by drying over anhydrous sodium sulfate and removal of the solvent under reduced pressure to give the crude product. The crude product was subjected to column chromatography (PE/EA= 1/1) to afford compound **15'** (2.51 g, 3.07 mmol, 76.2% yield).
ESI-MS: m/z 816.4 [M-H]⁻

### Synthesis of Compound 16':

Dried compound **15'** (2.51 g, 3.07 mmol, 1.0 eq) was added to a 100 mL round-bottom flask, and ultra-dry DCM (30 mL) was added to the flask with stirring to dissolve the compound. DIPEA (794 mg, 6.14 mmol, 2.0 eq) and DMAP (75 mg, 0.61 mmol, 0.2 eq) were added to the reaction, and the reaction was purged with nitrogen. The reaction was cooled to around 0 °C, and CEP-Cl (1.09 g, 4.61 mmol, 1.5 eq) was slowly added dropwise. The reaction was carried out at this temperature and under nitrogen protection for 1 hour until the compound **15'** was completely consumed. After completion of the reaction, the reaction was quenched by addition of saturated aqueous sodium bicarbonate to the reaction. The mixed system was extracted twice with dichloromethane, and the organic phases were combined. The organic phase was washed with water and saturated brine, followed by drying over anhydrous sodium sulfate and removal of the solvent under reduced pressure to give the crude product. The crude product was purified by column chromatography (PE/EA = 1/1) to afford compound **16'** (2.68 g, 2.63 mmol, 85.67% yield).
¹H NMR (400 MHz, DMSO-*d*₆) δ 11.29 (d, *J =* 17.5 Hz, 1H), 8.41 (dd, *J* = 30.3, 7.5 Hz, 1H), 8.00 (d, *J =* 7.6 Hz, 2H), 7.61 (t, *J =* 7.3 Hz, 1H), 7.57 - 7.41 (m, 2H), 7.36 - 7.11 (m, 9H), 6.88 - 6.81 (m, 4H), 5.72 (d, *J =* 25.0 Hz, 1H), 4.22 - 3.91 (m, 3H), 3.77 - 3.39 (m, 13H), 2.75 (dd, *J =* 10.7, 5.6 Hz, 1H), 2.62 - 2.31 (m, 1H), 1.62 - 1.51 (m, 2H), 1.35 - 1.18 (m, 20H), 1.12 - 1.04 (m, 9H), 0.96 (d, *J =* 6.7 Hz, 3H), 0.83 (t, *J =* 6.6 Hz, 3H). ³¹P NMR (162 MHz, DMSO-*d*₆) *δ* 149.74, 149.21. ESI-MS: m/z 1016.5 [M-H]⁻

### Example 33: Synthesis of Compound 21'

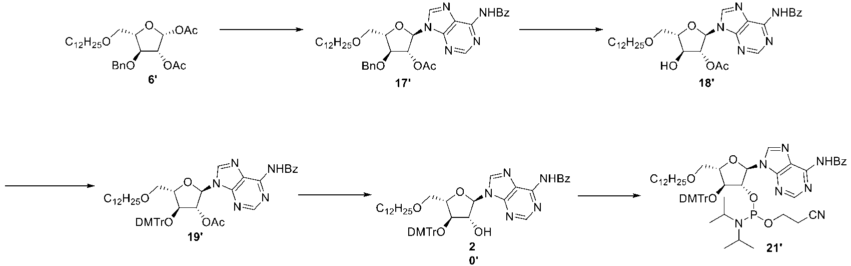

### Synthesis of Compound 17':

Compound **6'** (20.00 g, 40.60 mmol, 1.00 eq) was added to a 500 mL three-necked round-bottom flask, and ultra-dry ACN (250 mL) was added to the flask with stirring to dissolve the compounds. Subsequently, BSA (24.78 g, 121.80 mmol, 3.0 eq) and A(Bz) (19.43 g, 81.20 mmol, 2.0 eq) were added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 30 min. TMSOTf (9.02 g, 40.60 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound **6'** was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 1/1) to afford compound **17'** (19.03 g, 28.32 mmol, 69.75% yield).
¹H NMR (400 MHz, DMSO-*d*₆) δ 11.25 (s, 1H), 8.75 (s, 1H), 8.45 (s, 1H), 8.05 (d, *J =* 7.4 Hz, 2H), 7.62 (t, *J =* 7.4 Hz, 1H), 7.52 (t, *J =* 7.6 Hz, 2H), 7.37 - 7.28 (m, 5H), 6.32 (d, J = 1.7 Hz, 1H), 4.79 (d, J = 11.8 Hz, 1H), 4.61 (d, J = 11.8 Hz, 1H), 4.43 (dd, J = 10.3, 4.6 Hz, 1H), 3.85 - 3.68 (m, 2H), 3.48 - 3.41 (m, 2H), 2.12 (s, 3H), 1.54 - 1.41 (m, 2H), 1.34 - 1.11 (m, 20H), 0.85 (t, J *=* 6.7 Hz, 3H). ESI-MS: m/z 672.4 [M+H]⁺

### Synthesis of Compound 18':

Compound **17'** (19.03 g, 28.32 mmol, 1.0 eq) was added to a 500 mL three-necked flask and ultra-dry DCM (200 mL) was added to the reaction flask. The reaction system was cooled to -20 °C and stirred at this temperature for 30 min. 1.0 mol/L of boron trichloride in dichloromethane (85 mL, 3.0 eq) was slowly added dropwise to the reaction system and the reaction continued for 5 hours until the compound **17'** was completely consumed. The reaction was quenched with triethylamine and methanol at -20°C. The reaction was returned to room temperature, and water was added. The mixed system was extracted with dichloromethane, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 1/2) to afford compound **18'** (11.23 g, 19.31 mmol, 68.19% yield).

### Synthesis of Compound 19':

Compound **18'** (11.23 g, 19.31 mmol, 1.0eq) was added to a 250 mL round-bottom flask. Ultra-dry DCE (100 mL) was added to the reaction flask with stirring to completely dissolve the compound. DMTrCl (13.09 g, 38.62 mmol, 2.0 eq), silver nitrate (3.28 g, 19.31 mmol, 1.0 eq) and 2,4,6-trimethylpyridine (11.70 g, 96.55 mmol, 5.0 eq) were added to the reaction at room temperature and stirred well. The reaction was placed in an oil bath and heated to 80°C, then carried out overnight. The complete consumption of compound **18'** was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give the crude product. The crude product was subjected to column chromatography (PE/EA= 2/3) to afford compound **19'** (13.85 g, 15.67 mmol, 81.15% yield).
ESI-MS: m/z 882.4 [M-H]⁻

### Synthesis of Compound 20':

Compound **19'** (13.85 g, 15.67 mmol, 1.0 eq) was added to a 250 mL round-bottom flask. THF (150 mL) was added to the reaction, and the reaction was stirred until completely dissolved. To the reaction was added 5.4 mol/L NaOMe solution (3.48 mL, 18.80 mmol, 1.2 eq). The reaction was stirred at room temperature for 3 hours until the compound **19'** was completely consumed. After the reaction was completed, the reaction system was extracted twice with water and ethyl acetate, and the organic phases were combined. The combined organic phase was washed with water and saturated brine, followed by drying over anhydrous sodium sulfate and removal of the solvent under reduced pressure to give the crude product. The crude product was subjected to column chromatography to afford compound **20'** (12.13 g, 14.41 mmol, 91.96% yield).
ESI-MS: m/z 840.4 [M-H]⁻

### Synthesis of Compound 21':

Dried compound **20'** (2.0 g, 2.38 mmol, 1.0 eq) was added to a 50 mL round-bottom flask. Ultra-dry DCM (20 mL) was added to the flask with stirring to completely dissolve the compound. DIPEA (615 mg, 4.76 mmol, 2.0 eq) and DMAP (59 mg, 0.48 mmol, 0.2 eq) were added to the reaction and stirred for 15 min at room temperature. The reaction system was purged with nitrogen and carried out under a nitrogen protection. Compound CEP-Cl (845 mg, 3.57 mmol, 1.5 eq) was added dropwise to the reaction at room temperature. The reaction was carried out at room temperature for 30-60 min until the compound **20'** was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product. The crude product was purified by column chromatography to afford compound **21'** (1.92 g, 1.84 mmol, 77.31% yield).
¹H NMR (400 MHz, DMSO-*d*₆) δ 11.25 (d, *J =* 4.0 Hz, 1H), 8.72 (d, *J =* 5.9 Hz, 1H), 8.61 (d, *J* = 29.9 Hz, 1H), 8.14 (d, *J =* 7.6 Hz, 2H), 7.61 (t, *J =* 7.3 Hz, 1H), 7.52 (t, *J =* 7.6 Hz, 2H), 7.33 (t, *J* = 8.6 Hz, 2H), 7.22 - 7.14 (m, 7H), 6.81 - 6.61 (m, 4H), 6.03 (dd, *J =* 36.3, 3.4 Hz, 1H), 4.74 - 4.32 (m, 2H), 3.82 - 3.39 (m, 15H), 2.67 - 2.35 (m, 2H), 1.61 - 1.49 (m, 2H), 1.41 - 1.20 (m, 18H), 1.13 - 0.98 (m, 9H), 0.92 - 0.77 (m, 6H). ³¹P NMR (162 MHz, DMSO-*d*₆) δ 149.64, 148.91. ESI-MS: m/z 1040.5 [M-H]⁻

### Example 34: Synthesis of Compound 26'

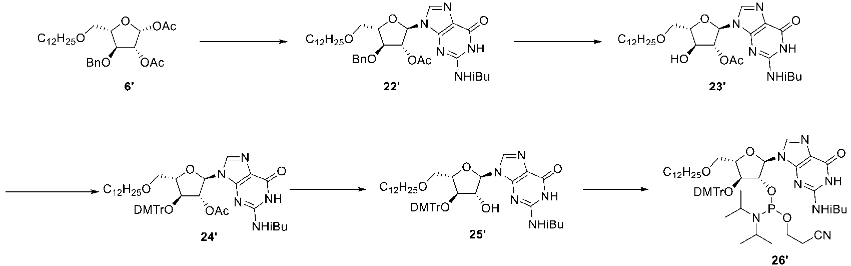

### Synthesis of Compound 22':

Compound **6'** (20 g, 40.60 mmol, 1.00 eq) was added to a 500 mL three-necked round-bottom flask, and ultra-dry ACN (200 mL) was added to the flask with stirring to dissolve the compounds. Subsequently, BSA (24.78 g, 121.8 mmol, 3.0 eq) and G(iBu) (17.96 g, 81.2 mmol, 2.0 eq) were added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 30 min. TMSOTf (9.02 g, 40.6 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound **6** was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 1/1) to afford compound **22'** (19.22 g, 29.40 mmol, 72.41% yield).
ESI-MS: m/z 654.4 [M+H]⁺

### Synthesis of Compound 23':

Compound **22'** (10.00 g, 15.29 mmol, 1.0 eq) was added to a 250 mL three-necked flask and ultra-dry DCM (100 mL) was added to the reaction flask. The reaction system was cooled to -20 °C and stirred at this temperature for 30 min. 1.0 mol/L of BCl₃ in dichloromethane (45.87 mL, 3.0 eq) was slowly added dropwise to the reaction system and the reaction continued for 5 hours until the compound **22'** was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 1/2) to afford compound **23'** (6.12 g, 10.86 mmol, 71.03% yield).
ESI-MS: m/z 564.3 [M+H]⁺

### Synthesis of Compound 24':

Compound **23'** (6.12 g, 10.86 mmol, 1.0eq) was added to a 250 mL round-bottom flask. Ultra-dry DCE (70 mL) was added to the reaction flask with stirring to completely dissolve the compound. DMTrCl (7.36 g, 21.72 mmol, 2.0 eq), AgNO₃ (1.84 g, 10.86 mmol, 1.0 eq), 2,4,6-trimethylpyridine (6.58 g, 54.30 mmol, 5.0 eq) were added to the reaction at room temperature and stirred well. The reaction was placed in an oil bath and heated to 80°C, then carried out overnight. The complete consumption of compound **23'** was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give the crude product. The crude product was subjected to column chromatography (PE/EA= 2/3) to afford compound **24'** (8.18 g, 9.46 mmol, 87.11% yield).
ESI-MS: m/z 864.5 [M-H]⁻

### Synthesis of Compound 25':

Compound **24'** (3.18 g, 3.67 mmol, 1.0 eq) was added to a 100 mL round-bottom flask. THF (30 mL) was added to the reaction, and the reaction was stirred until completely dissolved. To the reaction was added 5.4 mol/L NaOMe solution (0.81 mL, 4.40 mmol, 1.2 eq). The reaction was stirred at room temperature until the compound **24'** was completely consumed. After the reaction was completed, the reaction system was extracted twice with water and ethyl acetate, and the organic phases were combined. The combined organic phase was washed with water and saturated brine, followed by drying over anhydrous sodium sulfate and removal of the solvent under reduced pressure to give the crude product. The crude product was subjected to column chromatography to afford compound **25'** (2.68 g, 3.26 mmol, 88.83% yield).
ESI-MS: m/z 822.4 [M-H]⁻

### Synthesis of Compound 26':

Dried compound 25' (2.68 g, 3.26 mmol, 1.0 eq) was added to a 100 mL round-bottom flask. Ultra-dry DCM (30 mL) was added to the flask with stirring to completely dissolve the compound. DIPEA (843 mg, 6.52 mmol, 2.0 eq) and DMAP (79 mg, 0.65 mmol, 0.2 eq) were added to the reaction and stirred for 15 min at room temperature. The reaction system was purged with nitrogen and carried out under a nitrogen protection. Compound CEP-Cl (1.16 g, 4.89 mmol, 1.5 eq) was added dropwise to the reaction at room temperature. The reaction was carried out at room temperature for 30-60 min until the compound **25'** was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product. The crude product was purified by column chromatography to afford compound **26'** (2.98 g, 2.91 mmol, 89.26% yield).
¹H NMR (400 MHz, DMSO-*d*₆) *δ* 12.09 (s, 1H), 11.49 (s, 1H), 8.15 (d, *J =* 29.6 Hz, 1H), 7.53 - 7.12 (m, 9H), 6.92 - 6.87 (m, 4H), 5.73 (dd, *J* = 38.6, 4.8 Hz, 1H), 5.22 - 5.01 (m, 1H), 4.52 (t, *J =* 5.5 Hz, 1H), 3.82 - 3.67 (m, 8H), 3.66 - 3.39 (m, 3H), 3.26 - 3.21 (m, 2H), 3.10 (d, *J* = 8.4 Hz, 1H), 2.79 - 2.66 (m, 1H), 2.57 - 2.47 (m, 2H), 2.47 - 2.33 (m, 1H), 1.50 (d, *J* = 6.6 Hz, 2H), 1.36 - 1.16 (m, 18H), 1.14 - 0.99 (m, 13H), 0.89 - 0.73 (m, 8H). ³¹P NMR (162 MHz, DMSO-*d*₆) δ 151.33, 148.56. ESI-MS: m/z 1022.6 [M-H]⁻

### Example 35: Synthesis of Compound 33'

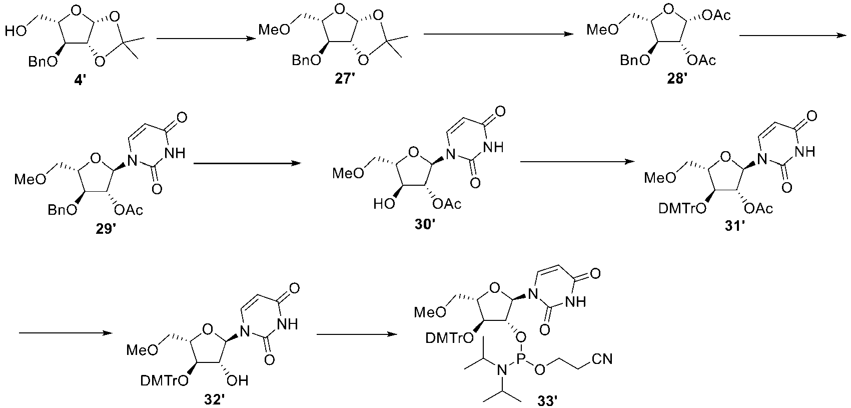

### Synthesis of Compound 27':

Compound **4'** (30.00 g, 103.02 mmol, 1.0 eq) was dissolved in DMF (300 mL) and stirred well. The reaction was cooled to 0 °C and stirred for 30 min. Then 60% NaH (4.94 g, 123.62 mmol, 1.2 eq) was added to the reaction and stirred for 30 min. Compound CH₃I (21.93 g, 154.53 mmol, 1.5 eq) was added slowly dropwise to the reaction system. Once the addition was completed, the reaction was returned to room temperature and carried out overnight at room temperature. The complete consumption of compound **4'** was monitored by TLC. The reaction system was poured into ice water to quench the reaction completely. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 100/9) to afford compound **27'** (28.32 g, 96.21 mmol, 93.39% yield).
ESI-MS: m/z 295.2 [M+H]⁺

### Synthesis of Compound 28':

Compound **27'** (28.32 g, 96.21 mmol, 1.0 eq) was added to a 500 mL round-bottom three-necked flask, and AcOH (300 mL) and Ac₂O (49.11 g, 481.05 mmol, 5.0 eq) were added to the reaction system and stirred to completely dissolve the compound. The reaction was cooled to 0°C and stirred at this temperature for 30 min. Concentrated sulfuric acid (3 mL) was added slowly dropwise to the reaction system, and the reaction system was kept at a temperature of 0-5°C at all times. Once the addition was completed, the reaction system was returned to room temperature and stirred for additional 8 hours until the compound **27'** was completely consumed. The reaction system was cooled to around -5°C and neutralized to a pH of about 7 with aqueous ammonia. Water was added to the reaction system, and the reaction system was extracted twice with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 10/2) to afford compound **28'** (21.82 g, 64.49 mmol, 67.03% yield).
ESI-MS: m/z 339.1 [M+H]⁺

### Synthesis of Compound 29':

Compound **28'** (5.0 g, 14.78 mmol, 1.00 eq) and uracil (3.31 g, 29.56 mmol, 2.0 eq) were added to a 250 mL three-necked round-bottom flask, and ultra-dry ACN (80 mL) was added to the flask with stirring to dissolve the compounds. Subsequently, BSA (9.02 g, 44.34 mmol, 3.0 eq) was added. The reaction system was placed in an oil bath and heated to 80 °C. The reaction system was stirred at this temperature for 1 hour. The entire process was conducted under a nitrogen atmosphere. When the reaction was completed, the reaction system was placed in an ice water bath at 0°C with stirring for 30 min. TMSOTf (3.29 g, 14.78 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound **28'** was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 5/2) to afford compound **29'** (5.03 g, 12.88 mmol, 87.14% yield).
ESI-MS: m/z 391.2 [M+H]⁺

### Synthesis of Compound 30':

Compound **29'** (5.03 g, 12.88 mmol, 1.0 eq) was added to a 250 mL three-necked flask and ultra-dry DCM (50 mL) was added to the reaction flask. The reaction system was cooled to -20 °C and stirred at this temperature for 30 min. Boron trichloride (1M in toluene, 38.64 mL, 38.64 mmol, 3.0 eq) was slowly added dropwise to the reaction system and the reaction continued for 5 hours until the compound **29'** was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 2/3) to afford compound 30' (2.44 g, 8.13 mmol, 63.13% yield).
ESI-MS: m/z 301.1 [M+H]⁺

### Synthesis of Compound 31':

Compound **30'** (2.44 g, 8.13 mmol, 1.0 eq) was added to a 100 mL round-bottom flask. Ultra-dry DCE (50 mL) was added to the reaction flask with stirring to completely dissolve the compound. DMTrCl (5.51 g, 16.26 mmol, 2.0 eq), AgNO₃ (1.38 g, 8.13 mmol, 1.0 eq) and 2,4,6-trimethylpyridine (4.93 g, 40.65 mmol, 5.0 eq) were added to the reaction at room temperature and stirred well. The reaction was placed in an oil bath and heated to 80°C, then carried out overnight. The complete consumption of compound **30'** was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give the crude product. The crude product was subjected to column chromatography (PE/EA= 20/7) to afford compound **31'** (4.33 g, 7.20 mmol, 88.56% yield).
ESI-MS: m/z 601.2 [M-H]⁻

### Synthesis of Compound 32':

Compound **31'** (4.33 g, 7.20 mmol, 1.0 eq) was added to a 100 mL round-bottom flask. THF (50 mL) was added to the reaction, and the reaction was stirred until completely dissolved. To the reaction was added 5.4 mol/L NaOMe solution (1.60 mL, 8.64 mmol, 1.2 eq). The reaction was stirred at room temperature until the compound **31'** was completely consumed. After the reaction was completed, the reaction system was extracted twice with water and ethyl acetate, and the organic phases were combined. The combined organic phase was washed with water and saturated brine, followed by drying over anhydrous sodium sulfate and removal of the solvent under reduced pressure to give the crude product. The crude product was subjected to column chromatography to afford compound **32'** (3.91 g, 6.99 mmol, 97.08% yield).
ESI-MS: m/z 559.2 [M-H]⁻

### Synthesis of Compound 33':

Dried compound **32'** (3.91 g, 6.99 mmol, 1.0 eq) was added to a 100 mL round-bottom flask. Ultra-dry DCM (40 mL) was added to the flask with stirring to completely dissolve the compound. DIPEA (1.81 g, 13.98 mmol, 2.0 eq) and DMAP (171 mg, 1.40 mmol, 0.2 eq) were added to the reaction and stirred for 15 min at room temperature. The reaction system was purged with nitrogen and carried out under a nitrogen protection. Compound CEP-Cl (1.27 g, 5.36 mmol, 1.5 eq) was added dropwise to the reaction at room temperature. The reaction was carried out at room temperature for 30-60 min until the compound **32'** was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product. The crude product was purified by column chromatography (PE/EA= 1/1) to afford compound **33'** (4.02 g, 5.29 mmol, 75.68% yield).
¹H NMR (400 MHz, DMSO-*d*₆) *δ* 11.33 (s, 1H), 7.88 (dd, *J =* 18.9, 8.2 Hz, 1H), 7.43 - 7.18 (m, 9H), 6.93 - 6.85 (m, 4H), 5.66 - 5.46 (m, 2H), 4.26 - 4.13 (m, 1H), 4.01 - 3.43 (m, 14H), 3.35 - 3.30 (d, *J* = 20 Hz, 3H), 2.72 - 2.69 (m, 1H), 2.63 - 2.42 (m, 1H), 1.08 (t, *J* = 6.1 Hz, 6H), 1.00 (t, *J =* 6.8 Hz, 6H); ³¹P NMR (162 MHz, DMSO-*d*₆) *δ* 151.23, 149.32. ESI-MS: m/z 759.3 [M-H]⁻

### Example 36: Synthesis of Compound 38'

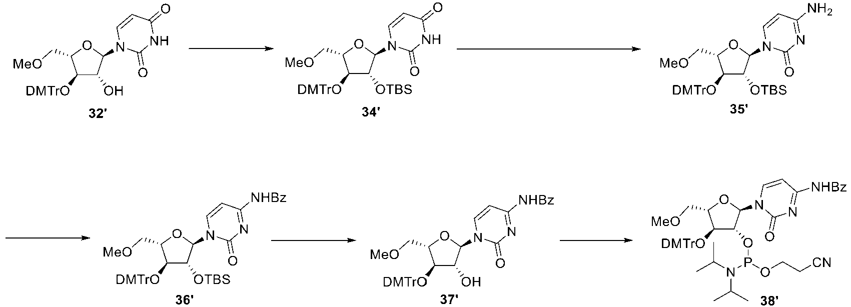

### Synthesis of Compound 34':

Compound **32'** (4.0 g, 7.14 mmol, 1.0 eq) was added to a 250 mL round-bottom flask. Ultra-dry DMF (50 mL) was added to the flask with stirring. Imidazole (1.94 g, 28.56 mmol, 4.0 eq) was added at room temperature and stirred for 10 min. Then, TBSCl (2.15 g, 14.28 mmol, 2.0 eq) was slowly added to the reaction system in batches and the reaction was carried out overnight at room temperature under nitrogen protection. The complete consumption of compound 32' was monitored by TLC and LCMS. The reaction was quenched by adding water. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 2/1) to afford compound **34'** (3.93 g, 5.82 mmol, 81.51% yield).
ESI-MS: m/z 673.3 [M-H]⁻

### Synthesis of Compound 35':

Compound **34'** (3.93 g, 5.82 mmol, 1.0 eq) was added to a 250 mL round-bottom flask. Ultra-dry ACN (40 mL) was added to the flask with stirring to completely dissolve the compound. TEA (1.18 g, 11.64 mmol, 2.0 eq) and DMAP (1.42 g, 11.64 mmol, 2.0 eq) were added to the reaction system and stirred well. The reaction was cooled to 0-5 °C, and the compound TPSCl (3.53 g, 11.64 mmol, 2.0 eq) was added slowly in batches. Once the addition was completed, the ice bath was removed, and the reaction was returned to room temperature. The reaction was stirred overnight at room temperature. The complete consumption of compound **34'** was monitored by TLC. At room temperature, aqueous ammonia (50 mL) was added to the reaction and stirred for about 12 hours until the intermediate was completely consumed. Saturated brine was added to the reaction, and the mixed system was extracted twice with ethyl acetate. The organic phases were combined. The organic phase was washed with water and saturated brine, followed by drying over anhydrous sodium sulfate and concentration under reduced pressure to give the crude product **35'** (6.21 g, calculated at 100% yield).
ESI-MS: m/z 672.3 [M-H]⁻

### Synthesis of Compound 36':

Crude compound **35'** (6.21 g, 1.0 eq) was added to a 250 mL round-bottom flask, and pyridine (60 mL) was added to the flask with stirring to completely dissolve the crude product **35'.** The reaction system was cooled to 0 °C, and BzCl (1.64 g, 11.64 mmol, 2.0 eq) was slowly added dropwise to the reaction system and stirred at 0 °C for 1 hour until the compound **35'** was completely consumed. The reaction was protected by nitrogen throughout. The reaction system was returned to room temperature, and the reaction was quenched by the addition of methanol and water. The mixed system was extracted twice with ethyl acetate and the organic phases were combined. The organic phase was washed with water and saturated brine, followed by drying over anhydrous sodium sulfate and removal of the solvent under reduced pressure to give the crude product. The crude product was subjected to column chromatography (PE/EA = 1/1) to afford compound **36'** (3.82 g, 4.91 mmol, 84.36% overall yield in 2 steps).
ESI-MS: m/z 776.3 [M-H]⁻

### Synthesis of Compound 37':

Compound **36'** (3.82 g, 4.91 mmol, 1.0 eq) was added to a 100 mL round-bottom flask and THF (40 mL) was added with stirring until completely dissolved. Triethylamine trihydrofluoride (5.0 mL) was neutralized to alkaline with triethylamine (20 mL) and added to the above reaction system. The reaction was placed in a 40°C oil bath and stirred overnight under nitrogen protection. The complete consumption of compound **36'** was monitored by TLC and LCMS. Water was added to the reaction, and the mixed system was extracted twice with ethyl acetate. The organic phases were combined. The organic phase was washed with water, saturated aqueous sodium bicarbonate solution and saturated brine, followed by drying over anhydrous sodium sulfate and removal of the solvent under reduced pressure to give the crude product. The crude product was subjected to column chromatography (PE/EA= 1/1) to afford compound **37'** (2.72 g, 4.10 mmol, 83.50% yield).
ESI-MS: m/z 662.3 [M-H]⁻

### Synthesis of Compound 38':

Dried compound **37'** (2.72 g, 4.10 mmol, 1.0 eq) was added to a 100 mL round-bottom flask, and ultra-dry DCM (30 mL) was added to the flask with stirring to dissolve the compound. DIPEA (1.06 g, 8.2 mmol, 2.0 eq) and DMAP (100 mg, 0.82 mmol, 0.2 eq) were added to the reaction, and the reaction was purged with nitrogen. The reaction was cooled to around 0 °C, and CEP-Cl (1.46 g, 6.15 mmol, 1.5 eq) was slowly added dropwise. The reaction was carried out at this temperature and under nitrogen protection for 1 hour until the compound **37'** was completely consumed. After completion of the reaction, the reaction was quenched by addition of saturated aqueous sodium bicarbonate to the reaction. The mixed system was extracted twice with dichloromethane, and the organic phases were combined. The organic phase was washed with water and saturated brine, followed by drying over anhydrous sodium sulfate and removal of the solvent under reduced pressure to give the crude product. The crude product was purified by column chromatography (PE/EA = 1/1) to afford compound **38'** (2.3 g, 2.66 mmol, 67.9% yield).
¹H NMR (400 MHz, DMSO-*d*₆) δ 11.16 (d, *J =* 24.4 Hz, 1H), 8.44 (dd, *J =* 30.5, 7.3 Hz, 1H), 7.98 (d, *J =* 7.7 Hz, 2H), 7.66 - 7.09 (m, 14H), 6.86 - 6.74 (m, 4H), 5.72 - 5.63 (m, 1H), 4.24 - 3.83 (m, 3H), 3.82 - 3.68 (m, 8H), 3.62 - 3.39 (m, 5H), 3.35 - 3.30 (d, *J =* 20 Hz, 3H), 2.76 - 2.68 (m, 1H), 2.52 - 2.31(m, 1H), 1.07 (t, *J* = 6.1 Hz, 6H), 1.01 (t, *J* = 6.8 Hz, 6H); ³¹P NMR (162 MHz, DMSO-*d*₆) δ 149.91, 149.03. ESI-MS: m/z 864.4 [M-H]⁻

### Example 37: Synthesis of Compound 43'

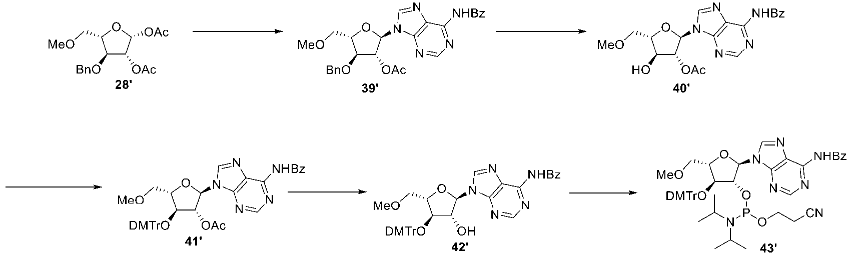

### Synthesis of Compound 39':

Compound **28'** (10.00 g, 29.55 mol, 1.00 eq) was added to a 500 mL three-necked round-bottom flask, and ultra-dry ACN (150 mL) was added to the flask with stirring to dissolve the compounds. Subsequently, BSA (18.03 g, 88.65 mmol, 3.0 eq) and A(Bz) (14.14 g, 59.10 mmol, 2.0 eq) were added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 30 min. TMSOTf (6.57 g, 29.55 mol, 1.0 eq) was slowly added dropwise to the reaction system. Once the addition was completed, the reaction system was placed in an oil bath and slowly warmed to 80°C, and the reaction was carried out at this temperature overnight. The complete consumption of compound **28'** was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 1/1) to afford compound **39'** (10.11 g, 19.53 mmol, 66.09% yield).
ESI-MS: m/z 518.2 [M+H]⁺

### Synthesis of Compound 40':

Compound **39'** (10.11 g, 19.53 mmol, 1.0 eq) was added to a 500 mL three-necked flask and ultra-dry DCM (200 mL) was added to the reaction flask. The reaction system was cooled to -20 °C and stirred at this temperature for 30 min. 1.0 mol/L of BCl₃ in dichloromethane (58.59 mL, 58.59 mmol, 3.0 eq) was slowly added dropwise to the reaction system and the reaction continued for 5 hours until the compound **39'** was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 1/2) to afford compound **40'** (5.21 g, 12.19 mmol, 62.42% yield).
ESI-MS: m/z 428.2 [M+H]⁺

### Synthesis of Compound 41':

Compound **40'** (5.21 g, 12.19 mmol, 1.0eq) was added to a 250 mL round-bottom flask. Ultra-dry DCE (80 mL) was added to the reaction flask with stirring to completely dissolve the compound. DMTrCl (8.26 g, 24.38 mmol, 2.0 eq), AgNO₃ (2.07 g, 12.19 mmol, 1.0 eq), 2,4,6-trimethylpyridine (7.39 g, 60.95 mmol, 5.0 eq) were added to the reaction at room temperature and stirred well. The reaction was placed in an oil bath and heated to 80°C, then carried out overnight. The complete consumption of compound **40'** was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give the crude product. The crude product was subjected to column chromatography (PE/EA= 2/3) to afford compound **41'** (7.75 g, 10.63 mmol, 87.20% yield).
ESI-MS: m/z 728.3 [M-H]⁻

### Synthesis of Compound 42':

Compound **41'** (7.75 g, 10.63 mmol, 1.0 eq) was added to a 250 mL round-bottom flask. THF (80 mL) was added to the reaction, and the reaction was stirred until completely dissolved. To the reaction was added 5.4 mol/L NaOMe solution (2.36 mL, 12.76 mmol, 1.2 eq). The reaction was stirred at room temperature until the compound **41'** was completely consumed. After the reaction was completed, the reaction system was extracted twice with water and ethyl acetate, and the organic phases were combined. The combined organic phase was washed with water and saturated brine, followed by drying over anhydrous sodium sulfate and removal of the solvent under reduced pressure to give the crude product. The crude product was subjected to column chromatography to afford compound **42'** (6.81 g, 9.92 mmol, 93.32% yield).
ESI-MS: m/z 686.3 [M-H]⁻

### Synthesis of Compound 43':

Dried compound **42'** (2.0 g, 2.91 mmol, 1.0 eq) was added to a 50 mL round-bottom flask. Ultra-dry DCM (20 mL) was added to the flask with stirring to completely dissolve the compound. DIPEA (752 mg, 5.82 mmol, 2.0 eq) and DMAP (71 mg, 0.58 mmol, 0.2 eq) were added to the reaction and stirred for 15 min at room temperature. The reaction system was purged with nitrogen and carried out under a nitrogen protection. Compound CEP-Cl (1.55 g, 6.56 mmol, 1.5 eq) was added dropwise to the reaction at room temperature. The reaction was carried out at room temperature for 30-60 min until the compound **42'** was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product. The crude product was purified by column chromatography to afford compound **43'** (2.24 g, 2.53 mmol, 86.94% yield).
¹H NMR (400 MHz, DMSO-*d*₆) δ 11.25 (s, 1H), 8.73 - 8.52 (m, 2H), 8.01 (d, *J =* 7.3 Hz, 2H), 7.69 - 7.51 (m, 3H), 7.38 - 7.32 (m, 2H), 7.28 - 7.11 (m, 7H), 6.87 - 6.72 (m, 4H), 6.03 (dd, *J* = 34.0, 3.2 Hz, 1H), 4.73 - 4.31 (m, 2H), 3.85 - 3.79 (m, 1H), 3.78 - 3.45 (m, 12H), 3.31 (d, *J* = 24.0, 3H), 2.71 - 2.56 (m, 2H), 1.12 - 0.88 (m, 12H); ³¹P NMR (162 MHz, DMSO-*d*₆) *δ* 150.71, 149.12; ESI-MS: m/z 886.4 [M-H]⁻

### Example 38: Synthesis of Compound 48'

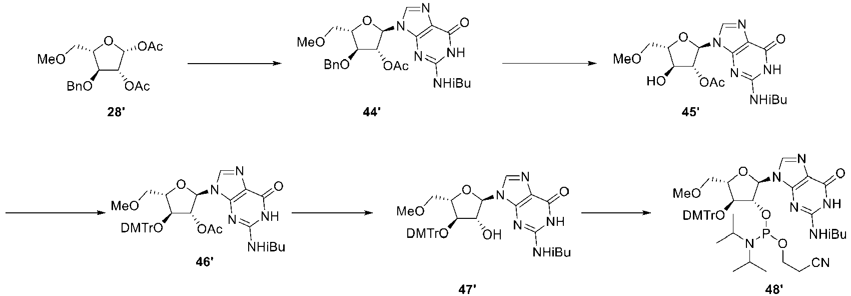

### Synthesis of Compound 44':

Compound **28'** (10.00 g, 29.55 mmol, 1.00 eq) was added to a 500 mL three-necked round-bottom flask, and ultra-dry ACN (150 mL) was added to the flask with stirring to dissolve the compounds. Subsequently, BSA (18.03 g, 88.65 mmol, 3.0 eq) and G(iBu) (13.07 g, 59.10 mmol, 2.0 eq) were added. The reaction system was placed in an oil bath and heated to 80 °C. The reaction system was stirred at this temperature for 1 hour. The entire process was conducted under a nitrogen atmosphere. When the reaction was completed, the reaction system was placed in an ice water bath at 0°C with stirring for 30 min. TMSOTf (6.57 g, 29.55 mmol, 1.0 eq) was slowly added dropwise to the reaction. Once the addition was completed, the reaction system was placed in an oil bath and slowly warmed to 80°C, and the reaction was carried out at this temperature overnight. The complete consumption of compound **28'** was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 1/1) to afford compound **44'** (9.62 g, 19.26 mmol, 65.18% yield).
ESI-MS: m/z 500.2 [M+H]⁺

### Synthesis of Compound 45':

Compound **44'** (9.62 g, 19.26 mmol, 1.0 eq) was added to a 250 mL three-necked flask and ultra-dry DCM (100 mL) was added to the reaction flask. The reaction system was cooled to -20 °C and stirred at this temperature for 30 min. 1.0 mol/L of BCl₃ in dichloromethane (57.78 mL, 57.78 mmol, 3.0 eq) was slowly added dropwise to the reaction system and the reaction continued for 5 hours until the compound **44'** was completely consumed. The reaction was quenched with triethylamine and methanol at -20°C. The reaction was returned to room temperature, and water was added. The mixed system was extracted with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 1/2) to afford compound **45'** (5.12 g, 12.51 mmol, 64.95% yield).
ESI-MS: m/z 410.2 [M+H]⁺

### Synthesis of Compound 46':

Compound **45'** (5.12 g, 12.51 mmol, 1.0eq) was added to a 250 mL round-bottom flask. Ultra-dry DCE (80 mL) was added to the reaction flask with stirring to completely dissolve the compound. DMTrCl (8.48 g, 25.02 mmol, 2.0 eq), AgNO₃ (2.13 g, 12.51 mmol, 1.0 eq), 2,4,6-trimethylpyridine (7.58 g, 62.55 mmol, 5.0 eq) were added to the reaction at room temperature and stirred well. The reaction was placed in an oil bath and heated to 80°C, then carried out overnight. The complete consumption of compound **45'** was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give the crude product. The crude product was subjected to column chromatography (PE/EA= 2/3) to afford compound **46'** (7.37 g, 10.35 mmol, 82.73% yield).
ESI-MS: m/z 710.3 [M-H]⁻

### Synthesis of Compound 47':

Compound **46'** (7.37 g, 10.35 mmol, 1.0 eq) was added to a 250 mL round-bottom flask. THF (80 mL) was added to the reaction, and the reaction was stirred until completely dissolved. To the reaction was added 5.4 mol/L NaOMe solution (2.30 mL, 12.42 mmol, 1.2 eq). The reaction was stirred at room temperature until the compound **46'** was completely consumed. After the reaction was completed, the reaction system was extracted twice with water and ethyl acetate, and the organic phases were combined. The combined organic phase was washed with water and saturated brine, followed by drying over anhydrous sodium sulfate and removal of the solvent under reduced pressure to give the crude product. The crude product was subjected to column chromatography to afford compound **47'** (6.58 g, 9.82 mmol, 91.48% yield).
ESI-MS: m/z 668.3 [M-H]⁻

### Synthesis of Compound 48':

Dried compound **47'** (2.00 g, 2.99 mmol, 1.0 eq) was added to a 50 mL round-bottom flask. Ultra-dry DCM (20 mL) was added to the flask with stirring to completely dissolve the compound. DIPEA (773 mg, 5.98 mmol, 2.0 eq) and DMAP (73 mg, 0.60 mmol, 0.2 eq) were added to the reaction and stirred for 15 min at room temperature. The reaction system was purged with nitrogen and carried out under a nitrogen protection. Compound CEP-Cl (1.06 g, 4.49 mmol, 1.5 eq) was added dropwise to the reaction at room temperature. The reaction was carried out at room temperature for 30-60 min until the compound **47'** was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product. The crude product was purified by column chromatography to afford compound **48'** (2.27 g, 2.61 mmol, 87.29% yield).
¹H NMR (400 MHz, DMSO-*d*₆) *δ* 12.08 (s, 1H), 11.52 (s, 1H), 8.22 (d, *J =* 29.6 Hz, 1H), 7.56 - 7.14 (m, 9H), 6.89 - 6.78 (m, 4H), 5.73 (dd, *J =* 38.6, 4.8 Hz, 1H), 5.23 - 5.11 (m, 1H), 4.51 (t, *J =* 5.5 Hz, 1H), 3.81 - 3.66 (m, 8H), 3.61 - 3.38 (m, 3H), 3.31 (d, *J =* 24.0, 3H), 3.23 - 3.19 (m, 1H), 3.11 (d, *J =* 8.4 Hz, 1H), 2.79 - 2.63 (m, 1H), 2.59 - 2.49 (m, 1H), 2.43 - 2.33 (m, 1H), 1.12 - 0.88 (m, 18H); ³¹P NMR (162 MHz, DMSO-*d*₆) *δ* 151.35, 149.21; ESI-MS: m/z 868.4 [M-H]⁻

### Example 39: Synthesis of Compound 56'

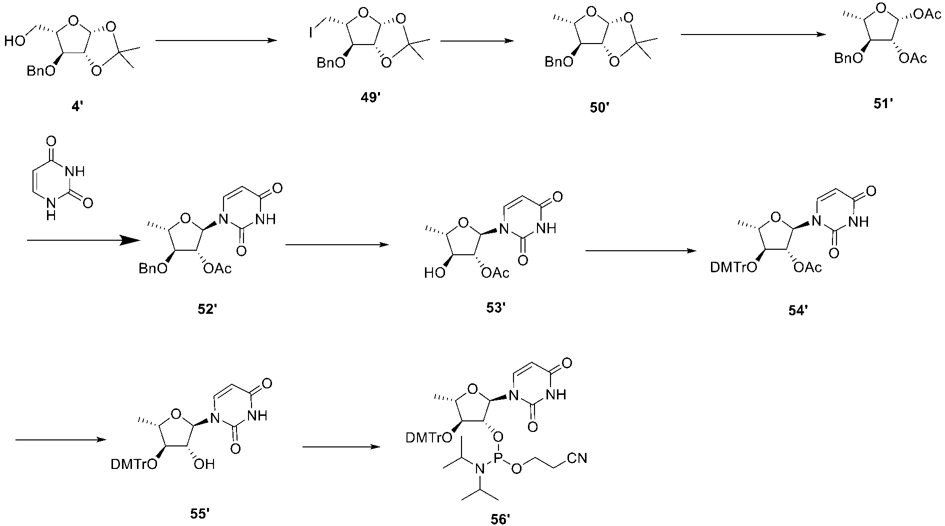

### Synthesis of Compound 49':

Compound **4'** (45.00 g, 160.53 mmol, 1.00 eq) was added to a 500 mL round-bottom flask and toluene (400 mL) was added to the reaction flask with stirring to dissolve the compound. Then imidazole (38.25 g, 561.86 mmol, 3.5 eq) and PPh₃ (50.53 g, 192.64 mmol, 1.2 eq) was added, followed by slow addition of I₂ (48.9 g, 192.64 mmol, 1.2eq). Once the addition was completed, the reaction system was placed in an oil bath and heated to 70 °C with stirring for 3 h. The complete consumption of compound **4'** was monitored by TLC and LCMS. The reaction was removed from the oil bath and cooled to room temperature. The reaction was quenched by the addition of aqueous sodium sulfite solution to the reaction system, and the system was extracted with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 1/4) to afford compound **49'** (54.40 g, 139.41 mmol, 86.8% yield).
ESI-MS: m/z 391.2 [M+H]⁺

### Synthesis of Compound 50':

Compound **49'** (54.40 g, 139.42 mmol, 1.0 eq) was dissolved in MeOH (500 mL), followed by the addition of K₂CO₃ (19.34 g, 139.42 mmol, 1.0 eq) and Pd/C (10% W, 5.4 g) to the reaction solution. Hydrogen gas was introduced and the reaction was stirred at room temperature until the compound **49'** was complete consumed. The system was filtered through diatomaceous earth, concentrated, extracted twice with ethyl acetate, filtered to remove salt, and concentrated under reduced pressure to give the crude product. The crude product was subjected to column chromatography (PE/EA= 4/1) to afford compound **50'** (33.20 g, 125.61 mmol, 90.1% yield).
¹H NMR (400 MHz, DMSO-*d*₆) *δ* 7.51 - 7.20 (m, 5H), 5.81 (d, *J* = 4.0 Hz, 1H), 4.72 - 4.64 (m, 2H), 4.49 (d, *J =* 12.0 Hz, 1H), 4.25 - 4.16 (m, 1H), 3.71 (d, *J =* 3.1 Hz, 1H), 1.38 (s, 3H), 1.25 (s, 3H), 1.20 (d, *J =* 6.4 Hz, 3H). ESI-MS: m/z 265.3 [M+H]⁺

### Synthesis of Compound 51':

Compound **50'** (33.20 g, 125.61 mmol, 1.0 eq) was added to a 500 mL round-bottom three-necked flask, and AcOH (300 mL) and Ac₂O (64.12 g, 628.05 mmol, 5.0 eq) were added to the reaction system and stirred to completely dissolve the compound. The reaction was cooled to 0°C and stirred at this temperature for 30 min. Concentrated sulfuric acid (10 mL) was added slowly dropwise to the reaction system, and the reaction system was kept at a temperature of 0-5°C at all times. Once the addition was completed, the reaction system was returned to room temperature and stirred for additional 8 hours until the compound **50'** was completely consumed. The reaction system was cooled to around -5°C and neutralized to a pH of about 7 with aqueous ammonia. Water was added to the reaction system, and the reaction system was extracted twice with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 4/1) to afford compound **51'** (21.20 g, 68.76 mmol, 54.7% yield).
ESI-MS: m/z 309.3 [M+H]⁺

### Synthesis of Compound 52':

Dried compound **51'** (10.00 g, 32.43 mmol, 1.00 eq) and uracil (7.27 g, 64.86 mmol, 2.0 eq) were added to a 500 mL three-necked round-bottom flask, and ultra-dry ACN (100 mL) was added to the flask with stirring to dissolve the compounds. Subsequently, BSA (19.79 g, 97.29 mmol, 3.0 eq) was added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 30 min. TMSOTf (7.21 g, 32.43 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound **51'** was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 1/3) to afford compound **52'** (9.61 g, 26.64 mmol, 82.1% yield).
¹H NMR (400 MHz, DMSO-*d*₆) *δ* 11.36 (s, 1H), 7.46 - 7.21 (m, 6H), 6.32 (d, *J =* 8.0 Hz, 1H), 5.90 (t, *J =* 9.0 Hz, 1H), 5.18 (s, 1H), 4.77 (d, *J* = 11.5 Hz, 1H), 4.58 (t, *J* = 11.0 Hz, 1H), 4.31 - 4.18 (m, 1H), 3.94 (d, *J =* 3.4 Hz, 1H), 2.11 (s, 3H), 1.33 (d, *J =* 6.3 Hz, 3H). ESI-MS: m/z 361.34 [M+H]⁺

### Synthesis of Compound 53':

Dried compound **52'** (9.61 g, 26.64 mmol, 1.0 eq) was added to a 500 mL three-necked flask and ultra-dry DCM (100 mL) was added to the reaction flask. The reaction system was cooled to -20 °C and stirred at this temperature for 30 min. BCl₃ (1M in toluene, 80 mL, 79.92 mmol, 3.0 eq) was slowly added dropwise to the reaction system. Once the dropwise addition was complete, the reaction was allowed to warm to -10°C and continued until the compound **52'** was completely consumed. The reaction was quenched with triethylamine and methanol at -10°C. The reaction was returned to room temperature, and water was added. The mixed system was extracted with dichloromethane, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 1/2) to afford compound **53'** (5.01 g, 18.5 mol, 69.4% yield).
¹H NMR (400 MHz, DMSO-*d*₆) δ 11.36 (s, 1H), 7.59 (s, 1H), 6.32 (d, *J =* 8.0 Hz, 1H), 5.90 (t, *J* = 9.0 Hz, 1H), 5.18 (s, 1H), 4.49 (d, *J* = 7.8 Hz, 1H), 4.31 - 4.18 (m, 1H), 3.94 (d, *J* = 3.4 Hz, 1H), 2.11 (s, 3H), 1.33 (d, *J* = 6.3 Hz, 3H). ESI-MS: m/z 271.24 [M+H]

### Synthesis of Compound 54':

Compound **53'** (5.01 g, 18.5 mmol, 1.0 eq) was added to a 100 mL round-bottom flask. Ultra-dry DCE (100 mL) was added to the reaction flask with stirring to completely dissolve the compound. DMTrCl (12.54 g, 37.0 mmol, 2.0 eq), AgNO₃ (3.14 g, 18.5 mmol, 1.0 eq) and 2,4,6-trimethylpyridine (11.21 g, 92.5 mmol, 5.0 eq) were added to the reaction at room temperature and stirred well. The reaction was placed in an oil bath and heated to 80°C, then carried out overnight. The complete consumption of compound **53'** was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give the crude product. The crude product was subjected to column chromatography (PE/EA= 1/1) to afford compound **54'** (7.30 g, 12.75 mmol, 70% yield).
¹H NMR (400 MHz, DMSO-*d*₆) δ 11.36 (s, 1H), 7.46 (d, *J =* 7.5 Hz, 3H), 7.39 - 7.22 (m, 7H), 6.92 (dd, *J* = 8.9, 7.3 Hz, 4H), 6.32 (d, *J* = 8.0 Hz, 1H), 5.90 (t, *J =* 9.0 Hz, 1H), 5.18 (s, 1H), 4.31 - 4.18 (m, 1H), 3.94 (d, *J =* 3.4 Hz, 1H), 3.81 (s, 6H), 2.11 (s, 3H), 1.33 (d, *J =* 6.3 Hz, 3H). ESI-MS: m/z 573.6 [M-H]⁻

### Synthesis of Compound 55':

Compound **54'** (7.30 g, 12.75 mmol, 1.0 eq) was dissolved in THF (100 mL), followed by the addition of NaOMe (826.51 mg, 15.3 mmol, 1.2 eq) to the reaction solution. The reaction was stirred at room temperature until the compound **54'** was completely consumed. Water was added to the reaction system, and the reaction system was extracted twice with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 1/2) to afford compound **55'** (6.21 g, 11.68 mmol, 91.61% yield).
¹H NMR (400 MHz, DMSO-*d*₆) δ 11.36 (s, 1H), 7.46 (d, *J =* 7.5 Hz, 3H), 7.39 - 7.22 (m, 7H), 6.92 (dd, *J* = 8.9, 7.3 Hz, 4H), 6.32 (d, *J* = 8.0 Hz, 1H), 5.90 (t, *J* = *9.0* Hz, 1H), 5.30 (d, *J* = 3.8 Hz, 1H), 5.18 (s, 1H), 4.31 - 4.18 (m, 1H), 3.94 (d, *J* = 3.4 Hz, 1H), 3.81 (s, 6H), 1.33 (d, *J* = 6.3 Hz, 3H). ESI-MS: m/z 531.6 [M-H]⁻

### Synthesis of Compound 56':

Dried compound **55'** (2.00 g, 3.77 mmol, 1.0 eq) was added to a 100ml round-bottom flask. Ultra-dry DCM (20 mL) was added to the flask with stirring to completely dissolve the compound. DIPEA (974.5 mg, 7.54 mmol, 2.0 eq) and DMAP (92.12 mg, 0.754 mmol, 0.2 eq) were added to the reaction and stirred for 15 min at room temperature. The reaction system was purged with nitrogen and carried out under a nitrogen protection. Compound CEP-Cl (1.34 g, 5.66 mmol, 1.5 eq) was added dropwise to the reaction at room temperature. The reaction was carried out at room temperature for 30-60 min until the compound **55'** was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product. The crude product was purified by column chromatography (PE/EA= 1/1) to afford compound **56'** (2.31 g, 3.15 mmol, 83.55% yield).
¹H NMR (400 MHz, DMSO-*d*₆) *δ* 11.36 (s, 1H), 7.46 (d, *J =* 7.5 Hz, 3H), 7.39 - 7.22 (m, 7H), 6.92 (dd, *J* = 8.9, 7.3 Hz, 4H), 6.32 (d, *J* = 8.0 Hz, 1H), 5.90 (t, *J* = 9.0 Hz, 1H), 5.18 (s, 1H), 4.31 - 4.18 (m, 1H), 3.99 - 3.80 (m, 9H), 2.95 - 2.70 (m, 2H), 2.60 - 2.49 (m, 2H),1.33 (d, *J* = 6.3 Hz, 3H), 1.12 - 0.92 (m, 12H). ³¹P NMR (162 MHz, DMSO-*d*₆) *δ* 150.02 (s), 149.73 (s). ESI-MS: m/z 731.8 [M-H]⁻

### Example 40: Synthesis of Compound 61'

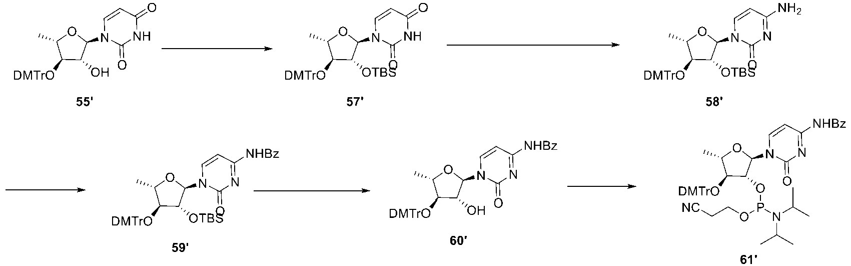

### Synthesis of Compound 57':

Compound **55'** (1.98 g, 3.74 mmol, 1.0 eq) was added to a 100 mL round-bottom flask. Ultra-dry DMF (30 mL) was added to the flask with stirring. Imidazole (1.02 g, 14.96 mmol, 4.0 eq) was added at room temperature and stirred for 10 min. Then, TBSCl (1.13 g, 7.48 mmol, 2.0 eq) was slowly added to the reaction system in batches and the reaction was carried out overnight at room temperature under nitrogen protection. The complete consumption of compound **55'** was monitored by TLC and LCMS. The reaction was quenched by adding water. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 1/1) to afford compound **57'** (2.15 g, 3.32 mmol, 88.77% yield).
ESI-MS: m/z 645.8 [M-H]⁻

### Synthesis of Compound 58':

Compound **57'** (2.15 g, 3.32 mmol, 1.0 eq) was added to a 100ml round-bottom flask. Ultra-dry ACN (30 mL) was added to the flask with stirring to completely dissolve the compound. TEA (672 mg, 6.64 mmol, 2.0 eq) and DMAP (811 mg, 6.64 mmol, 2.0 eq) were added to the reaction system and stirred well. The reaction was cooled to 0-5 °C, and the compound TPSCl (2.01 g, 6.64 mmol, 2.0 eq) was added slowly in batches. Once the addition was completed, the ice bath was removed, and the reaction was returned to room temperature. The reaction was stirred overnight at room temperature. The complete consumption of compound **57'** was monitored by TLC. At room temperature, aqueous ammonia (20 mL) was added to the reaction and stirred for about 12 hours until the intermediate was completely consumed. Saturated brine was added to the reaction, and the mixed system was extracted twice with ethyl acetate. The organic phases were combined. The organic phase was washed with water and saturated brine, followed by drying over anhydrous sodium sulfate and concentration under reduced pressure to give the crude product 58 (2.42 g, calculated at 100% yield).
ESI-MS: m/z 644.9 [M+H]⁺

### Synthesis of Compound 59':

Crude compound **58'** (2.42 g) was added to a 100 mL round-bottom flask, and pyridine (50 mL) was added to the flask with stirring to completely dissolve the crude product **58'.** The reaction system was cooled to 0 °C, and BzCl (933 mg, 6.64 mmol, 2.0 eq) was slowly added dropwise to the reaction system and stirred at 0 °C for 1 hour until the compound **58'** was completely consumed. The reaction was protected by nitrogen throughout. The reaction system was returned to room temperature, and the reaction was quenched by the addition of methanol and water. The mixed system was extracted twice with ethyl acetate, and the organic phases were combined. The organic phase was washed with water and saturated brine, followed by drying over anhydrous sodium sulfate and removal of the solvent under reduced pressure to give the crude product. The crude product was subjected to column chromatography (PE/EA = 1/1) to afford compound **59'** (1.81 g, 2.41 mmol, 72.59% overall yield in 2 steps).
ESI-MS: m/z 752.93 [M+-H]⁻

### Synthesis of Compound 60':

Compound **59'** (1.81 g, 2.41 mmol, 1.0 eq) was added to a 100 mL round-bottom flask and THF (20 mL) was added with stirring until completely dissolved. Triethylamine trihydrofluoride (5.0 mL) was neutralized to alkaline with triethylamine (17 mL) and added to the above reaction system. The reaction was placed in a 40°C oil bath and stirred overnight under nitrogen protection. The complete consumption of compound **59'** was monitored by TLC and LCMS. Water was added to the reaction, and the mixed system was extracted twice with ethyl acetate. The organic phases were combined. The organic phase was washed with water, saturated aqueous sodium bicarbonate solution and saturated brine, followed by drying over anhydrous sodium sulfate and removal of the solvent under reduced pressure to give the crude product. The crude product was subjected to column chromatography (PE/EA= 1/3) to afford compound **60'** (1.16 g, 1.83 mmol, 75.93% yield).
¹H NMR (400 MHz, DMSO-*d*₆) δ 11.36 (s, 1H), 7.46 (d, *J =* 7.5 Hz, 3H), 7.92 - 7.72 (m, 2H), 7.69 - 7.46 (m, 3H), 7.39 - 7.22 (m, 7H), 6.92 (dd, *J* = 8.9, 7.3 Hz, 4H), 6.32 (d, *J* = 8.0 Hz, 1H), 5.90 (t, *J* = 9.0 Hz, 1H), 5.30 (d, *J* = 3.8 Hz, 1H), 5.18 (s, 1H), 4.31 - 4.18 (m, 1H), 3.94 (d, *J* = 3.4 Hz, 1H), 3.81 (s, 6H), 1.33 (d, *J* = 6.3 Hz, 3H). ESI-MS: m/z 634.7 [M-H]⁻

### Synthesis of Compound 61':

Dried compound **60'** (1.16 g, 1.83 mmol, 1.0 eq) was added to a 100 mL round-bottom flask, and ultra-dry DCM (20 mL) was added to the flask with stirring to dissolve the compound. DIPEA (473 mg, 3.66 mmol, 2.0 eq) and DMAP (45 mg, 0.37 mmol, 0.2 eq) were added to the reaction, and the reaction was purged with nitrogen. The reaction was cooled to around 0 °C, and CEP-Cl (651 mg, 2.75 mmol, 1.5 eq) was slowly added dropwise. The reaction was carried out at this temperature and under nitrogen protection for 1 hour until the compound **60'** was completely consumed. After completion of the reaction, the reaction was quenched by addition of saturated aqueous sodium bicarbonate to the reaction. The mixed system was extracted twice with dichloromethane, and the organic phases were combined. The organic phase was washed with water and saturated brine, followed by drying over anhydrous sodium sulfate and removal of the solvent under reduced pressure to give the crude product. The crude product was purified by column chromatography (PE/EA = 1/1) to afford compound **61'** (1.22 g, 1.46 mmol, 79.78% yield).
¹H NMR (400 MHz, DMSO-*d*₆) δ 11.36 (s, 1H), 7.92 - 7.72 (m, 2H), 7.69 - 7.46 (m, 3H),7.41 (d, *J =* 7.5 Hz, 3H), 7.39 - 7.22 (m, 7H), 6.92 (dd, *J =* 8.9, 7.3 Hz, 4H), 6.32 (d, *J* = 8.0 Hz, 1H), 5.90 (t, *J* = 9.0 Hz, 1H), 5.18 (s, 1H), 4.31 - 4.18 (m, 1H), 3.99 - 3.80 (m, 9H), 2.95 - 2.70 (m, 2H), 2.60 - 2.49 (m, 2H),1.33 (d, *J =* 6.3 Hz, 3H), 1.12 - 0.92 (m, 12H). ³¹P NMR (162 MHz, DMSO-*d*₆) *δ* 149.92 (s), 149.69 (s). ESI-MS: m/z 834.9 [M-H]⁻

### Example 41: Synthesis of Compound 66'

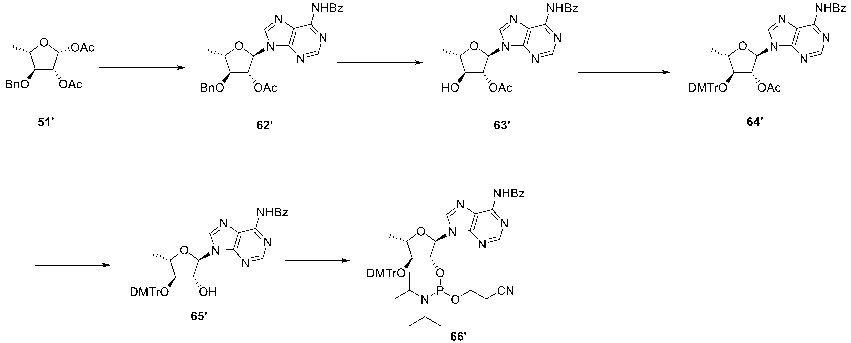

### Synthesis of Compound 62':

Compound **51'** (10.00 g, 32.43 mmol, 1.00 eq) and A(Bz) (15.52 g, 64.86 mmol, 2.0 eq) were added to a 500 mL three-necked round-bottom flask, and ultra-dry ACN (150 mL) was added to the flask with stirring to dissolve the compounds. Subsequently, BSA (19.79 g, 97.29 mmol, 3.0 eq) was added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 30 min. TMSOTf (7.21 g, 32.43 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound **51'** was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 1/5) to afford compound **62'** (11.40 g, 23.38 mmol, 72.1% yield).
¹H NMR (400 MHz, DMSO-*d*₆) *δ* 11.17 (s, 1H), 8.35 (s, 1H), 8.21 (s, 1H), 8.01 - 7.87 (m, 2H), 7.69 - 7.47 (m, 3H), 7.46 - 7.21 (m, 5H), 5.90 (t, *J* = 9.0 Hz, 1H), 5.18 (s, 1H), 4.77 (d, *J* = 11.5 Hz, 1H), 4.58 (t, *J =* 11.0 Hz, 1H), 4.31 - 4.18 (m, 1H), 3.94 (d, *J =* 3.4 Hz, 1H), 2.11 (s, 3H), 1.33 (d, *J =* 6.3 Hz, 3H). ESI-MS: m/z 488.5 [M+H]⁺

### Synthesis of Compound 63':

Compound **62'** (11.40 g, 23.38 mmol, 1.0 eq) was added to a 500 mL three-necked flask and ultra-dry DCM (100 mL) was added to the reaction flask. The reaction system was cooled to -80 °C and stirred at this temperature for 30 min. BCl₃ (1M in toluene, 70 mL, 70.14 mmol, 3.0 eq) was slowly added dropwise to the reaction system. Once the dropwise addition was complete, the reaction was allowed to warm to -10°C and continued until the compound **62'** was completely consumed. The reaction was quenched with triethylamine and methanol at -10°C. The reaction was returned to room temperature, and water was added. The mixed system was extracted with dichloromethane, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 1/10) to afford compound **63'** (4.80 g, 12.08 mmol, 51.67% yield).
ESI-MS: m/z 398.4 [M+H]⁺

### Synthesis of Compound 64':

Compound **63'** (4.80 g, 12.08 mmol, 1.0 eq) was added to a 250 mL round-bottom flask. Ultra-dry DCE (50 mL) was added to the reaction flask with stirring to completely dissolve the compound. DMTrCl (8.19 g, 24.16 mmol, 2.0 eq), AgNO₃ (2.05 g, 12.08 mmol, 1.0 eq) and 2,4,6-trimethylpyridine (7.32 g, 60.40 mmol, 5.0 eq) were added to the reaction at room temperature and stirred well. The reaction was placed in an oil bath and heated to 80°C, then carried out overnight. The complete consumption of compound **63'** was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give the crude product. The crude product was subjected to column chromatography (PE/EA= 1/3) to afford compound **64'** (6.20 g, 8.86 mmol, 73.34% yield).
ESI-MS: m/z 700.7 [M-H]⁻

### Synthesis of Compound 65':

Compound **64'** (6.20 g, 8.86 mmol, 1.0 eq) was dissolved in THF (100 mL), followed by the addition of aqueous ammonia to the reaction solution. The reaction was stirred at room temperature until the compound **64'** was completely consumed. Water was added to the reaction system, and the reaction system was extracted twice with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 1/5) to afford compound **65'** (5.21 g, 7.91 mmol, 90% yield).
¹H NMR (400 MHz, DMSO-*d*₆) *δ* 11.17 (s, 1H), 8.35 (s, 1H), 8.21 (s, 1H), 8.01 - 7.87 (m, 2H), 7.69 - 7.47 (m, 3H), 7.33 - 7.22 (m, 9H), 6.94 - 6.82 (m, 4H), 5.90 (t, *J* = 9.0 Hz, 1H), 5.23 (d, *J* = 3.8 Hz, 1H), 5.18 (s, 1H), 4.31 - 4.18 (m, 1H), 3.94 (d, *J =* 3.4 Hz, 1H), 3.82 (s, 6H), 1.33 (d, *J* = 6.3 Hz, 3H). ESI-MS: m/z 660.2 [M+H]⁻

### Synthesis of Compound 66':

Dried compound **65'** (5.21 g, 7.91 mmol, 1.0 eq) was added to a 100ml round-bottom flask. Ultra-dry DCM (50 mL) was added to the flask with stirring to completely dissolve the compound. DIPEA (2.04 g, 15.82 mmol, 2.0 eq) and DMAP (193 mg, 1.58 mmol, 0.2 eq) were added to the reaction and stirred for 15 min at room temperature. The reaction system was purged with nitrogen and carried out under a nitrogen protection. Compound CEP-Cl (2.81 g, 11.87 mmol, 1.5 eq) was added dropwise to the reaction at room temperature. The reaction was carried out at room temperature for 30-60 min until the compound **65'** was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product. The crude product was purified by column chromatography (PE/EA= 1/3) to afford compound **66'** (5.0 g, 5.83 mmol, 74% yield).
¹H NMR (400 MHz, DMSO-*d*₆) δ 11.17 (s, 1H), 8.35 (s, 1H), 8.21 (s, 1H), 8.01 - 7.87 (m, 2H), 7.69 - 7.47 (m, 3H), 7.33 - 7.22 (m, 9H), 6.94 - 6.82 (m, 4H), 5.90 (t, *J* = 9.0 Hz, 1H), 5.18 (s, 1H), 4.31 - 4.18 (m, 1H), 3.99 - 3.85 (m, 3H), 3.82 (s, 6H), 2.96 - 2.74 (m, 2H), 2.73 - 2.66 (m, 2H),1.33 (d, *J =* 6.3 Hz, 3H), 1.06 (d, *J* = 12.0 Hz, 12H). ³¹P NMR (162 MHz, DMSO-*d*₆) δ 150.91 (s), 149.82 (s). ESI-MS: m/z 858.9 [M-H]⁻

### Example 42: Synthesis of Compound 71'

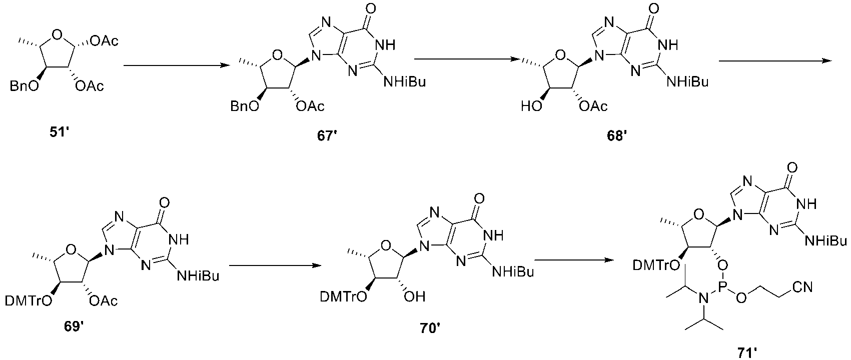

### Synthesis of Compound 67':

Compound **51'** (10.00 g, 32.43 mmol, 1.00 eq) and G(iBu) (14.3 g, 64.86 mmol, 2.0 eq) were added to a 500 mL three-necked round-bottom flask, and ultra-dry ACN (150 mL) was added to the flask with stirring to dissolve the compounds. Subsequently, BSA (19.8 g, 97.29 mmol, 3.0 eq) was added. The reaction system was placed in an oil bath and heated to 80 °C. The reaction system was stirred at this temperature for 1 hour. The entire process was conducted under a nitrogen atmosphere. When the reaction was complete, the reaction system was placed in an ice water bath at 0°C with stirring for 30 min. TMSOTf (7.21 g, 32.43 mmol, 1.0 eq) was slowly added dropwise to the reaction system. Once the addition was completed, the reaction system was placed in an oil bath and slowly warmed to 80°C, and the reaction was carried out at this temperature overnight. The complete consumption of compound **51'** was monitored by TLC and LCMS. The reaction was removed from the oil bath and cooled to room temperature. The reaction was quenched by the addition of saturated aqueous sodium bicarbonate to the reaction system, and the system was extracted with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 1/6) to afford compound **67'** (11.60 g, 24.71 mmol, 76.2% yield).
¹H NMR (400 MHz, DMSO-*d*₆) δ 12.09 (s, 1H), 11.53 (s, 1H), 7.91 (s, 1H), 7.46 - 7.21 (m, 5H), 5.90 (t, *J =* 9.0 Hz, 1H), 5.18 (s, 1H), 4.77 (d, *J =* 11.5 Hz, 1H), 4.58 (t, *J =* 11.0 Hz, 1H), 4.31 - 4.18 (m, 1H), 3.94 (d, *J* = 3.4 Hz, 1H), 2.87 - 2.53 (m, 1H), 2.11 (s, 3H), 1.33 (d, *J =* 6.3 Hz, 3H), 1.10 (d, *J* = 12.9 Hz, 6H). ESI-MS: m/z 470.5 [M+H]⁺

### Synthesis of Compound 68':

Compound **67'** (11.60 g, 24.71 mmol, 1.0 eq) was added to a 500 mL three-necked flask and ultra-dry DCM (150 mL) was added to the reaction flask. The reaction system was cooled to -78 °C and stirred at this temperature for 30 min. Boron trichloride (1M in toluene, 74 mL, 74.13 mmol, 3.0 eq) was slowly added dropwise to the reaction system. Once the dropwise addition was complete, the reaction was allowed to warm to -10°C and continued until the compound **67'** was completely consumed. The reaction was quenched with triethylamine and methanol at -78°C. The reaction was returned to room temperature, and water was added. The mixed system was extracted with dichloromethane, and the organic phases were combined. The organic phase was washed with water and saturated brine, followed by drying over anhydrous sodium sulfate and removal of the solvent under reduced pressure to give the crude product. The crude product was subjected to column chromatography (PE/EA= 1/20) to afford compound **68'** (5.61 g, 14.76 mmol, 60% yield).
ESI-MS: m/z 380.37 [M+H]⁺

### Synthesis of Compound 69':

Compound **68'** (5.61 g, 14.76 mmol, 1.0 eq) was added to a 100 mL round-bottom flask. Ultra-dry DCE (50 mL) was added to the reaction flask with stirring to completely dissolve the compound. DMTrCl (10.0 g, 29.52 mmol, 2.0 eq), AgNO₃ (2.51 g, 14.76 mmol, 1.0 eq) and 2,4,6-trimethylpyridine (8.9 g, 73.8 mmol, 5.0 eq) were added to the reaction at room temperature and stirred well. The reaction was placed in an oil bath and heated to 80°C, then carried out overnight. The complete consumption of compound **68'** was monitored by TLC and LCMS. The reaction was returned to room temperature and quenched by the addition of methanol. The reaction was diluted with ethyl acetate and filtered through diatomaceous earth to obtain a filtrate. The filter cake was washed twice with ethyl acetate. The filtrates were combined and concentrated under reduced pressure to give the crude product. The crude product was subjected to column chromatography (PE/EA= 1/10) to afford compound **69'** (7.20 g, 10.56 mmol, 71.5% yield).
ESI-MS: m/z 682.7[M-H]⁻

### Synthesis of Compound 70':

Compound **69'** (7.20 g, 10.56 mmol, 1.0 eq) was dissolved in THF (100 mL), followed by the addition of aqueous ammonia to the reaction solution. The reaction was stirred at room temperature until the compound **69'** was completely consumed. Water was added to the reaction system, and the reaction system was extracted twice with ethyl acetate. The organic phases were combined. The organic phase was washed with water and saturated brine, followed by drying over anhydrous sodium sulfate and removal of the solvent under reduced pressure to give the crude product. The crude product was subjected to column chromatography (PE/EA= 1/20) to afford compound **70'** (5.30 g, 8.29 mmol, 78.5% yield).
¹H NMR (400 MHz, DMSO-*d*₆) δ 12.09 (s, 1H), 11.53 (s, 1H), 7.91 (s, 1H), 7.38 - 7.20 (m, 9H), 6.96 - 6.74 (m, 4H), 5.90 (t, *J =* 9.0 Hz, 1H), 5.30 (d, *J =* 3.8 Hz, 1H), 5.18 (s, 1H), 4.31 - 4.18 (m, 1H), 3.94 (d, *J =* 3.4 Hz, 1H), 3.80 (s, 6H), 2.87 - 2.53 (m, 1H), 1.33 (d, *J =* 6.3 Hz, 3H), 1.10 (d, *J =* 12.9 Hz, 6H). ESI-MS: m/z 640.7 [M-H]⁻

### Synthesis of Compound 71':

Dried compound **70'** (2.00 g, 3.13 mmol, 1.0 eq) was added to a 100ml round-bottom flask. Ultra-dry DCM (20 mL) was added to the flask with stirring to completely dissolve the compound. DIPEA (809 mg, 6.26 mmol, 2.0 eq) and DMAP (77 mg, 0.63 mmol, 0.2 eq) were added to the reaction and stirred for 15 min at room temperature. The reaction system was purged with nitrogen and carried out under a nitrogen protection. Compound CEP-Cl (1.1 g, 4.70 mmol, 1.5 eq) was added dropwise to the reaction at room temperature. The reaction was carried out at room temperature for 30-60 min until the compound **70'** was completely consumed. Saturated aqueous sodium bicarbonate was added to the reaction system to quench the reaction. The reaction system was extracted twice with dichloromethane. The organic phases were combined. The combined organic phase was washed with water and saturated brine, followed by drying over anhydrous sodium sulfate and removal of the solvent under reduced pressure to give the crude product. The crude product was purified by column chromatography (PE/EA= 1/3) to afford compound **71'** (2.01 g, 2.38 mmol, 76% yield).
¹H NMR (400 MHz, DMSO-*d*₆) *δ* 12.09 (s, 1H), 11.53 (s, 1H), 7.91 (s, 1H), 7.38 - 7.20 (m, 9H), 6.96 - 6.74 (m, 4H), 5.90 (t, *J* = 9.0 Hz, 1H), 5.30 (d, *J* = 3.8 Hz, 1H), 5.18 (s, 1H), 4.31 - 4.18 (m, 1H), 4.00 - 3.83 (m, 3H), 3.80 (s, 6H), 2.93 - 2.53 (m, 5H), 1.33 (d, *J =* 6.3 Hz, 2H), 1.10 (d, *J =* 12.9 Hz, 6H), 1.06 (d, *J =* 12.0 Hz, 12H). ³¹P NMR (162 MHz, DMSO-*d*₆) *δ* 151.11 (s), 149.91 (s). ESI-MS: m/z 840.9 [M-H]⁻

**Example 43 below is the preparation example of the nucleoside compound shown in Formula 3-b of the present invention (Synthetic route), wherein the nucleoside compound shown in Formula 3-b is a TNA structural compound with 2'-phosphoramidite moiety, 3'-ODMTr, and 4'-modification.**

### Example 43:

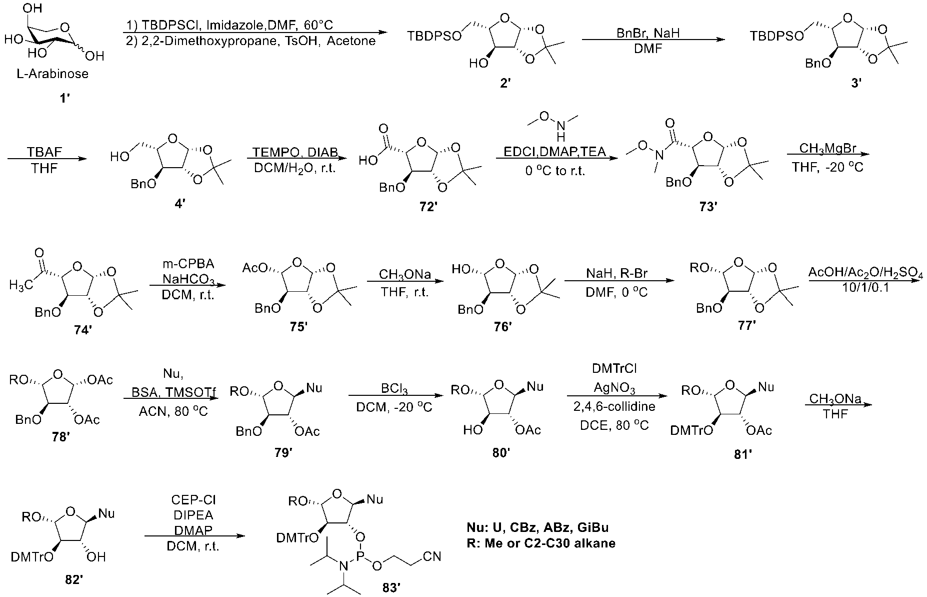

**Example 44 below is the preparation example of the nucleoside compound shown in Formula 4-b of the present invention (Synthetic route), wherein the nucleoside compound shown in Formula 4-b is a TNA structural compound with 2'-phosphoramidite moiety, 3'-ODMTr, and 4'-modification.**

### Example 44:

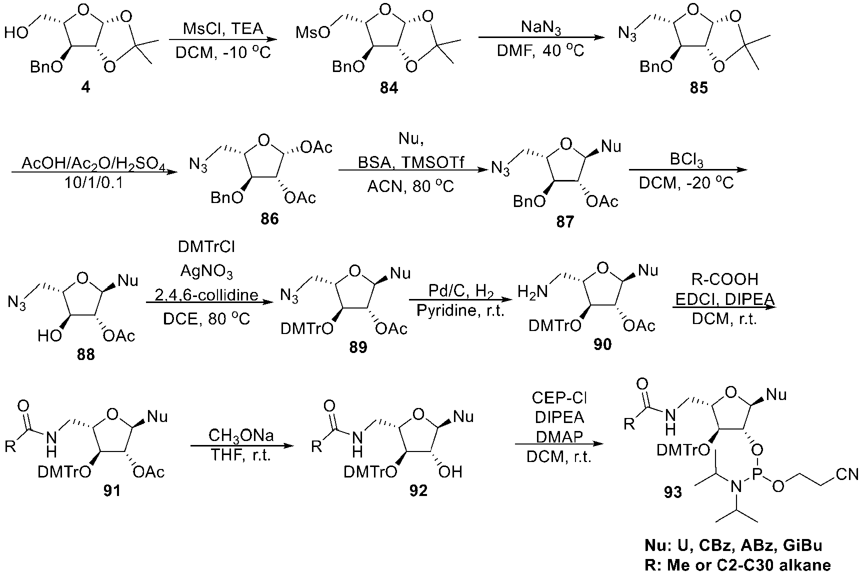

**Example 45 below is the preparation example of the nucleoside compound shown in Formula 5-b of the present invention (Synthetic route), wherein the nucleoside compound shown in Formula 5-b is a TNA structural compound with 2'-phosphoramidite moiety, 3'-ODMTr, and 4'-modification.**

### Example 45:

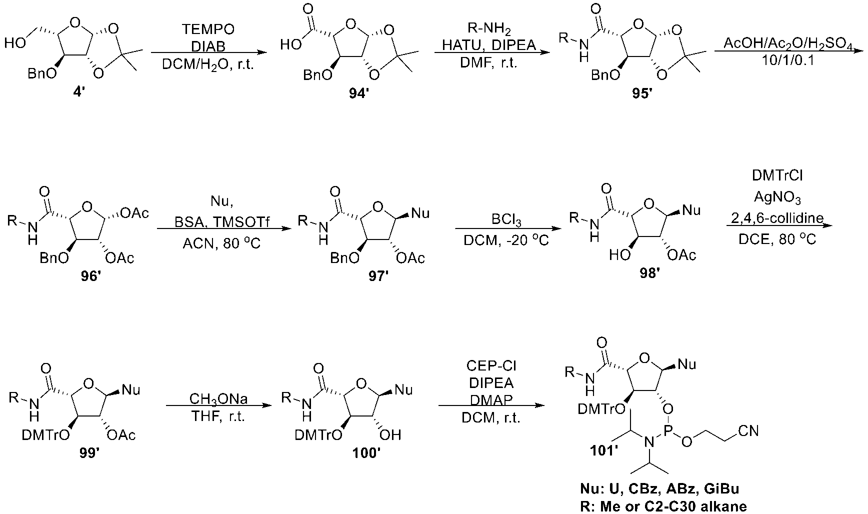

**Example 46 below is the preparation example of the nucleoside compound shown in Formula 6-b of the present invention (Synthetic route), wherein the nucleoside compound shown in Formula 6-b is a TNA structural compound with 2'-phosphoramidite moiety, 3'-ODMTr, and 4'-modification.**

### Example 46:

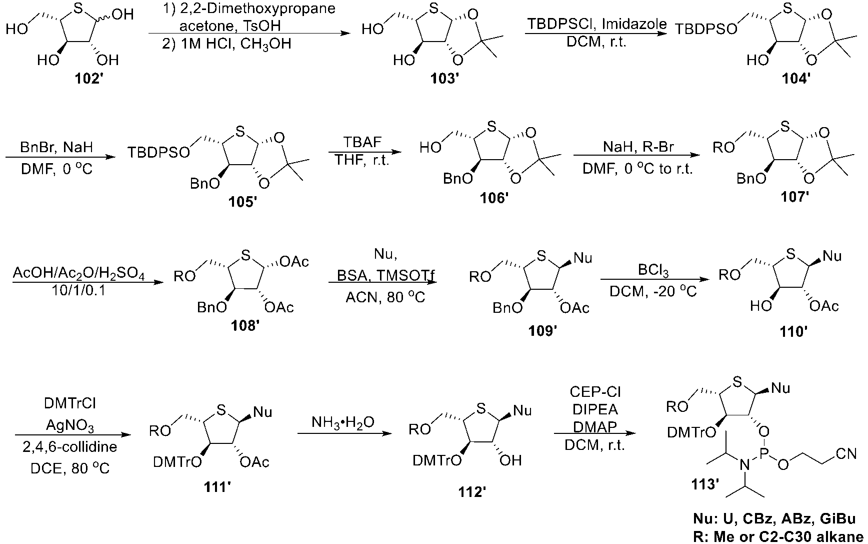

**Example 47 below is the preparation example of the nucleoside compound shown in Formula 7-b of the present invention (Synthetic route), wherein the nucleoside compound shown in Formula 7-b is a TNA structural compound with 2'-phosphoramidite moiety, 3'-ODMTr, and 4'-modification.**

### Example 47:

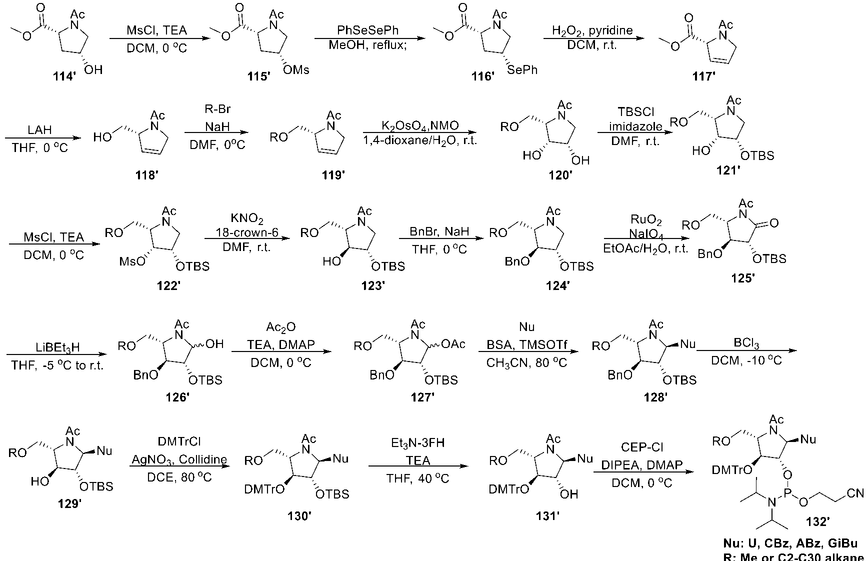

**Example 48 below is the preparation example of the nucleoside compound shown in Formula 8-b of the present invention (Synthetic route), wherein the nucleoside compound shown in Formula 8-b is a TNA structural compound with 1'-H, 2'-phosphoramidite moiety, 3'-ODMTr, and 4'-modification.**

### Example 48:

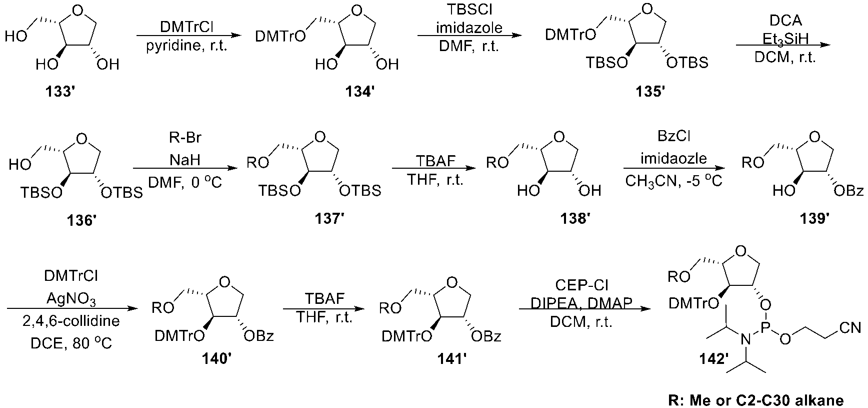

**Example 49 below is the preparation example of the nucleoside compound shown in Formula 9-b of the present invention (Synthetic route), wherein the nucleoside compound shown in Formula 9-b is a TNA structural compound with 2'-S-phosphoramidite moiety, 3'-ODMTr, and 4'-modification.**

### Example 49:

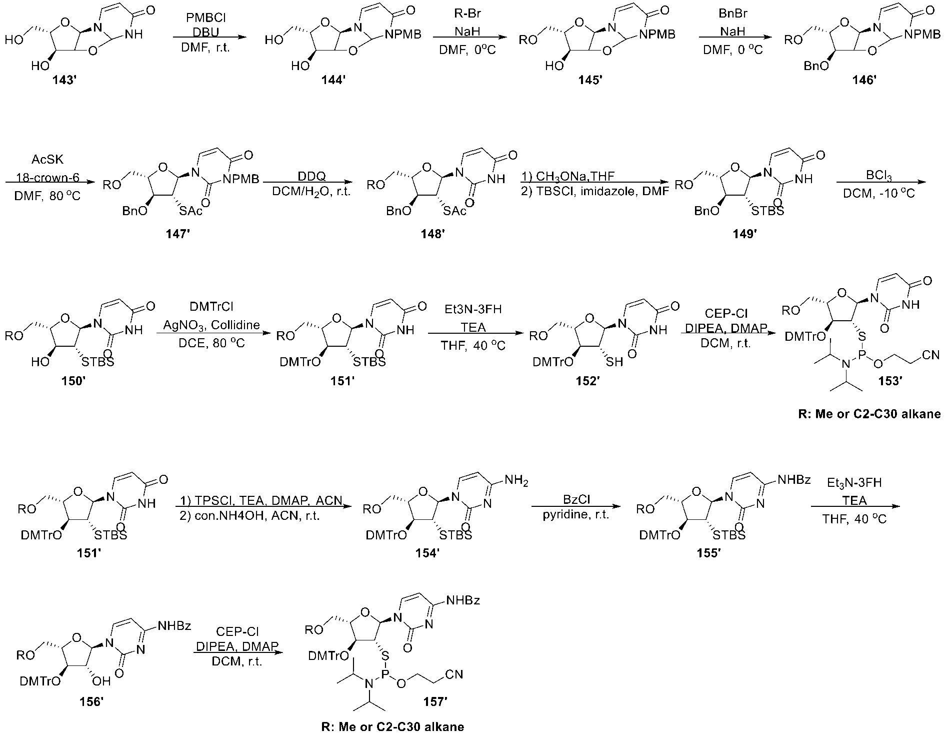

**Example 50 below is the preparation example of the nucleoside compound shown in Formula 10-b of the present invention (Synthetic route), wherein the nucleoside compound shown in Formula 10-b is a TNA structural compound with 2'-phosphoramidite moiety, 3'-SDMTr, and 4'-modification.**

### Example 50:

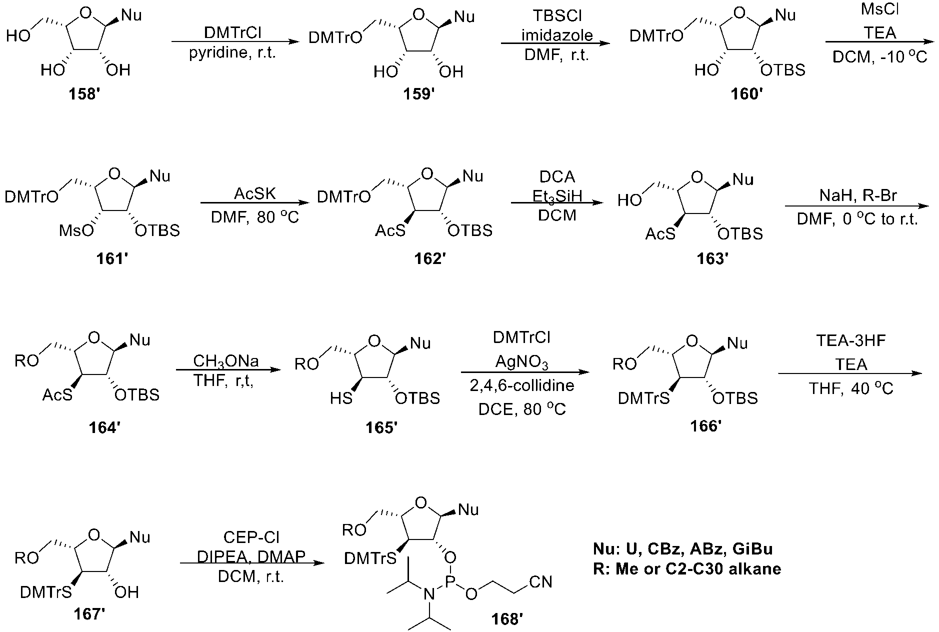

**Example 51 below is the preparation example of the nucleoside compound shown in Formula 11-b of the present invention (Synthetic route), wherein the nucleoside compound shown in Formula 11-b is a TNA structural compound with 2'-phosphoramidite moiety, 3'-ODMTr, and 4'-modification.**

### Example 51:

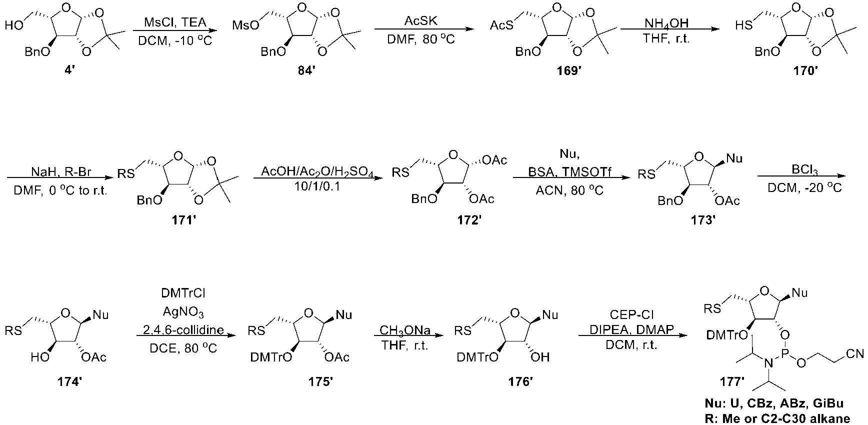

**Example 52 below is the preparation example of the nucleoside compound shown in Formula 12-b of the present invention (Synthetic route), wherein the nucleoside compound shown in Formula 12-b is a TNA structural compound with 2'-phosphoramidite moiety, 3'-ODMTr, and 4'-modification.**

### Example 52:

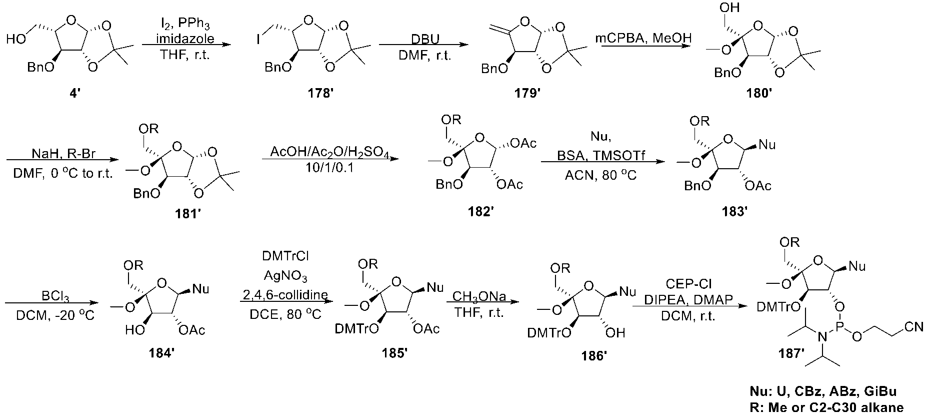

### Example 53: Synthesis of Compound 188'

**Nucleoside compound 188' is a TNA structural compound with 2'-phosphoramidite moiety, 3'-ODMTr, Nu=A, and R=C₁₄H₂₉. Preparation route refers to Examples 31 and 33, with the difference that in the synthesis of compound 5' from compound 4' in Example 31, the raw material C₁₂H₂₅Br is replaced by C₁₄H₂₉Br. All other steps are similar to those in Examples 31 and 33. The structure and nuclear magnetic resonance data of the obtained nucleoside compound 188' are as follows:**

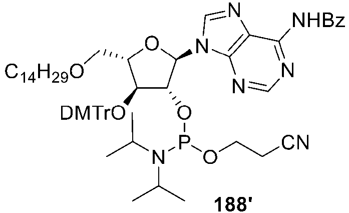

¹H NMR (400 MHz, DMSO-*d*₆) δ 11.26 (d, *J =* 4.0 Hz, 1H), 8.74 (d, *J =* 5.9 Hz, 1H), 8.56 (d, *J =* 29.9 Hz, 1H), 8.24 (d, *J =* 7.6 Hz, 2H), 7.65 (t, *J =* 7.3 Hz, 1H), 7.53 (t, *J =* 7.6 Hz, 2H), 7.36 (t, *J* = 8.6 Hz, 2H), 7.24 - 7.12 (m, 7H), 6.80 - 6.61 (m, 4H), 6.03 (dd, *J* = 36.3, 3.4 Hz, 1H), 4.74 - 4.32 (m, 2H), 3.82 - 3.39 (m, 15H), 2.67 - 2.35 (m, 2H), 1.61 - 1.49 (m, 2H), 1.41 - 1.20 (m, 22H), 1.03 - 0.96 (m, 9H), 0.90 - 0.76 (m, 6H). ³¹P NMR (162 MHz, DMSO-*d*₆) δ 149.66, 148.93. ESI-MS: m/z 1070.5 [M+H]⁺

### Example 54: Synthesis of Compound 189'

**Nucleoside compound 189' is a TNA structural compound with 2'-phosphoramidite moiety, 3'-ODMTr, Nu=A, and R=C₁₆H₃₃. Preparation route refers to Examples 31 and 33, with the difference that in the synthesis of compound 5' from compound 4' in Example 31, the raw material C₁₂H₂₅Br is replaced by C₁₆H₃₃Br. All other steps are similar to those in Examples 31 and 33. The structure and nuclear magnetic resonance data of the obtained nucleoside compound 189' are as follows:**

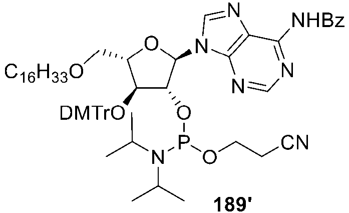

¹H NMR (400 MHz, DMSO-*d*₆) δ 11.19 (d, *J =* 4.0 Hz, 1H), 8.71 (d, *J =* 5.9 Hz, 1H), 8.49 (d, *J =* 29.9 Hz, 1H), 8.27 (d, *J =* 7.6 Hz, 2H), 7.35 (t, *J =* 7.3 Hz, 1H), 7.55 (t, *J =* 7.6 Hz, 2H), 7.37 (t, *J* = 8.6 Hz, 2H), 7.25 - 7.17 (m, 7H), 6.81 - 6.60 (m, 4H), 6.05 (dd, *J =* 36.3, 3.4 Hz, 1H), 4.75 - 4.33 (m, 2H), 3.89 - 3.37 (m, 15H), 2.69 - 2.32 (m, 2H), 1.62 - 1.45 (m, 2H), 1.43 - 1.22 (m, 26H), 1.13 - 0.79 (m, 9H), 0.91 - 0.77 (m, 6H). ³¹P NMR (162 MHz, DMSO-*d*₆) δ 149.29, 148.76. ESI-MS: m/z 1098.4 [M+H]⁺

The structures of some compounds prepared by the above preparation methods are shown in Table B below (nucleoside compounds shown in Formula 1-b).

| Compound and Structure | | Compound and Structure | |
|---|---|---|---|
| DT0- Nu | 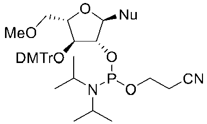 | DT33- Nu | 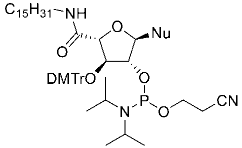 |
| DT1- Nu | 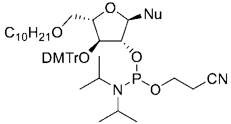 | DT34- Nu | 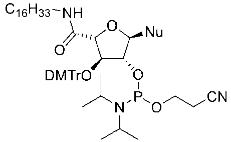 |
| DT2- Nu | 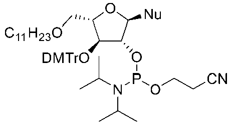 | DT35- Nu | 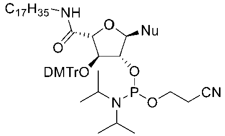 |
| DT3- Nu | 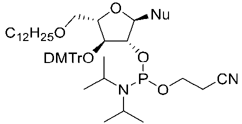 | DT36- Nu | 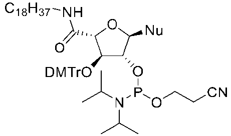 |
| DT4- Nu | 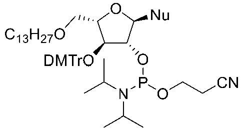 | DT37- Nu | 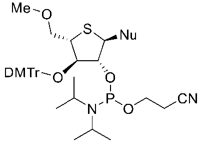 |
| DT5- Nu | 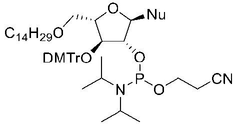 | DT38- Nu | 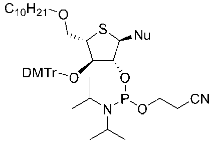 |
| DT6- Nu | 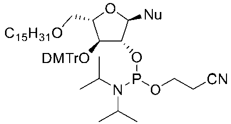 | DT39- Nu | 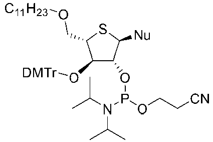 |
| DT7- Nu | 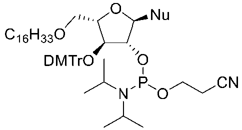 | DT40- Nu | 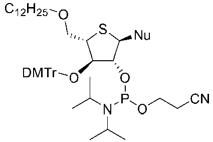 |
| DT8- Nu | 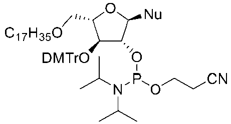 | DT41- Nu | 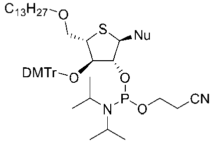 |
| DT9- Nu | 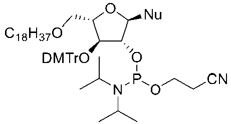 | DT42- Nu | 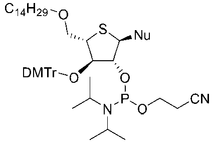 |
| DT10- Nu | 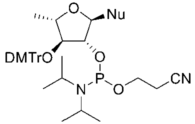 | DT43- Nu | 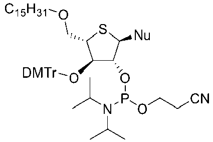 |
| DT11- Nu | 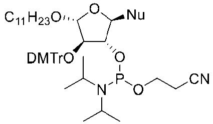 | DT44- Nu | 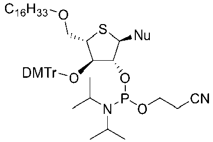 |
| DT12- Nu | 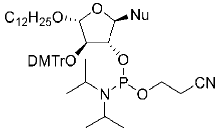 | DT45- Nu | 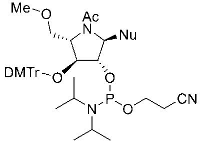 |
| DT13- Nu | 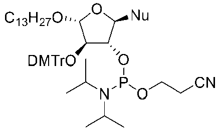 | DT46- without Nu | 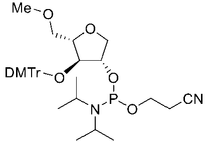 |
| DT14- Nu | 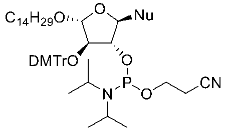 | DT47- without Nu | 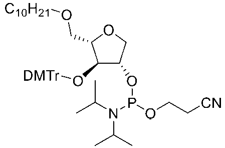 |
| DT15- Nu | 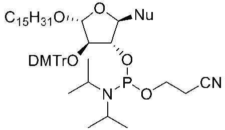 | DT48- without Nu | 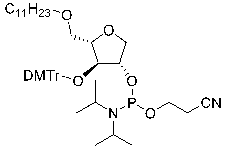 |
| DT16- Nu | 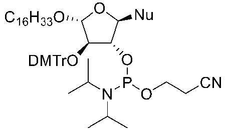 | DT49- without Nu | 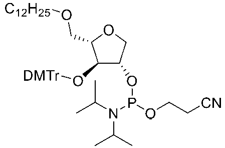 |
| DT17- Nu | 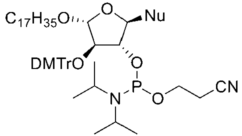 | DT50- without Nu | 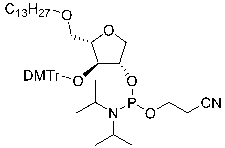 |
| DT18- Nu | 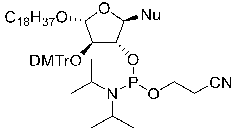 | DT51- without Nu | 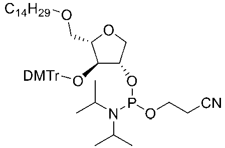 |
| DT19- Nu | 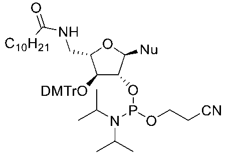 | DT52- without Nu | 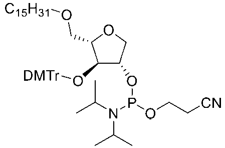 |
| DT20- Nu | 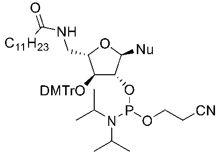 | DT53- without Nu | 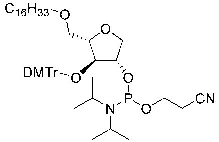 |
| DT21- Nu | 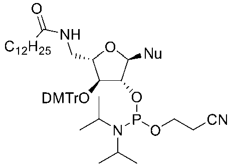 | DT54- Nu | 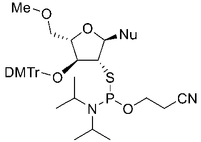 |
| DT22- Nu | 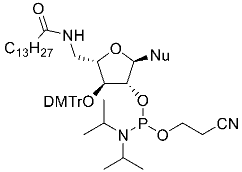 | DT55- Nu | 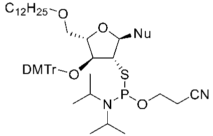 |
| DT23- Nu | 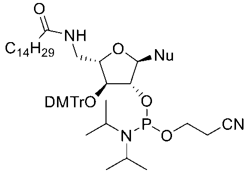 | DT56- Nu | 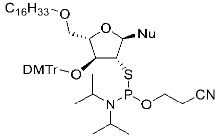 |
| DT24- Nu | 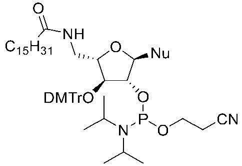 | DT57- Nu | 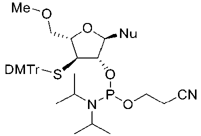 |
| DT25- Nu | 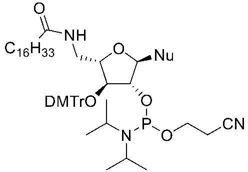 | DT58- Nu | 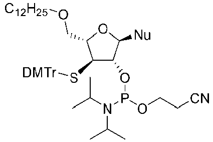 |
| DT26- Nu | 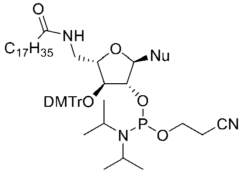 | DT59- Nu | 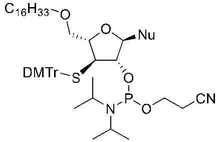 |
| DT27- Nu | 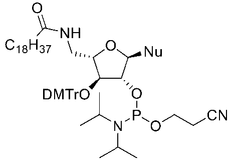 | DT60- Nu | 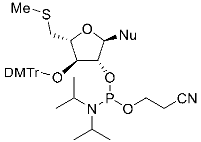 |
| DT28- Nu | 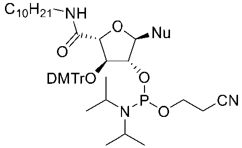 | DT61- Nu | 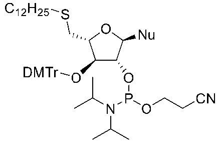 |
| DT29- Nu | 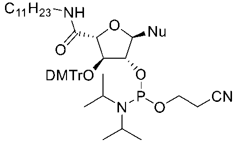 | DT62- Nu | 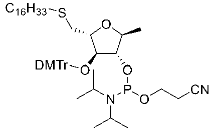 |
| DT30- Nu | 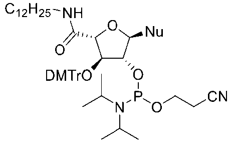 | DT63- Nu | 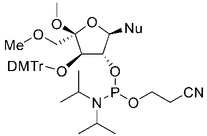 |
| DT31- Nu | 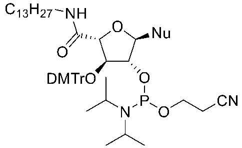 | DT64- Nu | 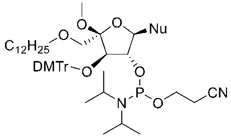 |
| DT32- Nu | 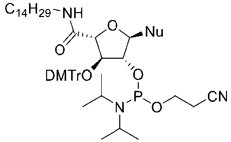 | DT65- Nu | 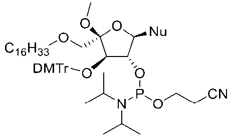 |

wherein Nu represents a base or is absent.

### Experimental section

The following experiments 1 to 7 are for nucleoside compounds of Formula 1-a, and the following experiments 8 to 13 are for nucleoside compounds of Formula 1-b.

### Experiment 1: Synthesis of siRNA Sequences for In Vitro Evaluation

The synthesis of siRNA is the same as the usual phosphoramidite solid-phase synthesis method, in which the synthesized phosphoramidite monomers synthesized as described above are used to replace the parent sequence protonucleotides in the synthesis of nucleotides modified at each of positions of the SS and AS chains.

The synthesis process is briefly described as follows: on the LK- 48E synthesizer (Linkun), starting with Universal CPG carrier, nucleoside phosphoramidite monomers were linked one by one according to the synthesis program. Except for the syntheses of LT3-A, U, G, and C (corresponding to compounds 21, 11, 26, and 16, respectively) as described above, all other nucleoside monomers raw materials, such as nucleoside phosphoramidite monomers, e.g., 2'-F RNA and 2'-O-methyl RNA, were purchased from Hongene Biotech. 5'-Ethylthio-1H-tetrazole (ETT) was used as an activator (0.6 M acetonitrile solution), 0.22 M PADS dissolved in a 1:1 v/v ratio and a solution of trimethylpyridine (Suzhou Koloma) was used as a sulfurizing reagent, and iodopyridine/water solution (Koloma) was used as an oxidizing agent.

After solid-phase synthesis was completed, the oligoribonucleotides were cleaved from this solid support, and a 3:1 solution of 28% ammonia and ethanol was used to soak for 16 h at 50 °C, followed by centrifugation, and the supernatant was transferred to another centrifuge tube, concentrated and evaporated to dryness, and then purified using C18 reversed-phase chromatography with a mobile phase of 0.1 M TEAA and acetonitrile, and DMTr was removed using a 3% trifluoroacetic acid solution. The target oligonucleotides were collected and lyophilized. They were identified as target products by LCMS and quantified by UV (260 nm).

The obtained single-stranded oligonucleotides, according to the equimolar ratio, were paired based on complementarity and annealed. The final obtained double-stranded siRNA was dissolved in 1xPBS and adjusted to the concentration required for the experiment and set aside.

The modification schemes used in Table 1 are as follows:
"m" is a 2'-OMe modification to one nucleotide adjacent to the left of the letter m, "f' is a 2'-F modification to one nucleotide adjacent to the left of the letter f, the "s" is a PS backbone modification between two nucleotides adjacent to the left and right of the letter s. "LT3" is a 4'-(*R*)-CH₂OC₁₂H₂₅-TNA modification.

Among them, CM0412-0000, which lacks nucleoside modifications on the sense strand compared to other groups, served as a control.

**Table 1: Sequences of siRNA molecules used for in vitro screening**

| | | |
|---|---|---|
| CM0412-0000 | SS | AmsAmsCmAmGmUmGfUmUfCfUfUmGmCmUmCmUmAmUmAmAm |
| | AS | UmsUfsAmUmAmGfAmGmCmAmAmGmAmAfCmAfCmUmGmUmUmsUmsUm |
| CM0412-0001 | SS | (LT3-A)sAmsCmAmGmUmGfUmUfCfUfUmGmCmUmCmUmAmUmAmAm |
| | AS | UmsUfsAmUmAmGfAmGmCmAmAmGmAmAfCmAfCmUmGmUmUmsUmsUm |
| CM0412-0002 | SS | Ams(LT3-A)sCmAmGmUmGfUmUfCfUfUmGmCmUmCmUmAmUmAmAm |
| | AS | UmsUfsAmUmAmGfAmGmCmAmAmGmAmAfCmAfCmUmGmUmUmsUmsUm |
| CM0412-0003 | SS | AmsAms(LT3-C)AmGmUmGfUmUfCfUfUmGmCmUmCmUmAmUmAmAm |
| | AS | UmsUfsAmUmAmGfAmGmCmAmAmGmAmAfCmAfCmUmGmUmUmsUmsUm |
| CM0412-0004 | SS | AmsAmsCm(LT3-A)GmUmGfUmUfCfUfUmGmCmUmCmUmAmUmAmAm |
| | AS | UmsUfsAmUmAmGfAmGmCmAmAmGmAmAfCmAfCmUmGmUmUmsUmsUm |
| CM0412-0005 | SS | AmsAmsCmAm(LT3-G)UmGfUmUfCfUfUmGmCmUmCmUmAmUmAmAm |
| | AS | UmsUfsAmUmAmGfAmGmCmAmAmGmAmAfCmAfCmUmGmUmUmsUmsUm |
| CM0412-0006 | SS | AmsAmsCmAmGm(LT3-U)GfUmUfCfUfUmGmCmUmCmUmAmUmAmAm |
| | AS | UmsUfsAmUmAmGfAmGmCmAmAmGmAmAfCmAfCmUmGmUmUmsUmsUm |
| CM0412-0007 | SS | AmsAmsCmAmGmUm(LT3-G)UmUfCfUfUmGmCmUmCmUmAmUmAmAm |
| | AS | UmsUfsAmUmAmGfAmGmCmAmAmGmAmAfCmAfCmUmGmUmUmsUmsUm |
| CM0412-0008 | SS | AmsAmsCmAmGmUmGf(LT3-U)UfCfUfUmGmCmUmCmUmAmUmAmAm |
| | AS | UmsUfsAmUmAmGfAmGmCmAmAmGmAmAfCmAfCmUmGmUmUmsUmsUm |
| CM0412-0009 | SS | AmsAmsCmAmGmUmGfUm(LT3-U)CfUfUmGmCmUmCmUmAmUmAmAm |
| | AS | UmsUfsAmUmAmGfAmGmCmAmAmGmAmAfCmAfCmUmGmUmUmsUmsUm |
| CM0412-0010 | SS | AmsAmsCmAmGmUmGfUmUfCf(LT3-U)UmGmCmUmCmUmAmUmAmAm |
| | AS | UmsUfsAmUmAmGfAmGmCmAmAmGmAmAfCmAfCmUmGmUmUmsUmsUm |
| CM0412-0011 | SS | AmsAmsCmAmGmUmGfUmUfCfUf(LT3-U)GmCmUmCmUmAmUmAmAm |
| | AS | UmsUfsAmUmAmGfAmGmCmAmAmGmAmAfCmAfCmUmGmUmUmsUmsUm |
| CM0412-0012 | SS | AmsAmsCmAmGmUmGfUmUfCfUfUm(LT3-G)CmUmCmUmAmUmAmAm |
| | AS | UmsUfsAmUmAmGfAmGmCmAmAmGmAmAfCmAfCmUmGmUmUmsUmsUm |
| CM0412-0013 | SS | AmsAmsCmAmGmUmGfUmUfCfUfUmGm(LT3-C)UmCmUmAmUmAmAm |
| | AS | UmsUfsAmUmAmGfAmGmCmAmAmGmAmAfCmAfCmUmGmUmUmsUmsUm |
| CM0412-0014 | SS | AmsAmsCmAmGmUmGfUmUfCfUfUmGmCm(LT3-U)CmUmAmUmAmAm |
| | AS | UmsUfsAmUmAmGfAmGmCmAmAmGmAmAfCmAfCmUmGmUmUmsUmsUm |
| CM0412-0015 | SS | AmsAmsCmAmGmUmGfUmUfCfUfUmGmCmUm(LT3-C)UmAmUmAmAm |
| | AS | UmsUfsAmUmAmGfAmGmCmAmAmGmAmAfCmAfCmUmGmUmUmsUmsUm |
| CM0412-0016 | SS | AmsAmsCmAmGmUmGfUmUfCfUfUmGmCmUmCm(LT3-U)AmUmAmAm |
| | AS | UmsUfsAmUmAmGfAmGmCmAmAmGmAmAfCmAfCmUmGmUmUmsUmsUm |
| CM0412-0017 | SS | AmsAmsCmAmGmUmGfUmUfCfUfUmGmCmUmCmUm(LT3-A)UmAmAm |
| | AS | UmsUfsAmUmAmGfAmGmCmAmAmGmAmAfCmAfCmUmGmUmUmsUmsUm |
| CM0412-0018 | SS | AmsAmsCmAmGmUmGfUmUfCfUfUmGmCmUmCmUmAm(LT3-U)AmAm |
| | AS | UmsUfsAmUmAmGfAmGmCmAmAmGmAmAfCmAfCmUmGmUmUmsUmsUm |
| CM0412-0019 | SS | AmsAmsCmAmGmUmGfUmUfCfUfUmGmCmUmCmUmAmUm(LT3-A)Am |
| | AS | UmsUfsAmUmAmGfAmGmCmAmAmGmAmAfCmAfCmUmGmUmUmsUmsUm |
| CM0412-0020 | SS | AmsAmsCmAmGmUmGfUmUfCfUfUmGmCmUmCmUmAmUmAm(LT3-A) |
| | AS | UmsUfsAmUmAmGfAmGmCmAmAmGmAmAfCmAfCmUmGmUmUmsUmsUm |
| CM0412-0021 | SS | AmsAmsCmAmGmUmGfUmUfCfUfUmGmCmUmCmUmAmUmAmAm |
| | AS | UmsUfsAmUmAmGfAmGmCmAmAmGmAmAfCmAfCmUmGmUmUms(LT3-U)sUm |
| CM0412-0022 | SS | AmsAmsCmAmGmUmGfUmUfCfUfUmGmCmUmCmUmAmUmAmAm |
| | AS | UmsUfsAmUmAmGfAmGmCmAmAmGmAmAfCmAfCmUmGmUmUmsUms(LT3-U) |

### Experiment 2: In Vitro Silencing Efficacy Against TTR mRNA by siRNA Duplexes Containing LT3-A, U, G and C

### Activity screening step:

### Cell culture and transfection:

Hepa1-6 cells (ATCC, Catalog No. CRL1830) were cultured in DMEM (Invitrogen, Catalog No. 11965092) supplemented with 10% FBS (Ausgenex, Catalog No. FBS500-S) and 1% penicillin-streptomycin under conditions of 37°C and 5% CO₂ until they reached near confluence. The cells were then trypsinized, resuspended in cell seeding medium (DMEM + 10% FBS), and seeded at a density of 5,000 cells per well in 90 µL into 96-well cell culture plates (Corning, Catalog No. 3904), followed by overnight incubation at 37°C with 5% CO₂. Transfection conditions were as follows: 4.6 µL of Opti-MEM (Gibco, catalog no. 1985-070) and 0.4 µL of Lipofectamine RNAiMax (Invitrogen, Carlsbad CA. Catalog no. 13778-150) were mixed with 5 µL of siRNAs per well and incubated for 15 min at room temperature. The mixture was then added to 10 µL per well in the 96-well plate laid out the day before. The cells were incubated at 37°C under 5% CO₂ for 48 hours. The final concentrations in the siRNAs experiments were 10 nM, 1 nM, and 0.1 nM.

### RNA extraction with reverse transcription into cDNA:

Cells were lysed for RNA extraction according to the instructions for Cells-to-Ct bulk lysis reagents (Invitrogen, catalog number 4391851C). 1) Aspirate off the old medium in each well and add 100 µL PBS to moisten and wash twice. 2) Carefully aspirate off the PBS. 50 µL of lysate was added to each well. 3) Shake at 500rpm for 5 min at room temperature. 4) Add 5µL of termination solution to each well and shake at 500rpm for 2 min at room temperature. If cDNA synthesis is performed immediately, the plate needs to be placed on ice.

Reverse transcription was performed according to the instructions for Cells-to-CT Bulk Fast Advanced RT Reagent (Invitrogen, catalog no. A39110): preparation of the mixture (per well: 25 µL 2× Fast Advanced RT Buffer, 2.5 µL 20× RT Fast Advanced Enzyme Mix and 22.5 µL total RNA). The cDNA was synthesized using Biometra TAdvanced 96SG (analytikjena) by the following steps: 30 min at 37°C, 5 min at 95°C, and maintained at 4°C. cDNA was stored at -20°C or analyze immediately by real-time PCR.

### Real-time PCR:

4.33 µL of cDNA and 0.17 µL of 60X GAPDH TaqMan probe (Invitrogencat, catalog no. 4448491), 0.5 µL of 20X TTR TaqMan probe (Invitrogen, catalog no. 4351370) and 5 µL of 2×TaqMan^{®} FAST PCR Master Mix (Applied Biosystems, Catalog No. 4444965) were mixed and added to a 96-well plate (Axygen, Catalog No. PCR-96-FLT-C) for real-time PCR detection. The instrument used was QuantStudio^{®} 7 (AppliedBiosystems), and the system program was 50 °C for 2 min, 95 °C for 20 s, and 40 cycles of 90 °C for 1 s, and 60 °C for 20 s. Unless otherwise stated, 3 transfection replicate wells were done for each siRNAs.

For data analysis, real-time data were analyzed using the ΔΔCt method and these data were normalized with the mock group data, which was calculated as follows: ΔCt = Ct (Target gene) -Ct (GAPDH), ΔΔCt = ΔCt (Assay samples) -ΔCt (Mock), Relative mRNA expression to Mock =2^{-ΔΔCt}, %Inhibition v.s. Mock = {1 -Expression fold (tested sample)/Expression fold (Mock)}*100. The final results are shown in Table 2.

**Table 2: q-PCR in vitro activity screening data**

| Name for siRNA | Inhibition Rate (10 nM) | SD | Inhibition Rate (1 nM) | SD | Inhibition Rate (0.1 nM) | SD |
|---|---|---|---|---|---|---|
| CM0412-0000 | 79.14% | 5.42% | 77.67% | 5.32% | 64.71% | 5.43% |
| CM0412-0001 | 82.26% | 1.01% | 77.02% | 8.56% | 68.42% | 2.39% |
| CM0412-0002 | 77.36% | 5.86% | 70.34% | 2.79% | 75.74% | 5.19% |
| CM0412-0003 | 84.84% | 4.09% | 68.67% | 17.48% | 67.59% | 3.44% |
| CM0412-0004 | 76.09% | 11.54% | 67.07% | 10.66% | 57.62% | 13.99% |
| CM0412-0005 | 78.42% | 3.79% | 70.48% | 5.36% | 61.35% | 4.82% |
| CM0412-0006 | 80.94% | 1.35% | 64.00% | 11.10% | 59.65% | 13.20% |
| CM0412-0007 | 81.59% | 3.72% | 76.49% | 6.25% | 67.44% | 3.4% |
| CM0412-0008 | 82.25% | 5.78% | 63.76% | 10.14% | 62.87% | 2.67% |
| CM0412-0009 | 84.81% | 6.67% | 72.94% | 3.07% | 40.12% | 7.60% |
| CM0412-0010 | 71.59% | 3.88% | 51.35% | 8.27% | 21.57% | 4.21% |
| CM0412-0011 | 72.89% | 3.00% | 45.49% | 2.87% | 4.74% | 22.35% |
| CM0412-0012 | 84.52% | 4.11% | 76.38% | 3.76% | 68.35% | 6.21% |
| CM0412-0013 | 78.09% | 12.28% | 81.50% | 1.17% | 77.51% | 5.44% |
| CM0412-0014 | 70.44% | 11.29% | 63.88% | 26.97% | 48.89% | 6.36% |
| CM0412-0015 | 87.02% | 7.47% | 71.48% | 11.57% | 55.77% | 12.13% |
| CM0412-0016 | 84.78% | 5.68% | 81.93% | 6.14% | 65.59% | 3.50% |
| CM0412-0017 | 83.25% | 2.67% | 77.85% | 2.98% | 68.10% | 6.56% |
| CM0412-0018 | 82.69% | 7.60% | 66.47% | 6.26% | 59.68% | 9.03% |
| CM0412-0019 | 73.74% | 2.84% | 71.23% | 13.05% | 62.11% | 5.70% |
| CM0412-0020 | 81.28% | 0.88% | 70.10% | 8.78% | 59.24% | 14.28% |
| CM0412-0021 | 79.85% | 9.08% | 67.13% | 10.86% | 53.45% | 3.95% |
| CM0412-0022 | 83.27% | 4.01% | 78.81% | 1.87% | 50.09% | 3.02% |

RESULTS: The modification (LT3-A, U, G, C) was able to increase the activity of siRNA sequences when incorporated into them. Even at a lower concentration (0.1 nM), some of the sequences still had a silencing efficiency of greater than 60%.

### Experiment 3: Synthesis of Single-Stranded Oligonucleotides for 3'-Exonuclease Stability Evaluation

The synthesis of oligonucleotide single strands is the same as the usual phosphoramidite solid-phase synthesis method, in which the synthesized phosphoramidite monomers (LT3) synthesized as described above are used to replace the parent sequence protonucleotides in the synthesis of a single-stranded oligonucleotide with LT3 modification at the 3'-terminus.

The synthesis process is briefly described as follows: on the LK- 48E synthesizer (Linkun), starting with Universal CPG (DNA chem) carrier, nucleoside phosphoramidite monomers were linked one by one according to the synthesis program. Except for the syntheses of LT3-A, U, G, and C (corresponding to compounds 21, 11, 26, and 16, respectively) as described above, all other nucleoside monomers raw materials, such as nucleoside phosphoramidite monomers, such as 2'-F RNA and 2'-O-methyl RNA, were purchased from Hongene Biotech. 5'-Ethylthio-1H-tetrazole (ETT) was used as an activator (0.6 M acetonitrile solution), 0.22 M PADS dissolved in a 1:1 v/v ratio and a solution of trimethylpyridine (Suzhou Koloma) was used as a sulfurizing reagent, and iodopyridine/water solution (Koloma) was used as an oxidizing agent.

After solid-phase synthesis was completed, the oligoribonucleotides were cleaved from this solid support, and a 3:1 solution of 28% ammonia and ethanol was used to soak for 16 h at 50 °C, followed by centrifugation, and the supernatant was transferred to another centrifuge tube, concentrated and evaporated to dryness, and then purified using C18 reversed-phase chromatography with a mobile phase of 0.1 M TEAA and acetonitrile, and DMTr was removed using a 3% trifluoroacetic acid solution. The target oligonucleotides were collected and lyophilized. They were identified as target products by LCMS, and quantified by UV (260nm). The obtained single-stranded oligonucleotides were dissolved in 1xPBS and adjusted to the concentration required for the experiment and set aside.

The modification schemes used in Table 3 are as follows:
"m" is a 2'-OMe modification to one nucleotide adjacent to the left of the letter m, "f" is a 2'-F modification to one nucleotide adjacent to the left of the letter f, the "s" is a PS backbone modification between two nucleotides adjacent to the left and right of the letter s. "LT3" is a 4'-(*R*)-CH₂OC₁₂H₂₅-TNA modification.

Among them, CM0412-1000, which lacks nucleoside compound modifications compared to the other groups, served as a control.

**Table 3: Oligonucleotide sequences used to examine 3'-exonuclease stability**

| | |
|---|---|
| CM0412-1000 | UmsUfsAmUmAmGfAmGmCmAmAmGmAmAfCmAfCmUmGmUmUmsUmsUm |
| CM0412-1001 | UmsUfsAmUmAmGfAmGmCmAmAmGmAmAfCmAfCmUmGmUmUmsUms**(LT3-U)** |
| CM0412-1002 | UmsUfsAmUmAmGfAmGmCmAmAmGmAmAfCmAfCmUmGmUmUmUm**(LT3-U)** |

### Experiment 4: 3'-Exonuclease Stability Assay of Single-Stranded Sequences

### Stability evaluation procedure:

The samples (oligonucleotide molecules CM0412-1000, CM0412-1001, CM0412-1002) were solubilized using PBS (BOSTER, Lot. 17F24C21) and the concentration of the compounds was determined using a microplate reader, and the working solution was diluted to 300 ng/µL using PBS.

Samples to be tested (CM0412-1000, CM0412-1001, CM0412-1002, see Table 3) were co-incubated with the enzyme system: 43 µL of the enzyme system (Sigma, Lot. P4506) as well as 7 µL of the samples to be tested were added to each tube, N = 2 (2 replicates per sample), vortex-mixed, and then incubated at 37°C in an incubator. 50 µL from each tube in the first step was placed in another EP tube and 5 µL of EDTA (0.5 M) solution was added to each tube, mixed, and immediately placed in liquid nitrogen for rapid cooling to terminate the reaction as a 0 hr self-control. The remaining samples were placed in a 37 °C incubator, and the samples were taken out at each time point, and 5 µL of EDTA (0.5 M) was added respectively, mixed well, and immediately placed in liquid nitrogen for rapid cooling to terminate the reaction.

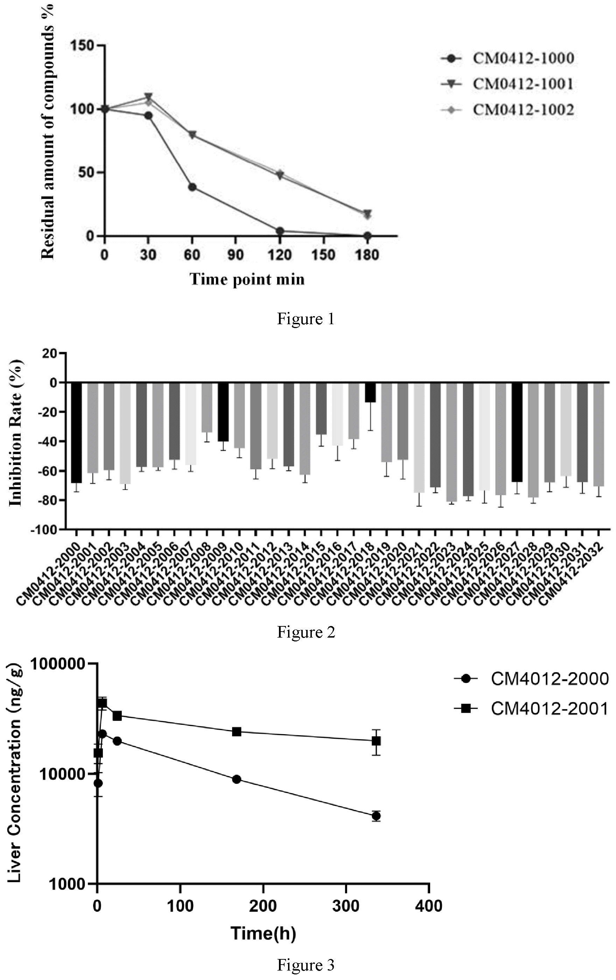

Pre-treatment of biological samples: Prepare RNA extractant: 25mL saturated phenol aqueous solution, 24mL chloroform, 1mL isoamyl alcohol, mix well to obtain. After thawing the above frozen samples at room temperature, 10 µL of internal standard compounds (compounds used for quality control, i.e., siRNA sequences: the antisense strand CmsUfsCmGmGfUmAfCmAm UmGmCfAmAfUmCmsCmsCm; and the sense strand for GmsGmGmAmUmUfGmCfAfUfGmUm AmAmCmCmGmsAmsGm), 150 µL Tris, and 150 µL extractant (phenol: chloroform: isoamyl alcohol = 25:24:1) were added to each tube. After vortex mixing, the mixture was centrifuged for 10 min, and the supernatant was taken for high-resolution LC-MS analysis. The residual amounts of the test samples at each time point are shown in Table 4 and Figure 1.

**Table 4: Residual amounts of test samples at each time point**

| Time point (min) | CM0412-1000 (% residual amount) | CM0412-1001 (% residual amount) | CM0412-1002 (% residual amount) |
|---|---|---|---|
| 0 | 100.0 | 100.0 | 100.0 |
| 30 | 95.1 | 109.4 | 105.2 |
| 60 | 38.6 | 79.5 | 79.8 |
| 120 | 4.1 | 47.1 | 49.5 |
| 180 | 0.3 | 17.4 | 15.7 |

RESULTS: This modification (LT3) significantly improves the stability of oligonucleotide single strands against 3'-exonucleases when added to single-stranded 3'-ends.

The remaining nucleoside compounds (LT0-LT2 and LT4-LT106) share identical or similar sugar backbone structures and feature the same 2,3-phosphodiester linkage pattern within the sequence; consequently, they all exhibit enhanced resistance to nucleases.

### Experiment 5: Synthesis of siRNA Sequences for In Vivo Evaluation in Wild-Type Mice

The synthesis of siRNA is the same as the usual phosphoramidite solid-phase synthesis method, in which the synthesized phosphoramidite monomers synthesized as described above are used to replace the parent sequence protonucleotides in the synthesis of nucleotides modified at each of positions of the SS and AS chains.

The synthesis process is briefly described as follows: on the LK- 48E synthesizer (Linkun), starting with Universal CPG (DNA chem) carrier, nucleoside phosphoramidite monomers were linked one by one according to the synthesis program. Except for LT3-A, U, G, C (corresponding to compounds 21, 11, 26 and 16, respectively), LT0-A, U, G, C (corresponding to compounds 43, 33, 48 and 38, respectively), LT5-A, U, G, C (corresponding to compounds 65, 55, 70 and 60, respectively), LT7-A, U, G, C (corresponding to compounds 87, 77, 82 and 82), LT16-A (corresponding to compound 116), LT25-A (corresponding to compound 104), LT41-A (corresponding to compound 135), LT88-A (corresponding to compound 154), LT93-A (corresponding to compound 175), LT97-A (corresponding to compound 205) as described above, other nucleoside monomers raw materials, such as nucleoside phosphoramidite monomers, such as 2'-F RNA and 2'-O-methyl RNA, were purchased from Hongene Biotech, and L96 was purchased from WuXi AppTec. 5'-Ethylthio-1H-tetrazole (ETT) was used as an activator (0.6 M acetonitrile solution), 0.22 M PADS dissolved in a 1:1 v/v ratio and a solution of trimethylpyridine (Suzhou Koloma) was used as a sulfurizing reagent, and iodopyridine/water solution (Koloma) was used as an oxidizing agent.

After solid-phase synthesis was completed, the oligoribonucleotides were cleaved from this solid support, and a 3:1 solution of 28% ammonia and ethanol was used to soak for 16 h at 50 °C, followed by centrifugation, and the supernatant was transferred to another centrifuge tube, concentrated and evaporated to dryness, and then purified using C18 reversed-phase chromatography with a mobile phase of 0.1 M TEAA and acetonitrile, and DMTr was removed using a 3% trifluoroacetic acid solution. The target oligonucleotides were collected and lyophilized. They were identified as target products by LCMS and quantified by UV (260nm).

The obtained single-stranded oligonucleotide was subjected to exchange into sodium acetate using a 3 kDa cutoff ultrafiltration tube, and according to the equimolar ratio, were paired based on complementarity and annealed. The final obtained double-stranded siRNA was dissolved in water and adjusted to the concentration required for the experiment and set aside.

The modification schemes used in Table 5 are as follows:
"m" is a 2'-OMe modification to one nucleotide adjacent to the left of the letter m, "f" is a 2'-F modification to one nucleotide adjacent to the left of the letter f, "s" is a PS backbone modification between two nucleotides adjacent to the left and right of the letter s, "LT3" is a 4'-(*R*)-CH₂OC₁₂H₂₅-TNA modification, "LT0" is 4'-*(R*)-CH₂OMe-TNA modification, "LT5" is 4'-*(R*)-CH₂OC₁₄H₂₉-TNA modification, "LT7" is 4'-*(R*)-CH₂OC₁₆H₃₃-TNA modification, "LT1, LT2, LT4, LT6", and "LT8 to LT106" modifications are detailed in the structural list of corresponding compounds in specific embodiments.

Among them, CM0412-2000, which lacks nucleoside modifications on the sense strand compared to other groups, served as a control.

**Table 5: Sequences of siRNA molecules used for in vivo evaluation in wild-type mice**

| | | |
|---|---|---|
| CM0412-2000 | SS | AmsAmsCmAmGmUmGfUmUfCfUfUmGmCmUmCmUmAmUmAmAm**L96** |
| | AS | UmsUfsAmUmAmGfAmGmCmAmAmGmAmAfCmAfCmUmGmUmUmsUmsUm |
| CM0412-2001 | SS | **(LT3-A)**sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2002 | SS | Ams**(LT3-A)**sCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2003 | SS | AmsAms**(LT3-C)**AmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2004 | SS | AmsAmsCm(**LT3-A**)GmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2005 | SS | AmsAmsCmAm(**LT3-G**)UmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2006 | SS | AmsAmsCmAmGm(**LT3-U**)GfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2007 | SS | AmsAmsCmAmGmUm(**LT3-G**)UmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2008 | SS | AmsAmsCmAmGmUmGf(**LT3-U**)UfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2009 | SS | AmsAmsCmAmGmUmGfUm(**LT3-U**)CfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2010 | SS | AmsAmsCmAmGmUmGfUmUfCf(**LT3-U**)UmGmCmUmCmUmAmUmAmAm **L96** |
| | AS | UmsUfsAmUmAmGfAmGmCmAmAmGmAmAfCmAfCmUmGmUmUmsUmsUm |
| CM0412-2011 | SS | AmsAmsCmAmGmUmGfUmUfCfUf(**LT3-U**)GmCmUmCmUmAmUmAmAm **L96** |
| | AS | UmsUfsAmUmAmGfAmGmCmAmAmGmAmAfCmAfCmUmGmUmUmsUmsUm |
| CM0412-2012 | SS | AmsAmsCmAmGmUmGfUmUfCfUfUm(**LT3-G**)CmUmCmUmAmUmAmAm **L96** |
| | AS | UmsUfsAmUmAmGfAmGmCmAmAmGmAmAfCmAfCmUmGmUmUmsUmsUm |
| CM0412-2013 | SS | AmsAmsCmAmGmUmGfUmUfCfUfUmGm(**LT3-C**)UmCmUmAmUmAmAm **L96** |
| | AS | UmsUfsAmUmAmGfAmGmCmAmAmGmAmAfCmAfCmUmGmUmUmsUmsUm |
| CM0412-2014 | SS | AmsAmsCmAmGmUmGfUmUfCfUfUmGmCm(**LT3-U**)CmUmAmUmAmAm **L96** |
| | AS | UmsUfsAmUmAmGfAmGmCmAmAmGmAmAfCmAfCmUmGmUmUmsUmsUm |
| CM0412-2015 | SS | AmsAmsCmAmGmUmGfUmUfCfUfUmGmCmUm(**LT3-C**)UmAmUmAmAm **L96** |
| | AS | UmsUfsAmUmAmGfAmGmCmAmAmGmAmAfCmAfCmUmGmUmUmsUmsUm |
| CM0412-2016 | SS | AmsAmsCmAmGmUmGfUmUfCfUfUmGmCmUmCm(**LT3-U**)AmUmAmAm **L96** |
| | AS | UmsUfsAmUmAmGfAmGmCmAmAmGmAmAfCmAfCmUmGmUmUmsUmsUm |
| CM0412-2017 | SS | AmsAmsCmAmGmUmGfUmUfCfUfUmGmCmUmCmUm(**LT3-A**)UmAmAm **L96** |
| | AS | UmsUfsAmUmAmGfAmGmCmAmAmGmAmAfCmAfCmUmGmUmUmsUmsUm |
| CM0412-2018 | SS | AmsAmsCmAmGmUmGfUmUfCfUfUmGmCmUmCmUmAm(**LT3-U**)AmAm **L96** |
| | AS | UmsUfsAmUmAmGfAmGmCmAmAmGmAmAfCmAfCmUmGmUmUmsUmsUm |
| CM0412-2019 | SS | AmsAmsCmAmGmUmGfUmUfCfUfUmGmCmUmCmUmAmUm(**LT3-A**)Am **L96** |
| | AS | UmsUfsAmUmAmGfAmGmCmAmAmGmAmAfCmAfCmUmGmUmUmsUmsUm |
| CM0412-2020 | SS | AmsAmsCmAmGmUmGfUmUfCfUfUmGmCmUmCmUmAmUmAm(**LT3-A**) **L96** |
| | AS | UmsUfsAmUmAmGfAmGmCmAmAmGmAmAfCmAfCmUmGmUmUmsUmsUm |
| CM0412-2021 | SS | (**LT0-A**)sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2022 | SS | **(LT5-A)**sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2023 | SS | **(LT7-A)**sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2024 | SS | **(LT9-A)**sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2025 | SS | **(LT16-A)**sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2026 | SS | **(LT25-A)**sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2027 | SS | **(LT41-A)**sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2028 | SS | **(LT88-A)**sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2029 | SS | **(LT93-A)**sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2030 | SS | **(LT97-A)**sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| CM0412-2031 | SS | AmsAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUms**(LT0-U)** |
| CM0412-2032 | SS | AmsAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUms**(LT0-U)**sUm |

### Experiment 6: Evaluation of In Vivo Activity of TTR-Targeted mRNA and siRNA Duplexes Containing LT-4'-TNA-Modified Nucleotides

*In vivo* evaluation of siRNA sequence activity in wild-type mice:
The activity of the above compounds (CM0412 - 2000 to CM0412 - 2032, see Table 5) was evaluated *in vivo* using wild-type C57BL/6 mice (Spivey (Beijing) Biotechnology Co. Ltd.).

6- to 8-week-old C57BL/6 mice received a single subcutaneous injection of the compound (from Table 5) at a dose of 1 mg/kg. Orbital blood samples were collected using EP tubes before administration and on days 7, 14, 21, 28, 35, 42, and 56 post administration. The blood samples were allowed to clot at room temperature for two hours, followed by centrifugation at 5500 rpm for 10 minutes at 4°C. The serum was then separated and collected for the determination of TTR levels in the animal serum.

Determination of TTR levels in serum was analyzed by TTR enzyme-linked immunosorbent assay (ELISA). ELISA was performed using Abcam Mouse Prealbumin ELISA Kit (ab282297), and all samples were tested according to the instructions of the test kit. Absorbance at 450 nm was measured using a Tecan SPARK microplate reader. A parametric standard curve was generated from the standards (supplied with the aforementioned ELISA kit), and serum TTR protein levels (in µg/mL) were determined based on this curve. The protein content of individual animals was compared to their corresponding pre-administration serum protein content to determine the percentage of TTR remaining relative to pre-administration.

The experimental results are shown in Figure 2.

RESULTS: This class of modified nucleotides can enhance the *in vivo* gene silencing activity of siRNA sequences and confers a certain degree of long-lasting effect.

### Experiment 7: Evaluation of Liver Exposure of TTR-Target mRNA and siRNA Duplexes containing LT-4'-TNA-Modified Nucleotides in Wild-Type Mice

Evaluation of Exposure Levels in Wild-Type Mouse Liver:
The exposure levels of the aforementioned compounds (Table 5) in the liver were compared using wild-type C57BL/6 mice (purchased from Sibeifu (Beijing) Biotechnology Co., Ltd.).

6- to 8-week-old C57BL/6 mice were administered a single subcutaneous injection of the compound (CM0412-2001) at a dose of 3 mg/kg. Mice were euthanized at 1 h, 4 h, 8 h, 24 h, Day 3, Day 7, and Day 14 post-administration, and liver tissues were collected after dissection.

After dissection, the liver was collected, and the median lobe of the liver was taken for experimentation. The collected liver tissue was homogenized using homogenization buffer (100 mM Tris and 1 mM MgCl₂, pH = 6.0). The volume of homogenization buffer added was based on a ratio of 1 mL buffer per 200 mg of liver tissue. Homogenization was performed using a 24-bead mill tissue homogenizer (Bertin Technologies (Montigny-le-Bretonneux, France) Precellys 24 bead homogenizer).

Prior to SPE sample preparation, an internal standard at a concentration of 2.5 mg/mL was added to all samples (1 mL of 0.5 mg/mL internal standard solution was added per well to 200 mL of liver homogenate). Samples were processed via solid-phase extraction (using 96-well Clarity OTX SPE plates (100 mg/well), purchased from Phenomenex (Torrance, CA, USA)) and then analyzed by liquid chromatography-mass spectrometry (LC-MS). The equipment used was: Waters ACQUITY Premier, ACQUITY Premier Oligonucleotide BEH C18 column, Waters ACQUITY Premier + Xevo TQ-Absolute LC-MS system.

Liver exposure levels were calculated based on the measured drug concentrations at each time point, and comparisons of exposure levels among the compounds were conducted.

The specific operations for solid-phase extraction were carried out according to the instructions. Buffers involved were prepared at room temperature, with details as provided above.
Equilibration buffer: 50 mM NaH₂PO4, 2 mM NaN₃, 10mM K₂EDTA in water (pH = 5.5)
Wash Buffer 1: 50 mM NaH₂PO₄ in 50:50 (v:v) water: acetonitrile (pH = 5.5)
Wash Buffer 2: 50 mM NH₄HCO₃ in 20:80 (v:v) water: acetonitrile (pH = 5.5)
Elution buffer: 100 mM NH₄HCO₃, 10 mM TCEP in 50:40:10 (v:v:v) water: acetonitrile: tetrahydrofuran (pH = 9)

For all buffer pH adjustments, ammonium hydroxide or glacial acetic acid was used.

The experimental results are shown in Figure 3.

RESULTS: This class of modified nucleotides can maintain or enhance the exposure level of siRNA sequences in the mouse liver.

### Experiment 8: Synthesis of siRNA Sequences for In Vitro Evaluation

The synthesis of siRNA is the same as the usual phosphoramidite solid-phase synthesis method, in which the synthesized phosphoramidite monomers synthesized as described above are used to replace the parent sequence protonucleotides in the synthesis of nucleotides modified at each of positions of the SS and AS chains.

The synthesis process is briefly described as follows: on the LK- 48E synthesizer (Linkun), starting with Universal CPG carrier, nucleoside phosphoramidite monomers were linked one by one according to the synthesis program. Except for syntheses ofDT3-A (corresponding to compound 21'), U (corresponding to compound 11'), G (corresponding to compound 26'), C (corresponding to compound 16'), DT0-A (corresponding to compound 43'), U (corresponding to compound 33'), G (corresponding to compound 48'), C (corresponding to compound 38'), DT10-A (corresponding to compound 66'), U (corresponding to compound 56'), G (corresponding to compound 71'), and C (corresponding to compound 61') as described above, other nucleoside monomers raw materials, such as nucleoside phosphoramidite monomers, such as 2'-F RNA and 2'-O-methyl RNA, were purchased from Hongene Biotech. 5'-Ethylthio-1H-tetrazole (ETT) was used as an activator (0.6 M acetonitrile solution), 0.22 M PADS dissolved in a 1:1 v/v ratio and a solution of trimethylpyridine (Suzhou Koloma) was used as a sulfurizing reagent, and iodopyridine/water solution (Koloma) was used as an oxidizing agent.

After solid-phase synthesis was completed, the oligoribonucleotides were cleaved from this solid support, and a 3:1 solution of 28% ammonia and ethanol was used to soak for 16 h at 50 °C, followed by centrifugation, and the supernatant was transferred to another centrifuge tube, concentrated and evaporated to dryness, and then purified using C18 reversed-phase chromatography with a mobile phase of 0.1 M TEAA and acetonitrile, and DMTr was removed using a 3% trifluoroacetic acid solution. The target oligonucleotides were collected and lyophilized. They were identified as target products by LCMS and quantified by UV (260 nm).

The obtained single-stranded oligonucleotides, according to the equimolar ratio, were paired based on complementarity and annealed. The final obtained double-stranded siRNA was dissolved in 1xPBS and adjusted to the concentration required for the experiment and set aside.

The modification schemes used in Table 6 are as follows:
"m" is a 2'-OMe modification to one nucleotide adjacent to the left of the letter m, "f" is a 2'-F modification to one nucleotide adjacent to the left of the letter f, the "s" is a PS backbone modification between two nucleotides adjacent to the left and right of the letter s. "DT3" is a 4'-(*S*)-CH₂OC₁₂H₂₅-TNA modification.

Among them, DM0412-0000, which lacks nucleoside modifications on the sense strand compared to other groups, served as a control.

**Table 6: Sequences of siRNA molecules used for in vitro screening**

| | | |
|---|---|---|
| DM0412-0000 | SS | AmsAmsCmAmGmUmGfUmUfCfUfUmGmCmUmCmUmAmUmAmAm |
| | AS | UmsUfsAmUmAmGfAmGmCmAmAmGmAmAfCmAfCmUmGmUmUmsUmsUm |
| DM0412-0001 | SS | **(DT3-A)**sAmsCmAmGmUmGfUmUfCfUfUmGmCmUmCmUmAmUmAmAm |
| | AS | UmsUfsAmUmAmGfAmGmCmAmAmGmAmAfCmAfCmUmGmUmUmsUmsUm |
| DM0412-0002 | SS | Ams**(DT3-A)**sCmAmGmUmGfUmUfCfUfUmGmCmUmCmUmAmUmAmAm |
| | AS | UmsUfsAmUmAmGfAmGmCmAmAmGmAmAfCmAfCmUmGmUmUmsUmsUm |
| DM0412-0003 | SS | AmsAms**(DT3-C)**AmGmUmGfUmUfCfUfUmGmCmUmCmUmAmUmAmAm |
| | AS | UmsUfsAmUmAmGfAmGmCmAmAmGmAmAfCmAfCmUmGmUmUmsUmsUm |
| DM0412-0004 | SS | AmsAmsCm**(DT3-A)**GmUmGfUmUfCfUfUmGmCmUmCmUmAmUmAmAm |
| | AS | UmsUfsAmUmAmGfAmGmCmAmAmGmAmAfCmAfCmUmGmUmUmsUmsUm |
| DM0412-0005 | SS | AmsAmsCmAm**(DT3-G)**UmGfUmUfCfUfUmGmCmUmCmUmAmUmAmAm |
| | AS | UmsUfsAmUmAmGfAmGmCmAmAmGmAmAfCmAfCmUmGmUmUmsUmsUm |
| DM0412-0006 | SS | AmsAmsCmAmGm**(DT3-U)**GfUmUfCfUfUmGmCmUmCmUmAmUmAmAm |
| | AS | UmsUfsAmUmAmGfAmGmCmAmAmGmAmAfCmAfCmUmGmUmUmsUmsUm |
| DM0412-0007 | SS | AmsAmsCmAmGmUm**(DT3-G)**UmUfCfUfUmGmCmUmCmUmAmUmAmAm |
| | AS | UmsUfsAmUmAmGfAmGmCmAmAmGmAmAfCmAfCmUmGmUmUmsUmsUm |
| DM0412-0008 | SS | AmsAmsCmAmGmUmGf**(DT3-U)**UfCfUfUmGmCmUmCmUmAmUmAmAm |
| | AS | UmsUfsAmUmAmGfAmGmCmAmAmGmAmAfCmAfCmUmGmUmUmsUmsUm |
| DM0412-0009 | SS | AmsAmsCmAmGmUmGfUm**(DT3-U)**CfUfUmGmCmUmCmUmAmUmAmAm |
| | AS | UmsUfsAmUmAmGfAmGmCmAmAmGmAmAfCmAfCmUmGmUmUmsUmsUm |
| DM0412-0010 | SS | AmsAmsCmAmGmUmGfUmUfCf**(DT3-U)**UmGmCmUmCmUmAmUmAmAm |
| | AS | UmsUfsAmUmAmGfAmGmCmAmAmGmAmAfCmAfCmUmGmUmUmsUmsUm |
| DM0412-0011 | SS | AmsAmsCmAmGmUmGfUmUfCfUf**(DT3-U)**GmCmUmCmUmAmUmAmAm |
| | AS | UmsUfsAmUmAmGfAmGmCmAmAmGmAmAfCmAfCmUmGmUmUmsUmsUm |
| DM0412-0012 | SS | AmsAmsCmAmGmUmGfUmUfCfUfUm**(DT3-G)**CmUmCmUmAmUmAmAm |
| | AS | UmsUfsAmUmAmGfAmGmCmAmAmGmAmAfCmAfCmUmGmUmUmsUmsUm |
| DM0412-0013 | SS | AmsAmsCmAmGmUmGfUmUfCfUfUmGm**(DT3-C)**UmCmUmAmUmAmAm |
| | AS | UmsUfsAmUmAmGfAmGmCmAmAmGmAmAfCmAfCmUmGmUmUmsUmsUm |
| DM0412-0014 | SS | AmsAmsCmAmGmUmGfUmUfCfUfUmGmCm**(DT3-U)**CmUmAmUmAmAm |
| | AS | UmsUfsAmUmAmGfAmGmCmAmAmGmAmAfCmAfCmUmGmUmUmsUmsUm |
| DM0412-0015 | SS | AmsAmsCmAmGmUmGfUmUfCfUfUmGmCmUm**(DT3-C)**UmAmUmAmAm |
| | AS | UmsUfsAmUmAmGfAmGmCmAmAmGmAmAfCmAfCmUmGmUmUmsUmsUm |
| DM0412-0016 | SS | AmsAmsCmAmGmUmGfUmUfCfUfUmGmCmUmCm**(DT3-U)**AmUmAmAm |
| | AS | UmsUfsAmUmAmGfAmGmCmAmAmGmAmAfCmAfCmUmGmUmUmsUmsUm |
| DM0412-0017 | SS | AmsAmsCmAmGmUmGfUmUfCfUfUmGmCmUmCmUm**(DT3-A)**UmAmAm |
| | AS | UmsUfsAmUmAmGfAmGmCmAmAmGmAmAfCmAfCmUmGmUmUmsUmsUm |
| DM0412-0018 | SS | AmsAmsCmAmGmUmGfUmUfCfUfUmGmCmUmCmUmAm**(DT3-U)**AmAm |
| | AS | UmsUfsAmUmAmGfAmGmCmAmAmGmAmAfCmAfCmUmGmUmUmsUmsUm |
| DM0412-0019 | SS | AmsAmsCmAmGmUmGfUmUfCfUfUmGmCmUmCmUmAmUm**(DT3-A)**Am |
| | AS | UmsUfsAmUmAmGfAmGmCmAmAmGmAmAfCmAfCmUmGmUmUmsUmsUm |
| DM0412-0020 | SS | AmsAmsCmAmGmUmGfUmUfCfUfUmGmCmUmCmUmAmUmAm**(DT3-A)** |
| | AS | UmsUfsAmUmAmGfAmGmCmAmAmGmAmAfCmAfCmUmGmUmUmsUmsUm |
| DM0412-0021 | SS | AmsAmsCmAmGmUmGfUmUfCfUfUmGmCmUmCmUmAmUmAmAm |
| | AS | UmsUfsAmUmAmGfAmGmCmAmAmGmAmAfCmAfCmUmGmUmUms**(DT3-U)**sUm |
| DM0412-0022 | SS | AmsAmsCmAmGmUmGfUmUfCfUfUmGmCmUmCmUmAmUmAmAm |
| | AS | UmsUfsAmUmAmGfAmGmCmAmAmGmAmAfCmAfCmUmGmUmUmsUms**(DT3-U)** |
| DM0412-0023 | SS | AmsAmsCmAmGmUmGfUmUfCfUfUmGmCmUmCmUmAmUmAm**(DT0-A)** |
| | AS | UmsUfsAmUmAmGfAmGmCmAmAmGmAmAfCmAfCmUmGmUmUmsUmsUm |
| DM0412-0024 | SS | AmsAmsCmAmGmUmGfUmUfCfUfUmGmCmUmCmUmAmUmAmAm |
| | AS | UmsUfsAmUmAmGfAmGmCmAmAmGmAmAfCmAfCmUmGmUmUmsUms**(DT0-U)** |
| DM0412-0025 | SS | AmsAmsCmAmGmUmGfUmUfCfUfUm**(DT0-G)**CmUmCmUmAmUmAmAm |
| | AS | UmsUfsAmUmAmGfAmGmCmAmAmGmAmAfCmAfCmUmGmUmUmsUmsUm |
| DM0412-0026 | SS | AmsAmsCmAmGmUmGfUmUfCfUfUmGm**(DT0**-**C)**UmCmUmAmUmAmAm |
| | AS | UmsUfsAmUmAmGfAmGmCmAmAmGmAmAfCmAfCmUmGmUmUmsUmsUm |
| DM0412-0027 | SS | AmsAmsCmAmGmUmGfUmUfCfUfUmGmCmUmCmUmAmUmAm**(DT10-A)** |
| | AS | UmsUfsAmUmAmGfAmGmCmAmAmGmAmAfCmAfCmUmGmUmUmsUmsUm |
| DM0412-0028 | SS | AmsAmsCmAmGmUmGfUmUfCfUfUmGmCmUmCmUmAmUmAmAm |
| | AS | UmsUfsAmUmAmGfAmGmCmAmAmGmAmAfCmAfCmUmGmUmUmsUms**(DT10-U)** |
| DM0412-0029 | SS | AmsAmsCmAmGmUmGfUmUfCfUfUm**(DT10-G)**CmUmCmUmAmUmAmAm |
| | AS | UmsUfsAmUmAmGfAmGmCmAmAmGmAmAfCmAfCmUmGmUmUmsUmsUm |
| DM0412-0030 | SS | AmsAmsCmAmGmUmGfUmUfCfUfUmGm**(DT10-C)**UmCmUmAmUmAmAm |
| | AS | UmsUfsAmUmAmGfAmGmCmAmAmGmAmAfCmAfCmUmGmUmUmsUmsUm |

### Experiment 9: In Vitro Silencing Efficacy Against TTR mRNA by siRNA Duplexes Containing DT3-Nu

### Activity screening step:

### Cell culture and transfection:

Hepa1-6 cells (ATCC, Catalog No. CRL1830) were cultured in DMEM (Invitrogen, Catalog No. 11965092) supplemented with 10% FBS (Ausgenex, Catalog No. FBS500-S) and 1% penicillin-streptomycin under conditions of 37°C and 5% CO₂ until they reached near confluence. The cells were then trypsinized, resuspended in cell seeding medium (DMEM + 10% FBS), and seeded at a density of 5,000 cells per well in 90 µL into 96-well cell culture plates (Corning, Catalog No. 3904), followed by overnight incubation at 37°C with 5% CO₂. Transfection conditions were as follows: 4.6 µL of Opti-MEM (Gibco, catalog no. 31985-070) and 0.4 µL of Lipofectamine RNAiMax (Invitrogen, Carlsbad CA. Catalog no. 13778-150) were mixed with 5 µL of siRNAs per well and incubated for 15 min at room temperature. The mixture was then added to 10 µL per well in the 96-well plate laid out the day before. The cells were incubated at 37°C under 5% CO₂ for 48 hours. The final concentrations in the siRNAs experiments were 10 nM, 1 nM, and 0.1 nM.

### RNA extraction with reverse transcription into cDNA:

Cells were lysed for RNA extraction according to the instructions for Cells-to-Ct bulk lysis reagents (Invitrogen, catalog number 4391851C). 1) Aspirate off the old medium in each well and add 100 µL PBS to moisten and wash twice. 2) Carefully aspirate off the PBS. 50 µL of lysate was added to each well. 3) Shake at 500rpm for 5 min at room temperature. 4) Add 5µL of termination solution to each well and shake at 500rpm for 2 min at room temperature. If cDNA synthesis is performed immediately, the plate needs to be placed on ice.

Reverse transcription was performed according to the instructions for Cells-to-CT Bulk Fast Advanced RT Reagent (Invitrogen, catalog no. A39110): preparation of the mixture (per well: 25 µL 2× Fast Advanced RT Buffer, 2.5 µL 20× RT Fast Advanced Enzyme Mix and 22.5 µL total RNA). The cDNA was synthesized using Biometra TAdvanced 96SG (analytikjena) by the following steps: 30 min at 37°C, 5 min at 95°C, and maintained at 4°C. cDNA was stored at -20°C or analyze immediately by real-time PCR.

### Real-time PCR:

4.33 µL of cDNA and 0.17 µL of 60X GAPDH TaqMan probe (Invitrogencat, cat#. 4448491), 0.5 µL of 20X TTR TaqMan probe (Invitrogen, catalog no. 4351370) and 5 µL of 2×TaqMan^{®} FAST PCR Master Mix (Applied Biosystems, Catalog No. 4444965) were mixed and added to a 96-well plate (Axygen, Catalog No. PCR-96-FLT-C) for real-time PCR detection. The instrument used was QuantStudio^{®} 7 (AppliedBiosystems), and the system program was 50 °C for 2 min, 95 °C for 20 s, and 40 cycles of 90 °C for 1 s, and 60 °C for 20 s. Unless otherwise stated, 3 transfection replicate wells were done for each siRNAs.

For data analysis, real-time data were analyzed using the ΔΔCt method and these data were normalized with the mock group data, which was calculated as follows: ΔCt = Ct (Target gene) -Ct (GAPDH), ΔΔCt = ΔCt (Assay samples) -ΔCt (Mock), Relative mRNA expression to Mock =2^{-ΔΔCt}, of Inhibition v.s. Mock = {1 -Expression fold (tested sample)/Expression fold (Mock)}*100. The final results are shown in Table 7.

**Table 7: q-PCR in vitro activity screening data**

| Name for siRNA | Inhibition Rate (10 nM) | SD | Inhibition Rate (1 nM) | SD | Inhibition Rate (0.1 nM) | SD |
|---|---|---|---|---|---|---|
| DM0412-0000 | 79.14% | 7.42% | 76.67% | 4.77% | 63.71% | 8.22% |
| DM0412-0001 | 81.22% | 1.87% | 74.11% | 4.50% | 66.44% | 7.06% |
| DM0412-0002 | 75.33% | 9.43% | 69.31% | 9.31% | 61.72% | 2.08% |
| DM0412-0003 | 83.12% | 0.27% | 65.78% | 2.98% | 61.11% | 5.23% |
| DM0412-0004 | 75.12% | 1.71% | 65.56% | 9.43% | 54.72% | 5.84% |
| DM0412-0005 | 79.11% | 6.01% | 71.42% | 5.02% | 62.33% | 7.28% |
| DM0412-0006 | 81.14% | 4.09% | 63.01% | 5.26% | 51.25% | 0.82% |
| DM0412-0007 | 80.29% | 5.17% | 74.12% | 9.59% | 68.51% | 4.95% |
| DM0412-0008 | 80.11% | 3.33% | 62.16% | 8.91% | 53.52% | 1.85% |
| DM0412-0009 | 85.22% | 9.30% | 72.12% | 8.69% | 50.52% | 8.27% |
| DM0412-0012 | 83.14% | 6.15% | 72.11% | 3.26% | 65.74% | 6.09% |
| DM0412-0013 | 74.02% | 1.80% | 61.52% | 7.74% | 57.21% | 3.36% |
| DM0412-0014 | 71.42% | 2.07% | 62.11% | 1.94% | 55.81% | 6.93% |
| DM0412-0015 | 85.01% | 5.21% | 72.68% | 0.33% | 65.72% | 4.11% |
| DM0412-0016 | 82.58% | 2.22% | 78.52% | 6.85% | 66.29% | 3.45% |
| DM0412-0017 | 81.65% | 3.17% | 75.15% | 2.54% | 61.12% | 7.37% |
| DM0412-0018 | 80.19% | 6.90% | 67.22% | 8.61% | 54.38% | 5.63% |
| DM0412-0019 | 74.24% | 8.50% | 69.33% | 0.67% | 60.52% | 8.20% |
| DM0412-0020 | 82.44% | 5.87% | 71.56% | 5.67% | 61.22% | 9.80% |
| DM0412-0021 | 74.35% | 4.82% | 68.17% | 4.91% | 54.42% | 0.65% |
| DM0412-0022 | 84.21% | 7.16% | 77.82% | 2.13% | 60.29% | 2.25% |
| DM0412-0023 | 85.68% | 1.33% | 73.31% | 2.21% | 63.72% | 5.19% |
| DM0412-0024 | 81.22% | 2.81% | 66.12% | 1.88% | 51.32% | 1.55% |
| DM0412-0025 | 87.90% | 2.87% | 77.15% | 4.27% | 62.63% | 5.85% |
| DM0412-0026 | 77.33% | 2.31% | 63.44% | 3.71% | 59.03% | 6.78% |
| DM0412-0027 | 87.15% | 2.09% | 76.22% | 4.72% | 66.35% | 6.32% |
| DM0412-0028 | 82.59% | 3.15% | 72.35% | 3.72% | 60.16% | 5.28% |
| DM0412-0029 | 85.89% | 4.26% | 73.37% | 5.16% | 63.09% | 4.45% |
| DM0412-0030 | 79.54% | 3.26% | 64.35% | 3.56% | 61.39% | 5.67% |

RESULTS: The modification (DT0-A, U, G, C; DT3-A, U, G, C; DT10-A, U, G, C) was able to increase the activity of siRNA sequences when added to them. Even at a lower concentration (0.1 nM), some of the sequences still had a silencing efficiency of greater than 60%.

### Experiment 10: Synthesis of Single-Stranded Oligonucleotides for 3'-Exonuclease Stability Evaluation

The synthesis of oligonucleotide single strands is the same as the usual phosphoramidite solid-phase synthesis method, in which the synthesized phosphoramidite monomers (DT3) synthesized as described above are used to replace the parent sequence protonucleotides in the synthesis of a single-stranded oligonucleotide with DT3 modification at the 3'-terminus.

The synthesis process is briefly described as follows: on the LK- 48E synthesizer (Linkun), starting with Universal CPG (DNA chem) carrier, nucleoside phosphoramidite monomers were linked one by one according to the synthesis program. Except for the synthesis of DT3-U (corresponding to compound 11') as described above, all other nucleoside monomers raw materials, such as nucleoside phosphoramidite monomers, such as 2'-F RNA and 2'-O-methyl RNA, were purchased from Hongene Biotech. 5'-Ethylthio-1H-tetrazole (ETT) was used as an activator (0.6 M acetonitrile solution), 0.22 M PADS dissolved in a 1:1 v/v ratio and a solution of trimethylpyridine (Suzhou Koloma) was used as a sulfurizing reagent, and iodopyridine/water solution (Koloma) was used as an oxidizing agent.

After solid-phase synthesis was completed, the oligoribonucleotides were cleaved from this solid support, and a 3:1 solution of 28% ammonia and ethanol was used to soak for 16 h at 50 °C, followed by centrifugation, and the supernatant was transferred to another centrifuge tube, concentrated and evaporated to dryness, and then purified using C18 reversed-phase chromatography with a mobile phase of 0.1 M TEAA and acetonitrile, and DMTr was removed using a 3% trifluoroacetic acid solution. The target oligonucleotides were collected and lyophilized. They were identified as target products by LCMS and quantified by UV (260nm). The obtained single-stranded oligonucleotides were dissolved in 1xPBS and adjusted to the concentration required for the experiment and set aside.

The modification schemes used in Table 8 are as follows:
"m" is a 2'-OMe modification to one nucleotide adjacent to the left of the letter m, "f" is a 2'-F modification to one nucleotide adjacent to the left of the letter f, the "s" is a PS backbone modification between two nucleotides adjacent to the left and right of the letter s. "DT3" is a 4'-(*S*)-CH₂OC₁₂H₂₅-TNA modification.

Among them, DM0412-0000, which lacks nucleoside compound modifications compared to the other groups, served as a control.

**Table 8: Oligonucleotide sequences used to examine 3'-exonuclease stability**

| | |
|---|---|
| DM0412-1000 | UmsUfsAmUmAmGfAmGmCmAmAmGmAmAfCmAfCmUmGmUmUmsUmsUm |
| DM0412-1001 | UmsUfsAmUmAmGfAmGmCmAmAmGmAmAfCmAfCmUmGmUmUmsUms**(DT3-U)** |
| DM0412-1002 | UmsUfsAmUmAmGfAmGmCmAmAmGmAmAfCmAfCmUmGmUmUmUm**(DT3-U)** |

### Experiment 11: 3'-Exonuclease Stability Assay of Single-Stranded Sequences

### Stability evaluation procedure:

The samples (oligonucleotide molecules DM0412-1000, DM0412-1001, DM0412-1002) were solubilized using PBS (BOSTER, Lot. 17F24C21) and the concentration of the compounds was determined using a microplate reader, and the working solution was diluted to 300 ng/µL using PBS.

Samples to be tested (DM0412-1000, DM0412-1001, DM0412-1002, see Table 8) were co-incubated with the enzyme system: 43 µL of the enzyme system (Sigma, Lot. P4506) as well as 7 µL of the samples to be tested were added to EP tube (2 replicates per sample), vortex-mixed, and then incubated at 37°C in an incubator.

Reaction termination: At each designated time point of the stability study, 50 µL was taken from the incubation tube and transferred to another EP tube. Then, 5 µL of EDTA (0.5 M) solution was added, mixed thoroughly, and the tube was immediately placed in liquid nitrogen for rapid cooling to terminate the reaction.

Biological Sample Pretreatment: After thawing the above frozen samples at room temperature, 10 µL of internal standard compounds (compounds used for quality control, i.e., siRNA sequences: the antisense strand CmsUfsCmGmGfUmAfCmAm UmGmCfAmAfUmCmsCmsCm; and the sense strand for GmsGmGmAmUmUfGmCfAfUfGmUm AmAmCmCmGmsAmsGm), 150 µL Tris, and 150 µL extractant (phenol: chloroform: isoamyl alcohol = 25:24:1) were added to each tube. After vortex mixing, the mixture was centrifuged for 10 min, and the supernatant was taken for high-resolution LC-MS analysis. The residual amounts of the test samples at each time point are shown in Table 9.

**Table 9: Residual amounts of test samples at each time point**

| Time point (min) | DM0412-1000 (% residual) | DM0412-1001 (% residual) | DM0412-1002 (% residual) |
|---|---|---|---|
| 0 | 100.0 | 100.0 | 100.0 |
| 30 | 92.1 | 99.4 | 97.2 |
| 60 | 40.5 | 78.5 | 76.8 |
| 120 | 5.7 | 50.2 | 48.2 |
| 180 | 0.5 | 20.3 | 18.1 |

RESULTS: This modification (DT3-U) significantly improves the stability of oligonucleotide single strands against 3'-exonucleases when added to single-stranded 3'-ends.

### Experiment 12: Synthesis of siRNA Sequences for In Vivo Evaluation in Wild-Type Mice

The synthesis process is briefly described as follows: on the LK- 48E synthesizer (Linkun), starting with Universal CPG (DNA chem) carrier, nucleoside phosphoramidite monomers were linked one by one according to the synthesis program. Except for syntheses of DT3-A (corresponding to compound 21'), U (corresponding to compound 11'), G (corresponding to compound 26'), C (corresponding to compound 16'), DT0-A (corresponding to compound 43'), U (corresponding to compound 33'), DT5-A (corresponding to compound 188'), DT7-A (corresponding to compound 189'), and DT10-A (corresponding to compound 66') as described above, other nucleoside monomers raw materials, such as nucleoside phosphoramidite monomers, such as 2'-F RNA and 2'-O-methyl RNA, were purchased from Hongene Biotech, L96 was purchased from WuXi AppTec. 5'-Ethylthio-1H-tetrazole (ETT) was used as an activator (0.6 M acetonitrile solution), 0.22 M PADS dissolved in a 1:1 v/v ratio and a solution of trimethylpyridine (Suzhou Koloma) was used as a sulfurizing reagent, and iodopyridine/water solution (Koloma) was used as an oxidizing agent.

After solid-phase synthesis was completed, the oligoribonucleotides were cleaved from this solid support, and a 3:1 solution of 28% ammonia and ethanol was used to soak for 16 h at 50 °C, followed by centrifugation, and the supernatant was transferred to another centrifuge tube, concentrated and evaporated to dryness, and then purified using C18 reversed-phase chromatography with a mobile phase of 0.1 M TEAA and acetonitrile, and DMTr was removed using a 3% trifluoroacetic acid solution. The target oligonucleotides were collected and lyophilized. They were identified as target products by LCMS and quantified by UV (260nm).

The obtained single-stranded oligonucleotide was subjected to exchange into sodium acetate using a 3 kDa cutoff ultrafiltration tube, and according to the equimolar ratio, were paired based on complementarity and annealed. The final obtained double-stranded siRNA was dissolved in water and adjusted to the concentration required for the experiment and set aside.

The modification schemes used in Table 10 are as follows:
"m" is a 2'-OMe modification to one nucleotide adjacent to the left of the letter m, "f" is a 2'-F modification to one nucleotide adjacent to the left of the letter f, "s" is a PS backbone modification between two nucleotides adjacent to the left and right of the letter s, "DT3" is a 4'-(*S*)-CH₂OC₁₂H₂₅-TNA modification, "DT0" is 4'-(*S*)-CH₂OMe-TNA modification, "DT5" is 4'-(*S*)- CH₂OC₁₄H₂₉-TNA modification, "DT7" is 4'-(*S*)-CH₂OC₁₆H₃₃-TNA modification, and "DT10" is 4'-(*S*)-CH₃-TNA modification.

Among them, DM0412-2000, which lacks nucleoside modifications on the sense strand compared to other groups, served as a control.

**Table 10: Sequences of siRNA molecules used for in vivo evaluation in wild-type mice**

| | | |
|---|---|---|
| DM0412-2000 | SS | AmsAmsCmAmGmUmGfUmUfCfUfUmGmCmUmCmUmAmUmAmAm**L96** |
| | AS | UmsUfsAmUmAmGfAmGmCmAmAmGmAmAfCmAfCmUmGmUmUmsUmsUm |
| DM0412-2001 | SS | **(DT3-A)**sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| DM0412-2002 | SS | Ams**(DT3-A)**sCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| DM0412-2003 | SS | AmsAms(**DT3-C**)AmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| DM0412-2004 | SS | AmsAmsCm(**DT3-A**)GmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| DM0412-2005 | SS | AmsAmsCmAm(**DT3-G**)UmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| DM0412-2006 | SS | AmsAmsCmAmGm(**DT3-U**)GfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| DM0412-2007 | SS | AmsAmsCmAmGmUm(**DT3-G**)UmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| DM0412-2008 | SS | AmsAmsCmAmGmUmGf(**DT3-U**)UfCfUfUmGmCmUmCmUmAmUmAmAm **L96** |
| | AS | UmsUfsAmUmAmGfAmGmCmAmAmGmAmAfCmAfCmUmGmUmUmsUmsUm |
| DM0412-2009 | SS | AmsAmsCmAmGmUmGfUm(**DT3-U**)CfUfUmGmCmUmCmUmAmUmAmAm **L96** |
| | AS | UmsUfsAmUmAmGfAmGmCmAmAmGmAmAfCmAfCmUmGmUmUmsUmsUm |
| DM0412-2010 | SS | AmsAmsCmAmGmUmGfUmUfCf(**DT3-U**)UmGmCmUmCmUmAmUmAmAm **L96** |
| | AS | UmsUfsAmUmAmGfAmGmCmAmAmGmAmAfCmAfCmUmGmUmUmsUmsUm |
| DM0412-2011 | SS | AmsAmsCmAmGmUmGfUmUfCfUf(**DT3-U**)GmCmUmCmUmAmUmAmAm **L96** |
| | AS | UmsUfsAmUmAmGfAmGmCmAmAmGmAmAfCmAfCmUmGmUmUmsUmsUm |
| DM0412-2012 | SS | AmsAmsCmAmGmUmGfUmUfCfUfUm(**DT3-G**)CmUmCmUmAmUmAmAm **L96** |
| | AS | UmsUfsAmUmAmGfAmGmCmAmAmGmAmAfCmAfCmUmGmUmUmsUmsUm |
| DM0412-2013 | SS | AmsAmsCmAmGmUmGfUmUfCfUfUmGm(**DT3-C**)UmCmUmAmUmAmAm **L96** |
| | AS | UmsUfsAmUmAmGfAmGmCmAmAmGmAmAfCmAfCmUmGmUmUmsUmsUm |
| DM0412-2014 | SS | AmsAmsCmAmGmUmGfUmUfCfUfUmGmCm(**DT3-U**)CmUmAmUmAmAm **L96** |
| | AS | UmsUfsAmUmAmGfAmGmCmAmAmGmAmAfCmAfCmUmGmUmUmsUmsUm |
| DM0412-2015 | SS | AmsAmsCmAmGmUmGfUmUfCfUfUmGmCmUm(**DT3-C**)UmAmUmAmAm **L96** |
| | AS | UmsUfsAmUmAmGfAmGmCmAmAmGmAmAfCmAfCmUmGmUmUmsUmsUm |
| DM0412-2016 | SS | AmsAmsCmAmGmUmGfUmUfCfUfUmGmCmUmCm(**DT3-U**)AmUmAmAm **L96** |
| | AS | UmsUfsAmUmAmGfAmGmCmAmAmGmAmAfCmAfCmUmGmUmUmsUmsUm |
| DM0412-2017 | SS | AmsAmsCmAmGmUmGfUmUfCfUfUmGmCmUmCmUm(**DT3-A**)UmAmAm **L96** |
| | AS | UmsUfsAmUmAmGfAmGmCmAmAmGmAmAfCmAfCmUmGmUmUmsUmsUm |
| DM0412-2018 | SS | AmsAmsCmAmGmUmGfUmUfCfUfUmGmCmUmCmUmAm(**DT3-U**)AmAm **L96** |
| | AS | UmsUfsAmUmAmGfAmGmCmAmAmGmAmAfCmAfCmUmGmUmUmsUmsUm |
| DM0412-2019 | SS | AmsAmsCmAmGmUmGfUmUfCfUfUmGmCmUmCmUmAmUm(**DT3-A**)Am **L96** |
| | AS | UmsUfsAmUmAmGfAmGmCmAmAmGmAmAfCmAfCmUmGmUmUmsUmsUm |
| DM0412-2020 | SS | AmsAmsCmAmGmUmGfUmUfCfUfUmGmCmUmCmUmAmUmAm(**DT3-A**) **L96** |
| | AS | UmsUfsAmUmAmGfAmGmCmAmAmGmAmAfCmAfCmUmGmUmUmsUmsUm |
| DM0412-2021 | SS | (**DT0-A**)sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| DM0412-2022 | SS | (**DT5-A**)sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| DM0412-2023 | SS | **(DT7-A)**sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| DM0412-2024 | SS | **(DT10-A)**sAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUmsUm |
| DM0412-2025 | SS | AmsAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUmsUms**(DT0-U)** |
| DM0412-2026 | SS | AmsAmsCmAmGmUmGfUmUfCfUfUmGmCmUmCmUmAmUmAmAm **L96** |
| | AS | UmsUfsAmUmAmGfAmGmCmAmAmGmAmAfCmAfCmUmGmUmUms**(DT0-U)**s Um |

### Experiment 13: Evaluation of In Vivo Activity of TTR-Targeted mRNA and siRNA Duplexes Containing DT-4'-TNA-Modified Nucleotides

### In vivo evaluation of siRNA sequence activity in wild-type mice:

The activity of the above compounds (DM0412 - 2000 to DM0412 - 2026, see Table 10) was evaluated *in vivo* using wild-type C57BL/6 mice (Spivey (Beijing) Biotechnology Co. Ltd.).

6- to 8-week-old C57BL/6 mice received a single subcutaneous injection of the compound (from Table 5) at a dose of 1 mg/kg. Orbital blood samples were collected using EP tubes before administration and on days 7, 14, 21, 28, 35, 42, and 56 post administration. The blood samples were allowed to clot at room temperature for two hours, followed by centrifugation at 5500 rpm for 10 minutes at 4°C. The serum was then separated and collected for the determination of TTR levels in the animal serum.

Determination of TTR levels in serum was analyzed by TTR enzyme-linked immunosorbent assay (ELISA). ELISA was performed using Abcam Mouse Prealbumin ELISA Kit (ab282297), and all samples were tested according to the instructions of the test kit. Absorbance at 450 nm was measured using a Tecan SPARK microplate reader. A parametric standard curve was generated from the standards (supplied with the aforementioned ELISA kit), and serum TTR protein levels (in µg/mL) were determined based on this curve. The protein content of individual animals was compared to their corresponding pre-administration serum protein content to determine the percentage of TTR remaining relative to pre-administration.

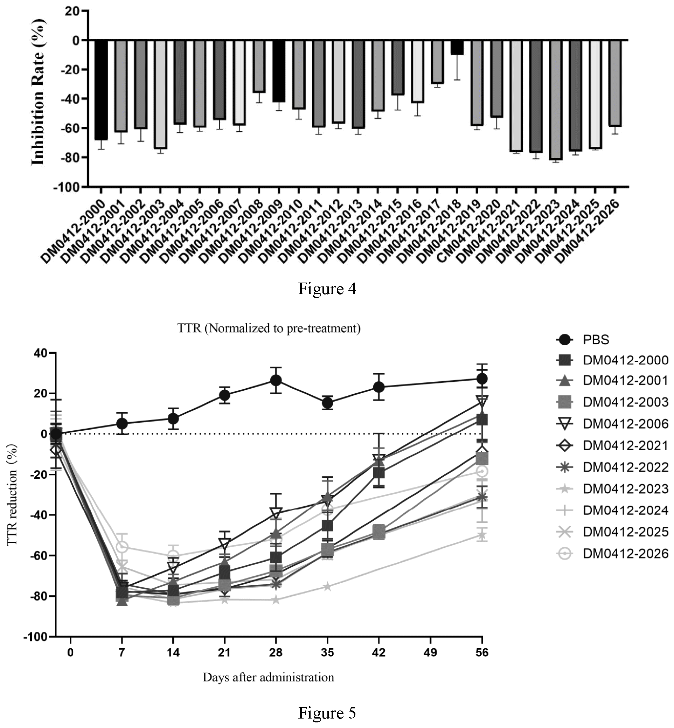

The experimental results are shown in Figures 4 and 5.

RESULTS: This class of modified nucleotides (DT3-A, U, G, and C; DT0-A and U; DT5-A; DT7-A; and DT10-A) can enhance the *in vivo* silencing activity of siRNA sequences. Some sequences maintained relatively high activity even on day 28 post-administration, indicating that the designed sequences possess a certain degree of long-lasting efficacy.

## Claims

1. A nucleoside compound shown in Formula (1), or a stereoisomer thereof: 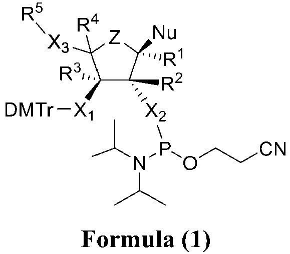 wherein:
R¹, R², R³, and R⁴ are each independently selected from the group consisting of hydrogen, halogen, cycloalkyl, C1-C6 alkyl, substituted C1-C6 alkyl, C1-C6 alkoxy, substituted C1-C6 alkoxy, C2-C6 alkenyl, substituted C2-C6 alkenyl, C2-C6 alkynyl, and substituted C2-C6 alkynyl;
R⁵ is selected from the group consisting of hydrogen, halogen, cyclopropyl, cyclobutyl, C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, and substituted C2-C30 alkynyl;
Nu represents a base or is absent;
Z is -O-, -S-, -NR¹⁰, or -CR¹⁰R¹¹, wherein R¹⁰ and R¹¹ are each independently selected from the group consisting of hydrogen, halogen, acyl, substituted or unsubstituted alkyl, substituted or unsubstituted aryl, substituted or unsubstituted heteroaryl, substituted or unsubstituted heterocyclyl, and substituted or unsubstituted cycloalkyl;
X₁ and X₂ are each independently selected from the group consisting of -(CH₂)ₙO- and -(CH₂)ₙS-, wherein n is any integer from 0 to 10;
X₃ is selected from the group consisting of -(CH₂)ₙO-, -(CH₂)ₙ-, -(CH₂)ₙS-, -(CH₂)ₙNH(CO)-, -(CH₂)ₙ(CO)NH-, and -(CH₂)ₙO(CH₂)ₙ-A-, wherein A is substituted or unsubstituted aryl, substituted or unsubstituted heteroaryl, substituted or unsubstituted heterocyclyl, or substituted or unsubstituted cycloalkyl, wherein n is any integer from 0 to 10.

2. The nucleoside compound according to claim 1, which is a compound selected from the group consisting of Formula 1-a and Formula 1-b: 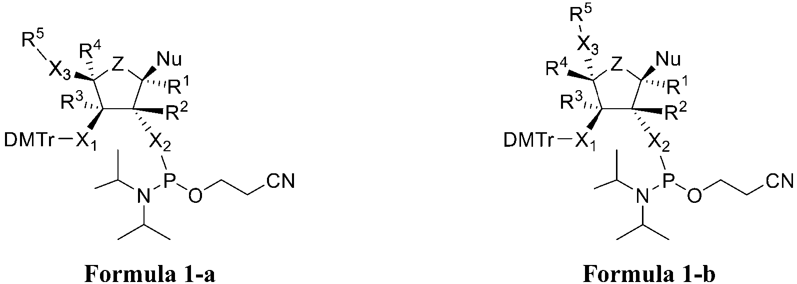 wherein R¹, R², R³, R⁴, R⁵, Nu, X₁, X₂, and X₃ are as defined in claim 1.

3. The nucleoside compound shown in Formula 1-a or Formula 1-b according to claim 2, wherein:
Z is -O-, -S-, or -N(COR')-, wherein R' is C1-C5 alkyl; preferably, R' is selected from the group consisting of methyl, ethyl, n-propyl, isopropyl, n-butyl, isobutyl, and tert-butyl;
X₁ and X₂ are each independently selected from the group consisting of -(CH₂)ₙO- and -(CH₂)ₙS-, wherein n is any integer from 0 to 5; preferably, n is selected from the group consisting of 0, 1, 2, 3, 4, and 5;
X₃ is selected from the group consisting of -(CH₂)ₙ-, -(CH₂)ₙO-, -(CH₂)ₙS-, -(CH₂)ₙNH(CO)-, -(CH₂)ₙ(CO)NH-, and -(CH₂)ₙO(CH₂)ₙ-A-, wherein A is substituted or unsubstituted aryl, substituted or unsubstituted heteroaryl, substituted or unsubstituted heterocyclyl, or substituted or unsubstituted cycloalkyl, wherein n is any integer from 0 to 5; preferably, n is selected from the group consisting of 0, 1, 2, 3, 4, and 5.

4. The nucleoside compound according to any one of claims 1 to 3, wherein:
Z is -O-, -S-, or -N(COCH₃)-;
X₁ is -O-, -S-, or -CH₂O-;
X₂ is -O- or -S-;
X₃ is -O-, -CH₂-, -CH₂O-, -CH₂S-, -CH₂NH(CO)-, -(CO)NH-, or -CH₂OCH₂-heteroaryl-; preferably, X₃ is -O-, -CH₂O-, -CH₂S-, -CH₂NH(CO)-, -(CO)NH-, or -CH₂OCH₂-heteroaryl-;
R¹, R², and R³ are hydrogen;
R⁴ is hydrogen or methoxy;
R⁵ is hydrogen, methyl, halogen, or C10-C18 alkyl; preferably, *R⁵* is methyl or C10-C18 alkyl; more preferably, R⁵ is methyl, C10 alkyl, C11 alkyl, C12 alkyl, C13 alkyl, C14 alkyl, C15 alkyl, C16 alkyl, C17 alkyl, or C18 alkyl; even further preferably, R⁵ is methyl, C10 straight-chain alkyl, C11 straight-chain alkyl, C12 straight-chain alkyl, C13 straight-chain alkyl, C14 straight-chain alkyl, C15 straight-chain alkyl, C16 straight-chain alkyl, C17 straight-chain alkyl, or C18 straight-chain alkyl.

5. The nucleoside compound according to any one of claims 1 to 4, wherein: Nu is a base selected from the group consisting of:

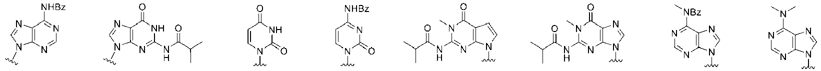

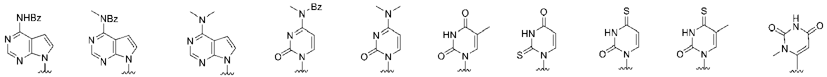

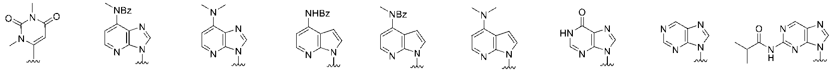

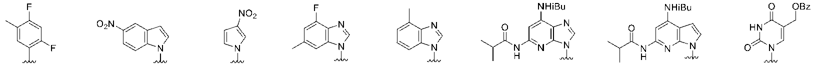

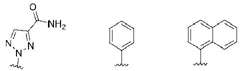

6. The nucleoside compound shown in Formula 1-a or Formula 1-b according to any one of claims 2-5, wherein the nucleoside compound shown in Formula 1-a or Formula 1-b is a nucleoside compound shown in Formula 2-a or Formula 2-b, respectively, 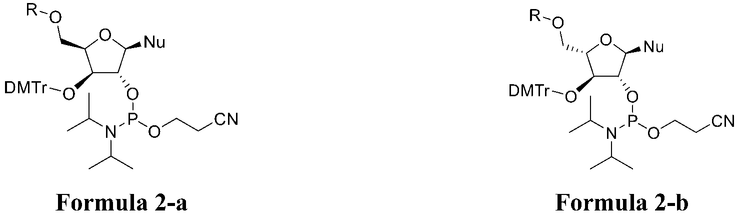
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined in claim 1.

7. The nucleoside compound shown in Formula 1-a according to any one of claims 2-5, wherein the nucleoside compound shown in Formula 1-a is selected from the group consisting of a nucleoside compound shown in Formula 3-a, Formula 5-a, Formula 8-a, or Formula 9-a, 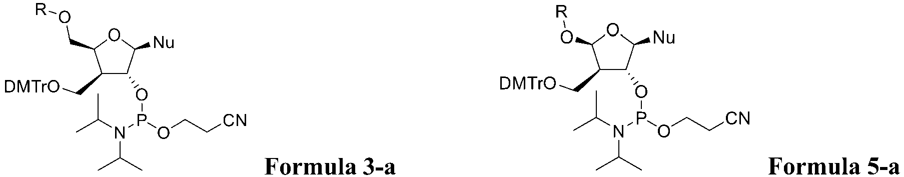 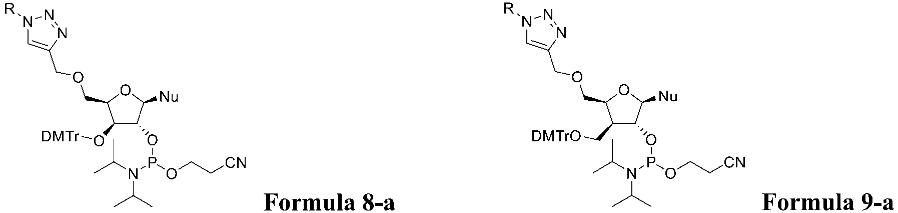
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined in claim 1.

8. The nucleoside compound shown in Formula 1-a or Formula 1-b according to any one of claims 2-5, wherein the nucleoside compound shown in Formula 1-a or Formula 1-b is a nucleoside compound shown in Formula 4-a or Formula 3-b, respectively, 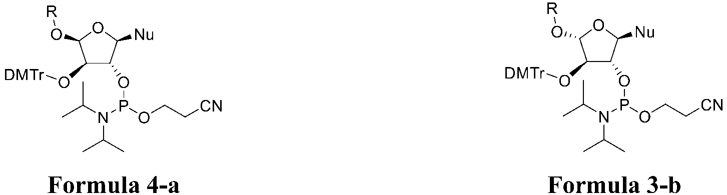
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined in claim 1.

9. The nucleoside compound shown in Formula 1-a or Formula 1-b according to any one of claims 2-5, wherein the nucleoside compound shown in Formula 1-a or Formula 1-b is a nucleoside compound shown in Formula 6-a or Formula 4-b, respectively, 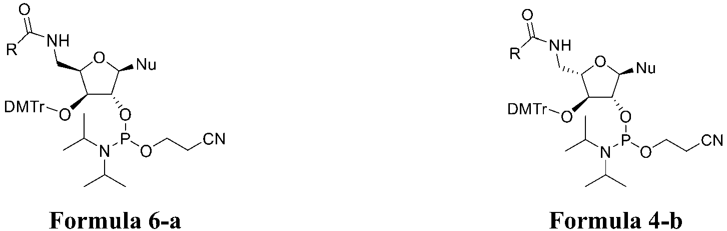
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined in claim 1.

10. The nucleoside compound shown in Formula 1-a or Formula 1-b according to any one of claims 2-5, wherein the nucleoside compound shown in Formula 1-a or Formula 1-b is a nucleoside compound shown in Formula 7-a or Formula 5-b, respectively, 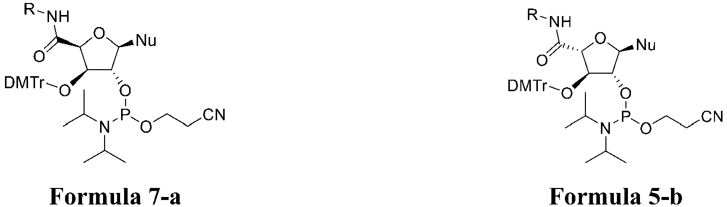
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined in claim 1.

11. The nucleoside compound shown in Formula 1-a or Formula 1-b according to any one of claims 2-5, wherein the nucleoside compound shown in Formula 1-a or Formula 1-b is a nucleoside compound shown in Formula 10-a or Formula 6-b, respectively, 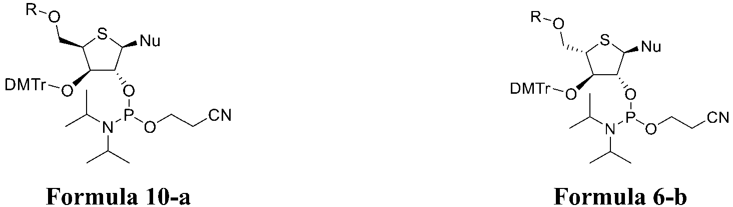
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined in claim 1.

12. The nucleoside compound shown in Formula 1-a or Formula 1-b according to any one of claims 2-5, wherein the nucleoside compound shown in Formula 1-a or Formula 1-b is a nucleoside compound shown in Formula 11-a or Formula 7-b, respectively, 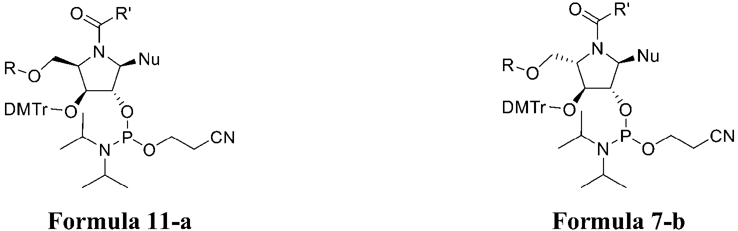
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
R' is C1-C5 alkyl; preferably, R' is methyl, ethyl, n-propyl, or isopropyl;
Nu is as defined in claim 1.

13. The nucleoside compound shown in Formula 1-a or Formula 1-b according to any one of claims 2-5, wherein the nucleoside compound shown in Formula 1-a or Formula 1-b is a nucleoside compound shown in Formula 12-a or Formula 8-b, respectively, 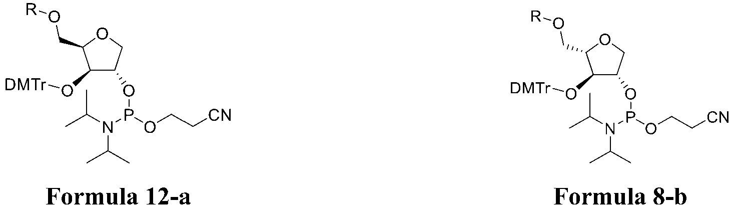 R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl.

14. The nucleoside compound shown in Formula 1-a or Formula 1-b according to any one of claims 2-5, wherein the nucleoside compound shown in Formula 1-a or Formula 1-b is a nucleoside compound shown in Formula 13-a or Formula 9-b, respectively, 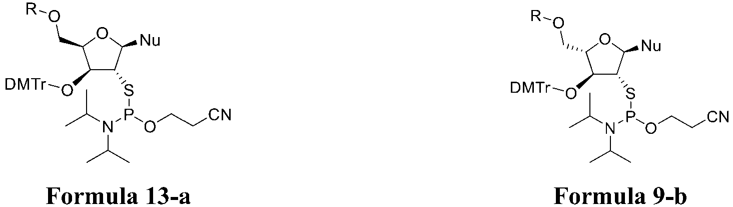
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined in claim 1.

15. The nucleoside compound shown in Formula 1-a or Formula 1-b according to any one of claims 2-5, wherein the nucleoside compound shown in Formula 1-a or Formula 1-b is a nucleoside compound shown in Formula 14-a or Formula 10-b, respectively, 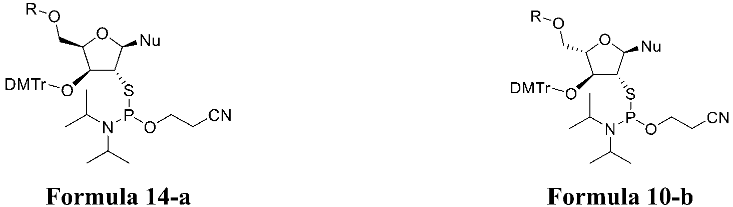
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined in claim 1.

16. The nucleoside compound shown in Formula 1-a or Formula 1-b according to any one of claims 2-5, wherein the nucleoside compound shown in Formula 1-a or Formula 1-b is a nucleoside compound shown in Formula 15-a or Formula 11-b, respectively, 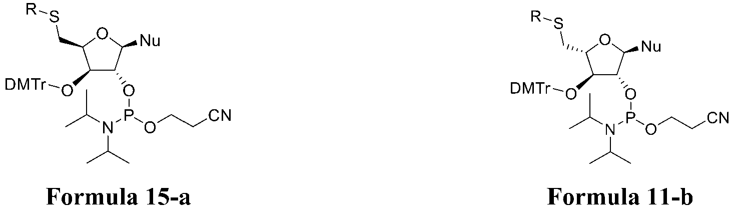
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined in claim 1.

17. The nucleoside compound shown in Formula 1-a or Formula 1-b according to any one of claims 2-5, wherein the nucleoside compound shown in Formula 1-a or Formula 1-b is a nucleoside compound shown in Formula 16-a or Formula 12-b, respectively, 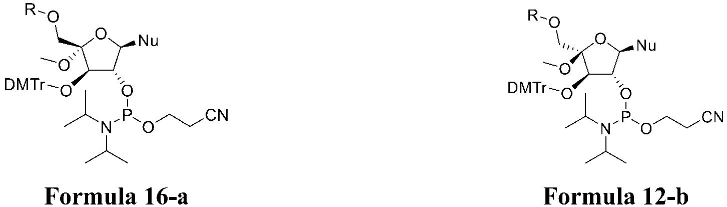
R is C1-C30 alkyl, substituted C1-C30 alkyl, C1-C30 alkoxy, substituted C1-C30 alkoxy, C2-C30 alkenyl, substituted C2-C30 alkenyl, C2-C30 alkynyl, or substituted C2-C30 alkynyl; preferably, R is methyl, C10 alkyl, C11 alkyl, C12 alkyl, C13 alkyl, C14 alkyl, C15 alkyl, C16 alkyl, C17 alkyl, or C18 alkyl;
Nu is as defined in claim 1.

18. The nucleoside compound shown in Formula 1-a according to claim 2 or 3, wherein the nucleoside compound shown in Formula 1-a is a nucleoside compound shown as follows:
| Compound No. and Structure | | Compound No. and Structure | |
|---|---|---|---|
| LT0- Nu | 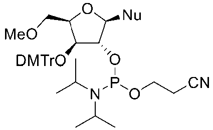 | LT54- Nu | 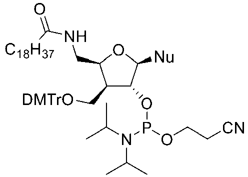 |
| LT1- Nu | 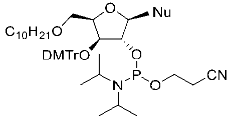 | LT55- Nu | 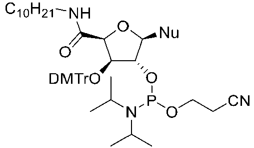 |
| LT2- Nu | 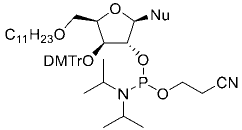 | LT56- Nu | 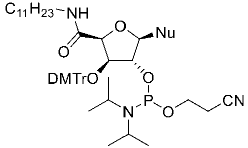 |
| LT3- Nu | 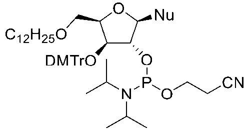 | LT57- Nu | 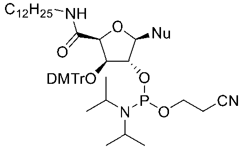 |
| LT4- Nu | 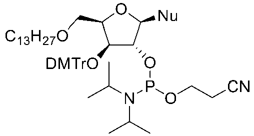 | LT58- Nu | 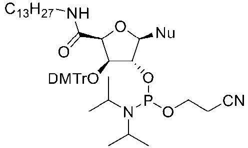 |
| LT5- Nu | 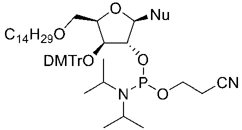 | LT59- Nu | 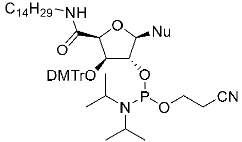 |
| LT6- Nu | 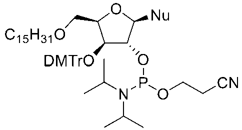 | LT60- Nu | 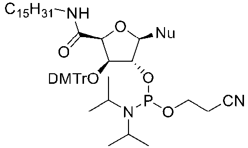 |
| LT7- Nu | 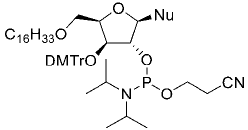 | LT61- Nu | 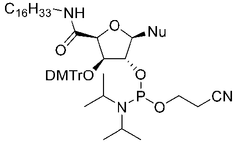 |
| LT8- Nu | 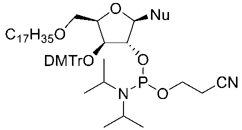 | LT62- Nu | 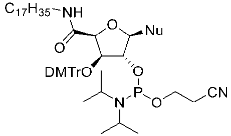 |
| LT9- Nu | 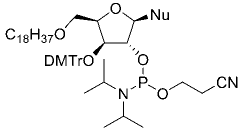 | LT63- Nu | 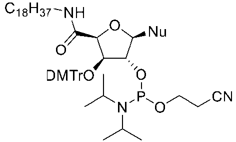 |
| LT10- Nu | 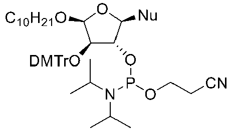 | LT64- Nu | 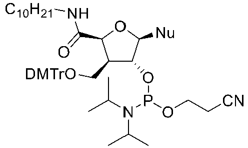 |
| LT11- Nu | 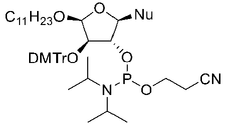 | LT65- Nu | 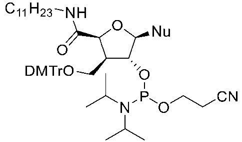 |
| LT12- Nu | 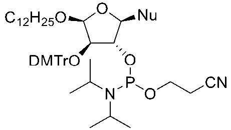 | LT66- Nu | 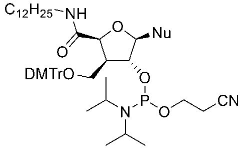 |
| LT13- Nu | 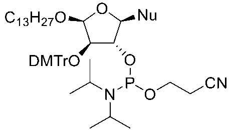 | LT67- Nu | 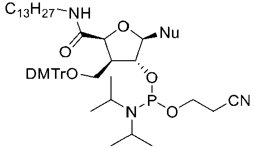 |
| LT14- Nu | 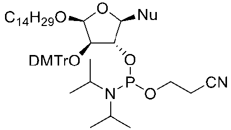 | LT68- Nu | 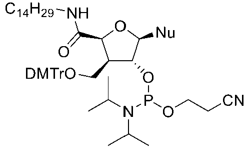 |
| LT15- Nu | 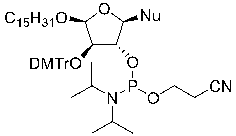 | LT69- Nu | 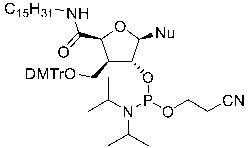 |
| LT16- Nu | 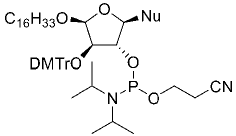 | LT70- Nu | 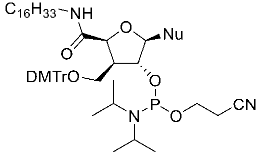 |
| LT17- Nu | 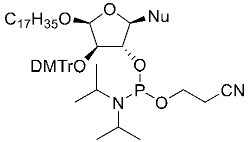 | LT71- Nu | 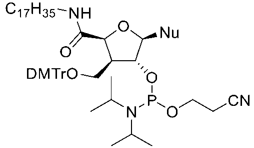 |
| LT18- Nu | 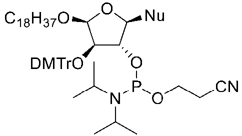 | LT72- Nu | 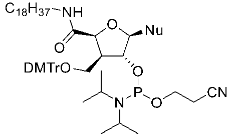 |
| LT19- Nu | 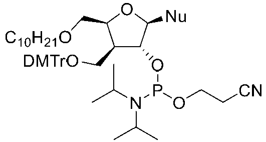 | LT73- Nu | 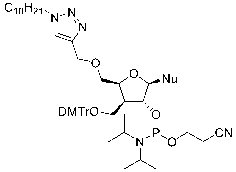 |
| LT20- Nu | 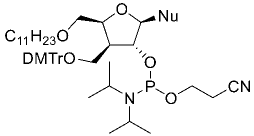 | LT74- Nu | 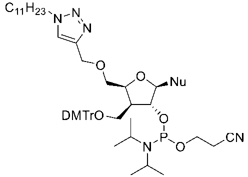 |
| LT21- Nu | 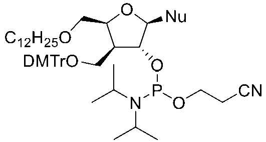 | LT75- Nu | 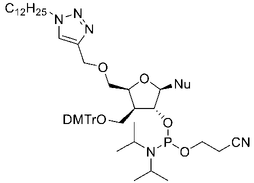 |
| LT22- Nu | 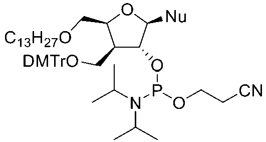 | LT76- Nu | 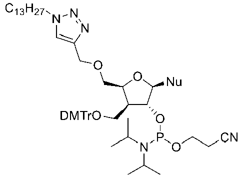 |
| LT23- Nu | 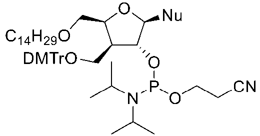 | LT77- Nu | 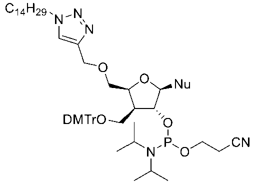 |
| LT24- Nu | 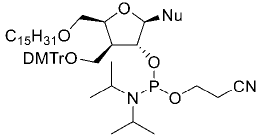 | LT78- Nu | 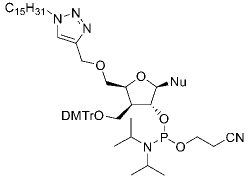 |
| LT25- Nu | 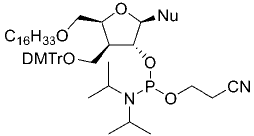 | LT79- Nu | 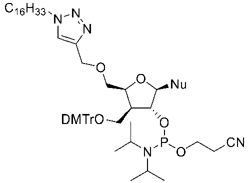 |
| LT26- Nu | 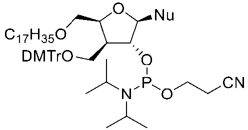 | LT80- Nu | 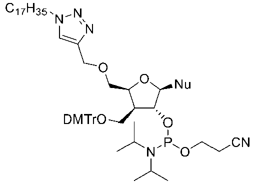 |
| LT27- Nu | 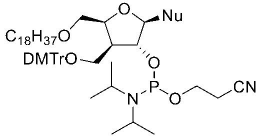 | LT81- Nu | 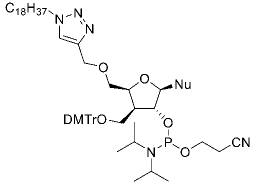 |
| LT28- Nu | 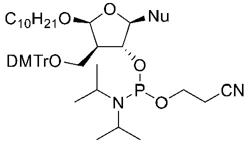 | LT82- Nu | 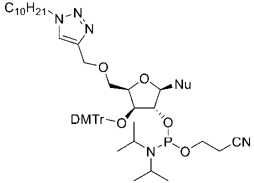 |
| LT29- Nu | 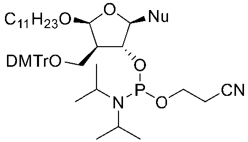 | LT83- Nu | 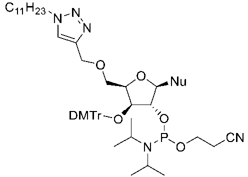 |
| LT30- Nu | 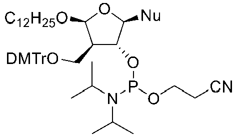 | LT84- Nu | 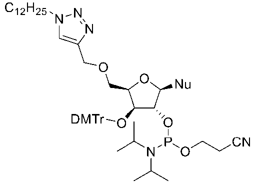 |
| LT31- Nu | 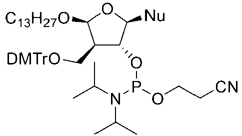 | LT85- Nu | 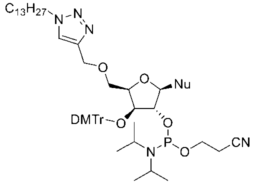 |
| LT32- Nu | 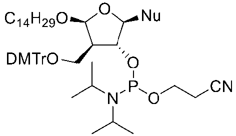 | LT86- Nu | 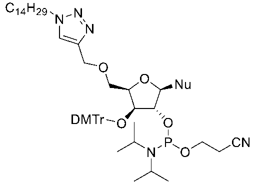 |
| LT33- Nu | 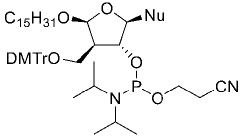 | LT87- Nu | 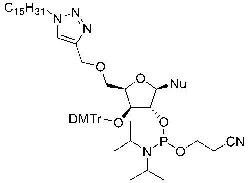 |
| LT34- Nu | 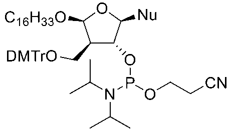 | LT88- Nu | 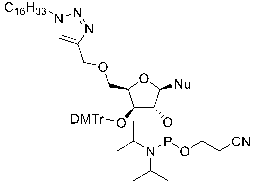 |
| LT35- Nu | 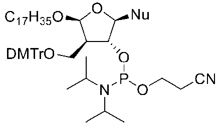 | LT89- Nu | 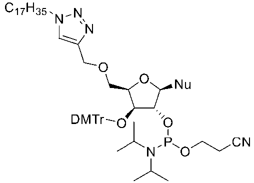 |
| LT36- Nu | 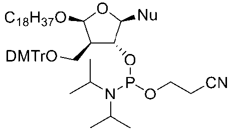 | LT90- Nu | 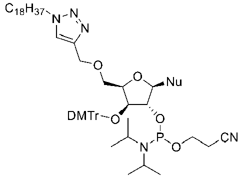 |
| LT37- Nu | 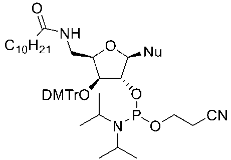 | LT91- Nu | 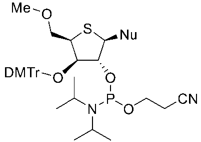 |
| LT38- Nu | 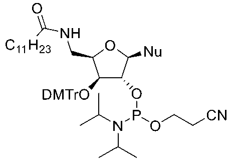 | LT92- Nu | 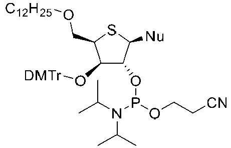 |
| LT39- Nu | 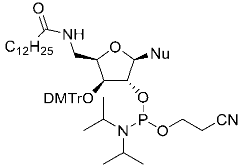 | LT93- Nu | 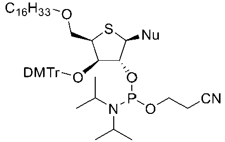 |
| LT40- Nu | 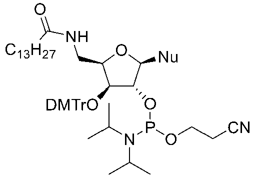 | LT94- Nu | 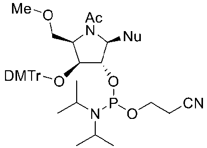 |
| LT41- Nu | 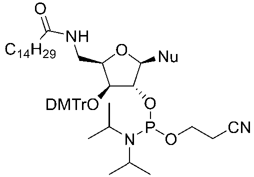 | LT95- without Nu | 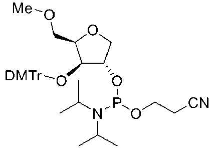 |
| LT42- Nu | 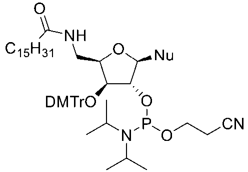 | LT96- without Nu | 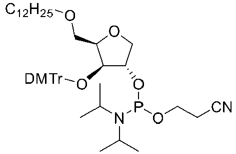 |
| LT43- Nu | 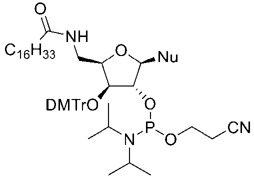 | LT97- without Nu | 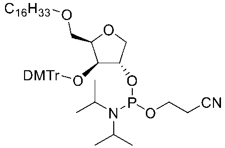 |
| LT44- Nu | 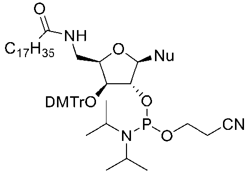 | LT98- Nu | 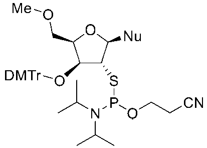 |
| LT45- Nu | 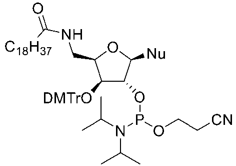 | LT99- Nu | 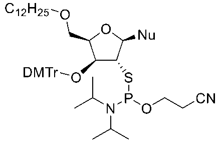 |
| LT46- Nu | 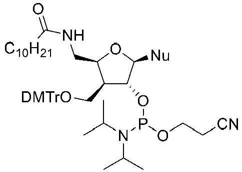 | LT100- Nu | 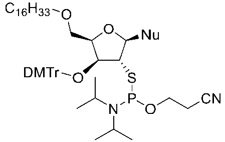 |
| LT47- Nu | 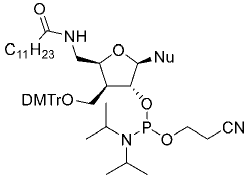 | LT101- Nu | 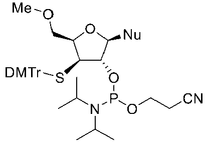 |
| LT48- Nu | 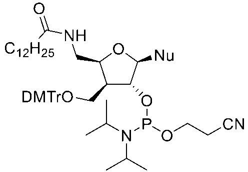 | LT102- Nu | 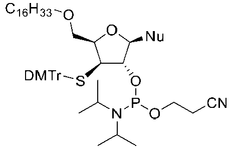 |
| LT49- Nu | 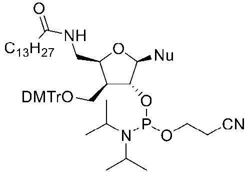 | LT103- Nu | 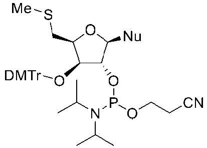 |
| LT50- Nu | 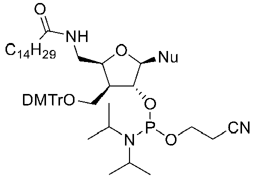 | LT104- Nu | 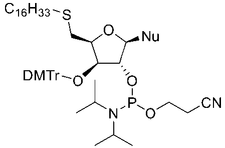 |
| LT51- Nu | 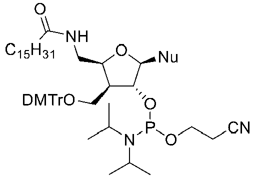 | LT105- Nu | 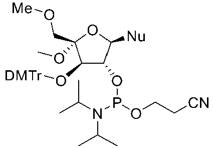 |
| LT52- Nu | 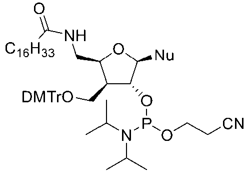 | LT106- Nu | 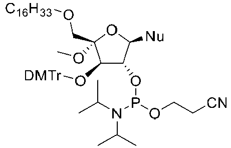 |
| LT53- Nu | 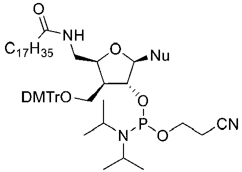 | | |
wherein, Nu is as defined in claim 1.

19. The nucleoside compound shown in Formula 1-a according to claim 2 or 3, wherein the nucleoside compound shown in Formula 1-a is a nucleoside compound shown as follows:

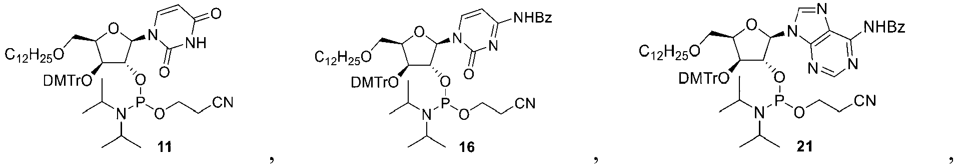

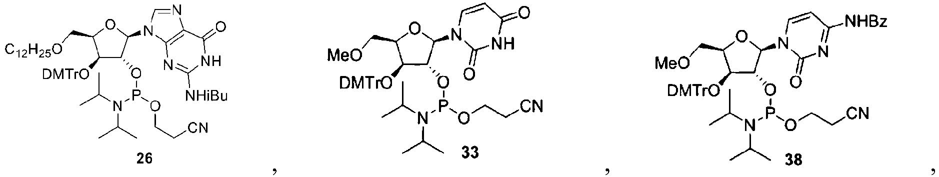

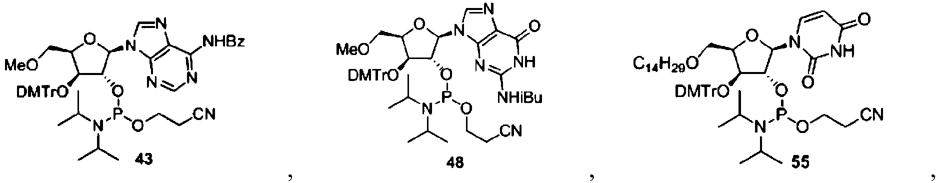

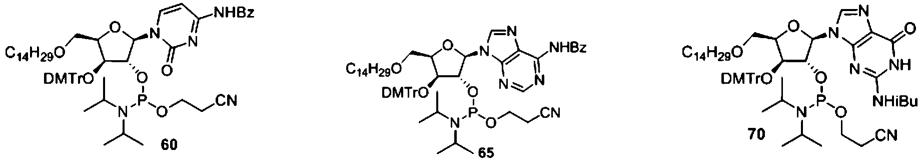

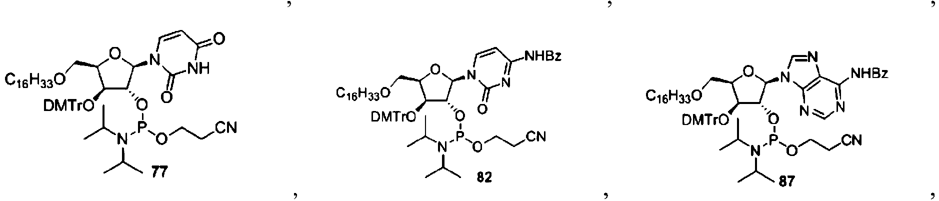

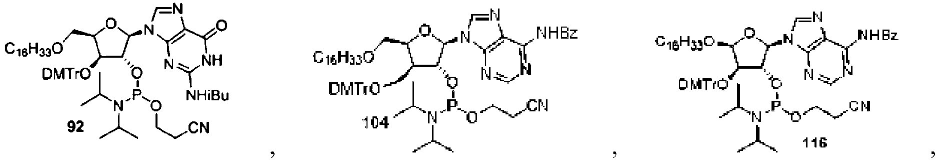

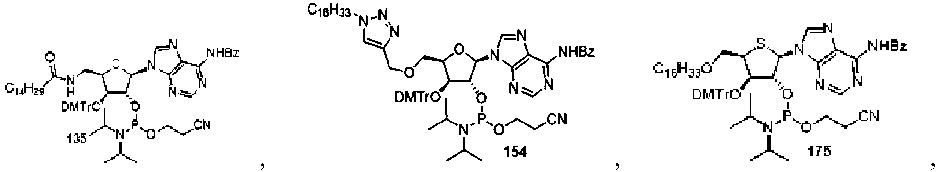

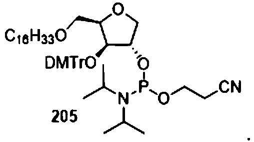

20. The nucleoside compound shown in Formula 1-b according to claim 2 or 3, wherein the nucleoside compound shown in Formula 1-b is a nucleoside compound shown as follows:
| Compound and Structure | | Compound and Structure | |
|---|---|---|---|
| DT0- Nu | 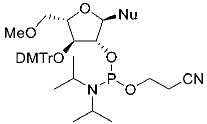 | DT33- Nu | 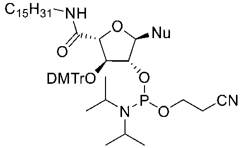 |
| DT1- Nu | 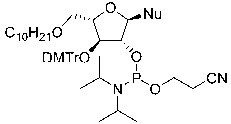 | DT34- Nu | 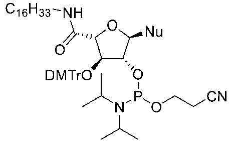 |
| DT2- Nu | 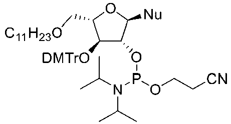 | DT35- Nu | 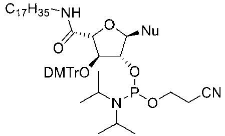 |
| DT3- Nu | 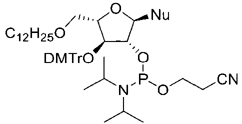 | DT36- Nu | 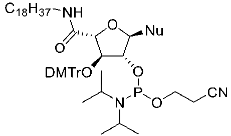 |
| DT4- Nu | 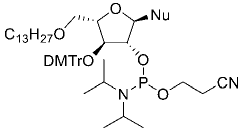 | DT37- Nu | 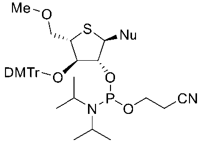 |
| DT5- Nu | 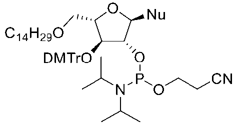 | DT38- Nu | 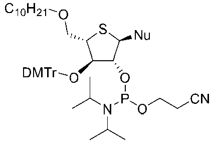 |
| DT6- Nu | 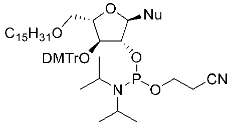 | DT39- Nu | 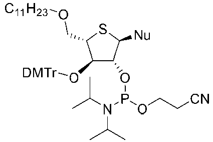 |
| DT7- Nu | 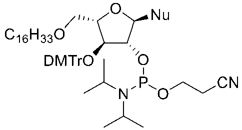 | DT40- Nu | 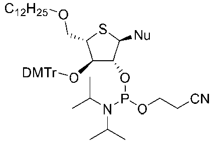 |
| DT8- Nu | 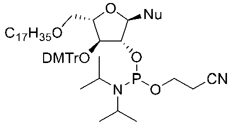 | DT41- Nu | 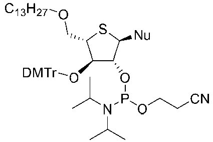 |
| DT9- Nu | 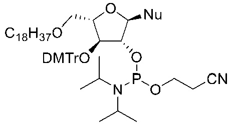 | DT42- Nu | 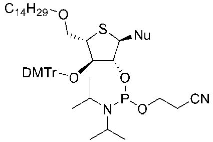 |
| DT10- Nu | 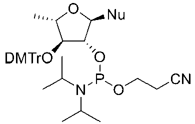 | DT43- Nu | 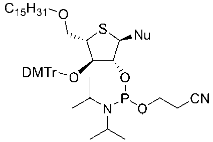 |
| DT11- Nu | 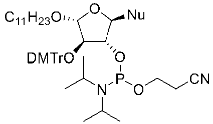 | DT44- Nu | 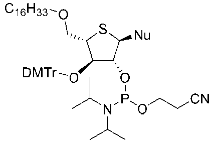 |
| DT12- Nu | 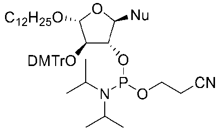 | DT45- Nu | 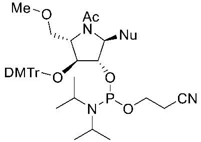 |
| DT13- Nu | 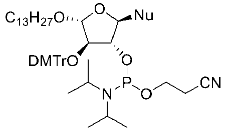 | DT46-without Nu | 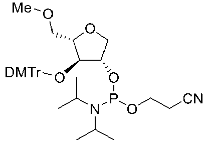 |
| DT14- Nu | 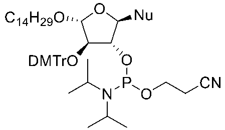 | DT47-without Nu | 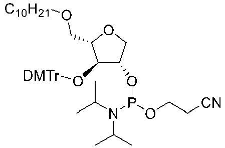 |
| DT15- Nu | 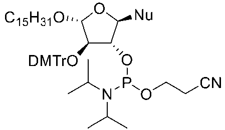 | DT48-without Nu | 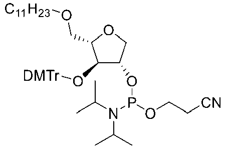 |
| DT16- Nu | 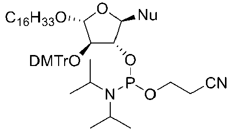 | DT49-without Nu | 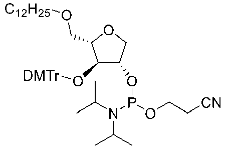 |
| DT17- Nu | 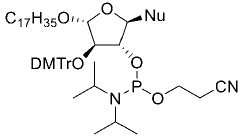 | DT50-without Nu | 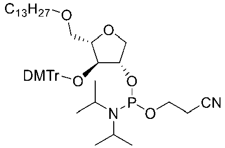 |
| DT18- Nu | 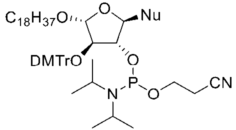 | DT51-without Nu | 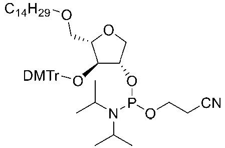 |
| DT19- Nu | 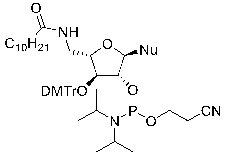 | DT52-without Nu | 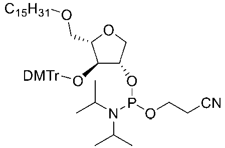 |
| DT20- Nu | 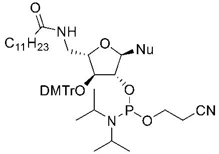 | DT53-without Nu | 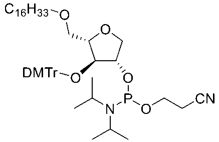 |
| DT21- Nu | 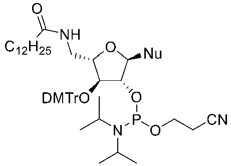 | DT54- Nu | 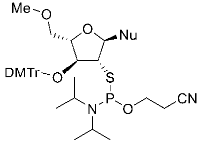 |
| DT22- Nu | 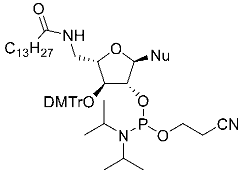 | DT55- Nu | 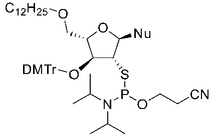 |
| DT23- Nu | 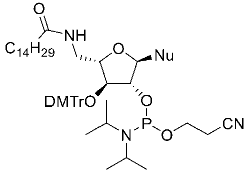 | DT56- Nu | 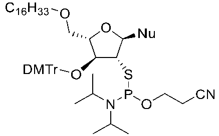 |
| DT24- Nu | 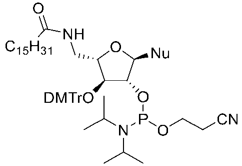 | DT57- Nu | 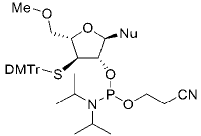 |
| DT25- Nu | 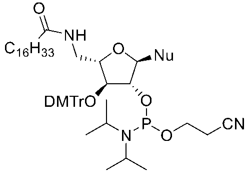 | DT58- Nu | 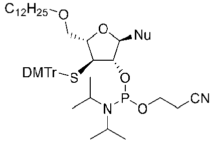 |
| DT26- Nu | 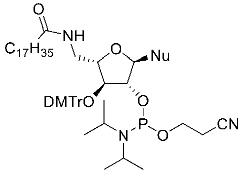 | DT59- Nu | 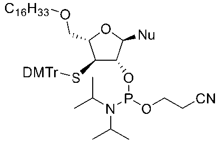 |
| DT27- Nu | 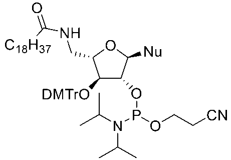 | DT60- Nu | 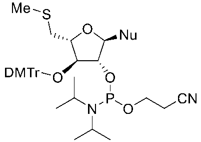 |
| DT28- Nu | 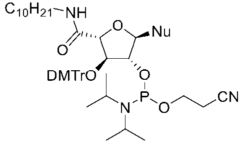 | DT61- Nu | 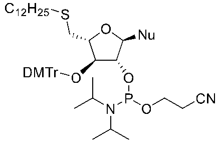 |
| DT29- Nu | 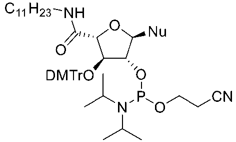 | DT62- Nu | 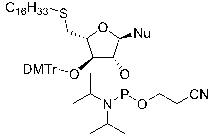 |
| DT30- Nu | 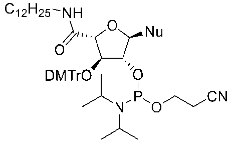 | DT63- Nu | 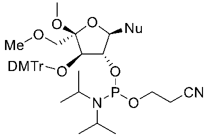 |
| DT31- Nu | 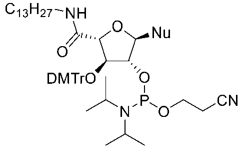 | DT64- Nu | 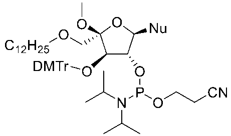 |
| DT32- Nu | 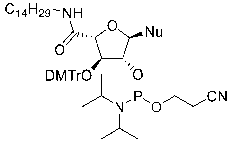 | DT65- Nu | 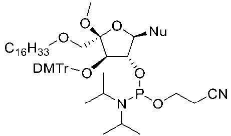 |
wherein, Nu is as defined in claim 1.

21. The nucleoside compound shown in Formula 1-b according to claim 2 or 3, wherein the nucleoside compound shown in Formula 1-b is a nucleoside compound shown as follows:

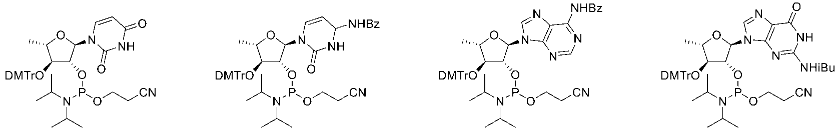

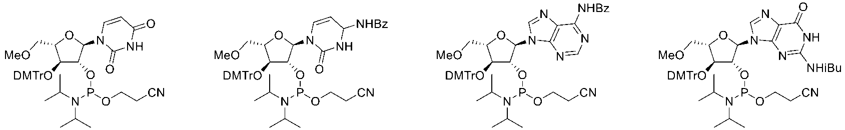

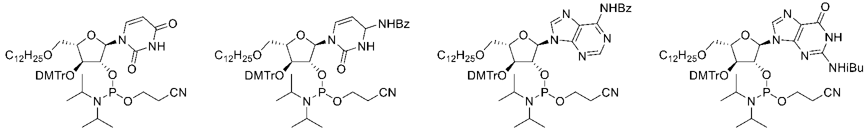

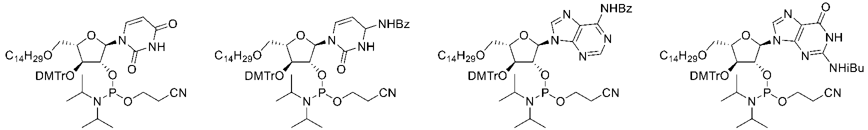

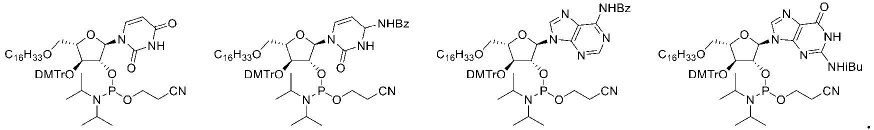

22. An oligonucleotide, wherein the oligonucleotide is prepared from at least one nucleoside compound shown in Formula 1-a or Formula 1-b according to any one of claims 2-21 as an intermediate; preferably, the oligonucleotide is siRNA, antisense nucleic acid, saRNA, miRNA, or nucleic acid aptamer.

23. The oligonucleotide according to claim 22, wherein the nucleoside compound in the oligonucleotide has a structure of the following Formula 17-a or Formula 18-a and is linked to the remainder of the oligonucleotide via 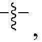 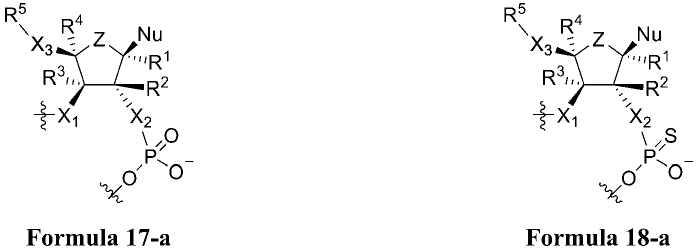
wherein R¹, R², R³, R⁴, R⁵, Z, X₁, X₂, X₃ and Nu are as defined in claim 1;
preferably,
the oligonucleotide is siRNA;
the nucleoside compound in the sense strand of siRNA has a structure shown in Formula 17-a or Formula 18-a; more preferably, the structure of Formula 17-a or Formula 18-a is the first, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, or twenty-first nucleotide at the 5' end of the sense strand of the siRNA; further preferably, the structure of Formula 17-a or Formula 18-a is the first, second, third, fourth, fifth, sixth, seventh, eighth, ninth, twelfth, thirteenth, fourteenth, or fifteenth nucleotide at the 5' end of the sense strand of the siRNA; even further preferably, the structure of Formula 17-a or Formula 18-a is the first nucleotide at the 5' end of the sense strand of the siRNA;
or, the nucleoside compound in the antisense strand of siRNA has a structure shown in Formula 17-a or Formula 18-a; more preferably, the structure of Formula 17-a or Formula 18-a is the first, second, third, fourth, fifth, or sixth nucleotide at the 3' end of the antisense strand of the siRNA; further preferably, the structure of Formula 17-a or Formula 18-a is the first or second nucleotide at the 3' end of the antisense strand of the siRNA.

24. The oligonucleotide according to claim 22, wherein the nucleoside compound in the oligonucleotide has a structure of the following Formula 13-b or Formula 14-b and is linked to the remainder of the oligonucleotide via 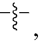 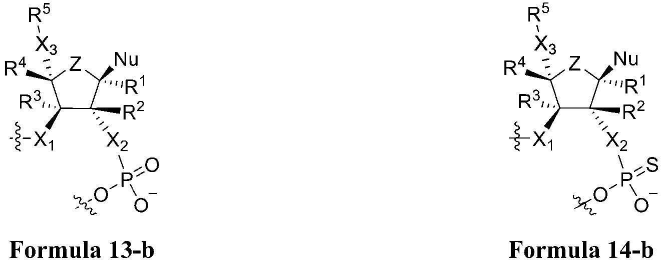
wherein R¹, R², R³, R⁴, R⁵, Z, X₁, X₂, X₃ and Nu are as defined in claim 1;
preferably,
the oligonucleotide is siRNA;
the nucleoside compound in the sense strand of the siRNA has a structure shown in Formula 13-b or Formula 14-b; more preferably, the structure of Formula 13-b or Formula 14-b is the first, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, or twenty-first nucleotide at the 5' end of the sense strand of the siRNA; further preferably, the structure of Formula 13-b or Formula 14-b is the first, second, third, fourth, fifth, sixth, seventh, eighth, ninth, twelfth, thirteenth, fourteenth, or fifteenth nucleotide at the 5' end of the sense strand of the siRNA; even further preferably, the structure of Formula 13-b or Formula 14-b is the first nucleotide at the 5' end of the sense strand of the siRNA;
or, the nucleoside compound in the antisense strand of siRNA has a structure shown in Formula 13-b or Formula 14-b; more preferably, the structure of Formula 13-b or Formula 14-b is the first, second, third, fourth, fifth, or sixth nucleotide at the 3' end of the antisense strand of the siRNA; further preferably, the structure of Formula 13-b or Formula 14-b is the first or second nucleotide at the 3' end of the antisense strand of the siRNA.

25. Use of the nucleoside compound shown in Formula 1-a or Formula 1-b according to any one of claims 2-21 for the preparation of oligonucleotide.

26. The use according to claim 25, wherein the nucleoside compound in the oligonucleotide has a structure of the following Formula 17-a or Formula 18-a and is linked to the remainder of the oligonucleotide via 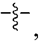 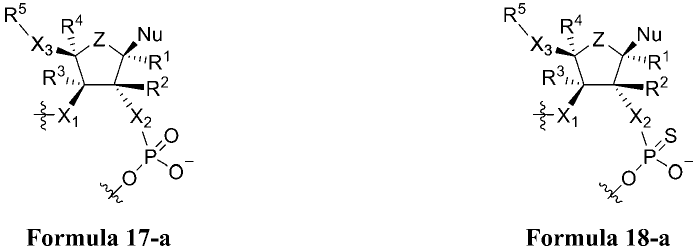
wherein R¹, R², R³, R⁴, R⁵, Z, X₁, X₂, X₃ and Nu are as defined in claim 1;
preferably,
the oligonucleotide is siRNA;
the nucleoside compound in the sense strand of siRNA has a structure shown in Formula 17-a or Formula 18-a; more preferably, the structure of Formula 17-a or Formula 18-a is the first, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, or twenty-first nucleotide at the 5' end of the sense strand of the siRNA; further preferably, the structure of Formula 17-a or Formula 18-a is the first, second, third, fourth, fifth, sixth, seventh, eighth, ninth, twelfth, thirteenth, fourteenth, or fifteenth nucleotide at the 5' end of the sense strand of the siRNA; even further preferably, the structure of Formula 17-a or Formula 18-a is the first nucleotide at the 5' end of the sense strand of the siRNA;
or, the nucleoside compound in the antisense strand of siRNA has a structure shown in Formula 17-a or Formula 18-a; more preferably, the structure of Formula 17-a or Formula 18-a is the first, second, third, fourth, fifth, or sixth nucleotide at the 3' end of the antisense strand of the siRNA; further preferably, the structure of Formula 17-a or Formula 18-a is the first or second nucleotide at the 3' end of the antisense strand of the siRNA.

27. The use according to claim 25, wherein the nucleoside compound in the oligonucleotide has a structure of the following Formula 13-b or Formula 14-b and is linked to the remainder of the oligonucleotide via 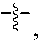 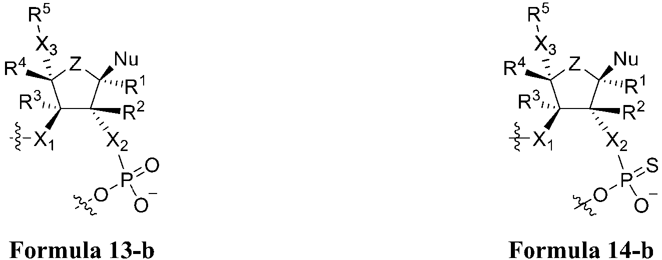
wherein R¹, R², R³, R⁴, R⁵, Z, X₁, X₂, X₃ and Nu are as defined in claim 1;
preferably,
the oligonucleotide is siRNA;
the nucleoside compound in the sense strand of siRNA has a structure shown in Formula 13-b or Formula 14-b; more preferably, the structure of Formula 13-b or Formula 14-b is the first, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, or twenty-first nucleotide at the 5' end of the sense strand of the siRNA; further preferably, the structure of Formula 13-b or Formula 14-b is the first, second, third, fourth, fifth, sixth, seventh, eighth, ninth, twelfth, thirteenth, fourteenth, or fifteenth nucleotide at the 5' end of the sense strand of the siRNA; even further preferably, the structure of Formula 13-b or Formula 14-b is the first nucleotide at the 5' end of the sense strand of the siRNA;
or, the nucleoside compound in the antisense strand of siRNA has a structure shown in Formula 13-b or Formula 14-b; more preferably, the structure of Formula 13-b or Formula 14-b is the first, second, third, fourth, fifth, or sixth nucleotide at the 3' end of the antisense strand of the siRNA; further preferably, the structure of Formula 13-b or Formula 14-b is the first or second nucleotide at the 3' end of the antisense strand of the siRNA.
